# EUROPEAN PATENT APPLICATION

(11) **EP 4 785 794 A1**
(43) Date of publication of application: **05.08.2026**
(21) Application number: 24872389.2
(22) Date of filing: 26.09.2024
(51) Int. Cl.: A01N 43/56, A01N 43/10, A01N 43/36, A01N 43/40, A01N 43/46, A01N 43/60, A01N 43/76, A01N 43/78, A01N 43/836, A01N 43/90, A01N 55/10, A01P 3/00, C07D 213/70, C07D 241/18, C07D 401/04, C07D 409/04, C07D 413/04, C07D 417/04, C07F 7/10

(54) **PLANT DISEASE CONTROL METHOD**

(30) Priority: 27.09.2023 JP 2023164815
(71) Applicant: SUMITOMO CHEMICAL COMPANY, LIMITED, Tokyo 103-6020 (JP)
(72) Inventor: ARAI, Keisuke, Takarazuka-shi, Hyogo 665-8555 (JP); NOKURA, Yoshihiko, Takarazuka-shi, Hyogo 665-8555 (JP); MAEHATA, Nao, Takarazuka-shi, Hyogo 665-8555 (JP)
(74) Representative: Vossius & Partner Patentanwälte Rechtsanwälte mbB
(86) International application number: PCT/JP2024/034415
(87) International publication number: WO 2025/070604

(57) **Abstract**

The present invention provides an excellent control method against plant diseases. A compound represented by formula (I) 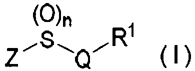 [wherein:
n represents 1 or 2;
R¹ represents a C3-C10 alicyclic hydrocarbon group or the like;
Q represents a group represented by Q1 or the like;
Z represents a C2-C6 chain hydrocarbon group or the like;
the group represented by Q1 represents a group represented by the following formula (wherein # represents the binding site to R¹, and • represents the binding site to the sulfur atom); 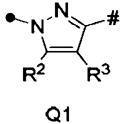 R² and R³ are identical to or different from each other, and each represent a hydrogen atom or a fluorine atom] or an N-oxide thereof, or a salt thereof can be used for controlling plant diseases.

## Description

### TECHNICAL FIELD

This application claims the priority to and the benefit of Japanese Patent Application No. 2023-164815 filed on September 27, 2023, the entire contents of which are incorporated herein by reference.

The present invention relates to methods for controlling plant diseases.

### BACKGROUND ART

Patent Document 1 discloses aryl sulfide compounds.

### CITATION LIST

### PATENT DOCUMENT

Patent Document 1: CN 112724092 A

### SUMMARY OF THE INVENTION

### PROBLEMS TO BE SOLVED BY THE INVENTION

An object of the present invention is to provide excellent control methods against plant diseases.

### MEANS TO SOLVE PROBLEMS

The present inventors have studied to find out an excellent control method against plant diseases. As a result, they have found that a compound represented by the following formula (I) has excellent control efficacy against plant diseases.

Namely, the present invention provides the followings.
[1] A method for controlling a plant disease which comprises applying a compound represented by formula (I) 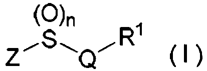 [wherein:
   n represents 1 or 2;
   R¹ represents a C3-C10 alicyclic hydrocarbon group, a 3-10 membered nonaromatic heterocyclic group {wherein said C3-C10 alicyclic hydrocarbon group and said 3-10 membered nonaromatic heterocyclic group are optionally substituted with one or more substituent(s) selected from Group A, and among atoms constituting said C3-C10 alicyclic hydrocarbon group and said 3-10 membered nonaromatic heterocyclic group, any atom which is not bound to Q optionally forms a C3-C7 alicyclic hydrocarbon or a 3-7 membered nonaromatic heterocycle as a spiro atom}, a 5-7 membered aromatic heterocyclic group optionally substituted with one or more substituent(s) selected from Group B1 (provided that a 1H-pyrazol-3-yl group is excluded when Q represents a group represented by Q6) {wherein among atoms constituting said 5-7 membered aromatic heterocyclic group (provided that a 1H-pyrazol-3-yl group is excluded when Q represents a group represented by Q6), any two adjacent atoms which are not bound to Q optionally form another benzene ring, C4-C7 alicyclic hydrocarbon, 4-7 membered nonaromatic heterocycle, or 5-7 membered aromatic heterocycle comprising said two adjacent atoms {wherein said benzene ring, said C4-C7 alicyclic hydrocarbon, said 4-7 membered nonaromatic heterocycle, and said 5-7 membered aromatic heterocycle are optionally substituted with one or more halogen atom(s)}}, or -C≡CR¹³;
   Q represents a group represented by Q1, a group represented by Q2, a group represented by Q3, a group represented by Q4, a group represented by Q5, or a group represented by Q6;
   Z represents a C2-C6 chain hydrocarbon group, a methyl group substituted with one or more fluorine atom(s), a C1-C2 alkyl group {wherein said C1-C2 alkyl group is substituted with a methoxy group or an ethoxy group}, a C3-C8 alicyclic hydrocarbon group, a 3-8 membered nonaromatic heterocyclic group {wherein said C3-C8 alicyclic hydrocarbon group and said 3-8 membered nonaromatic heterocyclic group are optionally substituted with one or more substituent(s) selected from Group C}, a 5-10 membered aromatic heterocyclic group optionally substituted with one or more substituent(s) selected from Group D, or NR¹⁴R¹⁵;
   the group represented by Q1, the group represented by Q2, the group represented by Q3, the group represented by Q4, the group represented by Q5, and the group represented by Q6 represent the groups represented by the following formulae (wherein # represents the binding site to R¹, and • represents the binding site to the sulfur atom); Q = 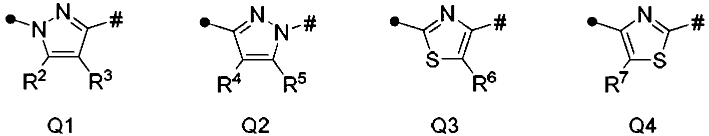 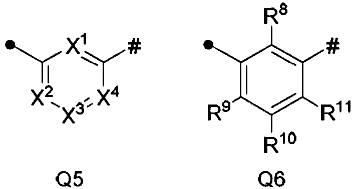
   R², R³, R⁴, R⁵, R⁶, and R⁷ are identical to or different from each other, and each represent a hydrogen atom or a fluorine atom;
   X¹ represents CR¹² or a nitrogen atom;
   X² represents CR¹⁶ or a nitrogen atom;
   X³ represents CR¹⁷ or a nitrogen atom;
   X⁴ represents CR¹⁸ or a nitrogen atom (provided that a combination wherein X¹ represents CR¹², X² represents CR¹⁶, X³ represents CR¹⁷, and X⁴ represents CR¹⁸ is excluded);
   R⁸, R⁹, R¹⁰, R¹¹, R¹², R¹⁶, R¹⁷, and R¹⁸ are identical to or different from each other, and each represent a methyl group optionally substituted with one or more fluorine atom(s), a halogen atom, or a hydrogen atom;
   R¹³ represents a C1-C6 chain hydrocarbon group optionally substituted with one or more substituent(s) selected from Group E, a C3-C10 alicyclic hydrocarbon group, a 3-10 membered nonaromatic heterocyclic group {wherein said C3-C10 alicyclic hydrocarbon group and said 3-10 membered nonaromatic heterocyclic group are optionally substituted with one or more substituent(s) selected from Group A}, a phenyl group, or a 5-7 membered aromatic heterocyclic group {wherein said phenyl group and said 5-7 membered aromatic heterocyclic group are optionally substituted with one or more substituent(s) selected from Group D}; and
   R¹⁴ and R¹⁵ are identical to or different from each other, and each represent a C1-C6 alkyl group, a methyl group substituted with one or more fluorine atom(s), or a C1-C2 alkyl group {wherein said C1-C2 alkyl group is substituted with a methoxy group or an ethoxy group};
   Group A: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group E}, a hydroxy group, a halogen atom, and a cyano group;
   Group B1: a group consisting of a C3-C10 alicyclic hydrocarbon group optionally substituted with one or more substituent(s) selected from Group D, a methyl group optionally substituted with one or more substituent(s) selected from Group F, a C2-C6 chain hydrocarbon group optionally substituted with one or more substituent(s) selected from Group E, a C1-C6 alkoxy group, a C1-C6 alkylsulfanyl group, a C1-C6 alkylsulfinyl group, a C1-C6 alkylsulfonyl group {wherein said C1-C6 alkoxy group, said C1-C6 alkylsulfanyl group, said C1-C6 alkylsulfinyl group, and said C1-C6 alkylsulfonyl group are optionally substituted with one or more halogen atom(s)}, a hydroxy group, a halogen atom, and a cyano group;
   Group C: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group E}, an oxo group, a thioxo group, a hydroxy group, a halogen atom, and a cyano group;
   Group D: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group E}, a halogen atom, and a cyano group;
   Group E: a group consisting of a C1-C3 alkoxy group, a halogen atom, and a cyano group;
   Group F: a group consisting of a methoxy group, a chlorine atom, and a cyano group]
   (hereinafter referred to as "Present compound Z") or an N-oxide thereof, or a salt thereof (hereinafter the Present compound Z or an N-oxide thereof, or a salt thereof is referred to as "Present compound W"), to a plant or soil for cultivating a plant.
[2] The method for controlling a plant disease according to [1], wherein the Present compound W in [1] is a compound, wherein
   R¹ represents a C3-C10 alicyclic hydrocarbon group, a 3-10 membered nonaromatic heterocyclic group {wherein said C3-C10 alicyclic hydrocarbon group and said 3-10 membered nonaromatic heterocyclic group are optionally substituted with one or more substituent(s) selected from Group A, and among atoms constituting said C3-C10 alicyclic hydrocarbon group and said 3-10 membered nonaromatic heterocyclic group, any atom which is not bound to Q optionally forms a C3-C7 alicyclic hydrocarbon or a 3-7 membered nonaromatic heterocycle as a spiro atom}, a 5-7 membered aromatic heterocyclic group optionally substituted with one or more substituent(s) selected from Group B (provided that a 1H-pyrazol-3-yl group is excluded when Q represents the group represented by Q6) {wherein among atoms constituting said 5-7 membered aromatic heterocyclic group (provided that a 1H-pyrazol-3-yl group is excluded when Q represents the group represented by Q6), any two adjacent atoms which are not bound to Q optionally form another C4-C7 alicyclic hydrocarbon, 4-7 membered nonaromatic heterocycle, or 5-7 membered aromatic heterocycle comprising said two adjacent atoms}, or -C≡CR¹³
      (hereinafter referred to as "Present compound N") or an N-oxide thereof, or a salt thereof (hereinafter the Present compound N or an N-oxide thereof, or a salt thereof is referred to as "Present compound");
   Group B: a group consisting of a methyl group optionally substituted with one or more substituent(s) selected from Group F, a C2-C6 chain hydrocarbon group optionally substituted with one or more substituent(s) selected from Group E, a C1-C6 alkoxy group, a C1-C6 alkylsulfanyl group, a C1-C6 alkylsulfinyl group, a C1-C6 alkylsulfonyl group {wherein said C1-C6 alkoxy group, said C1-C6 alkylsulfanyl group, said C1-C6 alkylsulfinyl group, and said C1-C6 alkylsulfonyl group are optionally substituted with one or more halogen atom(s)}, a hydroxy group, a halogen atom, and a cyano group.
[3] The method for controlling a plant disease according to [1], wherein the Present compound W in [1] is a compound or an N-oxide thereof, or a salt thereof wherein
   Q represents the group represented by Q1, the group represented by Q2, the group represented by Q5, or the group represented by Q6;
   R¹ represents a C3-C10 alicyclic hydrocarbon group, a 3-10 membered nonaromatic heterocyclic group (provided that when Q represents the group represented by Q2, then a carbon atom constituting said 3-10 membered nonaromatic heterocyclic group is bound to the group represented by Q2) {wherein said C3-C10 alicyclic hydrocarbon group and said 3-10 membered nonaromatic heterocyclic group (provided that when Q represents the group represented by Q2, then a carbon atom constituting said 3-10 membered nonaromatic heterocyclic group is bound to the group represented by Q2) are optionally substituted with one or more substituent(s) selected from Group A, and among atoms constituting said C3-C10 alicyclic hydrocarbon group and said 3-10 membered nonaromatic heterocyclic group (provided that when Q represents the group represented by Q2, then a carbon atom constituting said 3-10 membered nonaromatic heterocyclic group is bound to the group represented by Q2), any atom which is not bound to Q optionally forms a C3-C7 alicyclic hydrocarbon or a 3-7 membered nonaromatic heterocycle as a spiro atom}, a 5-7 membered aromatic heterocyclic group optionally substituted with one or more substituent(s) selected from Group B1 (provided that a 1H-pyrazol-3-yl group is excluded when Q represents the group represented by Q6, and when Q represents the group represented by Q2, then a carbon atom constituting said 5-7 membered aromatic heterocyclic group is bound to the group represented by Q2) {wherein among atoms constituting said 5-7 membered aromatic heterocyclic group (provided that a 1H-pyrazol-3-yl group is excluded when Q represents the group represented by Q6, and when Q represents the group represented by Q2, then a carbon atom constituting said 5-7 membered aromatic heterocyclic group is bound to the group represented by Q2), any two adjacent atoms which are not bound to Q optionally form another benzene ring, C4-C7 alicyclic hydrocarbon, 4-7 membered nonaromatic heterocycle, or 5-7 membered aromatic heterocycle comprising said two adjacent atoms {wherein said benzene ring, said C4-C7 alicyclic hydrocarbon, said 4-7 membered nonaromatic heterocycle, and said 5-7 membered aromatic heterocycle are optionally substituted with one or more halogen atom(s)}}, or -C≡CR¹³;
   Z represents a C2-C6 chain hydrocarbon group, a 5-6 membered aromatic heterocyclic group optionally substituted with one or more substituent(s) selected from Group D, or NR¹⁴R¹⁵;
   R², R³, R⁴, R⁵, R⁸, R⁹, R¹⁰, R¹¹, and R¹² each represent a hydrogen atom;
   X² and X⁴ each represent CH;
   X³ represents a nitrogen atom;
   R¹³ represents a C1-C6 chain hydrocarbon group or a phenyl group; and
   R¹⁴ and R¹⁵ are identical to or different from each other, and each represent a C1-C6 alkyl group.
[4] A compound represented by formula (II) 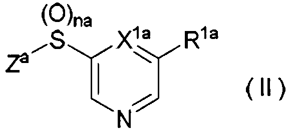 [wherein:
   na represents 1 or 2;
   X^{1a} represents CH or a nitrogen atom;
   R^{1a} represents a C3-C10 alicyclic hydrocarbon group, a 3-7 membered nonaromatic heterocyclic group {wherein said C3-C10 alicyclic hydrocarbon group and said 3-7 membered nonaromatic heterocyclic group are optionally substituted with one or more substituent(s) selected from Group A², and among atoms constituting said C3-C10 alicyclic hydrocarbon group and said 3-7 membered nonaromatic heterocyclic group, any atom which is not bound to the pyridine or pyrazine optionally forms a C3-C7 alicyclic hydrocarbon or a 3-7 membered nonaromatic heterocycle as a spiro atom}, or a 5-7 membered aromatic heterocyclic group {wherein said 5-7 membered aromatic heterocyclic group is optionally substituted with one or more substituent(s) selected from Group B1^{a}, and among atoms constituting said 5-7 membered aromatic heterocyclic group, any two adjacent atoms which are not bound to the pyridine or pyrazine optionally form another benzene ring, C4-C7 alicyclic hydrocarbon, 4-7 membered nonaromatic heterocycle, or 5-7 membered aromatic heterocycle comprising said two adjacent atoms {wherein said benzene ring, said C4-C7 alicyclic hydrocarbon, said 4-7 membered nonaromatic heterocycle, and said 5-7 membered aromatic heterocycle are optionally substituted with one or more halogen atom(s)}}; and
   Z^{a} represents a C2-C6 chain hydrocarbon group, a C3-C8 alicyclic hydrocarbon group optionally substituted with one or more substituent(s) selected from Group C^{a}, or a 5-10 membered aromatic heterocyclic group {wherein said 5-10 membered aromatic heterocyclic group is optionally substituted with one or more substituent(s) selected from Group D^{a}};
   Group A^{a}: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group E^{a}}, a hydroxy group, a halogen atom, and a cyano group;
   Group B1^{a}: a group consisting of a C3-C10 alicyclic hydrocarbon group optionally substituted with one or more substituent(s) selected from Group D^{a}, a methyl group optionally substituted with one or more substituent(s) selected from Group F^{a}, a C2-C6 chain hydrocarbon group optionally substituted with one or more substituent(s) selected from Group E^{a}, a C1-C6 alkoxy group, a C1-C6 alkylsulfanyl group, a C1-C6 alkylsulfinyl group, a C1-C6 alkylsulfonyl group {wherein said C1-C6 alkoxy group, said C1-C6 alkylsulfanyl group, said C1-C6 alkylsulfinyl group, and said C1-C6 alkylsulfonyl group are optionally substituted with one or more halogen atom(s)}, a hydroxy group, a halogen atom, and a cyano group;
   Group C^{a}: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group E^{a}}, an oxo group, a thioxo group, a hydroxy group, a halogen atom, and a cyano group;
   Group D^{a}: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group E^{a}}, a halogen atom, and a cyano group;
   Group E^{a}: a group consisting of a C1-C3 alkoxy group, a halogen atom, and a cyano group;
   Group F^{a}: a group consisting of a methoxy group, a chlorine atom, and a cyano group]
   (hereinafter referred to as "Compound Z of the present invention") or an N-oxide thereof, or a salt thereof (hereinafter the Compound Z of the present invention or an N-oxide thereof, or a salt thereof is referred to as "Compound W of the present invention").
[5] The compound according to [4], wherein
   R^{1a} represents a 3-7 membered nonaromatic heterocyclic group {wherein said 3-7 membered nonaromatic heterocyclic group is optionally substituted with one or more substituent(s) selected from Group A^{a}, and among atoms constituting said 3-7 membered nonaromatic heterocyclic group, any atom which is not bound to the pyridine or pyrazine optionally forms a C3-C7 alicyclic hydrocarbon or a 3-7 membered nonaromatic heterocycle as a spiro atom}, or a 5-7 membered aromatic heterocyclic group {wherein said 5-7 membered aromatic heterocyclic group is optionally substituted with one or more substituent(s) selected from Group B^{a}, and among atoms constituting said 5-7 membered aromatic heterocyclic group, any two adjacent atoms which are not bound to the pyridine or pyrazine optionally form another C4-C7 alicyclic hydrocarbon, 4-7 membered nonaromatic heterocycle, or 5-7 membered aromatic heterocycle comprising said two adjacent atoms} (hereinafter referred to as "Compound N of the present invention") or an N-oxide thereof, or a salt thereof (hereinafter the Compound N of the present invention or an N-oxide thereof, or a salt thereof is referred to as "Compound of the present invention");
   Group B^{a}: a group consisting of a methyl group optionally substituted with one or more substituent(s) selected from Group F^{a}, a C2-C6 chain hydrocarbon group optionally substituted with one or more substituent(s) selected from Group E^{a}, a C1-C6 alkoxy group, a C1-C6 alkylsulfanyl group, a C1-C6 alkylsulfinyl group, a C1-C6 alkylsulfonyl group {wherein said C1-C6 alkoxy group, said C1-C6 alkylsulfanyl group, said C1-C6 alkylsulfinyl group, and said C1-C6 alkylsulfonyl group are optionally substituted with one or more halogen atom(s)}, a hydroxy group, a halogen atom, and a cyano group.
[6] The compound or an N-oxide thereof, or a salt thereof according to [4], wherein
   R^{1a} represents a C3-C10 alicyclic hydrocarbon group optionally substituted with one or more C1-C6 chain hydrocarbon group(s), a 3-7 membered nonaromatic heterocyclic group {wherein said 3-7 membered nonaromatic heterocyclic group is optionally substituted with one or more substituent(s) selected from Group A^{a}2, and among atoms constituting said 3-7 membered nonaromatic heterocyclic group, any atom which is not bound to the pyridine or pyrazine optionally forms a C3-C7 alicyclic hydrocarbon as a spiro atom}, or a 5-7 membered aromatic heterocyclic group {wherein said 5-7 membered aromatic heterocyclic group is optionally substituted with one or more substituent(s) selected from Group B2^{a}, and among atoms constituting said 5-7 membered aromatic heterocyclic group, any two adjacent atoms which are not bound to the pyridine or pyrazine optionally form another benzene ring or C4-C7 alicyclic hydrocarbon comprising said two adjacent atoms}; and
   Z^{a} represents a C2-C6 chain hydrocarbon group or a 5-10 membered aromatic heterocyclic group {wherein said 5-10 membered aromatic heterocyclic group is optionally substituted with one or more substituent(s) selected from Group D^{a}};
   Group A^{a}2: a group consisting of a C1-C3 alkyl group and a halogen atom;
   Group B2^{a}: a group consisting of a C3-C6 alicyclic hydrocarbon group, a C1-C6 chain hydrocarbon group, and a halogen atom.
[7] A composition comprising the compound or an N-oxide thereof, or a salt thereof according to any one of [4] to [6], and an inert carrier.
[8] A composition comprising one or more ingredient(s) selected from the group consisting of Group (a), Group (b), Group (c), and Group (d), and the compound or an N-oxide thereof, or a salt thereof according to any one of [4] to [6]:
   Group (a): a group consisting of insecticidal active ingredients, miticidal active ingredients, and nematicidal active ingredients;
   Group (b): fungicidal active ingredients;
   Group (c): plant growth regulatory ingredients;
   Group (d): repellent ingredients.
[9] A method for controlling a plant disease which comprises applying an effective amount of the compound or an N-oxide thereof, or a salt thereof according to any one of [4] to [6] or an effective amount of the composition according to [8], to a plant or soil for cultivating a plant.
[10] Use of the compound or an N-oxide thereof, or a salt thereof according to any one of [4] to [6] or the composition according to [8] for controlling a plant disease.
[11] A seed or a vegetative reproductive organ holding an effective amount of the compound or an N-oxide thereof, or a salt thereof according to any one of [4] to [6] or an effective amount of the composition according to [8].

### EFFECT OF INVENTION

According to the present invention, plant diseases can be controlled.

### MODE FOR CARRYING OUT THE INVENTION

The substituents in the present invention are explained as follows.

The term of "halogen atom" represents a fluorine atom, a chlorine atom, a bromine atom, or an iodine atom.

When a substituent is substituted with two or more halogen atoms or substituents, these halogen atoms or substituents may be identical to or different from each other.

Regarding the expression of "optionally substituted with one or more substituent(s) selected from Group X" (wherein X represents any one of A, B, B1, C, D, E, F, A2, A^{a}, B^{a}, B1^{a}, B2^{a}, C^{a}, D^{a}, E^{a}, F^{a}, and A^{a}2) as described herein, when two or more substituents selected from Group X are present, these substituents may be identical to or different from each other.

The expression of "CX-CY" as described herein means that the number of carbon atom is X to Y. For example, the expression of "C1-C6" means that the number of carbon atom is 1 to 6.

The term of "chain hydrocarbon group" represents an alkyl group, an alkenyl group, or an alkynyl group.

Examples of the term of "alkyl group" include a methyl group, an ethyl group, a propyl group, an isopropyl group, a 1,1-dimethylpropyl group, a 1,2-dimethylpropyl group, a butyl group, a sec-butyl group, a tert-butyl group, a pentyl group, and a hexyl group.

Examples of the term of "alkenyl group" include a vinyl group, a 1-propenyl group, a 2-propenyl group, a 1-methyl-1-propenyl group, a 1-methyl-2-propenyl group, a 1,2-dimethyl-1-propenyl group, a 3-butenyl group, a 4-pentenyl group, and a 5-hexenyl group.

Examples of the term of "alkynyl group" include an ethynyl group, a 1-propynyl group, a 2-propynyl group, a 1-methyl-2-propynyl group, a 1,1-dimethyl-2-propynyl group, a 2-butynyl group, a 4-pentynyl group, and a 5-hexynyl group.

Examples of the term of "alkoxy group" include a methoxy group, an ethoxy group, a propoxy group, an isopropoxy group, a butoxy group, a tert-butoxy group, a pentyloxy group, and a hexyloxy group.

Examples of the term of "alkylsulfanyl group" include a methylsulfanyl group, an ethylsulfanyl group, an isopropylsulfanyl group, and a hexylsulfanyl group.

Examples of the term of "alkylsulfinyl group" include a methylsulfinyl group, an ethylsulfinyl group, an isopropylsulfinyl group, and a hexylsulfinyl group.

Examples of the term of "alkylsulfonyl group" include a methanesulfonyl group, an ethanesulfonyl group, an isopropanesulfonyl group, and a hexanesulfonyl group.

Examples of the term of "alicyclic hydrocarbon" include a cyclobutene ring, a cyclopentene ring, a cyclopentadiene ring, a cyclohexene ring, a cyclohexadiene ring, and a cycloheptene ring.

Examples of the term of "alicyclic hydrocarbon group" include a cycloalkyl group or a cycloalkenyl group.

Examples of the term of "cycloalkyl group" include a cyclopropyl group, a cyclobutyl group, a cyclopentyl group, and a cyclohexyl group.

Examples of the term of "cycloalkenyl group" include a cyclopentenyl group and a cyclohexenyl group.

Examples of the term of "nonaromatic heterocycle" include a dihydrofuran ring, a dihydrothiophene ring, a dihydropyrrole ring, a 1,3-dioxole ring, a pyran ring, and a dihydropyran ring.

Examples of the term of "aromatic heterocycle" include a furan ring, a thiophene ring, a pyrrole ring, a pyrazole ring, an imidazole ring, an oxazole ring, an isoxazole ring, a thiazole ring, an isothiazole ring, a triazole ring, an oxadiazole ring, a thiadiazole ring, a pyridine ring, a pyridazine ring, a pyrimidine ring, and a pyrazine ring.

Examples of the term of "aromatic heterocyclic group" include 5 membered aromatic heterocyclic groups such as a pyrrolyl group, a furanyl group, a thienyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, and a thiadiazolyl group; and 6 membered aromatic heterocyclic groups such as a pyridyl group, a pyridazinyl group, a pyrimidinyl group, a pyrazinyl group, a triazinyl group, and a tetrazinyl group.

Examples of the term of "nonaromatic heterocyclic group" include an aziridinyl group, an oxiranyl group, a thiiranyl group, an azetidinyl group, an oxetanyl group, a thietanyl group, a pyrrolidinyl group, a tetrahydrofuranyl group, a tetrahydrothienyl group, a pyrazolinyl group, a pyrazolidinyl group, an imidazolinyl group, an imidazolidinyl group, an oxazolinyl group, a thiazolinyl group, an oxazolidinyl group, a thiazolidinyl group, an isoxazolinyl group, an isoxazolidinyl group, an isothiazolinyl group, an isothiazolidinyl group, a dioxolanyl group, a dioxanyl group, a piperidyl group, a piperazinyl group, a morpholinyl group, a thiomorpholinyl group, a pyranyl group, a dihydropyranyl group, a tetrahydropyranyl group, a tetrahydrothiopyranyl group, an azepanyl group, an oxepanyl group, and a thiepanyl group.

The Present compound W or the Compound W of the present invention may optionally have one or more stereoisomer(s). Examples of the stereoisomer(s) include enantiomers, diastereomers, atropisomers, and geometric isomers. The present invention encompasses each stereoisomer and mixtures of stereoisomers at any ratio.

An N-oxide of the compound represented by formula (I) or formula (II) means a structure in which at least one nitrogen atom contained in the compound represented by formula (I) or formula (II) is substituted with an oxo group.

The compound represented by formula (I) or formula (II), or an N-oxide thereof may optionally be mixed with an acid such as hydrochloric acid, sulfuric acid, nitric acid, phosphoric acid, acetic acid, and benzoic acid to form an acid addition salt such as hydrochloride, sulfate, nitrate, phosphate, acetate, and benzoate.

Aspects of the Present compound Z include the following compounds.

[Aspect Z1] The Present compound Z, wherein Q represents the group represented by Q1.
[Aspect Z2] The Present compound Z, wherein
   Q represents the group represented by Q1; and
   R² and R³ each represent a hydrogen atom.
[Aspect Z3] The Present compound Z, wherein Q represents the group represented by Q2.
[Aspect Z4] The Present compound Z, wherein
   Q represents the group represented by Q2; and
   R⁴ and R⁵ each represent a hydrogen atom.
[Aspect Z5] The Present compound Z, wherein Q represents the group represented by Q3.
[Aspect Z6] The Present compound Z, wherein
   Q represents the group represented by Q3; and
   R⁶ represents a hydrogen atom.
[Aspect Z7] The Present compound Z, wherein Q represents the group represented by Q4.
[Aspect Z8] The Present compound Z, wherein
   Q represents the group represented by Q4; and
   R⁷ represents a hydrogen atom.
[Aspect Z9] The Present compound Z, wherein Q represents the group represented by Q5.
[Aspect Z10] The Present compound Z, wherein
   Q represents the group represented by Q5;
   X² represents CH; and
   X⁴ represents CH.
[Aspect Z11] The Present compound Z, wherein
   Q represents the group represented by Q5;
   X¹ represents CH;
   X² represents CH;
   X³ represents a nitrogen atom; and
   X⁴ represents CH.
[Aspect Z12] The Present compound Z, wherein
   Q represents the group represented by Q5;
   X¹ represents a nitrogen atom;
   X² represents CH;
   X³ represents a nitrogen atom; and
   X⁴ represents CH.
[Aspect Z13] The Present compound Z, wherein Q represents the group represented by Q6.
[Aspect Z14] The Present compound Z, wherein
   Q represents the group represented by Q6; and
   R⁸, R⁹, R¹⁰, and R¹¹ each represent a hydrogen atom.
[Aspect Z15] The Present compound Z, wherein Q represents the group represented by Q1, Q2, Q5, or Q6.
[Aspect Z16] The Present compound Z, wherein
   Q represents the group represented by Q1, Q2, Q5, or Q6; and
   R², R³, R⁴, R⁵, R⁸, R⁹, R¹⁰, R¹¹, R¹², R¹⁶, R¹⁷, and R¹⁸ each represent a hydrogen atom.
[Aspect Z17] The Present compound Z, wherein
   Q represents the group represented by Q1 or Q2; and
   R², R³, R⁴, and R⁵ each represent a hydrogen atom.
[Aspect Z18] The Present compound Z, wherein
   Q represents the group represented by Q5 or Q6; and
   R⁸, R⁹, R¹⁰, R¹¹, R¹², R¹⁶, R¹⁷, and R¹⁸ each represent a hydrogen atom.
[Aspect Z19] The Present compound Z or the compound according to any one of the Aspects Z1 to Z18, wherein
   Z represents a C2-C6 chain hydrocarbon group, a 5-6 membered aromatic heterocyclic group optionally substituted with one or more substituent(s) selected from Group D, or NR¹⁴R¹⁵; and
   R¹ represents a C3-C10 alicyclic hydrocarbon group optionally substituted with one or more C1-C6 chain hydrocarbon group(s), a 3-7 membered nonaromatic heterocyclic group {wherein said 3-7 membered nonaromatic heterocyclic group is optionally substituted with one or more substituent(s) selected from Group A2, and among atoms constituting said 3-7 membered nonaromatic heterocyclic group, any atom which is not bound to Q optionally forms a C3-C7 alicyclic hydrocarbon as a spiro atom}, a 5-7 membered aromatic heterocyclic group optionally substituted with one or more substituent(s) selected from Group B2 (provided that a 1H-pyrazol-3-yl group is excluded when Q represents the group represented by Q6) {wherein among atoms constituting said 5-7 membered aromatic heterocyclic group (provided that a 1H-pyrazol-3-yl group is excluded when Q represents the group represented by Q6), any two adjacent atoms which are not bound to Q optionally form another benzene ring or C4-C7 alicyclic hydrocarbon comprising said two adjacent atoms}, or -C≡CR¹³;

   Group A2: a group consisting of a C1-C3 alkyl group and a halogen atom;
   Group B2: a group consisting of a C3-C6 alicyclic hydrocarbon group, a C1-C6 chain hydrocarbon group, and a halogen atom.
[Aspect Z20] The Present compound Z or the compound according to any one of the Aspects Z1 to Z18, wherein
   Z represents a C2-C6 chain hydrocarbon group, a 5-6 membered aromatic heterocyclic group optionally substituted with one or more substituent(s) selected from Group A2, or NR¹⁴R¹⁵;
   R¹ represents a C3-C10 alicyclic hydrocarbon group optionally substituted with one or more C1-C6 chain hydrocarbon group(s), a 3-7 membered nonaromatic heterocyclic group {wherein said 3-7 membered nonaromatic heterocyclic group is optionally substituted with one or more substituent(s) selected from Group A2, and among atoms constituting said 3-7 membered nonaromatic heterocyclic group, any atom which is not bound to Q optionally forms a C3-C7 alicyclic hydrocarbon as a spiro atom}, or a 5-6 membered aromatic heterocyclic group optionally substituted with one or more substituent(s) selected from Group B2 (provided that a 1H-pyrazol-3-yl group is excluded when Q represents the group represented by Q6) {wherein among atoms constituting said 5-6 membered aromatic heterocyclic group (provided that a 1H-pyrazol-3-yl group is excluded when Q represents the group represented by Q6), any two adjacent atoms which are not bound to Q optionally form another benzene ring or C4-C7 alicyclic hydrocarbon comprising said two adjacent atoms}, or -C≡CR¹³;
   R¹⁴ and R¹⁵ are identical to or different from each other, and each represent a C1-C6 alkyl group; and
   R¹³ represents a C1-C6 chain hydrocarbon group or a phenyl group.
[Aspect Z21] The Present compound Z, or the compound according to any one of the Aspect Z3, Z4, Z15, Z16, or Z17, wherein
   R¹ represents a C3-C10 alicyclic hydrocarbon group, a 3-10 membered nonaromatic heterocyclic group (provided that when Q represents the group represented by Q2, then a carbon atom constituting said 3-10 membered nonaromatic heterocyclic group is bound to the group represented by Q2) {wherein said C3-C10 alicyclic hydrocarbon group and said 3-10 membered nonaromatic heterocyclic group (provided that when Q represents the group represented by Q2, then a carbon atom constituting said 3-10 membered nonaromatic heterocyclic group is bound to the group represented by Q2) are optionally substituted with one or more substituent(s) selected from Group A, and among atoms constituting said C3-C10 alicyclic hydrocarbon group and said 3-10 membered nonaromatic heterocyclic group (provided that when Q represents the group represented by Q2, then a carbon atom constituting said 3-10 membered nonaromatic heterocyclic group is bound to the group represented by Q2), any atom which is not bound to Q optionally forms a C3-C7 alicyclic hydrocarbon or a 3-7 membered nonaromatic heterocycle as a spiro atom}, a 5-7 membered aromatic heterocyclic group optionally substituted with one or more substituent(s) selected from Group B1 (provided that a 1H-pyrazol-3-yl group is excluded when Q represents the group represented by Q6, and when Q represents the group represented by Q2, then a carbon atom constituting said 5-7 membered aromatic heterocyclic group is bound to the group represented by Q2) {wherein among atoms constituting said 5-7 membered aromatic heterocyclic group (provided that a 1H-pyrazol-3-yl group is excluded when Q represents the group represented by Q6, and when Q represents the group represented by Q2, then a carbon atom constituting said 5-7 membered aromatic heterocyclic group is bound to the group represented by Q2), any two adjacent atoms which are not bound to Q optionally form another benzene ring, C4-C7 alicyclic hydrocarbon, 4-7 membered nonaromatic heterocycle, or 5-7 membered aromatic heterocycle comprising said two adjacent atoms {wherein said benzene ring, said C4-C7 alicyclic hydrocarbon, said 4-7 membered nonaromatic heterocycle, and said 5-7 membered aromatic heterocycle are optionally substituted with one or more halogen atom(s)}}, or -C≡CR¹³.
[Aspect Z22] The Present compound Z, or the compound according to any one of the Aspect Z3, Z4, Z15, Z16, or Z17, wherein
   Z represents a C2-C6 chain hydrocarbon group, a 5-6 membered aromatic heterocyclic group optionally substituted with one or more substituent(s) selected from Group D, or NR¹⁴R¹⁵; and
   R¹ represents a C3-C10 alicyclic hydrocarbon group optionally substituted with one or more C1-C6 chain hydrocarbon group(s), a 3-7 membered nonaromatic heterocyclic group (provided that when Q represents the group represented by Q2, then a carbon atom constituting said 3-7 membered nonaromatic heterocyclic group is bound to the group represented by Q2) {wherein said 3-7 membered nonaromatic heterocyclic group (provided that when Q represents the group represented by Q2, then a carbon atom constituting said 3-7 membered nonaromatic heterocyclic group is bound to the group represented by Q2) is optionally substituted with one or more substituent(s) selected from Group A2, and among atoms constituting said 3-7 membered nonaromatic heterocyclic group (provided that when Q represents the group represented by Q2, then a carbon atom constituting said 3-7 membered nonaromatic heterocyclic group is bound to the group represented by Q2), any atom which is not bound to Q optionally forms a C3-C7 alicyclic hydrocarbon as a spiro atom}, a 5-7 membered aromatic heterocyclic group optionally substituted with one or more substituent(s) selected from Group B2 (provided that a 1H-pyrazol-3-yl group is excluded when Q represents the group represented by Q6, and when Q represents the group represented by Q2, then a carbon atom constituting said 5-7 membered aromatic heterocyclic group is bound to the group represented by Q2) {wherein among atoms constituting said 5-7 membered aromatic heterocyclic group (provided that a 1H-pyrazol-3-yl group is excluded when Q represents the group represented by Q6, and when Q represents the group represented by Q2, then a carbon atom constituting said 5-7 membered aromatic heterocyclic group is bound to the group represented by Q2), any two adjacent atoms which are not bound to Q optionally form another benzene ring or C4-C7 alicyclic hydrocarbon comprising said two adjacent atoms}, or -C≡CR¹³.
[Aspect Z23] The Present compound Z, or the compound according to any one of the Aspect Z3, Z4, Z15, Z16, or Z17, wherein
   Z represents a C2-C6 chain hydrocarbon group, a 5-6 membered aromatic heterocyclic group optionally substituted with one or more substituent(s) selected from Group A2, or NR¹⁴R¹⁵;
   R¹ represents a C3-C10 alicyclic hydrocarbon group optionally substituted with one or more C1-C6 chain hydrocarbon group(s), a 3-7 membered nonaromatic heterocyclic group (provided that when Q represents the group represented by Q2, then a carbon atom constituting said 3-7 membered nonaromatic heterocyclic group is bound to the group represented by Q2) {wherein said 3-7 membered nonaromatic heterocyclic group (provided that when Q represents the group represented by Q2, then a carbon atom constituting said 3-7 membered nonaromatic heterocyclic group is bound to the group represented by Q2) is optionally substituted with one or more substituent(s) selected from Group A2, and among atoms constituting said 3-7 membered nonaromatic heterocyclic group (provided that when Q represents the group represented by Q2, then a carbon atom constituting said 3-7 membered nonaromatic heterocyclic group is bound to the group represented by Q2), any atom which is not bound to Q optionally forms a C3-C7 alicyclic hydrocarbon as a spiro atom}, or a 5-6 membered aromatic heterocyclic group optionally substituted with one or more substituent(s) selected from Group B2 (provided that a 1H-pyrazol-3-yl group is excluded when Q represents the group represented by Q6, and when Q represents the group represented by Q2, then a carbon atom constituting said 5-6 membered aromatic heterocyclic group is bound to the group represented by Q2) {wherein among atoms constituting said 5-6 membered aromatic heterocyclic group (provided that a 1H-pyrazol-3-yl group is excluded when Q represents the group represented by Q6, and when Q represents the group represented by Q2, then a carbon atom constituting said 5-6 membered aromatic heterocyclic group is bound to the group represented by Q2), any two adjacent atoms which are not bound to Q optionally form another benzene ring or C4-C7 alicyclic hydrocarbon comprising said two adjacent atoms}, or -C≡CR¹³;
   R¹⁴ and R¹⁵ are identical to or different from each other, and each represent a C1-C6 alkyl group; and
   R¹³ represents a C1-C6 chain hydrocarbon group or a phenyl group.

Aspects of the Present compound N include the following compounds.
[Aspect 1] The Present compound N, wherein Z represents a C2-C6 chain hydrocarbon group.
[Aspect 2] The Present compound N, wherein Z represents a C2-C6 alkyl group.
[Aspect 3] The Present compound N, wherein Z represents an ethyl group or a propyl group.
[Aspect 4] The Present compound N, wherein Z represents a methyl group substituted with one or more fluorine atom(s).
[Aspect 5] The Present compound N, wherein Z represents a trifluoromethyl group.
[Aspect 6] The Present compound N, wherein Z represents a C1-C2 alkyl group {wherein said C1-C2 alkyl group is substituted with a methoxy group or an ethoxy group}.
[Aspect 7] The Present compound N, wherein Z represents a 2-methoxyethyl group or a 2-ethoxyethyl group.
[Aspect 8] The Present compound N, wherein Z represents a C3-C8 alicyclic hydrocarbon group.
[Aspect 9] The Present compound N, wherein Z represents a cyclopropyl group, a cyclopentyl group, or a cyclohexyl group.
[Aspect 10] The Present compound N, wherein Z represents a 3-8 membered nonaromatic heterocyclic group.
[Aspect 11] The Present compound N, wherein Z represents a 3-tetrahydrofuranyl group or a 3-tetrahydrothienyl group.
[Aspect 12] The Present compound N, wherein Z represents a 5-10 membered aromatic heterocyclic group optionally substituted with one or more substituent(s) selected from Group D.
[Aspect 13] The Present compound N, wherein Z represents a 5 membered aromatic heterocyclic group optionally substituted with one or more substituent(s) selected from Group D.
[Aspect 14] The Present compound N, wherein Z represents a 6 membered aromatic heterocyclic group optionally substituted with one or more substituent(s) selected from Group D.
[Aspect 15] The Present compound N, wherein Z represents a 2-pyridyl group, a 3-pyridyl group, a 4-pyridyl group, a 2-pyrimidinyl group, or a 2-pyrazinyl group.
[Aspect 16] The Present compound N, wherein Z represents a 2-furyl group, a 3-furyl group, a 2-thienyl group, or a 3-thienyl group.
[Aspect 17] The Present compound N, wherein Z represents NR¹⁴R¹⁵.
[Aspect 18] The Present compound N, wherein Z represents a dimethylamino group.
[Aspect 19] The Present compound N, wherein Z represents a 5-6 membered aromatic heterocyclic group.
[Aspect 20] The Present compound N, wherein Z represents a C2-C6 chain hydrocarbon group or a 5-6 membered aromatic heterocyclic group.
[Aspect 21] The Present compound N, wherein Z represents a C2-C6 alkyl group or a 5 membered aromatic heterocyclic group.
[Aspect 22] The Present compound N, wherein Z represents a C2-C6 alkyl group or a 6 membered aromatic heterocyclic group.
[Aspect 23] The Present compound N, wherein Z represents an ethyl group, a propyl group, a 2-pyridyl group, a 3-pyridyl group, a 4-pyridyl group, a 2-pyrimidinyl group, a 2-pyrazinyl group, a 2-furyl group, a 3-furyl group, a 2-thienyl group, or a 3-thienyl group.
[Aspect 24] The Present compound N, wherein Q represents the group represented by Q1.
[Aspect 25] The Present compound N, wherein
   Q represents the group represented by Q1; and
   R² and R³ each represent a hydrogen atom.
[Aspect 26] The compound according to the Aspect 1, wherein
   Q represents the group represented by Q1; and
   R² and R³ each represent a hydrogen atom.
[Aspect 27] The compound according to the Aspect 2, wherein
   Q represents the group represented by Q1; and
   R² and R³ each represent a hydrogen atom.
[Aspect 28] The compound according to the Aspect 3, wherein
   Q represents the group represented by Q1; and
   R² and R³ each represent a hydrogen atom.
[Aspect 29] The compound according to the Aspect 12, wherein
   Q represents the group represented by Q1; and
   R² and R³ each represent a hydrogen atom.
[Aspect 30] The compound according to the Aspect 13, wherein
   Q represents the group represented by Q1; and
   R² and R³ each represent a hydrogen atom.
[Aspect 31] The compound according to the Aspect 14, wherein
   Q represents the group represented by Q1; and
   R² and R³ each represent a hydrogen atom.
[Aspect 32] The compound according to the Aspect 15, wherein
   Q represents the group represented by Q1; and
   R² and R³ each represent a hydrogen atom.
[Aspect 33] The compound according to the Aspect 16, wherein
   Q represents the group represented by Q1; and
   R² and R³ each represent a hydrogen atom.
[Aspect 34] The compound according to the Aspect 19, wherein
   Q represents the group represented by Q1; and
   R² and R³ each represent a hydrogen atom.
[Aspect 35] The compound according to the Aspect 20, wherein
   Q represents the group represented by Q1; and
   R² and R³ each represent a hydrogen atom.
[Aspect 36] The compound according to the Aspect 21, wherein
   Q represents the group represented by Q1; and
   R² and R³ each represent a hydrogen atom.
[Aspect 37] The compound according to the Aspect 22, wherein
   Q represents the group represented by Q1; and
   R² and R³ each represent a hydrogen atom.
[Aspect 38] The compound according to the Aspect 23, wherein
   Q represents the group represented by Q1; and
   R² and R³ each represent a hydrogen atom.
[Aspect 39] The Present compound N, wherein Q represents the group represented by Q2.
[Aspect 40] The Present compound N, wherein
   Q represents the group represented by Q2; and
   R⁴ and R⁵ each represent a hydrogen atom.
[Aspect 41] The Present compound N, wherein Q represents the group represented by Q3.
[Aspect 42] The Present compound N, wherein
   Q represents the group represented by Q3; and
   R⁶ represents a hydrogen atom.
[Aspect 43] The Present compound N, wherein Q represents the group represented by Q4.
[Aspect 44] The Present compound N, wherein
   Q represents the group represented by Q4; and
   R⁷ represents a hydrogen atom.
[Aspect 45] The Present compound N, wherein Q represents the group represented by Q5.
[Aspect 46] The Present compound N, wherein
   Q represents the group represented by Q5;
   X² represents CR¹⁶; and
   X⁴ represents CR1¹⁸,
[Aspect 47] The Present compound N, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 48] The compound according to the Aspect 1, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 49] The compound according to the Aspect 2, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 50] The compound according to the Aspect 3, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 51] The compound according to the Aspect 12, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 52] The compound according to the Aspect 13, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 53] The compound according to the Aspect 14, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 54] The compound according to the Aspect 15, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 55] The compound according to the Aspect 16, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 56] The compound according to the Aspect 19, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 57] The compound according to the Aspect 20, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 58] The compound according to the Aspect 21, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 59] The compound according to the Aspect 22, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 60] The compound according to the Aspect 23, wherein
   Q represents the group represented by Q5;
   X² represents CR¹⁶;
   X⁴ represents CR¹⁸; and
   R¹², R¹⁶, R¹⁷, and R¹⁸ each represent a hydrogen atom.
[Aspect 61] The Present compound N, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 62] The compound according to the Aspect 1, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 63] The compound according to the Aspect 2, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 64] The compound according to the Aspect 3, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 65] The compound according to the Aspect 12, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 66] The compound according to the Aspect 13, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 67] The compound according to the Aspect 14, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 68] The compound according to the Aspect 15, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 69] The compound according to the Aspect 16, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 70] The compound according to the Aspect 19, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 71] The compound according to the Aspect 20, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 72] The compound according to the Aspect 21, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 73] The compound according to the Aspect 22, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 74] The compound according to the Aspect 23, wherein
   Q represents the group represented by Q5;
   X² represents CR¹⁶;
   X³ represents a nitrogen atom;
   X⁴ represents CR¹⁸; and
   R¹², R¹⁶, and R¹⁸ each represent a hydrogen atom.
[Aspect 75] The Present compound N, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 76] The compound according to the Aspect 1, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 77] The compound according to the Aspect 2, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 78] The compound according to the Aspect 3, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 79] The compound according to the Aspect 12, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 80] The compound according to the Aspect 13, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 81] The compound according to the Aspect 14, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 82] The compound according to the Aspect 15, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 83] The compound according to the Aspect 16, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 84] The compound according to the Aspect 19, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 85] The compound according to the Aspect 20, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 86] The compound according to the Aspect 21, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 87] The compound according to the Aspect 22, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 88] The compound according to the Aspect 23, wherein
   Q represents the group represented by Q5;
   X¹ represents CR¹²;
   X² represents CR¹⁶;
   X³ represents a nitrogen atom;
   X⁴ represents CR¹⁸; and
   R¹², R¹⁶, and R¹⁸ each represent a hydrogen atom.
[Aspect 89] The Present compound N, wherein Q represents the group represented by Q6.
[Aspect 90] The Present compound N, wherein
   Q represents the group represented by Q6; and
   R⁸, R⁹, R¹⁰, and R¹¹ each represent a hydrogen atom.
[Aspect 91] The compound according to the Aspect 1, wherein
   Q represents the group represented by Q6; and
   R⁸, R⁹, R¹⁰, and R¹¹ each represent a hydrogen atom.
[Aspect 92] The compound according to the Aspect 2, wherein
   Q represents the group represented by Q6; and
   R⁸, R⁹, R¹⁰, and R¹¹ each represent a hydrogen atom.
[Aspect 93] The compound according to the Aspect 3, wherein
   Q represents the group represented by Q6; and
   R^{Θ}, R⁹, R¹⁰, and R¹¹ each represent a hydrogen atom.
[Aspect 94] The compound according to the Aspect 12, wherein
   Q represents the group represented by Q6; and
   R⁸, R⁹, R¹⁰, and R¹¹ each represent a hydrogen atom.
[Aspect 95] The compound according to the Aspect 13, wherein
   Q represents the group represented by Q6; and
   R⁸, R⁹, R¹⁰, and R¹¹ each represent a hydrogen atom.
[Aspect 96] The compound according to the Aspect 14, wherein
   Q represents the group represented by Q6; and
   R⁸, R⁹, R¹⁰, and R¹¹ each represent a hydrogen atom.
[Aspect 97] The compound according to the Aspect 15, wherein
   Q represents the group represented by Q6; and
   R⁸, R⁹, R¹⁰, and R¹¹ each represent a hydrogen atom.
[Aspect 98] The compound according to the Aspect 16, wherein
   Q represents the group represented by Q6; and
   R⁸, R⁹, R¹⁰, and R¹¹ each represent a hydrogen atom.
[Aspect 99] The compound according to the Aspect 19, wherein
   Q represents the group represented by Q6; and
   R⁸, R⁹, R¹⁰, and R¹¹ each represent a hydrogen atom.
[Aspect 100] The compound according to the Aspect 20, wherein
   Q represents the group represented by Q6; and
   R⁸, R⁹, R¹⁰, and R¹¹ each represent a hydrogen atom.
[Aspect 101] The compound according to the Aspect 21, wherein
   Q represents the group represented by Q6; and
   R⁸, R⁹, R¹⁰, and R¹¹ each represent a hydrogen atom.
[Aspect 102] The compound according to the Aspect 22, wherein
   Q represents the group represented by Q6; and
   R⁸, R⁹, R¹⁰, and R¹¹ each represent a hydrogen atom.
[Aspect 103] The compound according to the Aspect 23, wherein
   Q represents the group represented by Q6; and
   R⁸, R⁹, R¹⁰, and R¹¹ each represent a hydrogen atom.
[Aspect 104] The Present compound N, wherein Q represents the group represented by Q1 or the group represented by Q2.
[Aspect 105] The Present compound N, wherein
   Q represents the group represented by Q1 or the group represented by Q2; and
   R², R³, R⁴, and R⁵ each represent a hydrogen atom.
[Aspect 106] The Present compound N, wherein Q represents the group represented by Q2, the group represented by Q3, or the group represented by Q4.
[Aspect 107] The Present compound N, wherein
   Q represents the group represented by Q2, the group represented by Q3, or the group represented by Q4; and
   R⁴, R⁵, R⁶, and R⁷ each represent a hydrogen atom.
[Aspect 108] The Present compound N, wherein
   Q represents the group represented by Q3 or the group represented by Q4; and
   R⁶ and R⁷ each represent a hydrogen atom.
[Aspect 109] The Present compound N, wherein Q represents the group represented by Q5 or the group represented by Q6.
[Aspect 110] The Present compound N, wherein
   Q represents the group represented by Q5 or the group represented by Q6;
   X² represents CR¹⁶; and
   X⁴ represents CR¹⁸,
[Aspect 111] The Present compound N, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 112] The compound according to the Aspect 1, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 113] The compound according to the Aspect 2, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 114] The compound according to the Aspect 3, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 115] The compound according to the Aspect 12, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 116] The compound according to the Aspect 13, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 117] The compound according to the Aspect 14, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 118] The compound according to the Aspect 15, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 119] The compound according to the Aspect 16, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 120] The compound according to the Aspect 19, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 121] The compound according to the Aspect 20, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 122] The compound according to the Aspect 21, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 123] The compound according to the Aspect 22, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 124] The compound according to the Aspect 23, wherein
   Q represents the group represented by Q5 or the group represented by Q6;
   X² represents CR¹⁶;
   X⁴ represents CR¹⁸; and
   R⁸, R⁹, R¹⁰, R¹¹, R¹², R¹⁶, R¹⁷, and R¹⁸ each represent a hydrogen atom.
[Aspect 125] The Present compound N, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 126] The compound according to the Aspect 1, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 127] The compound according to the Aspect 2, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 128] The compound according to the Aspect 3, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 129] The compound according to the Aspect 12, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 130] The compound according to the Aspect 13, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 131] The compound according to the Aspect 14, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 132] The compound according to the Aspect 15, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 133] The compound according to the Aspect 16, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 134] The compound according to the Aspect 19, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 135] The compound according to the Aspect 20, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 136] The compound according to the Aspect 21, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 137] The compound according to the Aspect 22, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 138] The compound according to the Aspect 23, wherein
   Q represents the group represented by Q5 or the group represented by Q6;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 139] The Present compound N, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 140] The compound according to the Aspect 1, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 141] The compound according to the Aspect 2, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 142] The compound according to the Aspect 3, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 143] The compound according to the Aspect 12, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 144] The compound according to the Aspect 13, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 145] The compound according to the Aspect 14, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 146] The compound according to the Aspect 15, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 147] The compound according to the Aspect 16, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 148] The compound according to the Aspect 19, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 149] The compound according to the Aspect 20, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 150] The compound according to the Aspect 21, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 151] The compound according to the Aspect 22, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 152] The compound according to the Aspect 23, wherein
   Q represents the group represented by Q5 or the group represented by Q6;
   X¹ represents CR¹²;
   X² represents CR¹⁶;
   X³ represents a nitrogen atom;
   X⁴ represents CR¹⁸; and
   R⁸, R⁹, R¹⁰, R¹¹, R¹², R¹⁶, and R¹⁸ each represent a hydrogen atom.
[Aspect 153] The Present compound N, wherein Q represents the group represented by Q1, the group represented by Q2, the group represented by Q5, or the group represented by Q6.
[Aspect 154] The Present compound N, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 155] The compound according to the Aspect 1, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 156] The compound according to the Aspect 2, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 157] The compound according to the Aspect 3, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 158] The compound according to the Aspect 12, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 159] The compound according to the Aspect 13, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 160] The compound according to the Aspect 14, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 161] The compound according to the Aspect 15, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 162] The compound according to the Aspect 16, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 163] The compound according to the Aspect 19, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 164] The compound according to the Aspect 20, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 165] The compound according to the Aspect 21, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 166] The compound according to the Aspect 22, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 167] The compound according to the Aspect 23, wherein
   Q represents the group represented by Q1, the group represented by Q2, the group represented by Q5, or the group represented by Q6;
   X² represents CR¹⁶;
   X⁴ represents CR¹⁸; and
   R², R³, R⁴, R⁵, R⁸, R⁹, R¹⁰, R¹¹, R¹², R¹⁶, R¹⁷, and R¹⁸ each represent a hydrogen atom.
[Aspect 168] The Present compound N, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 169] The compound according to the Aspect 1, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 170] The compound according to the Aspect 2, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 171] The compound according to the Aspect 3, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 172] The compound according to the Aspect 12, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 173] The compound according to the Aspect 13, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 174] The compound according to the Aspect 14, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 175] The compound according to the Aspect 15, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 176] The compound according to the Aspect 16, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 177] The compound according to the Aspect 19, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 178] The compound according to the Aspect 20, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 179] The compound according to the Aspect 21, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 180] The compound according to the Aspect 22, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 181] The compound according to the Aspect 23, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸; and
   R², R³, R⁴, R⁵, R⁸, R⁹, R¹⁰, R¹¹, R¹², R¹⁶, and R¹⁸ each represent a hydrogen atom.
[Aspect 182] The Present compound N, wherein n represents 1.
[Aspect 183] The Present compound N, wherein n represents 2.
[Aspect 184] The Present compound N, wherein
   Q represents the group represented by Q1; and
   n represents 2.
[Aspect 185] The Present compound N, wherein
   Q represents the group represented by Q1;
   R² and R³ each represent a hydrogen atom; and
   n represents 2.
[Aspect 186] The compound according to the Aspect 1, wherein
   Q represents the group represented by Q1;
   R² and R³ each represent a hydrogen atom; and
   n represents 2.
[Aspect 187] The compound according to the Aspect 2, wherein
   Q represents the group represented by Q1;
   R² and R³ each represent a hydrogen atom; and
   n represents 2.
[Aspect 188] The compound according to the Aspect 3, wherein
   Q represents the group represented by Q1;
   R² and R³ each represent a hydrogen atom; and
   n represents 2.
[Aspect 189] The compound according to the Aspect 12, wherein
   Q represents the group represented by Q1;
   R² and R³ each represent a hydrogen atom; and
   n represents 2.
[Aspect 190] The compound according to the Aspect 13, wherein
   Q represents the group represented by Q1;
   R² and R³ each represent a hydrogen atom; and
   n represents 2.
[Aspect 191] The compound according to the Aspect 14, wherein
   Q represents the group represented by Q1;
   R² and R³ each represent a hydrogen atom; and
   n represents 2.
[Aspect 192] The compound according to the Aspect 15, wherein
   Q represents the group represented by Q1;
   R² and R³ each represent a hydrogen atom; and
   n represents 2.
[Aspect 193] The compound according to the Aspect 16, wherein
   Q represents the group represented by Q1;
   R² and R³ each represent a hydrogen atom; and
   n represents 2.
[Aspect 194] The compound according to the Aspect 19, wherein
   Q represents the group represented by Q1;
   R² and R³ each represent a hydrogen atom; and
   n represents 2.
[Aspect 195] The compound according to the Aspect 20, wherein
   Q represents the group represented by Q1;
   R² and R³ each represent a hydrogen atom; and
   n represents 2.
[Aspect 196] The compound according to the Aspect 21, wherein
   Q represents the group represented by Q1;
   R² and R³ each represent a hydrogen atom; and
   n represents 2.
[Aspect 197] The compound according to the Aspect 22, wherein
   Q represents the group represented by Q1;
   R² and R³ each represent a hydrogen atom; and
   n represents 2.
[Aspect 198] The compound according to the Aspect 23, wherein
   Q represents the group represented by Q1;
   R² and R³ each represent a hydrogen atom; and
   n represents 2.
[Aspect 199] The Present compound N, wherein
   Q represents the group represented by Q2; and
   n represents 2.
[Aspect 200] The Present compound N, wherein
   Q represents the group represented by Q2;
   R⁴ and R⁵ each represent a hydrogen atom; and
   n represents 2.
[Aspect 201] The Present compound N, wherein
   Q represents the group represented by Q3; and
   n represents 2.
[Aspect 202] The Present compound N, wherein
   Q represents the group represented by Q3;
   R⁶ represents a hydrogen atom; and
   n represents 2.
[Aspect 203] The Present compound N, wherein
   Q represents the group represented by Q4; and
   n represents 2.
[Aspect 204] The Present compound N, wherein
   Q represents the group represented by Q4;
   R⁷ represents a hydrogen atom; and
   n represents 2.
[Aspect 205] The Present compound N, wherein
   Q represents the group represented by Q5; and
   n represents 2.
[Aspect 206] The Present compound N, wherein
   Q represents the group represented by Q5;
   X² represents CR¹⁶;
   X⁴ represents CR¹⁸; and
   n represents 2.
[Aspect 207] The Present compound N, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 208] The compound according to the Aspect 1, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 209] The compound according to the Aspect 2, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 210] The compound according to the Aspect 3, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 211] The compound according to the Aspect 12, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 212] The compound according to the Aspect 13, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and n represents 2.
[Aspect 213] The compound according to the Aspect 14, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 214] The compound according to the Aspect 15, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 215] The compound according to the Aspect 16, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 216] The compound according to the Aspect 19, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 217] The compound according to the Aspect 20, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 218] The compound according to the Aspect 21, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 219] The compound according to the Aspect 22, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 220] The compound according to the Aspect 23, wherein
   Q represents the group represented by Q5;
   X² represents CR¹⁶;
   X⁴ represents CR¹⁸;
   R¹², R¹⁶, R¹⁷, and R¹⁸ each represent a hydrogen atom; and
   n represents 2.
[Aspect 221] The Present compound N, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 222] The compound according to the Aspect 1, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 223] The compound according to the Aspect 2, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 224] The compound according to the Aspect 3, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 225] The compound according to the Aspect 12, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 226] The compound according to the Aspect 13, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 227] The compound according to the Aspect 14, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 228] The compound according to the Aspect 15, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 229] The compound according to the Aspect 16, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 230] The compound according to the Aspect 19, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 231] The compound according to the Aspect 20, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 232] The compound according to the Aspect 21, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 233] The compound according to the Aspect 22, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 234] The compound according to the Aspect 23, wherein
   Q represents the group represented by Q5;
   X² represents CR¹⁶;
   X³ represents a nitrogen atom;
   X⁴ represents CR¹⁸;
   R¹², R¹⁶, and R¹⁸ each represent a hydrogen atom; and n represents 2.
[Aspect 235] The Present compound N, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 236] The compound according to the Aspect 1, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 237] The compound according to the Aspect 2, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 238] The compound according to the Aspect 3, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 239] The compound according to the Aspect 12, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 240] The compound according to the Aspect 13, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 241] The compound according to the Aspect 14, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 242] The compound according to the Aspect 15, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 243] The compound according to the Aspect 16, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 244] The compound according to the Aspect 19, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 245] The compound according to the Aspect 20, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 246] The compound according to the Aspect 21, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 247] The compound according to the Aspect 22, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and
   n represents 2.
[Aspect 248] The compound according to the Aspect 23, wherein
   Q represents the group represented by Q5;
   X¹ represents CR¹²;
   X² represents CR¹⁶;
   X³ represents a nitrogen atom;
   X⁴ represents CR¹⁸;
   R¹², R¹⁶, and R¹⁸ each represent a hydrogen atom; and n represents 2.
[Aspect 249] The Present compound N, wherein
   Q represents the group represented by Q6; and
   n represents 2.
[Aspect 250] The Present compound N, wherein
   Q represents the group represented by Q6;
   R⁸, R⁹, R¹⁰, and R¹¹ each represent a hydrogen atom; and
   n represents 2.
[Aspect 251] The compound according to the Aspect 1, wherein
   Q represents the group represented by Q6;
   R⁸, R⁹, R¹⁰, and R¹¹ each represent a hydrogen atom; and
   n represents 2.
[Aspect 252] The compound according to the Aspect 2, wherein
   Q represents the group represented by Q6;
   R⁸, R⁹, R¹⁰, and R¹¹ each represent a hydrogen atom; and
   n represents 2.
[Aspect 253] The compound according to the Aspect 3, wherein
   Q represents the group represented by Q6;
   R⁸, R⁹, R¹⁰, and R¹¹ each represent a hydrogen atom; and
   n represents 2.
[Aspect 254] The compound according to the Aspect 12, wherein
   Q represents the group represented by Q6;
   R⁸, R⁹, R¹⁰, and R¹¹ each represent a hydrogen atom; and
   n represents 2.
[Aspect 255] The compound according to the Aspect 13, wherein
   Q represents the group represented by Q6;
   R⁸, R⁹, R¹⁰, and R¹¹ each represent a hydrogen atom; and
   n represents 2.
[Aspect 256] The compound according to the Aspect 14, wherein
   Q represents the group represented by Q6;
   R⁸, R⁹, R¹⁰, and R¹¹ each represent a hydrogen atom; and
   n represents 2.
[Aspect 257] The compound according to the Aspect 15, wherein
   Q represents the group represented by Q6;
   R⁸, R⁹, R¹⁰, and R¹¹ each represent a hydrogen atom; and
   n represents 2.
[Aspect 258] The compound according to the Aspect 16, wherein
   Q represents the group represented by Q6;
   R⁸, R⁹, R¹⁰, and R¹¹ each represent a hydrogen atom; and
   n represents 2.
[Aspect 259] The compound according to the Aspect 19, wherein
   Q represents the group represented by Q6;
   R⁸, R⁹, R¹⁰, and R¹¹ each represent a hydrogen atom; and
   n represents 2.
[Aspect 260] The compound according to the Aspect 20, wherein
   Q represents the group represented by Q6;
   R⁸, R⁹, R¹⁰, and R¹¹ each represent a hydrogen atom; and
   n represents 2.
[Aspect 261] The compound according to the Aspect 21, wherein
   Q represents the group represented by Q6;
   R⁸, R⁹, R¹⁰, and R¹¹ each represent a hydrogen atom; and
   n represents 2.
[Aspect 262] The compound according to the Aspect 22, wherein
   Q represents the group represented by Q6;
   R⁸, R⁹, R¹⁰, and R¹¹ each represent a hydrogen atom; and
   n represents 2.
[Aspect 263] The compound according to the Aspect 23, wherein
   Q represents the group represented by Q6;
   R⁸, R⁹, R¹⁰, and R¹¹ each represent a hydrogen atom; and
   n represents 2.
[Aspect 264] The Present compound N, wherein
   Q represents the group represented by Q1 or the group represented by Q2; and
   n represents 2.
[Aspect 265] The Present compound N, wherein
   Q represents the group represented by Q1 or the group represented by Q2;
   R², R³, R⁴, and R⁵ each represent a hydrogen atom; and
   n represents 2.
[Aspect 266] The Present compound N, wherein
   Q represents the group represented by Q2, the group represented by Q3, or the group represented by Q4; and
   n represents 2.
[Aspect 267] The Present compound N, wherein
   Q represents the group represented by Q2, the group represented by Q3, or the group represented by Q4;
   R⁴, R⁵, R⁶, and R⁷ each represent a hydrogen atom; and
   n represents 2.
[Aspect 268] The Present compound N, wherein
   Q represents the group represented by Q3 or the group represented by Q4;
   R⁶ and R⁷ each represent a hydrogen atom; and
   n represents 2.
[Aspect 269] The Present compound N, wherein
   Q represents the group represented by Q5 or the group represented by Q6; and
   n represents 2.
[Aspect 270] The Present compound N, wherein
   Q represents the group represented by Q5 or the group represented by Q6;
   X² represents CR¹⁶;
   X⁴ represents CR¹⁸; and
   n represents 2.
[Aspect 271] The Present compound N, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 272] The compound according to the Aspect 1, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 273] The compound according to the Aspect 2, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 274] The compound according to the Aspect 3, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 275] The compound according to the Aspect 12, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 276] The compound according to the Aspect 13, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 277] The compound according to the Aspect 14, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 278] The compound according to the Aspect 15, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 279] The compound according to the Aspect 16, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 280] The compound according to the Aspect 19, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 281] The compound according to the Aspect 20, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 282] The compound according to the Aspect 21, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 283] The compound according to the Aspect 22, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 284] The compound according to the Aspect 23, wherein
   Q represents the group represented by Q5 or the group represented by Q6;
   X² represents CR¹⁶;
   X⁴ represents CR¹⁸;
   R⁸, R⁹, R¹⁰, R¹¹, R¹², R¹⁶, R¹⁷, and R¹⁸ each represent a hydrogen atom; and
   n represents 2.
[Aspect 285] The Present compound N, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 286] The compound according to the Aspect 1, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 287] The compound according to the Aspect 2, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 288] The compound according to the Aspect 3, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 289] The compound according to the Aspect 12, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 290] The compound according to the Aspect 13, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 291] The compound according to the Aspect 14, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 292] The compound according to the Aspect 15, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 293] The compound according to the Aspect 16, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 294] The compound according to the Aspect 19, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 295] The compound according to the Aspect 20, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 296] The compound according to the Aspect 21, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 297] The compound according to the Aspect 22, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 298] The compound according to the Aspect 23, wherein
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 299] The Present compound N, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 300] The compound according to the Aspect 1, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 301] The compound according to the Aspect 2, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 302] The compound according to the Aspect 3, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 303] The compound according to the Aspect 12, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and n represents 2.
[Aspect 304] The compound according to the Aspect 13, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 305] The compound according to the Aspect 14, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 306] The compound according to the Aspect 15, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 307] The compound according to the Aspect 16, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 308] The compound according to the Aspect 19, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 309] The compound according to the Aspect 20, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 310] The compound according to the Aspect 21, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 311] The compound according to the Aspect 22, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 312] The compound according to the Aspect 23, wherein
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   n represents 2.
[Aspect 313] The Present compound N, wherein
   Q represents the group represented by Q1, the group represented by Q2, the group represented by Q5, or the group represented by Q6; and
   n represents 2.
[Aspect 314] The Present compound N, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 315] The compound according to the Aspect 1, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 316] The compound according to the Aspect 2, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 317] The compound according to the Aspect 3, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 318] The compound according to the Aspect 12, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 319] The compound according to the Aspect 13, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 320] The compound according to the Aspect 14, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 321] The compound according to the Aspect 15, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 322] The compound according to the Aspect 16, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 323] The compound according to the Aspect 19, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 324] The compound according to the Aspect 20, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 325] The compound according to the Aspect 21, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 326] The compound according to the Aspect 22, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 327] The compound according to the Aspect 23, wherein
   Q represents the group represented by Q1, the group represented by Q2, the group represented by Q5, or the group represented by Q6;
   X² represents CR¹⁶;
   X⁴ represents CR¹⁸;
   R², R³, R⁴, R⁵, R⁸, R⁹, R¹⁰, R¹¹, R¹², R¹⁶, R¹⁷, and R¹⁸ each represent a hydrogen atom; and
   n represents 2.
[Aspect 328] The Present compound N, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 329] The compound according to the Aspect 1, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 330] The compound according to the Aspect 2, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 331] The compound according to the Aspect 3, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 332] The compound according to the Aspect 12, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 333] The compound according to the Aspect 13, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 334] The compound according to the Aspect 14, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 335] The compound according to the Aspect 15, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 336] The compound according to the Aspect 16, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 337] The compound according to the Aspect 19, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 338] The compound according to the Aspect 20, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R^{B}, R⁹, R¹⁰, R¹¹, R¹², R¹⁶, and R¹⁸ each represent a hydrogen atom; and n represents 2.
[Aspect 339] The compound according to the Aspect 21, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 340] The compound according to the Aspect 22, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 341] The compound according to the Aspect 23, wherein
   Q represents the group represented by Q1, the group represented by Q2, the group represented by Q5, or the group represented by Q6;
   X¹ represents CR¹²;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   n represents 2.
[Aspect 342] The Present compound N, wherein R¹ represents a C3-C10 alicyclic hydrocarbon group {wherein said C3-C10 alicyclic hydrocarbon group is optionally substituted with one or more substituent(s) selected from Group A2, and among atoms constituting said C3-C10 alicyclic hydrocarbon group, any atom which is not bound to Q optionally forms a C3-C7 alicyclic hydrocarbon or a 3-7 membered nonaromatic heterocycle as a spiro atom};
   Group A2: a group consisting of a C1-C3 alkyl group and a halogen atom.
[Aspect 343] The Present compound N, wherein R¹ represents a C5-C7 alicyclic hydrocarbon group optionally substituted with one or more substituent(s) selected from Group A2.
[Aspect 344] The Present compound N, wherein R¹ represents a cyclopropyl group, a cyclohexyl group, a 1-cyclopentenyl group, a 1-cyclohexenyl group, or a 4,4-dimethyl-1-cyclohexene-1-yl group.
[Aspect 345] The Present compound N, wherein R¹ represents a 3-10 membered nonaromatic heterocyclic group {wherein said 3-10 membered nonaromatic heterocyclic group is optionally substituted with one or more substituent(s) selected from Group A2, and among atoms constituting said 3-10 membered nonaromatic heterocyclic group, any atom which is not bound to Q optionally forms a C3-C7 alicyclic hydrocarbon or a 3-7 membered nonaromatic heterocycle as a spiro atom}.
[Aspect 346] The Present compound N, wherein R¹ represents a 5-7 membered nonaromatic heterocyclic group optionally substituted with one or more substituent(s) selected from Group A2.
[Aspect 347] The Present compound N, wherein R¹ represents a 1-piperidyl group or a 1-azepanyl group.
[Aspect 348] The Present compound N, wherein R¹ represents a C3-C10 alicyclic hydrocarbon group or a 3-10 membered nonaromatic heterocyclic group {wherein said C3-C10 alicyclic hydrocarbon group and said 3-10 membered nonaromatic heterocyclic group are optionally substituted with one or more substituent(s) selected from Group A2, and among atoms constituting said C3-C10 alicyclic hydrocarbon group and said 3-10 membered nonaromatic heterocyclic group, any atom which is not bound to Q optionally forms a C3-C7 alicyclic hydrocarbon or a 3-7 membered nonaromatic heterocycle as a spiro atom}.
[Aspect 349] The Present compound N, wherein R¹ represents a C5-C7 alicyclic hydrocarbon group or a 5-7 membered nonaromatic heterocyclic group {wherein said C5-C7 alicyclic hydrocarbon group and said 5-7 membered nonaromatic heterocyclic group are optionally substituted with one or more substituent(s) selected from Group A2}.
[Aspect 350] The Present compound N, wherein R¹ represents a 5-7 membered aromatic heterocyclic group optionally substituted with one or more substituent(s) selected from Group B (provided that a 1H-pyrazol-3-yl group is excluded when Q represents the group represented by Q6) {wherein among atoms constituting said 5-7 membered aromatic heterocyclic group (provided that a 1H-pyrazol-3-yl group is excluded when Q represents the group represented by Q6), any two adjacent atoms which are not bound to Q optionally form another C4-C7 alicyclic hydrocarbon, 4-7 membered nonaromatic heterocycle, or 5-7 membered aromatic heterocycle comprising said two adjacent atoms}.
[Aspect 351] The Present compound N, wherein R¹ represents a 5-6 membered aromatic heterocyclic group optionally substituted with one or more substituent(s) selected from Group A2.
[Aspect 352] The Present compound N, wherein R¹ represents a 2-pyridyl group, a 3-pyridyl group, a 4-pyridyl group, a 5-chloro-6-fluoropyridin-3-yl group, a 6-chloro-5-methylpyridin-3-yl group, a 6-chloro-5-fluoropyridin-3-yl group, a 5-fluoro-6-methylpyridin-2-yl group, a 5-fluoro-4-methylpyridin-2-yl group, or a 6-fluoro-5-methylpyridin-3-yl group.
[Aspect 353] The Present compound N, wherein R¹ represents a 1H-pyrazol-1-yl group, a 1H-pyrazol-3-yl group, a 1H-pyrazol-4-yl group, a 2-thienyl group, a 2-pyridyl group, a 3-pyridyl group, or a 4-pyridyl group {wherein said 1H-pyrazol-1-yl group, said 1H-pyrazol-3-yl group, said 1H-pyrazol-4-yl group, said 2-thienyl group, said 2-pyridyl group, said 3-pyridyl group, and said 4-pyridyl group are optionally substituted with one or more substituent(s) selected from Group A2}.
[Aspect 354] The Present compound N, wherein R¹ represents a 5 membered aromatic heterocyclic group optionally substituted with one or more substituent(s) selected from Group A2.
[Aspect 355] The Present compound N, wherein R¹ represents a 6 membered aromatic heterocyclic group optionally substituted with one or more substituent(s) selected from Group A2.
[Aspect 356] The Present compound N, wherein R¹ represents -C≡CR¹³ .
[Aspect 357] The Present compound N, wherein
   R¹ represents -C≡CR¹³; and
   R¹³ represents a C1-C6 chain hydrocarbon group or a C3-C7 alicyclic hydrocarbon group.
[Aspect 358] The Present compound N, wherein R¹ represents a 3,3-dimethyl-1-butyn-1-yl group, a 1-cyclohexen-1-yl group, or a 1-cyclopenten-1-yl group.
[Aspect 359] The Present compound N, wherein R¹ represents a 3-10 membered nonaromatic heterocyclic group {wherein said 3-10 membered nonaromatic heterocyclic group is optionally substituted with one or more substituent(s) selected from Group A2, and among atoms constituting said 3-10 membered nonaromatic heterocyclic group, any atom which is not bound to Q optionally forms a C3-C7 alicyclic hydrocarbon or a 3-7 membered nonaromatic heterocycle as a spiro atom}, a 5-7 membered aromatic heterocyclic group optionally substituted with one or more substituent(s) selected from Group B, or -C≡CR¹³.
[Aspect 360] The Present compound N, wherein
   R¹ represents a 5-7 membered nonaromatic heterocyclic group optionally substituted with one or more substituent(s) selected from Group A2, a 5-7 membered aromatic heterocyclic group optionally substituted with one or more substituent(s) selected from Group A2, or -C≡CR¹³; and
   R¹³ represents a C1-C6 chain hydrocarbon group or a C3-C7 alicyclic hydrocarbon group.
[Aspect 361] The Present compound N, wherein
   R¹ represents a 1-cyclopenten-1-yl group, a 1-cyclohexen-1-yl group, a 4,4-dimethyl-1-cyclohexen-1-yl group, a 1H-pyrazol-1-yl group, a 2-pyridyl group, a 3-pyridyl group, a 4-pyridyl group {wherein said 1H-pyrazol-1-yl group, said 2-pyridyl group, said 3-pyridyl group, and said 4-pyridyl group are optionally substituted with one or more substituent(s) selected from Group A2}, or -C≡CR¹³; and
   R¹³ represents a 3,3-dimethyl-1-butyn-1-yl group, a 1-cyclohexen-1-yl group, or a 1-cyclopenten-1-yl group.
[Aspect 362] The Present compound N, wherein
   Z represents a C2-C6 chain hydrocarbon group or a 5-6 membered aromatic heterocyclic group optionally substituted with one or more substituent(s) selected from Group D;
   Q represents the group represented by Q5 or the group represented by Q6;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   R¹ represents a 3-10 membered nonaromatic heterocyclic group {wherein said 3-10 membered nonaromatic heterocyclic group is optionally substituted with one or more substituent(s) selected from Group A2, and among atoms constituting said 3-10 membered nonaromatic heterocyclic group, any atom which is not bound to Q optionally forms a C3-C7 alicyclic hydrocarbon or a 3-7 membered nonaromatic heterocycle as a spiro atom}, or a 5-7 membered aromatic heterocyclic group optionally substituted with one or more substituent(s) selected from Group B.
[Aspect 363] The Present compound N, wherein
   Z represents a C2-C6 alkyl group or a 5-6 membered aromatic heterocyclic group;
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   R¹ represents a 5-7 membered nonaromatic heterocyclic group optionally substituted with one or more substituent(s) selected from Group A2 or a 5-6 membered aromatic heterocyclic group optionally substituted with one or more substituent(s) selected from Group A2.
[Aspect 364] The Present compound N, wherein
   Z represents an ethyl group, a propyl group, a 2-pyridyl group, a 3-pyridyl group, a 4-pyridyl group, a 2-pyrimidinyl group, a 2-pyrazinyl group, a 2-furyl group, a 3-furyl group, a 2-thienyl group, or a 3-thienyl group;
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom;
   R¹ represents a 3-bromo-1H-pyrazol-1-yl group, a 4-chloro-3-methyl-1H-pyrazol-1-yl group, a 4-bromo-3-methyl-1H-pyrazol-1-yl group, a 5-chloro-6-fluoropyridin-3-yl group, a 6-chloro-5-methylpyridin-3-yl group, a 6-chloro-5-fluoropyridin-3-yl group, a 5-fluoro-6-methylpyridin-2-yl group, a 5-fluoro-4-methylpyridin-2-yl group, or a 6-fluoro-5-methylpyridin-3-yl group; and
   n represents 2.
[Aspect 365] The Present compound N, wherein
   Z represents an ethyl group, a propyl group, or a 2-thienyl group;
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   R¹ represents a 1H-pyrazol-1-yl group, a 2-pyridyl group, or a 3-pyridyl group {wherein said 1H-pyrazol-1-yl group, said 2-pyridyl group, and said 3-pyridyl group are optionally substituted with one or more substituent(s) selected from Group A2}.
[Aspect 366] The Present compound N, wherein
   Z represents an ethyl group, a propyl group, a 2-furyl group, a 3-furyl group, or a 2-thienyl group;
   Q represents the group represented by Q5 or the group represented by Q6;
   X¹ represents CR¹²;
   X² represents CR¹⁶;
   X³ represents a nitrogen atom;
   X⁴ represents CR¹⁸;
   R⁸, R⁹, R¹⁰, R¹¹, R¹², R¹⁶, and R¹⁸ each represent a hydrogen atom; and
   R¹ represents a 1-piperidyl group, a 4-methyl-piperidin-1-yl group, a 4,4-dimethyl-piperidin-1-yl group, a 1-azepanyl group, a 1H-pyrazol-1-yl group, a 2-pyridyl group, or a 3-pyridyl group {wherein said 1H-pyrazol-1-yl group, said 2-pyridyl group, and said 3-pyridyl group are optionally substituted with one or more substituent(s) selected from Group A2}.
[Aspect 367] The compound according to the Aspect 365, wherein n represents 2.
[Aspect 368] The compound according to the Aspect 365, wherein
   Q represents the group represented by Q5; and
   n represents 2.
[Aspect 369] The compound according to the Aspect 366, wherein n represents 2.
[Aspect 370] The compound according to the Aspect 366, wherein
   Q represents the group represented by Q5; and
   n represents 2.
[Aspect 371] The Present compound N, wherein
   Z represents a C2-C6 chain hydrocarbon group or a 5-6 membered aromatic heterocyclic group optionally substituted with one or more substituent(s) selected from Group D;
   Q represents the group represented by Q1, the group represented by Q2, the group represented by Q5, or the group represented by Q6;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   R¹ represents a 3-10 membered nonaromatic heterocyclic group {wherein said 3-10 membered nonaromatic heterocyclic group is optionally substituted with one or more substituent(s) selected from Group A2, and among atoms constituting said 3-10 membered nonaromatic heterocyclic group, any atom which is not bound to Q optionally forms a C3-C7 alicyclic hydrocarbon or a 3-7 membered nonaromatic heterocycle as a spiro atom}, or a 5-7 membered aromatic heterocyclic group optionally substituted with one or more substituent(s) selected from Group B.
[Aspect 372] The Present compound N, or the compound according to any one of the Aspects 1 to 341, wherein R¹ represents a C3-C10 alicyclic hydrocarbon group, a 3-10 membered nonaromatic heterocyclic group (provided that when Q represents the group represented by Q2, then a carbon atom constituting said 3-10 membered nonaromatic heterocyclic group is bound to the group represented by Q2) {wherein said C3-C10 alicyclic hydrocarbon group and said 3-10 membered nonaromatic heterocyclic group (provided that when Q represents the group represented by Q2, then a carbon atom constituting said 3-10 membered nonaromatic heterocyclic group is bound to the group represented by Q2) are optionally substituted with one or more substituent(s) selected from Group A, and among atoms constituting said C3-C10 alicyclic hydrocarbon group and said 3-10 membered nonaromatic heterocyclic group (provided that when Q represents the group represented by Q2, then a carbon atom constituting said 3-10 membered nonaromatic heterocyclic group is bound to the group represented by Q2), any atom which is not bound to Q optionally forms a C3-C7 alicyclic hydrocarbon or a 3-7 membered nonaromatic heterocycle as a spiro atom}, a 5-7 membered aromatic heterocyclic group optionally substituted with one or more substituent(s) selected from Group B (provided that a 1H-pyrazol-3-yl group is excluded when Q represents the group represented by Q6, and when Q represents the group represented by Q2, then a carbon atom constituting said 5-7 membered aromatic heterocyclic group is bound to the group represented by Q2) {wherein among atoms constituting said 5-7 membered aromatic heterocyclic group (provided that a 1H-pyrazol-3-yl group is excluded when Q represents the group represented by Q6, and when Q represents the group represented by Q2, then a carbon atom constituting said 5-7 membered aromatic heterocyclic group is bound to the group represented by Q2), any two adjacent atoms which are not bound to Q optionally form another C4-C7 alicyclic hydrocarbon, 4-7 membered nonaromatic heterocycle, or 5-7 membered aromatic heterocycle comprising said two adjacent atoms}, or -C≡CR¹³.
[Aspect 373] The Present compound N, wherein R¹ represents a 3-10 membered nonaromatic heterocyclic group (provided that when Q represents the group represented by Q2, then a carbon atom constituting said 3-10 membered nonaromatic heterocyclic group is bound to the group represented by Q2) {wherein said 3-10 membered nonaromatic heterocyclic group (provided that when Q represents the group represented by Q2, then a carbon atom constituting said 3-10 membered nonaromatic heterocyclic group is bound to the group represented by Q2) is optionally substituted with one or more substituent(s) selected from Group A2, and among atoms constituting said 3-10 membered nonaromatic heterocyclic group (provided that when Q represents the group represented by Q2, then a carbon atom constituting said 3-10 membered nonaromatic heterocyclic group is bound to the group represented by Q2), any atom which is not bound to Q optionally forms a C3-C7 alicyclic hydrocarbon or a 3-7 membered nonaromatic heterocycle as a spiro atom}.
[Aspect 374] The Present compound N, wherein R¹ represents a 5-7 membered nonaromatic heterocyclic group optionally substituted with one or more substituent(s) selected from Group A2 (provided that when Q represents the group represented by Q2, then a carbon atom constituting said 5-7 membered nonaromatic heterocyclic group is bound to the group represented by Q2).
[Aspect 375] The Present compound N, wherein
   Q represents the group represented by Q1, the group represented by Q3, the group represented by Q4, the group represented by Q5, or the group represented by Q6; and
   R¹ represents a 1-piperidyl group or a 1-azepanyl group.
[Aspect 376] The Present compound N, wherein R¹ represents a C3-C10 alicyclic hydrocarbon group, or a 3-10 membered nonaromatic heterocyclic group (provided that when Q represents the group represented by Q2, then a carbon atom constituting said 3-10 membered nonaromatic heterocyclic group is bound to the group represented by Q2) {wherein said C3-C10 alicyclic hydrocarbon group and said 3-10 membered nonaromatic heterocyclic group (provided that when Q represents the group represented by Q2, then a carbon atom constituting said 3-10 membered nonaromatic heterocyclic group is bound to the group represented by Q2) are optionally substituted with one or more substituent(s) selected from Group A2, and among atoms constituting said C3-C10 alicyclic hydrocarbon group and said 3-10 membered nonaromatic heterocyclic group (provided that when Q represents the group represented by Q2, then a carbon atom constituting said 3-10 membered nonaromatic heterocyclic group is bound to the group represented by Q2), any atom which is not bound to Q optionally forms a C3-C7 alicyclic hydrocarbon or a 3-7 membered nonaromatic heterocycle as a spiro atom}.
[Aspect 377] The Present compound N, wherein R¹ represents a C5-C7 alicyclic hydrocarbon group or a 5-7 membered nonaromatic heterocyclic group (provided that when Q represents the group represented by Q2, then a carbon atom constituting said 5-7 membered nonaromatic heterocyclic group is bound to the group represented by Q2) {wherein said C5-C7 alicyclic hydrocarbon group and said 5-7 membered nonaromatic heterocyclic group (provided that when Q represents the group represented by Q2, then a carbon atom constituting said 5-7 membered nonaromatic heterocyclic group is bound to the group represented by Q2) are optionally substituted with one or more substituent(s) selected from Group A2}.
[Aspect 378] The Present compound N, wherein R¹ represents a 5-7 membered aromatic heterocyclic group optionally substituted with one or more substituent(s) selected from Group B (provided that a 1H-pyrazol-3-yl group is excluded when Q represents the group represented by Q6, and when Q represents the group represented by Q2, then a carbon atom constituting said 5-7 membered aromatic heterocyclic group is bound to the group represented by Q2) {wherein among atoms constituting said 5-7 membered aromatic heterocyclic group (provided that a 1H-pyrazol-3-yl group is excluded when Q represents the group represented by Q6, and when Q represents the group represented by Q2, then a carbon atom constituting said 5-7 membered aromatic heterocyclic group is bound to the group represented by Q2), any two adjacent atoms which are not bound to Q optionally form another C4-C7 alicyclic hydrocarbon, 4-7 membered nonaromatic heterocycle, or 5-7 membered aromatic heterocycle comprising said two adjacent atoms}.
[Aspect 379] The Present compound N, wherein R¹ represents a 5-6 membered aromatic heterocyclic group optionally substituted with one or more substituent(s) selected from Group A2 (provided that a 1H-pyrazol-3-yl group is excluded when Q represents the group represented by Q6, and when Q represents the group represented by Q2, then a carbon atom constituting said 5-6 membered aromatic heterocyclic group is bound to the group represented by Q2).
[Aspect 380] The Present compound N, wherein
   Q represents the group represented by Q1, the group represented by Q3, the group represented by Q4, the group represented by Q5, or the group represented by Q6; and
   R¹ represents a 1H-pyrazol-1-yl group, a 1H-pyrazol-3-yl group, a 1H-pyrazol-4-yl group, a 2-thienyl group, a 2-pyridyl group, a 3-pyridyl group, or a 4-pyridyl group {wherein said 1H-pyrazol-1-yl group, said 1H-pyrazol-3-yl group, said 1H-pyrazol-4-yl group, said 2-thienyl group, said 2-pyridyl group, said 3-pyridyl group, and said 4-pyridyl group are optionally substituted with one or more substituent(s) selected from Group A2}.
[Aspect 381] The Present compound N, wherein R¹ represents a 5 membered aromatic heterocyclic group optionally substituted with one or more substituent(s) selected from Group A2 (provided that a 1H-pyrazol-3-yl group is excluded when Q represents the group represented by Q6, and when Q represents the group represented by Q2, then a carbon atom constituting said 5 membered aromatic heterocyclic group is bound to the group represented by Q2).
[Aspect 382] The Present compound N, wherein R¹ represents a 6 membered aromatic heterocyclic group optionally substituted with one or more substituent(s) selected from Group A2 (provided that when Q represents the group represented by Q2, then a carbon atom constituting said 6 membered aromatic heterocyclic group is bound to the group represented by Q2).
[Aspect 383] The Present compound N, wherein R¹ represents a 3-10 membered nonaromatic heterocyclic group (provided that when Q represents the group represented by Q2, then a carbon atom constituting said 3-10 membered nonaromatic heterocyclic group is bound to the group represented by Q2) {wherein said 3-10 membered nonaromatic heterocyclic group (provided that when Q represents the group represented by Q2, then a carbon atom constituting said 3-10 membered nonaromatic heterocyclic group is bound to the group represented by Q2) is optionally substituted with one or more substituent(s) selected from Group A2, and among atoms constituting said 3-10 membered nonaromatic heterocyclic group (provided that when Q represents the group represented by Q2, then a carbon atom constituting said 3-10 membered nonaromatic heterocyclic group is bound to the group represented by Q2), any atom which is not bound to Q optionally forms a C3-C7 alicyclic hydrocarbon or a 3-7 membered nonaromatic heterocycle as a spiro atom}, a 5-7 membered aromatic heterocyclic group optionally substituted with one or more substituent(s) selected from Group B (provided that a 1H-pyrazol-3-yl group is excluded when Q represents the group represented by Q6, and when Q represents the group represented by Q2, then a carbon atom constituting said 5-7 membered aromatic heterocyclic group is bound to the group represented by Q2), or -C≡CR¹³.
[Aspect 384] The Present compound N, wherein
   R¹ represents a 5-7 membered nonaromatic heterocyclic group optionally substituted with one or more substituent(s) selected from Group A2 (provided that when Q represents the group represented by Q2, then a carbon atom constituting said 5-7 membered nonaromatic heterocyclic group is bound to the group represented by Q2), a 5-7 membered aromatic heterocyclic group optionally substituted with one or more substituent(s) selected from Group A2 (provided that a 1H-pyrazol-3-yl group is excluded when Q represents the group represented by Q6, and when Q represents the group represented by Q2, then a carbon atom constituting said 5-7 membered aromatic heterocyclic group is bound to the group represented by Q2), or -C≡CR¹³; and
   R¹³ represents a C1-C6 chain hydrocarbon group or a C3-C7 alicyclic hydrocarbon group.
[Aspect 385] The Present compound N, wherein
   Q represents the group represented by Q1, the group represented by Q3, the group represented by Q4, the group represented by Q5, or the group represented by Q6;
   R¹ represents a 1-cyclopenten-1-yl group, a 1-cyclohexen-1-yl group, a 4,4-dimethyl-1-cyclohexen-1-yl group, a 1H-pyrazol-1-yl group, a 2-pyridyl group, a 3-pyridyl group, a 4-pyridyl group {wherein said 1H-pyrazol-1-yl group, said 2-pyridyl group, said 3-pyridyl group, and said 4-pyridyl group are optionally substituted with one or more substituent(s) selected from Group A2}, or -C≡CR¹³; and
   R¹³ represents a 3,3-dimethyl-1-butyn-1-yl group, a 1-cyclohexen-1-yl group, or a 1-cyclopenten-1-yl group.
[Aspect 386] The Present compound N, wherein
   Z represents a C2-C6 chain hydrocarbon group or a 5-6 membered aromatic heterocyclic group optionally substituted with one or more substituent(s) selected from Group D;
   Q represents the group represented by Q1, the group represented by Q2, the group represented by Q5, or the group represented by Q6;
   X² represents CR¹⁶;
   X³ represents a nitrogen atom;
   X⁴ represents CR¹⁸;
   R², R³, R⁴, R⁵, R⁸, R⁹, R¹⁰, R¹¹, R¹², R¹⁶, and R¹⁸ each represent a hydrogen atom; and
   R¹ represents a 3-10 membered nonaromatic heterocyclic group (provided that when Q represents the group represented by Q2, then a carbon atom constituting said 3-10 membered nonaromatic heterocyclic group is bound to the group represented by Q2) {wherein said 3-10 membered nonaromatic heterocyclic group (provided that when Q represents the group represented by Q2, then a carbon atom constituting said 3-10 membered nonaromatic heterocyclic group is bound to the group represented by Q2) is optionally substituted with one or more substituent(s) selected from Group A2, and among atoms constituting said 3-10 membered nonaromatic heterocyclic group (provided that when Q represents the group represented by Q2, then a carbon atom constituting said 3-10 membered nonaromatic heterocyclic group is bound to the group represented by Q2), any atom which is not bound to Q optionally forms a C3-C7 alicyclic hydrocarbon or a 3-7 membered nonaromatic heterocycle as a spiro atom}, or a 5-7 membered aromatic heterocyclic group optionally substituted with one or more substituent(s) selected from Group B.

Aspects of the Compound Z of the present invention include the following compounds.
[Aspect CZ1] The Compound Z of the present invention, wherein Z² represents a C2-C6 chain hydrocarbon group.
[Aspect CZ2] The Compound Z of the present invention, wherein Z^{a} represents a C2-C6 alkyl group.
[Aspect CZ3] The Compound Z of the present invention, wherein Z^{a} represents an ethyl group or a propyl group.
[Aspect CZ4] The Compound Z of the present invention, wherein Z^{a} represents a C3-C8 alicyclic hydrocarbon group.
[Aspect CZ5] The Compound Z of the present invention, wherein Z^{a} represents a cyclopropyl group, a cyclopentyl group, or a cyclohexyl group.
[Aspect CZ6] The Compound Z of the present invention, wherein Z^{a} represents a 5-10 membered aromatic heterocyclic group optionally substituted with one or more substituent(s) selected from Group D^{a}.
[Aspect CZ7] The Compound Z of the present invention, wherein Z^{a} represents a 5 membered aromatic heterocyclic group.
[Aspect CZ8] The Compound Z of the present invention, wherein Z^{a} represents a 2-furyl group, a 3-furyl group, a 2-thienyl group, or a 3-thienyl group.
[Aspect CZ9] The Compound Z of the present invention, wherein Z^{a} represents a 6 membered aromatic heterocyclic group.
[Aspect CZ10] The Compound Z of the present invention, wherein Z^{a} represents a 2-pyridyl group, a 3-pyridyl group, a 4-pyridyl group, a 2-pyrimidinyl group, or a 2-pyrazinyl group.
[Aspect CZ11] The Compound Z of the present invention, wherein Z^{a} represents a 5-6 membered aromatic heterocyclic group.
[Aspect CZ12] The Compound Z of the present invention, wherein Z^{a} represents a C2-C6 alkyl group or a 5-6 membered aromatic heterocyclic group.
[Aspect CZ13] The Compound Z of the present invention, wherein Z^{a} represents an ethyl group, a propyl group, a 2-furyl group, a 3-furyl group, a 2-thienyl group, a 3-thienyl group, a 2-pyridyl group, a 3-pyridyl group, a 4-pyridyl group, a 2-pyrimidinyl group, or a 2-pyrazinyl group.
[Aspect CZ14] The Compound Z of the present invention, wherein Z^{a} represents an ethyl group, a propyl group, or a 2-thienyl group.
[Aspect CZ15] The Compound Z of the present invention, wherein X^{1a} represents CH.
[Aspect CZ16] The Compound Z of the present invention, wherein X^{1a} represents a nitrogen atom.
[Aspect CZ17] The Compound Z of the present invention, wherein R^{1a} represents a 3-7 membered nonaromatic heterocyclic group {wherein said 3-7 membered nonaromatic heterocyclic group is optionally substituted with one or more substituent(s) selected from Group A^{a}, and among atoms constituting said 3-7 membered nonaromatic heterocyclic group, any atom which is not bound to the pyridine or pyrazine optionally forms a C3-C7 alicyclic hydrocarbon or a 3-7 membered nonaromatic heterocycle as a spiro atom}.
[Aspect CZ18] The Compound Z of the present invention, wherein R^{1a} represents a 5-7 membered nonaromatic heterocyclic group optionally substituted with one or more substituent(s) selected from Group A^{a}2;
   Group A^{a}2: a group consisting of a C1-C3 alkyl group and a halogen atom.
[Aspect CZ19] The Compound Z of the present invention, wherein R^{1a} represents a 1-piperidyl group, a 4-methyl-piperidin-1-yl group, a 4,4-dimethyl-piperidin-1-yl group, or a 1-azepanyl group.
[Aspect CZ20] The Compound Z of the present invention, wherein R^{1a} represents a 5-7 membered aromatic heterocyclic group {wherein said 5-7 membered aromatic heterocyclic group is optionally substituted with one or more substituent(s) selected from Group B1^{a}, and among atoms constituting said 5-7 membered aromatic heterocyclic group, any two adjacent atoms which are not bound to the pyridine or pyrazine optionally form another benzene ring, C4-C7 alicyclic hydrocarbon, 4-7 membered nonaromatic heterocycle, or 5-7 membered aromatic heterocycle comprising said two adjacent atoms {wherein said benzene ring, said C4-C7 alicyclic hydrocarbon, said 4-7 membered nonaromatic heterocycle, and said 5-7 membered aromatic heterocycle are optionally substituted with one or more halogen atom(s)}}.
[Aspect CZ21] The Compound Z of the present invention, wherein R^{1a} represents a 5 membered aromatic heterocyclic group optionally substituted with one or more substituent(s) selected from Group A^{a}2 {wherein among atoms constituting said 5 membered aromatic heterocycle, any two adjacent atoms which are not bound to the pyridine or pyrazine optionally form another benzene ring or C4-C7 alicyclic hydrocarbon comprising said two adjacent atoms {wherein said benzene ring and said C4-C7 alicyclic hydrocarbon are optionally substituted with one or more halogen atom(s)}}.
[Aspect CZ22] The Compound Z of the present invention, wherein R^{1a} represents a 1H-pyrazol-1-yl group, a 1H-pyrazol-3-yl group, or a 1H-pyrazol-4-yl group {wherein said 1H-pyrazol-1-yl group, said 1H-pyrazol-3-yl group, and said 1H-pyrazol-4-yl group are optionally substituted with one or more substituent(s) selected from Group A^{a}2}.
[Aspect CZ23] The Compound Z of the present invention, wherein R^{1a} represents a 1H-pyrazol-1-yl group optionally substituted with one or more substituent(s) selected from Group A^{a}2.
[Aspect CZ24] The Compound Z of the present invention, wherein R^{1a} represents a 6 membered aromatic heterocyclic group optionally substituted with one or more substituent(s) selected from Group A^{a}2.
[Aspect CZ25] The Compound Z of the present invention, wherein R^{1a} represents a 2-pyridyl group, a 3-pyridyl group, or a 4-pyridyl group {wherein said 2-pyridyl group, said 3-pyridyl group, and said 4-pyridyl group are optionally substituted with one or more substituent(s) selected from Group A^{a}2}
[Aspect CZ26] The Compound Z of the present invention, wherein R^{1a} represents a 5-6 membered aromatic heterocyclic group optionally substituted with one or more substituent(s) selected from Group A^{a}2 {wherein among atoms constituting said 5-6 membered aromatic heterocycle, any two adjacent atoms which are not bound to the pyridine or pyrazine optionally form another benzene ring or C4-C7 alicyclic hydrocarbon comprising said two adjacent atoms {wherein said benzene ring and said C4-C7 alicyclic hydrocarbon are optionally substituted with one or more halogen atom(s)}}.
[Aspect CZ27] The Compound Z of the present invention, wherein R^{1a} represents a 1H-pyrazol-1-yl group, a 1H-pyrazol-3-yl group, a 1H-pyrazol-4-yl group, a 2-pyridyl group, a 3-pyridyl group, or a 4-pyridyl group {wherein said 1H-pyrazol-1-yl group, said 1H-pyrazol-3-yl group, said 1H-pyrazol-4-yl group, said 2-pyridyl group, said 3-pyridyl group, and said 4-pyridyl group are optionally substituted with one or more substituent(s) selected from Group A^{a}2}.
[Aspect CZ28] The Compound Z of the present invention, wherein na represents 1.
[Aspect CZ29] The Compound Z of the present invention, wherein na represents 2.
[Aspect CZ30] The compound according to the Aspect CZ15, wherein na represents 1.
[Aspect CZ31] The compound according to the Aspect CZ16, wherein na represents 1.
[Aspect CZ32] The compound according to the Aspect CZ15, wherein na represents 2.
[Aspect CZ33] The compound according to the Aspect CZ16, wherein na represents 2.
[Aspect CZ34] The Compound Z of the present invention, wherein
   Z^{a} represents a C2-C6 alkyl group or a 5 membered aromatic heterocyclic group; and
   R^{1a} represents a 6-7 membered nonaromatic heterocyclic group or a 5-6 membered aromatic heterocyclic group {wherein said 6-7 membered nonaromatic heterocyclic group and said 5-6 membered aromatic heterocyclic group are optionally substituted with one or more substituent(s) selected from Group A^{a}2}.
[Aspect CZ35] The compound according to the Aspect CZ34, wherein
   na represents 2; and
   X^{1a} represents CH.
[Aspect CZ36] The Compound Z of the present invention, wherein
   Z^{a} represents an ethyl group, a propyl group, or a 2-thienyl group; and
   R^{1a} represents a 1-piperidyl group, a 4-methyl-piperidin-1-yl group, a 4,4-dimethyl-piperidin-1-yl group, a 1-azepanyl group, a 1H-pyrazol-1-yl group, a 1H-pyrazol-3-yl group, a 1H-pyrazol-4-yl group, a 2-pyridyl group, a 3-pyridyl group, or a 4-pyridyl group {wherein said 1H-pyrazol-1-yl group, said 1H-pyrazol-3-yl group, said 1H-pyrazol-4-yl group, said 2-pyridyl group, said 3-pyridyl group, and said 4-pyridyl group are optionally substituted with one or more substituent(s) selected from Group A^{a}2}.
[Aspect CZ37] The compound according to the Aspect CZ36, wherein
   na represents 2; and
   X^{1a} represents CH.
[Aspect CZ38] The Compound Z of the present invention, wherein
   Z^{a} represents a C2-C6 alkyl group or a 5 membered aromatic heterocyclic group; and
   R^{1a} represents a 6-7 membered nonaromatic heterocyclic group or a 6 membered aromatic heterocyclic group {wherein said 6-7 membered nonaromatic heterocyclic group and said 6 membered aromatic heterocyclic group are optionally substituted with one or more substituent(s) selected from Group A^{a}2}.
[Aspect CZ39] The Compound Z of the present invention, wherein
   Z^{a} represents an ethyl group, a propyl group, or a 2-thienyl group; and
   R^{1a} represents a 1-piperidyl group, a 4-methyl-piperidin-1-yl group, a 4,4-dimethyl-piperidin-1-yl group, a 1-azepanyl group, a 2-pyridyl group, a 3-pyridyl group, or a 4-pyridyl group {wherein said 2-pyridyl group, said 3-pyridyl group, and said 4-pyridyl group are optionally substituted with one or more substituent(s) selected from Group A^{a}2}.
[Aspect CZ40] The compound according to the Aspect CZ38, wherein
   na represents 2; and
   X^{1a} represents CH.
[Aspect CZ41] The compound according to the Aspect CZ39, wherein
   na represents 2; and
   X^{1a} represents CH.

Aspects of the Compound N of the present invention include the following compounds.
[Aspect C1] The Compound N of the present invention, wherein Z^{a} represents a C2-C6 chain hydrocarbon group.
[Aspect C2] The Compound N of the present invention, wherein Z^{a} represents a C2-C6 alkyl group.
[Aspect C3] The Compound N of the present invention, wherein Z^{a} represents an ethyl group or a propyl group.
[Aspect C4] The Compound N of the present invention, wherein Z^{a} represents a C3-C8 alicyclic hydrocarbon group.
[Aspect C5] The Compound N of the present invention, wherein Z^{a} represents a cyclopropyl group, a cyclopentyl group, or a cyclohexyl group.
[Aspect C6] The Compound N of the present invention, wherein Z² represents a 5-10 membered aromatic heterocyclic group optionally substituted with one or more substituent(s) selected from Group D^{a}.
[Aspect C7] The Compound N of the present invention, wherein Z^{a} represents a 5 membered aromatic heterocyclic group.
[Aspect C8] The Compound N of the present invention, wherein Z^{a} represents a 2-furyl group, a 3-furyl group, a 2-thienyl group, or a 3-thienyl group.
[Aspect C9] The Compound N of the present invention, wherein Z^{a} represents a 6 membered aromatic heterocyclic group.
[Aspect C10] The Compound N of the present invention, wherein Z² represents a 2-pyridyl group, a 3-pyridyl group, a 4-pyridyl group, a 2-pyrimidinyl group, or a 2-pyrazinyl group.
[Aspect C11] The Compound N of the present invention, wherein Z^{a} represents a 5-6 membered aromatic heterocyclic group.
[Aspect C12] The Compound N of the present invention, wherein Z^{a} represents a C2-C6 alkyl group or a 5-6 membered aromatic heterocyclic group.
[Aspect C13] The Compound N of the present invention, wherein Z^{a} represents an ethyl group, a propyl group, a 2-furyl group, a 3-furyl group, a 2-thienyl group, a 3-thienyl group, a 2-pyridyl group, a 3-pyridyl group, a 4-pyridyl group, a 2-pyrimidinyl group, or a 2-pyrazinyl group.
[Aspect C14] The Compound N of the present invention, wherein Z^{a} represents an ethyl group, a propyl group, or a 2-thienyl group.
[Aspect C15] The Compound N of the present invention, wherein X^{1a} represents CH.
[Aspect C16] The Compound N of the present invention, wherein X^{1a} represents a nitrogen atom.
[Aspect C17] The Compound N of the present invention, wherein R^{1a} represents a 3-7 membered nonaromatic heterocyclic group {wherein said 3-7 membered nonaromatic heterocyclic group is optionally substituted with one or more substituent(s) selected from Group A^{a}, and among atoms constituting said 3-7 membered nonaromatic heterocyclic group, any atom which is not bound to the pyridine or pyrazine optionally forms a C3-C7 alicyclic hydrocarbon or a 3-7 membered nonaromatic heterocycle as a spiro atom}.
[Aspect C18] The Compound N of the present invention, wherein R^{1a} represents a 5-7 membered nonaromatic heterocyclic group optionally substituted with one or more substituent(s) selected from Group A^{a}2;
   Group A^{a}2: a group consisting of a C1-C3 alkyl group and a halogen atom.
[Aspect C19] The Compound N of the present invention, wherein R^{1a} represents a 1-piperidyl group, a 4-methyl-piperidin-1-yl group, a 4,4-dimethyl-piperidin-1-yl group, or a 1-azepanyl group.
[Aspect C20] The Compound N of the present invention, wherein R^{1a} represents a 5-7 membered aromatic heterocyclic group {wherein said 5-7 membered aromatic heterocyclic group is optionally substituted with one or more substituent(s) selected from Group B^{a}, and among atoms constituting said 5-7 membered aromatic heterocyclic group, any two adjacent atoms which are not bound to the pyridine or pyrazine optionally form another C4-C7 alicyclic hydrocarbon, 4-7 membered nonaromatic heterocycle, or 5-7 membered aromatic heterocycle comprising said two adjacent atoms}.
[Aspect C21] The Compound N of the present invention, wherein R^{1a} represents a 5 membered aromatic heterocyclic group optionally substituted with one or more substituent(s) selected from Group A^{a}2.
[Aspect C22] The Compound N of the present invention, wherein R^{1a} represents a 1H-pyrazol-1-yl group, a 1H-pyrazol-3-yl group, or a 1H-pyrazol-4-yl group {wherein said 1H-pyrazol-1-yl group, said 1H-pyrazol-3-yl group, and said 1H-pyrazol-4-yl group are optionally substituted with one or more substituent(s) selected from Group A^{a}2}.
[Aspect C23] The Compound N of the present invention, wherein R^{1a} represents a 1H-pyrazol-1-yl group optionally substituted with one or more substituent(s) selected from Group A^{a}2.
[Aspect C24] The Compound N of the present invention, wherein R^{1a} represents a 6 membered aromatic heterocyclic group optionally substituted with one or more substituent(s) selected from Group A^{a}2.
[Aspect C25] The Compound N of the present invention, wherein R^{1a} represents a 2-pyridyl group, a 3-pyridyl group, or a 4-pyridyl group {wherein said 2-pyridyl group, said 3-pyridyl group, and said 4-pyridyl group are optionally substituted with one or more substituent(s) selected from Group A^{a}2}.
[Aspect C26] The Compound N of the present invention, wherein R^{1a} represents a 5-6 membered aromatic heterocyclic group optionally substituted with one or more substituent(s) selected from Group A^{a}2.
[Aspect C27] The Compound N of the present invention, wherein R^{1a} represents a 1H-pyrazol-1-yl group, a 1H-pyrazol-3-yl group, a 1H-pyrazol-4-yl group, a 2-pyridyl group, a 3-pyridyl group, or a 4-pyridyl group {wherein said 1H-pyrazol-1-yl group, said 1H-pyrazol-3-yl group, said 1H-pyrazol-4-yl group, said 2-pyridyl group, said 3-pyridyl group, and said 4-pyridyl group are optionally substituted with one or more substituent(s) selected from Group A^{a}2}.
[Aspect C28] The Compound N of the present invention, wherein na represents 1.
[Aspect C29] The Compound N of the present invention, wherein na represents 2.
[Aspect C30] The compound according to the Aspect C15, wherein na represents 1.
[Aspect C31] The compound according to the Aspect C16, wherein na represents 1.
[Aspect C32] The compound according to the Aspect C15, wherein na represents 2.
[Aspect C33] The compound according to the Aspect C16, wherein na represents 2.
[Aspect C34] The Compound N of the present invention, wherein
   Z^{a} represents a C2-C6 alkyl group or a 5 membered aromatic heterocyclic group; and
   R^{1a} represents a 6-7 membered nonaromatic heterocyclic group or a 5-6 membered aromatic heterocyclic group {wherein said 6-7 membered nonaromatic heterocyclic group and said 5-6 membered aromatic heterocyclic group are optionally substituted with one or more substituent(s) selected from Group A^{a}2}.
[Aspect C35] The compound according to the Aspect C34, wherein
   na represents 2; and
   X^{1a} represents CH.
[Aspect C36] The Compound N of the present invention, wherein
   Z^{a} represents an ethyl group, a propyl group, or a 2-thienyl group; and
   R^{1a} represents a 1-piperidyl group, a 4-methyl-piperidin-1-yl group, a 4,4-dimethyl-piperidin-1-yl group, a 1-azepanyl group, a 1H-pyrazol-1-yl group, a 1H-pyrazol-3-yl group, a 1H-pyrazol-4-yl group, a 2-pyridyl group, a 3-pyridyl group, or a 4-pyridyl group {wherein said 1H-pyrazol-1-yl group, said 1H-pyrazol-3-yl group, said 1H-pyrazol-4-yl group, said 2-pyridyl group, said 3-pyridyl group, and said 4-pyridyl group are optionally substituted with one or more substituent(s) selected from Group A^{a}2}.
[Aspect C37] The compound according to the Aspect C36, wherein
   na represents 2; and
   X^{1a} represents CH.
[Aspect C38] The Compound N of the present invention, wherein
   Z^{a} represents a C2-C6 alkyl group or a 5 membered aromatic heterocyclic group; and
   R^{1a} represents a 6-7 membered nonaromatic heterocyclic group or a 6 membered aromatic heterocyclic group {wherein said 6-7 membered nonaromatic heterocyclic group and said 6 membered aromatic heterocyclic group are optionally substituted with one or more substituent(s) selected from Group A^{a}2}.
[Aspect C39] The Compound N of the present invention, wherein
   Z^{a} represents an ethyl group, a propyl group, or a 2-thienyl group; and
   R^{1a} represents a 1-piperidyl group, a 4-methyl-piperidin-1-yl group, a 4,4-dimethyl-piperidin-1-yl group, a 1-azepanyl group, a 2-pyridyl group, a 3-pyridyl group, or a 4-pyridyl group {wherein said 2-pyridyl group, said 3-pyridyl group, and said 4-pyridyl group are optionally substituted with one or more substituent(s) selected from Group A^{a}2}.
[Aspect C40] The compound according to the Aspect C38, wherein
   na represents 2; and
   X^{1a} represents CH.
[Aspect C41] The compound according to the Aspect C39, wherein
   na represents 2; and
   X^{1a} represents CH.
[Aspect A1] A method for controlling Phakopsora pachyrhizi which comprises applying the Present compound to a soybean or soil for cultivating a soybean.
[Aspect A2] A method for controlling Phakopsora pachyrhizi which comprises applying the Present compound W to a soybean or soil for cultivating a soybean.
[Aspect AC1] A method for controlling Phakopsora pachyrhizi which comprises applying the Compound of the present invention to a soybean or soil for cultivating a soybean.
[Aspect AC2] A seed or a vegetative reproductive organ holding an effective amount of the Present compound.
[Aspect AC3] A method for controlling Phakopsora pachyrhizi which comprises applying the Compound W of the present invention to a soybean or soil for cultivating a soybean.
[Aspect AC4] A seed or a vegetative reproductive organ holding an effective amount of the Present compound W.

Next, production methods of the Present compounds W (including Compounds W of the present invention) are described.

### Production method A

A compound represented by formula (I-b) (hereinafter referred to as "Compound (I-b)") or a compound represented by formula (I-c) (hereinafter referred to as "Compound (I-c)") may be prepared by reacting a compound represented by formula (I-a) (hereinafter referred to as "Compound (I-a)") with an oxidizing agent. 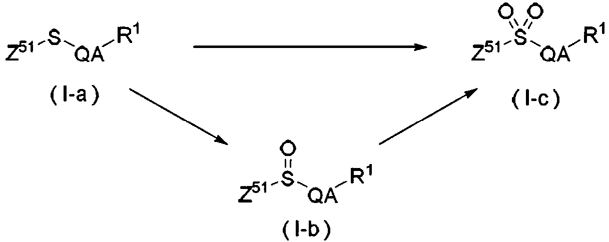 [wherein:
QA represents the group represented by Q2, the group represented by Q3, the group represented by Q4, the group represented by Q5, or the group represented by Q6;
Z⁵¹ represents a C2-C6 chain hydrocarbon group, a methyl group substituted with one or more fluorine atom(s), a C1-C2 alkyl group {wherein said C1-C2 alkyl group is substituted with a methoxy group or an ethoxy group}, a C3-C8 alicyclic hydrocarbon group, a 3-8 membered nonaromatic heterocyclic group {wherein said C3-C8 alicyclic hydrocarbon group and said 3-8 membered nonaromatic heterocyclic group are optionally substituted with one or more substituent(s) selected from Group C}, or a 5-10 membered aromatic heterocyclic group optionally substituted with one or more substituent(s) selected from Group D; and
the other symbols are the same as defined above.]

First, a method for producing the Compound (I-b) from the Compound (I-a) is described.

The reaction is usually carried out in a solvent. Examples of the solvent to be used in the reaction include halogenated hydrocarbons such as dichloromethane, chloroform, and chlorobenzene (hereinafter collectively referred to as "halogenated hydrocarbons"); nitriles such as acetonitrile and propionitrile (hereinafter collectively referred to as "nitriles"); alcohols such as methanol and ethanol (hereinafter collectively referred to as "alcohols"); acetic acid; water; and mixtures of two or more of them.

Examples of the oxidizing agent to be used in the reaction include sodium periodate, m-chloroperbenzoic acid (hereinafter referred to as "mCPBA"), and hydrogen peroxide.

When hydrogen peroxide is used as the oxidizing agent, a base or a catalyst may be used as needed.

Examples of the base to be used in the reaction include sodium carbonate. When a base is used in the reaction, the base is usually used at a ratio of 0.01 to 1 mol relative to 1 mol of the Compound (I-a).

Examples of the catalyst to be used in the reaction include sodium tungstate. When a catalyst is used in the reaction, the catalyst is usually used at a ratio of 0.01 to 0.5 mol relative to 1 mol of the Compound (I-a).

In the reaction, the oxidizing agent is usually used at a ratio of 1 to 1.2 mol relative to 1 mol of the Compound (I-a).

The reaction temperature is usually within the range of -20 to 80°C. The reaction time is usually within the range of 0.1 to 12 hour(s).

When the reaction is completed, to the reaction mixture is added water, the resulting mixture is subjected to extraction with organic solvent(s), and the resulting organic layer is washed with an aqueous solution of a reducing agent (for example, sodium sulfite or sodium thiosulfate) and an aqueous solution of a base (for example, sodium hydrogen carbonate) as needed. The resulting organic layer may be dried and/or concentrated to give the Compound (I-b).

Next, a method for producing the Compound (I-c) from the Compound (I-b) is described.

The reaction is usually carried out in a solvent. Examples of the solvent to be used in the reaction include halogenated hydrocarbons, nitriles, alcohols, acetic acid, water, and mixtures of two or more of them.

Examples of the oxidizing agent to be used in the reaction include mCPBA and hydrogen peroxide.

When hydrogen peroxide is used as the oxidizing agent, a base or a catalyst may be used as needed.

Examples of the base to be used in the reaction include sodium carbonate. When a base is used in the reaction, the base is usually used at a ratio of 0.01 to 1 mol relative to 1 mol of the Compound (I-b).

Examples of the catalyst to be used in the reaction include sodium tungstate. When a catalyst is used in the reaction, the catalyst is usually used at a ratio of 0.01 to 0.5 mol relative to 1 mol of the Compound (I-b).

In the reaction, the oxidizing agent is usually used at a ratio of 1 to 2 mol relative to 1 mol of the Compound (I-b).

The reaction temperature is usually within the range of -20 to 120°C. The reaction time is usually within the range of 0.1 to 12 hour(s).

When the reaction is completed, to the reaction mixture is added water, the resulting mixture is subjected to extraction with organic solvent(s), and the resulting organic layer is washed with an aqueous solution of a reducing agent (for example, sodium sulfite or sodium thiosulfate) and an aqueous solution of a base (for example, sodium hydrogen carbonate) as needed. The resulting organic layer may be dried and/or concentrated to give the Compound (I-c).

Also, the Compound (I-c) may be prepared in one step reaction (one-pot) by reacting the Compound (I-a) with an oxidizing agent.

The reaction may be carried out according to the method for producing the Compound (I-c) from the Compound (I-b) by usually using the oxidizing agent at a ratio of 2 to 5 mol relative to 1 mol of the Compound (I-a).

### Production method B

A compound represented by formula (I-d) (hereinafter referred to as "Compound (I-d)") may be prepared by reacting a compound represented by formula (B1) (hereinafter referred to as "Compound (B1)") with a compound represented by formula (M1) (hereinafter referred to as "Compound (M1)") in the presence of a base. 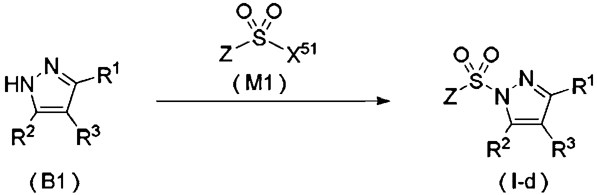 [wherein:
X⁵¹ represents a chlorine atom or OS(O)₂Z; and
the other symbols are the same as defined above.]

The reaction is usually carried out in a solvent. Examples of the solvent to be used in the reaction include hydrocarbons such as hexane, toluene, and xylene (hereinafter collectively referred to as "hydrocarbons"); ethers such as methyl tert-butyl ether (hereinafter referred to as "MTBE"), tetrahydrofuran (hereinafter referred to as "THF"), and dimethoxyethane (hereinafter referred to as "DME") (hereinafter collectively referred to as "ethers"); halogenated hydrocarbons; amides such as dimethylformamide (hereinafter referred to as "DMF") and N-methylpyrrolidone (hereinafter collectively referred to as "amides"); esters such as methyl acetate and ethyl acetate (hereinafter collectively referred to as "esters"); nitriles; water; and mixtures of two or more of them.

Examples of the base to be used in the reaction include organic bases such as triethylamine and pyridine (hereinafter collectively referred to as "organic bases"); alkali metal carbonates such as sodium carbonate and potassium carbonate (hereinafter collectively referred to as "alkali metal carbonates"); alkali metal hydrogen carbonates such as sodium hydrogen carbonate and potassium hydrogen carbonate (hereinafter collectively referred to as "alkali metal hydrogen carbonates"); alkali metal hydroxides such as lithium hydroxide, sodium hydroxide, potassium hydroxide, and cesium hydroxide (hereinafter collectively referred to as "alkali metal hydroxides"); alkali metal hydrides such as lithium hydride and sodium hydride (hereinafter collectively referred to as "alkali metal hydrides"); and alkali metal alkoxides such as sodium tert-butoxide and potassium tert-butoxide (hereinafter collectively referred to as "alkali metal alkoxides").

In the reaction, the Compound (M1) is usually used at a ratio of 1 to 10 mol, and the base is usually used at a ratio of 1 to 10 mol, relative to 1 mol of the Compound (B1).

The reaction temperature is usually within the range of -20 to 150°C. The reaction time is usually within the range of 0.1 to 120 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-d).

The Compound (M1) is a commercially available compound or may be prepared by using known method(s).

### Production method B-1

A compound represented by formula (I-d-A) (hereinafter referred to as "Compound (I-d-A)") may be prepared by reacting the Compound (B1) with a compound represented by formula (M1-A) (hereinafter referred to as "Compound (M1-A)") in the presence of a base. 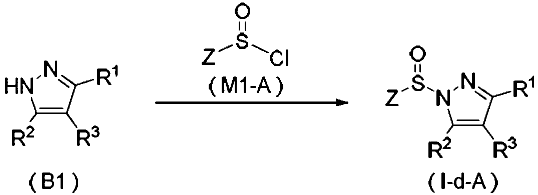 [wherein the symbols are the same as defined above.]

The reaction may be carried out according to the Production method B by using the Compound (M1-A) instead of the Compound (M1).

The Compound (M1-A) is a commercially available compound or may be prepared by using known method(s) such as the method(s) described in Synthesis, 1987, 1, 72-73.

### Production method C

The Compound (I-a) may be prepared by reacting a compound represented by formula (B2) (hereinafter referred to as "Compound (B2)") with a compound represented by formula (M2) (hereinafter referred to as "Compound (M2)") in the presence of a base. 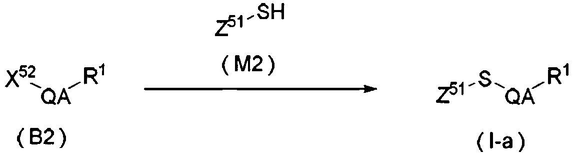 [wherein:
X⁵² represents a halogen atom, a mesyloxy group, or a trifuryloxy group; and
the other symbols are the same as defined above.]

The reaction is usually carried out in a solvent. Examples of the solvent to be used in the reaction include alcohols, nitriles, ethers, hydrocarbons, amides, water, and mixtures of two or more of them.

Examples of the base to be used in the reaction include alkali metal hydrides, alkali metal carbonates, organic bases, sodium hydride, and tripotassium phosphate.

When X⁵² represents a bromine atom, an iodine atom, or a trifuryloxy group, a metal catalyst and/or a ligand may be used as needed.

Examples of the metal catalyst to be used in the reaction include palladium catalysts such as palladium(II) acetate and [1,1'-bis(diphenylphosphino)ferrocene]palladium(II) dichloride; nickel catalysts such as bis(cyclooctadiene)nickel(0) and nickel(II) chloride; and copper catalysts such as copper(I) iodide and copper(I) chloride. When a metal catalyst is used in the reaction, the metal catalyst is usually used at a ratio of 0.01 to 1 mol relative to 1 mol of the Compound (B2).

Examples of the ligand to be used in the reaction include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, and 1,10-phenanthroline. When a ligand is used in the reaction, the ligand is usually used at a ratio of 0.01 to 1 mol relative to 1 mol of the Compound (B2).

In the reaction, the Compound (M2) is usually used at a ratio of 1 to 10 mol, and the base is usually used at a ratio of 1 to 10 mol, relative to 1 mol of the Compound (B2).

The reaction temperature is usually within the range of -20°C to 200°C. The reaction time is usually within the range of 0.1 to 72 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-a).

The Compound (M2) is known or may be prepared according to known method(s).

### Production method D

The Compound (I-c) may also be prepared by reacting the Compound (B2) with a compound represented by formula (M3) (hereinafter referred to as "Compound (M3)"). 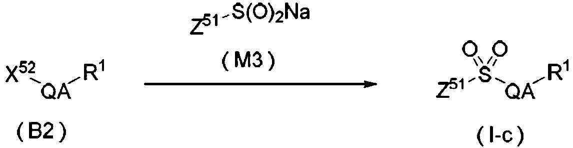 [wherein the symbols are the same as defined above.]

The reaction is usually carried out in a solvent. Examples of the solvent to be used in the reaction include hydrocarbons, ethers, halogenated hydrocarbons, amides, nitriles, dimethyl sulfoxide (hereinafter referred to as "DMSO"), water, and mixtures of two or more of them.

In the reaction, the Compound (M3) is usually used at a ratio of 1 to 3 mol relative to 1 mol of the Compound (B2).

The reaction temperature is usually within the range of 0 to 150°C. The reaction time is usually within the range of 0.1 to 48 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-c).

The Compound (M3) is known or may be prepared according to known method(s).

### Production method E

The Compound (I-a) may also be prepared by reacting a compound represented by formula (B3) (hereinafter referred to as "Compound (B3)") with a compound represented by formula (M4) (hereinafter referred to as "Compound (M4)") in the presence of a base. 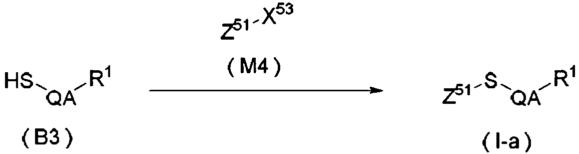 [wherein:
X⁵³ represents a chlorine atom, a bromine atom, an iodine atom, a mesyloxy group, or a trifuryloxy group; and
the other symbols are the same as defined above.]

The reaction is usually carried out in a solvent. Examples of the solvent to be used in the reaction include hydrocarbons, ethers, halogenated hydrocarbons, amides, esters, nitriles, water, and mixtures of two or more of them.

Examples of the base to be used in the reaction include organic bases, alkali metal carbonates, alkali metal hydrogen carbonates, sodium hydride, and tripotassium phosphate.

In the reaction, a metal catalyst and/or a ligand may be used as needed.

Examples of the metal catalyst to be used in the reaction include copper catalysts such as copper(I) iodide, copper(I) bromide, copper(I) chloride, copper(I) oxide, copper(I) trifluoromethanesulfonate benzene complex, tetrakis(acetonitrile)copper(I) hexafluorophosphate, and copper(I) thiophene-2-carboxylate; nickel catalysts such as bis(cyclooctadiene)nickel(0) and nickel(II) chloride; and palladium catalysts such as palladium acetate, tris(dibenzylideneaceton)dipalladium(0), tetrakis(triphenylphosphine)palladium(0), and [1,1'-bis(diphenylphosphino)ferrocene]palladium(II) dichloride. When a metal catalyst is used in the reaction, the metal catalyst is usually used at a ratio of 0.01 to 1 mol relative to 1 mol of the Compound (B3).

Examples of the ligand to be used in the reaction include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, N,N'-dimethylethylenediamine, and N,N-dimethylglycine hydrochloride. When a ligand is used in the reaction, the ligand is usually used at a ratio of 0.01 to 1 mol relative to 1 mol of the Compound (B3).

In the reaction, the Compound (M4) is usually used at a ratio of 1 to 10 mol, and the base is usually used at a ratio of 1 to 10 mol, relative to 1 mol of the Compound (B3).

The reaction temperature is usually within the range of 0 to 150°C. The reaction time is usually within the range of 0.1 to 48 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-a).

The Compound (M4) is known or may be prepared according to known method(s).

### Production method F

A compound represented by formula (I-e) (hereinafter referred to as "Compound (I-e)") may be prepared by reacting a compound represented by formula (B4) (hereinafter referred to as "Compound (B4)") with a compound represented by formula (M5) (hereinafter referred to as "Compound (M5)") in the presence of a base. 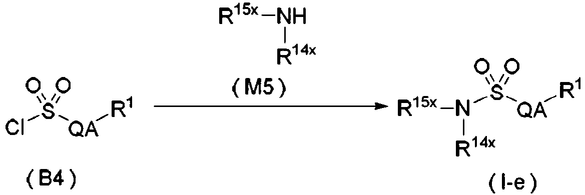 [wherein:
R^{14X} and R^{15X} are identical to or different from each other, and each represent a C1-C6 alkyl group, a methyl group substituted with one or more fluorine atom(s), or a C1-C2 alkyl group {wherein said C1-C2 alkyl group is substituted with a methoxy group or an ethoxy group}; or
R^{14X} and R^{15X} are optionally combined with the nitrogen atom to which they are attached to form a 3-8 membered nonaromatic heterocycle {wherein said 3-8 membered nonaromatic heterocycle is optionally substituted with one or more substituent(s) selected from Group C}, or a 5-10 membered aromatic heterocycle optionally substituted with one or more substituent(s) selected from Group D; and
the other symbols are the same as defined above.]

The reaction is usually carried out in a solvent. Examples of the solvent to be used in the reaction include hydrocarbons, ethers, halogenated hydrocarbons, amides, esters, nitriles, water, and mixtures of two or more of them.

Examples of the base to be used in the reaction include organic bases, alkali metal carbonates, alkali metal hydrogen carbonates, sodium hydride, and tripotassium phosphate.

In the reaction, the Compound (M5) is usually used at a ratio of 1 to 10 mol, and the base is usually used at a ratio of 1 to 10 mol, relative to 1 mol of the Compound (B4).

The reaction temperature is usually within the range of 0 to 150°C. The reaction time is usually within the range of 0.1 to 48 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-e).

The Compound (M5) is known or may be prepared according to known method(s).

### Production method F-1

A compound represented by formula (I-e-A) (hereinafter referred to as "Compound (I-e-A)") may be prepared by a step which comprises reacting the Compound (B3) with an oxidizing agent to give a compound represented by formula (B3-A) (hereinafter referred to as "Compound (B3-A)") (hereinafter referred to as "Step (1)"), and a step which comprises reacting the Compound (B3-A) with the Compound (M5) in the presence of a base (hereinafter referred to as "Step (2)"). 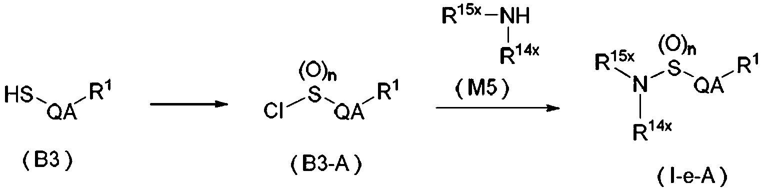 [wherein the symbols are the same as defined above.]

The Step (1) may be carried out according to, for example, the method described in WO 2016/204096 pamphlet, the method described in Tetrahedron Letters, 2017, 58, 2244-2247, the method described in Synthesis, 1987, 1, 72-73, the method described in Organic Letter, 2019 ,21, 1484-1487, the method described in Bioorganic & Medicinal Chemistry Letter, 2013, 23, 3723-3727, or the like.

The Step (2) may be carried out according to the Production method F by using the Compound (B3-A) instead of the Compound (B4).

### Production method G

A compound represented by formula (I-f) (hereinafter referred to as "Compound (I-f)") may be prepared by reacting a compound represented by formula (B5) (hereinafter referred to as "Compound (B5)") with a compound represented by formula (M6) (hereinafter referred to as "Compound (M6)") in the presence of a palladium catalyst and a base. 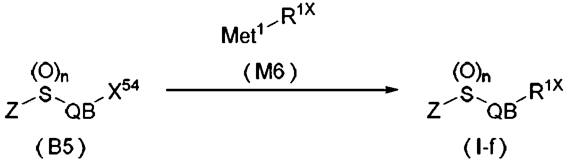 [wherein:
QB represents the group represented by Q1, the group represented by Q3, the group represented by Q4, the group represented by Q5, or the group represented by Q6;
X⁵⁴ represents a chlorine atom, a bromine atom, an iodine atom, or a trifuryloxy group;
Met¹ represents B(OH)₂ or a 4,4,5,5-tetramethyl-1,3,2-dioxaborolan-2-yl group;
R^{1X} represents a C3-C10 alicyclic hydrocarbon group, a 3-10 membered nonaromatic heterocyclic group {wherein said C3-C10 alicyclic hydrocarbon group and said 3-10 membered nonaromatic heterocyclic group are optionally substituted with one or more substituent(s) selected from Group A, and among atoms constituting said C3-C10 alicyclic hydrocarbon group and said 3-10 membered nonaromatic heterocyclic group, any atom which is not bound to Met¹ or QB optionally forms a C3-C7 alicyclic hydrocarbon or a 3-7 membered nonaromatic heterocycle as a spiro atom}, or a 5-7 membered aromatic heterocyclic group optionally substituted with one or more substituent(s) selected from Group B1 (provided that a 1H-pyrazol-3-yl group is excluded when QB represents the group represented by Q6) {wherein among atoms constituting said 5-7 membered aromatic heterocyclic group (provided that a 1H-pyrazol-3-yl group is excluded when QB represents the group represented by Q6), any two adjacent atoms which are not bound to Met¹ or QB optionally form another benzene ring, C4-C7 alicyclic hydrocarbon, 4-7 membered nonaromatic heterocycle, or 5-7 membered aromatic heterocycle comprising said two adjacent atoms} (provided that in R^{1X}, the atom which is bound to Met¹ or QB is a carbon atom); and
the other symbols are the same as defined above.]

The reaction is usually carried out in a solvent. Examples of the solvent to be used in the reaction include hydrocarbons, ethers, halogenated hydrocarbons, amides, esters, nitriles, DMSO, water, and mixtures of two or more of them.

Examples of the palladium catalyst to be used in the reaction include palladium(II) acetate and [1,1'-bis(diphenylphosphino)ferrocene]palladium(II) dichloride.

Examples of the base to be used in the reaction include organic bases, alkali metal carbonates, alkali metal hydrogen carbonates, sodium fluoride, and tripotassium phosphate.

In the reaction, the Compound (M6) is usually used at a ratio of 0.5 to 2 mol, the palladium catalyst is usually used at a ratio of 0.01 to 0.3 mol, and the base is usually used at a ratio of 1 to 10 mol, relative to 1 mol of the Compound (B5).

The reaction temperature is usually within the range of 0 to 150°C. The reaction time is usually within the range of 0.1 to 120 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (I-f).

The Compound (M6) is known or may be prepared according to known method(s).

### Production method H

The Compound (I-f) may also be prepared by reacting a compound represented by formula (B6) (hereinafter referred to as "Compound (B6)") with a compound represented by formula (M7) (hereinafter referred to as "Compound (M7)") in the presence of a palladium catalyst and a base. 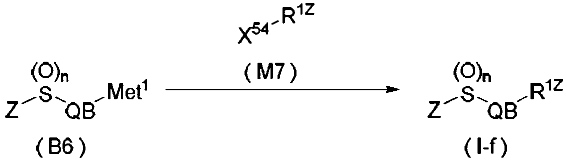 [wherein:
R^{1Z} represents a C3-C10 alicyclic hydrocarbon group, a 3-10 membered nonaromatic heterocyclic group {wherein said C3-C10 alicyclic hydrocarbon group and said 3-10 membered nonaromatic heterocyclic group are optionally substituted with one or more substituent(s) selected from Group A, and among atoms constituting said C3-C10 alicyclic hydrocarbon group and said 3-10 membered nonaromatic heterocyclic group, any atom which is not bound to X⁵⁴ or QB optionally forms a C3-C7 alicyclic hydrocarbon or a 3-7 membered nonaromatic heterocycle as a spiro atom}, or a 5-7 membered aromatic heterocyclic group optionally substituted with one or more substituent(s) selected from Group B1 (provided that a 1H-pyrazol-3-yl group is excluded when QB represents the group represented by Q6) {wherein among atoms constituting said 5-7 membered aromatic heterocyclic group, any two adjacent atoms which are not bound to X⁵⁴ or QB optionally form another benzene ring, C4-C7 alicyclic hydrocarbon, 4-7 membered nonaromatic heterocycle, or 5-7 membered aromatic heterocycle comprising said two adjacent atoms} (provided that in R^{1Z}, the atom which is bound to X⁵⁴ or QB is a carbon atom); and
the other symbols are the same as defined above.]

The reaction may be carried out according to the Production method G by using the Compound (M7) instead of the Compound (B5), and using the Compound (B6) instead of the Compound (M6).

The Compound (M7) is known or may be prepared according to known method(s).

### Production method I

A compound represented by formula (I-g) (hereinafter referred to as "Compound (I-g)") may be prepared by reacting a compound represented by formula (B7) (hereinafter referred to as "Compound (B7)") with a compound represented by formula (M8) (hereinafter referred to as "Compound (M8)") in the presence of a base. 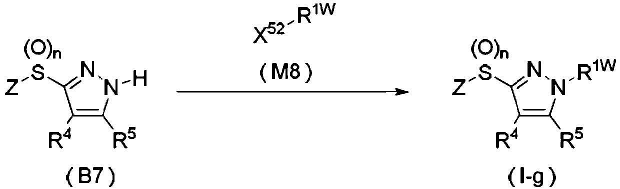 [wherein:
R^{1W} represents a C3-C10 alicyclic hydrocarbon group, a 3-10 membered nonaromatic heterocyclic group {wherein said C3-C10 alicyclic hydrocarbon group and said 3-10 membered nonaromatic heterocyclic group are optionally substituted with one or more substituent(s) selected from Group A, and among atoms constituting said C3-C10 alicyclic hydrocarbon group and said 3-10 membered nonaromatic heterocyclic group, any atom which is not bound to X⁵² or the pyrazole indicated in the Compound (I-g) optionally forms a C3-C7 alicyclic hydrocarbon or a 3-7 membered nonaromatic heterocycle as a spiro atom}, or a 5-7 membered aromatic heterocyclic group optionally substituted with one or more substituent(s) selected from Group B1 {wherein among atoms constituting said 5-7 membered aromatic heterocyclic group, any two adjacent atoms which are not bound to X⁵² or the pyrazole indicated in the Compound (I-g) optionally forms another benzene ring, C4-C7 alicyclic hydrocarbon, 4-7 membered nonaromatic heterocycle, or 5-7 membered aromatic heterocycle comprising said two adjacent atoms} (provided that in R^{1W}, the atom which is bound to X⁵² or the pyrazole indicated in the Compound (I-g) is a carbon atom); and
the other symbols are the same as defined above.]

The reaction may be carried out according to the Production method C by using the Compound (B7) instead of the Compound (M2), and using the Compound (M8) instead of the Compound (B2).

The Compound (M8) is known or may be prepared according to known method(s).

### Production method J

A compound represented by formula (I-h) (hereinafter referred to as "Compound (I-h)") may be prepared by reacting a compound represented by formula (B8) (hereinafter referred to as "Compound (B8)") with a compound represented by formula (M9) (hereinafter referred to as "Compound (M9)") in the presence of a base. 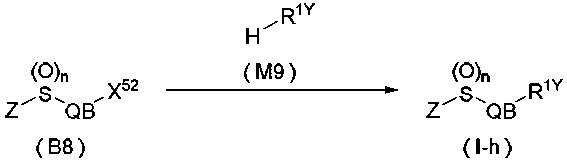 [wherein:
R^{1Y} represents a 3-10 membered nonaromatic heterocyclic group optionally substituted with one or more substituent(s) selected from Group A {wherein among atoms constituting said 3-10 membered nonaromatic heterocyclic group, any atom which is not bound to the hydrogen atom indicated in the Compound (M9) or QB optionally forms a C3-C7 alicyclic hydrocarbon or a 3-7 membered nonaromatic heterocycle as a spiro atom}, or a 5-7 membered aromatic heterocyclic group optionally substituted with one or more substituent(s) selected from Group B1 (provided that a 1H-pyrazol-3-yl group is excluded when QB represents the group represented by Q6) {wherein among atoms constituting said 5-7 membered aromatic heterocyclic group, any two adjacent atoms which are not bound to the hydrogen atom indicated in the Compound (M9) or QB optionally form another benzene ring, C4-C7 alicyclic hydrocarbon, 4-7 membered nonaromatic heterocycle, or 5-7 membered aromatic heterocycle comprising said two adjacent atoms} (provided that in R^{1Y}, the atom which is bound to the hydrogen atom indicated in the Compound (M9) or QB is a nitrogen atom); and
the other symbols are the same as defined above.]

The reaction may be carried out according to the Production method C by using the Compound (B8) instead of the Compound (B2), and using the Compound (M9) instead of the Compound (M2).

The Compound (M9) is known or may be prepared according to known method(s).

### Production method K

A compound represented by formula (I-i) (hereinafter referred to as "Compound (I-i)") may be prepared by reacting the Compound (B5) with a compound represented by formula (M10) (hereinafter referred to as "Compound (M10)") in the presence of a palladium catalyst and a base. 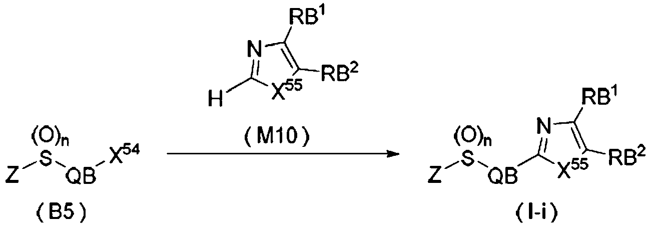 [wherein:
X⁵⁵ represents an oxygen atom or a sulfur atom;
RB¹ and RB² are identical to or different from each other, and each represent one substituent selected from Group B1, or a hydrogen atom; or
RB¹ and RB² are optionally combined with the carbon atoms to which they are attached to form a benzene ring, a C4-C7 alicyclic hydrocarbon, a 4-7 membered nonaromatic heterocycle, or a 5-7 membered aromatic heterocycle; and
the other symbols are the same as defined above.]

The reaction may be carried out according to the Production method G by using the Compound (M10) instead of the Compound (M6).

The Compound (M10) is known or may be prepared according to known method(s).

### Production method L

A compound represented by formula (I-j) (hereinafter referred to as "Compound (I-j)") may be prepared by reacting the Compound (B5) with a compound represented by formula (M11) (hereinafter referred to as "Compound (M11)") in the presence of a metal catalyst and a base. 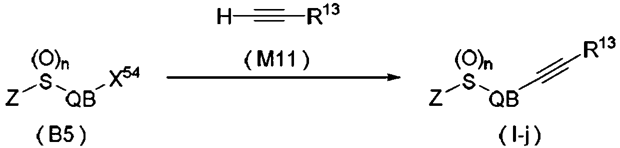 [wherein the symbols are the same as defined above.]

The reaction is usually carried out in a solvent. Examples of the solvent to be used in the reaction include hydrocarbons, ethers, halogenated hydrocarbons, amides, esters, nitriles, and mixtures of two or more of them.

Examples of the metal catalyst to be used in the reaction include bis(triphenylphosphine)palladium(II) dichloride.

In the reaction, a metal catalyst such as copper(I) iodide may be additionally used as needed.

Examples of the base to be used in the reaction include organic bases.

In the reaction, the Compound (M11) is usually used at a ratio of 1 to 10 mol, the metal catalyst is usually used at a ratio of 0.01 to 1 mol, and the base is usually used at a ratio of 1 to 10 mol, relative to 1 mol of the Compound (B5).

The reaction temperature is usually within the range of 0 to 150°C. The reaction time is usually within the range of 0.1 to 120 hour(s).

When the reaction is completed, the reaction mixture may be subjected to a work-up such as concentrating and drying it to give the Compound (I-j).

The Compound (M11) is known or may be prepared according to known method(s).

### Production method M

A compound represented by formula (B7-2) (hereinafter referred to as "Compound (B7-2)") may be prepared by a step which comprises reacting the Compound (B7) with hydroxylamine-O-sulfonic acid to give a compound represented by formula (B7-1) (hereinafter referred to as "Compound (B7-1)") (hereinafter referred to as "Step (B7-1)"), and a step which comprises reacting the Compound (B7-1) with the Compound (B7-3) in the presence of a base (hereinafter referred to as "Step (B7-2)"). 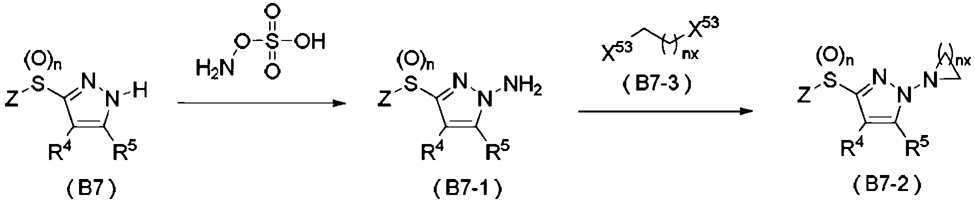 [wherein:
nx represent an integer of 1 to 8; and
the other symbols are the same as defined above.]

The Step (B7-1) may be carried out according to, for example, the method described in Journal of Organic Chemistry, 2021, 86(19), 13465-13474, the method described in Organic & Biomolecular Chemistry, 2003, 1(23), 4268-4274, the method described in Organic Letters, 2018, 20(9), 2628-2631, or the like.

The Step (B7-2) is usually carried out in a solvent. Examples of the solvent to be used in the reaction include hydrocarbons, ethers, halogenated hydrocarbons, amides, esters, nitriles, water, and mixtures of two or more of them.

Examples of the base to be used in the reaction include organic bases, alkali metal carbonates, alkali metal hydrogen carbonates, sodium hydride, and tripotassium phosphate.

In the reaction, the Compound (B7-3) is usually used at a ratio of 1 to 10 mol, and the base is usually used at a ratio of 1 to 10 mol, relative to 1 mol of the Compound (B7-1).

The reaction temperature is usually within the range of 0 to 150°C. The reaction time is usually within the range of 0.1 to 48 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to give the Compound (B7-2).

The Compound (B7-3) is a commercially available compound or may be prepared according to known method(s).

### Production method N

A compound represented by formula (B7-4) (hereinafter referred to as "Compound (B7-4)") may be prepared by reacting the Compound (B7) with a compound represented by formula (B7-5) (hereinafter referred to as "Compound (B7-5)") in the presence of a metal catalyst and a base. 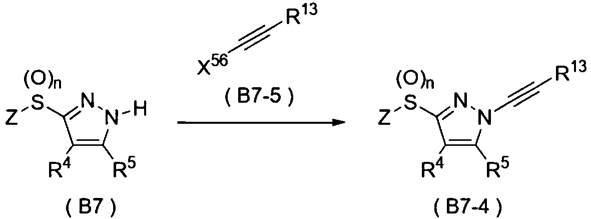 [wherein:
X⁵⁶ represents a chlorine atom, a bromine atom, or an iodine atom; and
the other symbols are the same as defined above.]

The reaction may be carried out according to, for example, the method described in Synthesis, 2011, 5, 816-820, the method described in Journal of Organic Chemistry, 2010, 75, 3, 980-983, or the like.

### Reference Production method A

A compound represented by formula (B1-a) (hereinafter referred to as "Compound (B1-a)") may be prepared by reacting a compound represented by formula (C1) (hereinafter referred to as "Compound (C1)") with hydrazine. 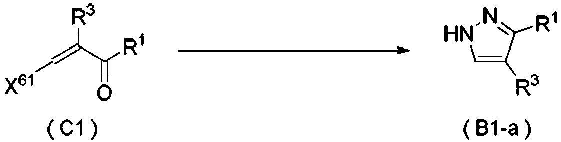 [wherein:
X⁶¹ represents a hydroxy group, a dimethylamino group, or a diethylamino group; and
the other symbols are the same as defined above.]

The reaction may be carried out according to, for example, the method described in Bioorganic and Medicinal Chemistry, 2014, 22, 4189. or J. Med. Chem., 2003, 46, 5416.

### Reference Production method B

A compound represented by formula (C1-a) (hereinafter referred to as "Compound (C1-a)") may be prepared by reacting a compound represented by formula (C2) (hereinafter referred to as "Compound (C2)") with a compound represented by formula (M21) (hereinafter referred to as "Compound (M21)"). 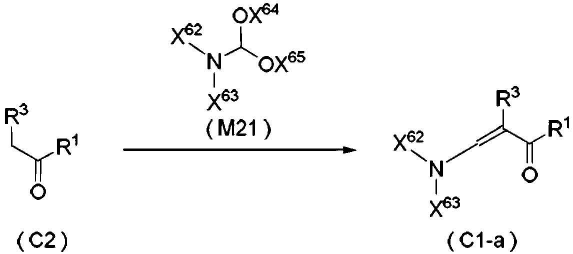 [wherein:
X⁶² and X⁶³ are identical to or different from each other, and each represent a methyl group or an ethyl group;
X⁶⁴ and X⁶⁵ are identical to or different from each other, and each represent a C1-C4 alkyl group or a benzyl group; and
the other symbols are the same as defined above.]

The reaction may be carried out according to, for example, the method described in European Journal of Medicinal Chemistry, 2011, 46, 5817.

The Compound (C2) and the Compound (M21) are commercially available compounds or may be prepared by using known method(s).

### Reference Production method C

A compound represented by formula (C1-b) (hereinafter referred to as "Compound (C1-b)") may be prepared by reacting the Compound (C2) with a compound represented by formula (M22) (hereinafter referred to as "Compound (M22)") in the presence of a base. 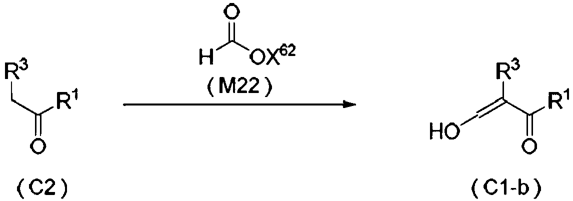 [wherein the symbols are the same as defined above.]

The reaction may be carried out according to, for example, the method described in J. Am. Chem. Soc., 2017, 139, 2421.

The Compound (M22) is a commercially available compound or may be prepared by using known method(s).

### Reference Production method D

A compound represented by formula (B2-a) (hereinafter referred to as "Compound (B2-a)") may be prepared by reacting a compound represented by formula (C3) (hereinafter referred to as "Compound (C3)") with a compound represented by formula (M23) (hereinafter referred to as "Compound (M23)") in the presence of a palladium catalyst and a base. 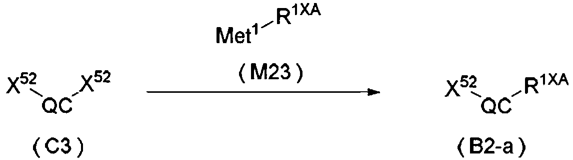 [wherein:
QC represents the group represented by Q3, the group represented by Q4, the group represented by Q5, or the group represented by Q6;
R^{1XA} represents a C3-C10 alicyclic hydrocarbon group, a 3-10 membered nonaromatic heterocyclic group {wherein said C3-C10 alicyclic hydrocarbon group and said 3-10 membered nonaromatic heterocyclic group are optionally substituted with one or more substituent(s) selected from Group A, and among atoms constituting said C3-C10 alicyclic hydrocarbon group and said 3-10 membered nonaromatic heterocyclic group, any atom which is not bound to Met¹ or QC optionally forms a C3-C7 alicyclic hydrocarbon or a 3-7 membered nonaromatic heterocycle as a spiro atom}, or a 5-7 membered aromatic heterocyclic group optionally substituted with one or more substituent(s) selected from Group B1 (provided that a 1H-pyrazol-3-yl group is excluded when QC represents the group represented by Q6) {wherein among atoms constituting said 5-7 membered aromatic heterocyclic group, any two adjacent atoms which are not bound to Met¹ or QC optionally form another benzene ring, C4-C7 alicyclic hydrocarbon, 4-7 membered nonaromatic heterocycle, or 5-7 membered aromatic heterocycle comprising said two adjacent atoms} (provided that in R^{1XA}, the atom which is bound to Met¹ or QC is a carbon atom); and
the other symbols are the same as defined above.]

The reaction may be carried out according to the Production method G by using the Compound (C3) instead of the Compound (B5), and using the Compound (M23) instead of the Compound (M6).

The Compound (C3) and the Compound (M23) are known or may be prepared according to known method(s).

### Reference Production method E

A compound represented by formula (B2-b) (hereinafter referred to as "Compound (B2-b)") may be prepared by reacting a compound represented by formula (C4) (hereinafter referred to as "Compound (C4)") with a compound represented by formula (M24) (hereinafter referred to as "Compound (M24)") in the presence of a palladium catalyst and a base. 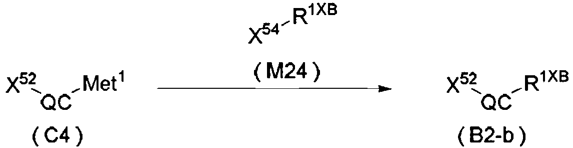 [wherein:
R^{1XB} represents a C3-C10 alicyclic hydrocarbon group, a 3-10 membered nonaromatic heterocyclic group {wherein said C3-C10 alicyclic hydrocarbon group and said 3-10 membered nonaromatic heterocyclic group are optionally substituted with one or more substituent(s) selected from Group A, and among atoms constituting said C3-C10 alicyclic hydrocarbon group and said 3-10 membered nonaromatic heterocyclic group, any atom which is not bound to X⁵⁴ or QC optionally forms a C3-C7 alicyclic hydrocarbon or a 3-7 membered nonaromatic heterocycle as a spiro atom}, or a 5-7 membered aromatic heterocyclic group optionally substituted with one or more substituent(s) selected from Group B1 (provided that a 1H-pyrazol-3-yl group is excluded when QC represents the group represented by Q6) {wherein among atoms constituting said 5-7 membered aromatic heterocyclic group, any two adjacent atoms which are not bound to X⁵⁴ or QC optionally form another benzene ring, C4-C7 alicyclic hydrocarbon, 4-7 membered nonaromatic heterocycle, or 5-7 membered aromatic heterocycle comprising said two adjacent atoms} (provided that in R^{1XB}, the atom which is bound to X⁵⁴ or QC is a carbon atom); and
the other symbols are the same as defined above.]

The reaction may be carried out according to the Production method G by using the Compound (M24) instead of the Compound (B5), and using the Compound (C4) instead of the Compound (M6).

The Compound (C4) and the Compound (M24) are known or may be prepared according to known method(s).

### Reference Production method E-1

A compound represented by formula (B2-c) (hereinafter referred to as "Compound (B2-c)") may be prepared by reacting a compound represented by formula (C3-A) (hereinafter referred to as "Compound (C3-A)") with the Compound (M8) in the presence of a base. 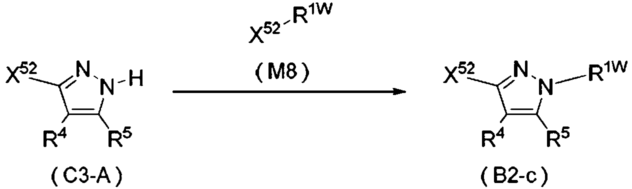 [wherein the symbols are the same as defined above.]

The reaction may be carried out according to the Production method C by using the Compound (C3-A) instead of the Compound (M2), and using the Compound (M8) instead of the Compound (B2).

### Reference Production method F

The Compound (B3) may be prepared by reacting the Compound (B2) with a sulfating agent in the presence of a base. 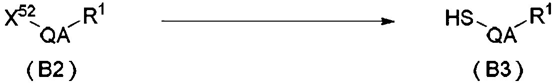 [wherein the symbols are the same as defined above.]

The reaction may be carried out according to the Production method C by using a sulfating agent such as hydrogen sulfide instead of the Compound (M2).

### Reference Production method G

The Compound (B4) may be prepared from a compound represented by formula (C5) (hereinafter referred to as "Compound (C5)"). 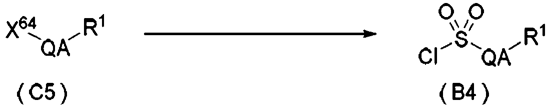 [wherein:
X⁶⁴ represents SH or S(O)₂OH; and
the other symbols are the same as defined above.]

The reaction may be carried out according to, for example, the method described in WO 2016/204096 pamphlet, the method described in Tetrahedron Letters, 2017, 58, 2244-2247, or the like.

The Compound (C5) is known or may be prepared according to known method(s).

### Reference Production method H

A compound represented by formula (B8-a) (hereinafter referred to as "Compound (B8-a)") may be prepared by reacting the Compound (C3) with the Compound (M2) in the presence of a base. 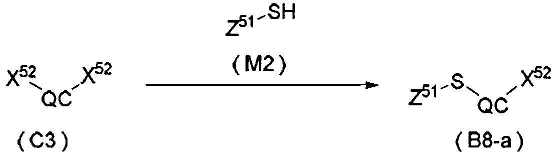 [wherein the symbols are the same as defined above.]

The reaction may be carried out according to the Production method C by using the Compound (C3) instead of the Compound (B2).

### Reference Production method I

A compound represented by formula (B8-b) (hereinafter referred to as "Compound (B8-b)") or a compound represented by formula (B8-c) (hereinafter referred to as "Compound (B8-c)") may be prepared by reacting the Compound (B8-a) with an oxidizing agent. 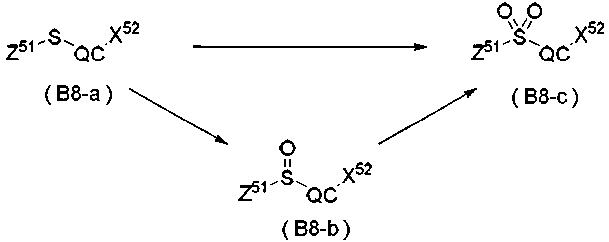 [wherein the symbols are the same as defined above.]

The reaction may be carried out according to the Production method A by using the Compound (B8-a) instead of the Compound (I-a), and using the Compound (B8-b) instead of the Compound (I-b).

### Reference Production method J

A compound represented by formula (B6-a) (hereinafter referred to as "Compound (B6-a)") may be prepared by reacting the Compound (B5) with bis(pinacolato)diboron in the presence of a base and a palladium catalyst. 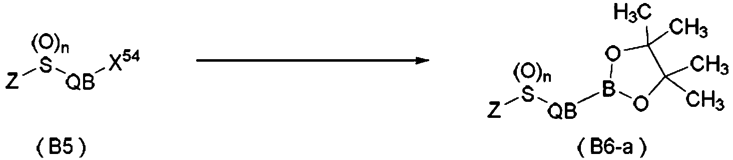 [wherein the symbols are the same as defined above.]

The reaction may be carried out according to the Production method G by using bis(pinacolato)diboron instead of the Compound (M6).

### Reference Production method K

A compound represented by formula (B7-a) (hereinafter referred to as "Compound (B7-a)") may be prepared by carrying out a first step which comprise reacting a compound represented by formula (C6) (hereinafter referred to as "Compound (C6)") with butyllithium or isopropylmagnesium chloride, then reacting the resulting mixture with a disulfide or a thiosulfonic acid ester to give a compound represented by formula (C7) (hereinafter referred to as "Compound (C7)"), and a second step which comprises reacting the Compound (C7) with an acid such as hydrochloric acid and trifluoroacetic acid or a base such as potassium hydroxide and sodium methoxide. 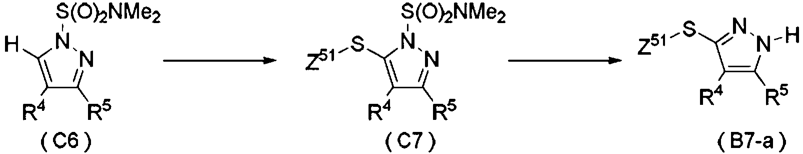 [wherein the symbols are the same as defined above.]

These reactions may be carried out according to the method described in WO 2008/129054 pamphlet or the method described in J. Med. Chem., 2016, 59, 2760.

The Compound (C6) is a commercially available compound or may be prepared according to the method described in J. Med. Chem., 2016, 59, 2760.

### Reference Production method M

A compound represented by formula (B7-c) (hereinafter referred to as "Compound (B7-c)") or a compound represented by formula (B7-b) (hereinafter referred to as "Compound (B7-b)") may be prepared by reacting the Compound (B7-a) with an oxidizing agent. 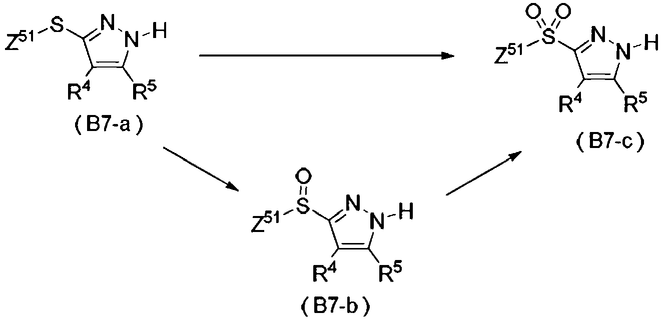 [wherein the symbols are the same as defined above.]

The reaction may be carried out according to the Production method A by using the Compound (B7-a) instead of the Compound (I-a), and using the Compound (B7-b) instead of the Compound (I-b).

### Reference Production method N

A compound represented by formula (B8-A) (hereinafter referred to as "Compound (B8-A)") may be prepared by reacting a compound represented by formula (B1-A) (hereinafter referred to as "Compound (B1-A)") with a compound represented by formula (M1-A2) (hereinafter referred to as "Compound (M1-A2)") in the presence of a base. 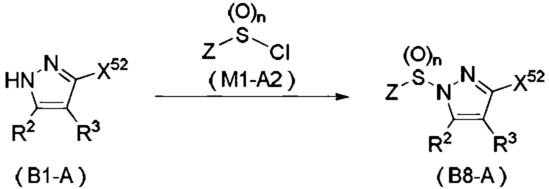 [wherein the symbols are the same as defined above.]

The reaction may be carried out according to the Production method B by using the Compound (M1-A2) instead of the Compound (M1).

The Compound (M1-A2) is a commercially available compound or may be prepared according to the production method of the Compound (M1-A).

The Compound (B1-A) is a commercially available compound or may be prepared by using known method(s).

The Compound of the present invention or the Compound W of the present invention may be mixed with or used in combination with one or more ingredient(s) selected from the group consisting of the following Group (a), Group (b), Group (c), and Group (d) (hereinafter referred to as "Present ingredient").

When the Compound of the present invention or the Compound W of the present invention is mixed with or used in combination with the Present ingredient, they are used simultaneously, separately, or at time intervals with each other.

When the Compound of the present invention or the Compound W of the present invention is used simultaneously with the Present ingredient, the Compound of the present invention or the Compound W of the present invention and the Present ingredient may be contained in separate formulations or contained in one formulation.

One aspect of the present invention provides a composition comprising one or more ingredient(s) selected from the group consisting of Group (a), Group (b), Group (c), and Group (d) (i.e., Present ingredient), and the Compound of the present invention.

One aspect of the present invention provides a composition comprising one or more ingredient(s) selected from the group consisting of Group (a), Group (b), Group (c), and Group (d) (i.e., Present ingredient), and the Compound W of the present invention (hereinafter referred to as "Composition A").

Group (a) is a group consisting of acetylcholinesterase inhibitors (for example, carbamate insecticides and organophosphate insecticides), GABA-gated chloride channel blockers (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mite growth inhibitors, microbial disruptors of insect midgut membranes, inhibitors of mitochondrial ATP synthase, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, mitochondrial complexes I, II, III, and IV electron transport inhibitors, voltage-dependent sodium channel blockers, inhibitors of acetyl CoA carboxylase, ryanodine receptor modulators (for example, diamide insecticides), chordotonal organ modulators, and microbial insecticides, and other insecticidal active ingredients, miticidal active ingredients, and nematicidal active ingredients. These ingredients are described in the classification on the basis of action mechanism by IRAC.

Group (b) is a group consisting of nucleic acids synthesis inhibitors (for example, phenylamide fungicides and acylamino acid fungicides), cell division and cytoskeleton inhibitors (for example, MBC fungicides), respiration inhibitors (for example, QoI fungicides and QiI fungicides), amino acids synthesis and protein synthesis inhibitors (for example, anilino-pyrimidine fungicides), signal transduction inhibitors, lipid synthesis and membrane synthesis inhibitors, sterol biosynthesis inhibitors (for example, DMI fungicides such as triazole fungicides), cell wall biosynthesis inhibitors, melanin synthesis inhibitors, plant defense inducers, fungicides with multi-site contact activity, microbial fungicides, and other fungicidal active ingredients. These ingredients are described in the classification on the basis of action mechanism by FRAC.

Group (c) is a group of plant growth regulatory ingredients (including mycorrhizal fungi and root nodule bacteria).

Group (d) is a group of repellent ingredients.

Hereinafter, examples of the combination of the Present ingredient and the Compound W of the present invention are described. For example, "alanycarb + SX" indicates a combination of alanycarb and SX.

The abbreviation of "SX" indicates any one of the Compound W of the present invention selected from the Compound groups SX1 to SX2159. Also, all of the following Present ingredient are known ingredients, and may be obtained from commercially available formulations, or may be prepared by known methods. When the Present ingredient is a microorganism, it may also be available from a bacterial authority depository. Further, the number in parentheses represents the CAS RN (registered trademark).

Combinations of the Present ingredient in the above Group (a) and the Compound W of the present invention:
abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, bentioflumin + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, bisulflufen + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cybenzoxasulfyl + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC(2-methyl-4,6-dinitrophenol) + SX, doramectin + SX, dried leaves of Dryopteris filix-mas + SX, emamectin-benzoate + SX, empenthrin + SX, endosulfan + SX, EPN(O-ethyl O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, flupyroxystrobin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hvla peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoflualanam + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, ledprona + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pioxaniliprole + SX, piperflanilide + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfiflumin + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tiapyrachlor + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vadescana + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, N-[4-chloro-2-(pyridin-3-yl)-1,3-thiazol-5-yl]-N-ethyl-3-(methanesulfonyl)propanamide + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, Bacillus thuringiensis Cry1Ab protein + SX, Bacillus thuringiensis CrylAc protein + SX, Bacillus thuringiensis CrylFa protein + SX, Bacillus thuringiensis Cry1A.105 protein + SX, Bacillus thuringiensis Cry2Ab protein + SX, Bacillus thuringiensis Vip3A protein + SX, Bacillus thuringiensis mCry3A protein + SX, Bacillus thuringiensis Cry3Ab protein + SX, Bacillus thuringiensis Cry3Bb protein + SX, Bacillus thuringiensis Cry34Ab1/Cry35Ab1 protein + SX, Adoxophyes orana GV(granulovirus) strain BV-0001 + SX, Anticarsia gemmatalis MNPV(multiple nucleocapsid nucleopolyhedrovirus) + SX, Autographa californica MNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV(nucleopolyhedrovirus) strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua MNPV + SX, Spodoptera littoralis MNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306 + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

Combination of the Present ingredient in the above Group (b) and the Compound W of the present invention:
acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Allium sativum + SX, extract of the cotyledons of lupine plantlets ("BLAD") + SX, extract of Equisetum arvense + SX, extract of Melaleuca alternifolia + SX, extract of Reynoutria sachalinensis + SX, extract of Tropaeolum majus + SX, famoxadone + SX, fenamidone + SX, fenaminstrobin + SX, fenarimol + SX, fenbuconazole + SX, feneptamidoquin + SX, fenfuram + SX, fenhexamid + SX, fenopyramid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, galquin + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl) (2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)amino]-N-(2,2-dimethylcyclobutyl)-5-methylthiazole-4-carboxamide + SX, 2-[(2,6-difluoropyridin-4-yl)amino]-N-(2,2-dimethylcyclobutyl)-5-methylthiazole-4-carboxamide + SX, 2-{[(oxetan-3-yl)carbonyl](2,6-difluoropyridin-4-yl)amino}-N-(2,2-dimethylcyclobutyl)-5-methylthiazole-4-carboxamide + SX, 2-{[(oxolan-3-yl)carbonyl](2,6-difluoropyridin-4-yl)amino}-N-(2,2-dimethylcyclobutyl)-5-methylthiazole-4-carboxamide + SX, 2-{[(oxan-4-yl)carbonyl](2,6-difluoropyridin-4-yl)amino}-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (SS)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2-methylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2,2-dimethylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(5-fluoro-2-methoxyphenyl)-2-(2-cyanoethoxy)ethyl]-5-[1-(isopropoxyimino)ethyl]-2,6-dioxo-3,6-dihydropyrimidin-1(2H)-yl}propan-2-yl)-2-methylpropanamide + SX, methyl ({5-[1-(2,6-difluoro-4-isopropylphenyl)-1H-pyrazol-3-yl]-2-methylphenyl}methyl)carbamate + SX, methyl ({5-[1-(2,6-difluoro-4-cyclopropylphenyl)-1H-pyrazol-3-yl]-2-methylphenyl}methyl)carbamate + SX, methyl ({5-[1-(2,6-difluoro-4-methoxyphenyl)-1H-pyrazol-3-yl]-2-methylphenyl}methyl)carbamate + SX, methyl (Z)-2-(5-cyclopentyl-2-methylphenoxy)-3-methoxyprop-2-enoate + SX, methyl (Z)-2-(5-cyclohexyl-2-methylphenoxy)-3-methoxyprop-2-enoate + SX, methyl (Z)-2-[(3-isopropyl-1H-pyrazol-1-yl)-2-methylphenoxy]-3-methoxyprop-2-enoate + SX, 1-(4,5-dimethyl-1H-benzimidazol-1-yl)-4,4-difluoro-3,3-dimethyl-3,4-dihydroisoquinoline + SX, 1-(4,5-dimethyl-1H-benzimidazol-1-yl)-4,4,5-trifluoro-3,3-dimethyl-3,4-dihydroisoquinoline + SX, 1-(pyrazolo[1,5-a]pyridin-3-yl)-4,4-difluoro-3,3-dimethyl-3,4-dihydroisoquinoline + SX, 1-(6,7-dimethylpyrazolo[1,5-a]pyridin-3-yl)-6-fluoro-3,3-dimethylisoquinolin-4(3H)-one + SX, methyl (2Z)-3-methoxy-2-[(4-methyl[1,1'-biphenyl]-3-yl)oxy]prop-2-enoate + SX, Agrobacterium radiobactor strain K1026 + SX, Agrobacterium radiobactor strain K84 + SX, Bacillus amyloliquefaciens strain PTA-4838 (Aveo (registered trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

Combination of the Present ingredient in the above Group (c) and the Compound W of the present invention:
1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butylate + SX, methyl 5-(trifluoromethyl)benzo[b]thiophene-2-carboxylate + SX, 3-[(6-chloro-4-phenylquinazolin-2-yl)amino]propan-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combination of the Present ingredient in the above Group (d) and the Compound W of the present invention:
anthraquinone + SX, deet + SX, icaridin + SX.

The ratio of the Compound W of the present invention to the Present ingredient includes, but not limited thereto, as a ratio by weight (the Compound W of the present invention : the Present ingredient) 1,000 : 1 to 1 : 1,000, 500 : 1 to 1 : 500, 100 : 1 to 1 : 100, 50 : 1, 20 : 1, 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9, 1 : 10, 1 : 20, 1 : 50, and the others.

The Present compound W, the Compound W of the present invention, or the Composition A can control plant diseases caused by plant pathogenic microorganisms such as fungi, Oomycete, Phytomyxea, and bacteria. Examples of the fungi include Ascomycota, Basidiomycota, Blastocladiomycota, Chytridiomycota, Mucoromycota, and Olpidiomycota. Specific examples thereof include the followings. The scientific name of plant pathogenic microorganism which causes each disease is shown in parentheses.
Rice diseases:
   blast (Pyricularia oryzae), brown spot (Cochliobolus miyabeanus), sheath blight (Rhizoctonia solani), bakanae disease (Gibberella fujikuroi), downy mildew (Sclerophthora macrospora), false blast and head blight (Epicoccum nigrum), seedling blight (Trichoderma viride and Rhizopus oryzae), pseudo sheath blight (Waitea circinate, Ceratobasidium setariae, and Thanatephorus cucumeris), Rice false smut (Ustilaginoidea virens), and Kernel smut (Tilletia horrida and Tilletia barclayana);
Wheat diseases:
   powdery mildew (Blumeria graminis), fusarium blight (Fusarium graminearum, Fusarium avenaceum, Fusarium culmorum, and Microdochium nivale), stripe rust (Puccinia striiformis), stem rust (Puccinia graminis), leaf rust (Puccinia recondita), snow mould (Microdochium nivale and Microdochium majus), typhula snow blight (Typhula incarnata and Typhula ishikariensis), Sclerotinia snow blight (Sclerotinia borealis), Browning root rot (Pythium spp.), loose smut (Ustilago tritici), stinking smut (Tilletia caries and Tilletia controversa), eyespot (Pseudocercosporella herpotrichoides), leaf blotch (Septoria tritici), glume blotch (Stagonospora nodorum), tan spot (Pyrenophora tritici-repentis), rhizoctonia seeding blight (Rhizoctonia solani), take-all disease (Gaeumannomyces graminis), and blast (Pyricularia graminis-tritici);
Barley diseases:
   powdery mildew (Blumeria graminis), fusarium head blight (Fusarium graminearum, Fusarium avenaceum, Fusarium culmorum, and Microdochium nivale), stripe rust (Puccinia striiformis), stem rust (Puccinia graminis), dwarf leaf rust (Puccinia hordei), loose smut (Ustilago nuda), scald (Rhynchosporium secalis), net blotch (Pyrenophora teres), spot blotch (Cochliobolus sativus), stripe (Pyrenophora graminea), Ramularia disease (Ramularia collo-cygni), and rhizoctonia seeding blight (Rhizoctonia solani);
Corn diseases:
   rust (Puccinia sorghi), southern rust (Puccinia polysora), northern leaf blight (Setosphaeria turcica), tropical rust (Physopella zeae), southern leaf blight (Cochliobolus heterostrophus), anthracnose (Colletotrichum graminicola), gray leaf spot (Cercospora zeae-maydis), eyespot (Kabatiella zeae), phaeosphaeria leaf spot (Phaeosphaeria maydis), diplodia rot (Stenocarpella maydis and Stenocarpella macrospora), stalk rot (Fusarium graminearum, Fusarium verticilioides, and Colletotrichum graminicola), smut (Ustilago maydis), Physoderma brown spot and Physoderma stalk rot (Physoderma maydis), and tar spot disease (Phyllachora maydis);
Cotton diseases:
   anthracnose (Colletotrichum gossypii), grey mildew (Ramularia areola), alternaria leaf spot (Alternaria macrospora and Alternaria gossypii), and black root rot (Thielaviopsis basicola);
Coffee diseases:
   rust (Hemileia vastatrix) and leaf spot (Cercospora coffeicola);
Rape seed diseases:
   sclerotinia rot (Sclerotinia sclerotiorum), gray leaf spot (Alternaria brassicae), root rot (Phoma lingam), and light leaf spot (Pyrenopeziza brassicae);
Sugar cane diseases:
   rust (Puccinia melanocephela and Puccinia kuehnii), and smut (Ustilago scitaminea);
Sunflower diseases:
   rust (Puccinia helianthi) and downy mildew (Plasmopara halstedii);
Citrus diseases:
   melanose (Diaporthe citri), scab (Elsinoe fawcetti), green mold (Penicillium digitatum), blue mold (Penicillium italicum), Phytophthora rot (Phytophthora parasitica and Phytophthora citrophthora), aspergillus rot (Aspergillus niger), sour rot (Geotrichum candidum var. citri-aurantii), and sooty spot (Cladosporium cladosporioides);
Apple diseases:
   blossom blight (Monilinia mali), valsa canker (Valsa ceratosperma), powdery mildew (Podosphaera leucotricha), alternaria leaf spot (Alternaria alternata apple pathotype), scab (Venturia inaequalis), bitter rot (Glomerella cingulata and Colletotrichum acutatum), blotch (Diplocarpon mali), ring rot (Botryosphaeria berengeriana), crown rot (Phytophtora cactorum), and rust (Gymnosporangium juniperi-virginianae and Gymnosporangium yamadae);
Pear diseases:
   scab (Venturia nashicola and Venturia pirina), black spot (Alternaria alternata Japanese pear pathotype), and rust (Gymnosporangium haraeanum);
Peach diseases:
   brown rot (Monilinia fructicola), scab (Cladosporium carpophilum), Phomopsis rot (Phomopsis sp.), leaf curl (Taphrina deformans), and powdery mildew (Podosphaera leucotricha) ;
Grapes diseases:
   anthracnose (Elsinoe ampelina), ripe rot (Glomerella cingulata and Colletotrichum acutatum), powdery mildew (Uncinula necator), rust (Neophysopella sp.), black rot (Guignardia bidwellii), downy mildew (Plasmopara viticola), leaf blight (Pseudocercospora vitis), and white rot (Coniella castaneicola);
Japanese persimmon diseases: anthracnose (Gloeosporium kaki and Colletotrichum acutatum),
   leaf spot (Cercospora kaki and Mycosphaerella nawae), and powdery mildew (Phyllactinia kakicola);
Fig disease:
   rust (Phakopsora nishidana);
Diseases of gourd family:
   anthracnose (Colletotrichum lagenarium), powdery mildew (Sphaerotheca fuliginea), gummy stem blight (Didymella bryoniae), Corynespora leaf spot (Corynespora cassiicola), fusarium wilt (Fusarium oxysporum), downy mildew (Pseudoperonospora cubensis), phytophthora rot (Phytophthora capsici), damping-off (Pythium sp.), and Black root rot (Phomopsis sp.);
Tomato diseases:
   early blight (Alternaria solani), leaf mold (Cladosporium fulvum), Cercospora leaf mold (Pseudocercospora fuligena), late blight (Phytophthora infestans), and powdery mildew (Leveillula taurica);
Eggplant diseases:
   brown spot (Phomopsis vexans), powdery mildew (Erysiphe cichoracearum), black blight (Corynespora melongenae), leaf mold (Mycovellosiella nattrassii), and brown leaf spot (Thanatephorus cucumeris);
Cruciferous vegetables diseases:
   alternaria leaf spot (Alternaria japonica), white spot (Cercosporella brassicae), clubroot (Plasmodiophora brassicae), downy mildew (Peronospora parasitica), white rust (Albugo candida), and Alternaria sooty spot (Alternaria brassicicola);
Welsh onion diseases:
   rust (Puccinia allii), black spot (Alternaria porri), white rot (Sclerotium cepivorum), leaf blight (Botrytis squamosa), leaf spot (Stemphylium vesicarium and Stemphylium botryosum), southern blight (Sclerotium rolfsii), leaf blight (Botrytis squamosa);
Soybean diseases:
   purple stain (Cercospora kikuchii), sphaceloma scab (Elsinoe glycines), pod and stem blight (Diaporthe phaseolorum var. sojae), rust (Phakopsora pachyrhizi), target spot (Corynespora cassiicola), anthracnose (Colletotrichum glycines and Colletotrichum truncatum), Rhizoctonia rot (Rhizoctonia solani), septoria brown spot (Septoria glycines), Cercospora leaf spot (Cercospora sojina), stem rot (Sclerotinia sclerotiorum), powdery mildew (Microsphaera diffusa), phytophthora stem and root rot (Phytophthora sojae), downy mildew (Peronospora manshurica), sudden death syndrome (Fusarium virguliforme), red crown rot (Calonectria ilicicola), and Diaporthe/Phomopsis complex (Diaporthe longicolla, Diaporthe phaseolorum, Phomopsis longicolla), and pod anomalies caused by various causative bacteria;
Kidney bean diseases:
   stem rot (Sclerotinia sclerotiorum), rust (Uromyces appendiculatus), angular leaf spot (Phaeoisariopsis griseola), anthracnose (Colletotrichum lindemuthianum), and Fusarium root-rot (Fusarium solani);
Peanut diseases:
   leaf spot (Cercospora personata), brown leaf spot (Cercospora arachidicola), southern blight (Sclerotium rolfsii), and Cylindrocladium black rot (Calonectria ilicicola);
Garden pea diseases:
   powdery mildew (Erysiphe pisi) and root rot (Fusarium solani);
Potato diseases:
   early blight (Alternaria solani), late blight (Phytophthora infestans), Pink rot (Phytophthora erythroseptica), powdery scab (Spongospora subterranea f. sp. subterranea), verticillium wilt (Verticillium albo-atrum, Verticillium dahliae, and Verticillium nigrescens), dry rot (Fusarium solani), and potato wart (Synchytrium endobioticum);
Strawberry diseases:
   powdery mildew (Sphaerotheca humuli) and crown rot (Glomerella cingulata and Colletotrichum acutatum);
Tea diseases:
   net blister blight (Exobasidium reticulatum), white scab (Elsinoe leucospila), gray blight (Pestalotiopsis sp.), and anthracnose (Colletotrichum theae-sinensis);
Tobacco diseases:
   brown spot (Alternaria longipes), anthracnose (Colletotrichum tabacum), blue mold (Peronospora tabacina), and black shank (Phytophthora nicotianae);
Sugar beet diseases:
   cercospora leaf spot (Cercospora beticola), leaf blight (Thanatephorus cucumeris), root rot (Thanatephorus cucumeris), aphanomyces root rot (Aphanomyces cochlioides), and rust (Uromyces betae);
Rose diseases:
   black spot (Diplocarpon rosae) and powdery mildew (Sphaerotheca pannosa);
Chrysanthemum diseases:
   leaf blight (Septoria chrysanthemi-indici) and white rust (Puccinia horiana);
Onion diseases:
   botrytis leaf blight (Botrytis cinerea, Botrytis byssoidea, and Botrytis squamosa), gray-mold neck rot (Botrytis allii), and small sclerotial neck rot (Botrytis squamosa);
Various crops diseases:
   Botrytis rot (Botrytis cinerea), sclerotinia rot (Sclerotinia sclerotiorum), white rot (Sclerotium cepivorum), southern blight (Sclerotium rolfsii), and seedling blight (Pythium aphanidermatum, Pythium irregulare, and Pythium ultimum);
Japanese radish disease:
   alternaria leaf spot (Alternaria brassicicola);
Sweet potato diseases:
   stem rot (Fusarium oxysporum f. sp. batatas), foot rot (Diaporthe destruens), and black rot (Ceratocystis fimbriata);
Turfgrass diseases:
   dollar spot (Sclerotinia homoeocarpa), brown patch and large patch (Rhizoctonia solani), pythium blight (Pythium aphanidermatum), and anthracnose (Colletotrichum caudatum);
Banana disease:
   Sigatoka disease (Mycosphaerella fijiensis and Mycosphaerella musicola);
Lentils disease:
   ascochyta blight (Ascochyta lentis);
Chickpea disease:
   ascochyta blight (Ascochyta rabiei);
Green pepper diseases:
   anthracnose (Colletotrichum scovillei), powdery mildew (Leveillula taurica), and southern blight (Sclerotium rolfsii);
Mango disease:
   anthracnose (Colletotrichum acutatum);
Cacao diseases:
   Witches' broom (Moniliophthora perniciosa) and Frosty pod rot (Moniliophthora roreri);
Pecan disease:
   scab (Venturia effusa);
Lychee diseases:
   downy mildew (Peronophythora litchii) and anthracnose (Colletotrichum siemense);
Fruit trees diseases:
   white root rot (Rosellinia necatrix) and violet root rot (Helicobasidium mompa);
Postharvest disease of fruits (for example, apple and pear) :
   Mucor rot diseases (Mucor piriformis);
Seed diseases or diseases in the early stages of the growth of various plants caused by Aspergillus spp., Penicillium spp., Fusarium spp., Gibberella spp., Tricoderma spp., Thielaviopsis spp., Rhizopus spp., Mucor spp., Corticium spp., Phoma spp., Rhizoctonia spp. or Diplodia spp., or the like;
Viral diseases:
   Lettuce big-vein disease transmitted by Olpidium brassicae, and viral diseases of several crops transmitted by Polymyxa spp. (e.g., Polymyxa betae and Polymyxa graminis);
Diseases caused by bacteria:
   bacterial seedling blight of rice (Burkholderia plantarii), bacterial palea browning of rice (Pantoea ananatis), Bacterial leaf blight of rice (Xanthomonas oryzae pv. oryzae.), bacterial spot of cucumber (Pseudomonas syringae pv. lachrymans), bacterial wilt of eggplant (Ralstonia solanacearum), canker of citrus (Xanthomonas citri), bacterial soft rot of Chinese cabbage (Erwinia carotovora), scab of potato (Streptomyces scabiei), black leg of potato (Pectobacterium carotovorum and Dickeya sp.), bacterial leaf spot of peach (Pseudomona syringae, Erwinia nigrifluens, and Xanthomonas campestris), bacterial canker of Japanese apricot (Prunus mume) (Pseudomonas syringae pv. morsprunorum and Erwinia sp.), Goss's wilt of corn (Clavibacter michiganensis), Pierce's disease of grapes, olive, peach, and the like (Xylella fastidiosa), and crown gall of Rosaceae plants such as apple, peach, and cherries (Agrobacterium tumefaciens);
and the others.

A rust fungus of soybean having an amino acid substitution of F129L in the mitochondrial cytochrome b protein means a rust fungus of soybean (scientific name: Phakopsora pachyrhizi) which has a mutation in the mitochondrial cytochrome b gene encoding the mitochondrial cytochrome protein, and as a result of said mutation, has an amino acid substitution of F129L, thereby shows resistance to QoI fungicides.

The method for controlling a plant disease of the present invention is carried out by applying an effective amount of the Present compound, the Present compound W, the Compound of the present invention, the Compound W of the present invention, or the Composition A to a plant or soil for cultivating a plant. Examples of the treatment method include foliage treatment, soil treatment, and seed treatment.

The Present compound, the Present compound W, the Compound of the present invention, the Compound W of the present invention, or the Composition A is usually used by mixing it with inert carrier(s) such as solid carrier(s) and liquid carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, or the like. In addition to these formulations, the Present compound, the Present compound W, the Compound of the present invention, the Compound W of the present invention, or the Composition A may be used by formulating it into a dosage form described in Manual on development and use of FAO and WHO Specifications for pesticides, FAO Plant Production and Protection Papers- 271-276, prepared by the FAO/WHO Joint Meeting on Pesticide Specifications, 2016, ISSN:0259-2517.

These formulations usually comprise 0.0001% to 99% by weight ratio of the Present compound, the Present compound W, the Compound of the present invention, the Compound W of the present invention, or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Also, adjuvant(s) may be used as ingredient(s) for enhancing or assisting the efficacy of the Present compound, the Present compound W, the Compound of the present invention, the Compound W of the present invention, or the Composition A. Specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

In the present invention, the plants include whole plants and specific parts of plants. Examples of the specific parts of plants include foliages, flowers, ears, fruits, tree stems, branches, tree crowns, seeds, vegetative reproductive organs, and nursery plants.

A vegetative reproductive organ means a part of plant such as root, stem, and leaf which has a growth capability even when said part is separated from the plant body and placed into soil. Examples of the vegetative reproductive organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Nursery plants include seedlings and saplings.

Examples of a method for controlling plant diseases by applying an effective amount of the Present compound, the Present compound W, the Compound of the present invention, the Compound W of the present invention, or the Composition A to soil include a method of applying an effective amount of the Present compound, the Present compound W, the Compound of the present invention, the Compound W of the present invention, or the Composition A to soil before planting plants or after planting plants. Specifically, examples of the application method include planting hole treatment (for example, spraying into planting holes and soil mixing after planting hole treatment), plant foot treatment (for example, plant foot spraying, soil mixing after plant foot treatment, irrigation at plant foot, and plant foot treatment at a later seeding raising stage), planting furrow treatment (for example, planting furrow spraying and soil mixing after planting furrow treatment), planting row treatment (for example, planting row spraying, soil mixing after planting row treatment, and planting row spraying at a growing stage), planting row treatment at the time of sowing (for example, planting row spraying at the time of sowing and soil mixing after planting row treatment at the time of sowing), broadcast treatment (for example, overall soil surface spraying and soil mixing after broadcast treatment), side-article treatment, treatment of water surface (for example, application to water surface and application to water surface after flooding), other soil spraying treatment (for example, spraying of a granular formulation on leaves at a growing stage, spraying under a canopy or around a tree stem, spraying on the soil surface, mixing with surface soil, spraying into seed holes, spraying on the ground surfaces of furrows, and spraying between plants), other irrigation treatment (for example, soil irrigation, irrigation at a seedling raising stage, chemical solution injection treatment, irrigation of a plant part just above the ground, chemical solution drip irrigation, and chemigation), seedling raising box treatment (for example, spraying into a seedling raising box, irrigation of a seedling raising box, and flooding into a seedling raising box with chemical solution), seedling raising tray treatment (for example, spraying on a seedling raising tray, irrigation of a seedling raising tray, and flooding into a seedling raising tray with chemical solution), seedbed treatment (for example, spraying on a seedbed, irrigation of a seedbed, spraying on a lowland rice nursery, and immersion of seedlings), seedbed soil incorporation treatment (for example, mixing with seedbed soil, mixing with seedbed soil before sowing, spraying at sowing before covering with soil, spraying at sowing after covering with soil, and mixing with covering with soil), and other treatment (for example, mixing with culture soil, plowing under, mixing with surface soil, mixing with soil at the place where raindrops fall from a canopy, treatment at a planting position, spraying of a granule formulation on flower clusters, and mixing with a paste fertilizer).

Examples of the application to seeds (or seed treatments) include an application of the Present compound, the Present compound W, the Compound of the present invention, the Compound W of the present invention, or the Composition A to seeds or vegetative reproductive organs, and specific examples thereof include spraying treatment in which a suspension of the Present compound, the Present compound W, the Compound of the present invention, the Compound W of the present invention, or the Composition A is sprayed onto seed surface or the vegetative reproductive organ surface in the form of mist; smearing treatment in which the Present compound, the Present compound W, the Compound of the present invention, the Compound W of the present invention, or the Composition A is coated on a surface of seeds or the vegetative reproductive organs; a soaking treatment in which the seeds or vegetative reproductive organs are soaked into the solution of the Present compound, the Present compound W, the Compound of the present invention, the Compound W of the present invention, or the Composition A for a certain time; and a method for coating the seeds or the vegetative reproductive organs with a carrier containing the Present compound, the Present compound W, the Compound of the present invention, the Compound W of the present invention, or the Composition A (for example, film coating treatment and pellet coating treatment). Examples of the above-described vegetative reproductive organ include particularly seed potato.

As used herein, seeds or vegetative reproductive organs holding the Present compound, the Present compound W, the Compound of the present invention, the Compound W of the present invention, or the Composition A mean seeds or vegetative reproductive organs in the state where the Present compound, the Present compound W, the Compound of the present invention, the Compound W of the present invention, or the Composition A is adhered to a surface of the seeds or the vegetative reproductive organs. The above-described seeds or vegetative reproductive organs holding the Present compound, the Present compound W, the Compound of the present invention, the Compound W of the present invention, or the Composition A may be adhered by any other materials that are different from the Present compound, the Present compound W, the Compound of the present invention, the Compound W of the present invention, or the Composition A before or after being adhered by the Present compound, the Present compound W, the Compound of the present invention, the Compound W of the present invention, or the Composition A to the seeds or vegetative reproductive organs.

Also, when the Composition A is adhered in a form of layer(s) to a surface of seeds or vegetative reproductive organs, the layer(s) is/are composed of one layer or multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs holding the Present compound, the Present compound W, the Compound of the present invention, the Compound W of the present invention, or the Composition A can be obtained, for example, by applying the formulations comprising the Present compound, the Present compound W, the Compound of the present invention, the Compound W of the present invention, or the Composition A by the above-described application method to seeds or vegetative reproductive organs.

The amount of the Present compound, the Present compound W, the Compound of the present invention, the Compound W of the present invention, or the Composition A to be applied varies depending on the climate condition, the dosage form, the application period, the application method, the application site, diseases to be controlled, crops to be protected, and the like, and is usually within the range of 1 g to 500 g per 1000 m² of area for cultivating plants in case of foliage treatment or soil treatment. In case of seed treatment, the amount of the Present compound, the Present compound W, the Compound of the present invention, the Compound W of the present invention, or the Composition A is usually within the range of 0.001 g to 100 g per 1 Kg of seed. When the Present compound, the Present compound W, the Compound of the present invention, the Compound W of the present invention, or the Composition A is formulated into an emulsifiable concentrate, a wettable powder, a flowable, or the like, such formulation is usually applied after diluting it with water in such a way that a concentration of the active ingredient is within the range of 0.01 ppm to 10000 ppm, and then sparging it. In the case of being formulated into a dust formulation, a granule, or the like, such formulation is usually used as itself without diluting it.

The Present compound, the Present compound W, the Compound of the present invention, the Compound W of the present invention, or the Composition A may be used as an agent for controlling plant diseases in croplands such as fields, paddy fields, grasses, and orchards. Examples of the plants include the followings.

corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

### EXAMPLES

Hereinafter, the present invention is illustrated more in detail by Preparation Examples, Reference Preparation Examples, Formulation Examples, and Test Examples, but the present invention is not limited to these Examples only.

In the present description, Me represents a methyl group, Et represents an ethyl group, Pr represents a propyl group, i-Pr represents an isopropyl group, c-Pr represents a cyclopropyl group, Bu represents a butyl group, t-Bu represents a tert-butyl group, 2-Thie represents a 2-thienyl group, 3-Thie represents a 3-thienyl group, 2-Fura represents a 2-furanyl group, 3-Fura represents a 3-furanyl group, 2-Py represents a 2-pyridyl group, 3-Py represents a 3-pyridyl group, 4-Py represents a 4-pyridyl group, 1-Pyraz represents a pyrazol-1-yl group, 3-Pyraz represents a pyrazol-3-yl group, 4-Pyraz represents a pyrazol-4-yl group, 5-Pyraz represents a pyrazol-5-yl group, 2-Thiaz represents a thiazol-2-yl group, 5-Thiaz represents a thiazol-5-yl group, 2-Imidaz represents an imidazol-2-yl group, 4-Imidaz represents an imidazol-4-yl group, and 2-Pyrazin represents a pyrazin-2-yl group. When these groups have substituent(s), the substituent(s) is/are indicated before the symbols of these groups with the substitution position(s). For example, 5-OMe-2-Pyrazin represents a 5-methoxypyrazin-2-yl group, 5-Br-2-Py represents a 5-bromo-2-pyridyl group, and 1,5-Me₂-3-Pyraz represents a 1,5-dimethylpyrazol-3-yl group.

When a physical property of a compound is measured by liquid chromatography / mass spectrometry (hereinafter referred to as "LC/MS"), the measured molecular ion value [M+H]⁺ or [M-H]⁻, and retention time (hereinafter referred to as "RT") are described. The conditions of liquid chromatography (hereinafter referred to as "LC") and mass spectrometry (hereinafter referred to as "MS") are as follows.

### [LC conditions]

Column: InertSustain C18, inner diameter: 2.1 mm, length: 50 mm, particle size: 2 µm (manufactured by GL Sciences Inc.)
Mobile phase: Solution A: 5 mM aqueous solution of ammonium acetate, Solution B: methanol
Flow rate: 0.3 mL/min
Pump: LC-30AD (manufactured by Shimadzu Corporation) two machines (high pressure gradient)
Gradient conditions: sending a solution with the concentration gradient described in Table LC1.

**Table LC1**

| Time (min) | Solution A (%) | Solution B (%) |
|---|---|---|
| 0.2 | 90 | 10 |
| 4.00 | 5 | 95 |
| 6.00 | 5 | 95 |
| 6.01 | 90 | 10 |

### [MS conditions]

Detector: LCMS-8030 (manufactured by Shimadzu Corporation)
Ionization Method: ESI method

First, Preparation Examples of the Present compounds (including the Compounds of the present invention) are shown below.

### Preparation Example 1

Under nitrogen atmosphere, to a mixture of the Intermediate 1 (0.14 g), triethylamine (0.17 mL), and THF (2 mL) was added ethanesulfonyl chloride (0.12 mL), and the resulting mixture was stirred at room temperature for 2 hours. The mixture was subjected to silica gel column chromatography (hexane : ethyl acetate = 1 : 1) to give the Present compound 1 represented by the following formula (0.23 g). 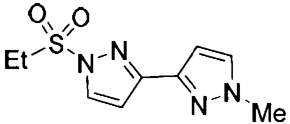 Present compound 1: LC/MS [M+H]⁺ = 241, RT = 3.31 min

### Preparation Example 1-1

The compounds prepared according to the Preparation Example 1 and physical properties thereof are shown below.

A compound represented by formula (IA-1) 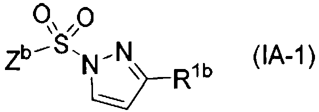 (hereinafter referred to as "Compound (IA-1)") wherein the combination of Z^{b} and R^{1b} represents any one combination indicated in Table A-1.

**Table A-1**

| Present compound | Z^{b} | R^{1b} | LC/MS [M+H]⁺ | RT |
|---|---|---|---|---|
| 2 | Et | 2-Py | 238 | 3.63 min |
| 3 | Et | 3-Py | 238 | 3.45 min |
| 4 | Et | 4-Py | 238 | 3.46 min |
| 5 | Et | Benzothiazol-2-yl | 294 | 4.37 min |
| 6 | Et | 1,5-Me₂-3-Pyraz | 255 | 3.55 min |
| 7 | Et | 2-Thie | 243 | 3.98 min |
| 8 | Et | 2-Fura | 227 | 3.77 min |
| 9 | Et | 5-I-2-Thie | 369 | 4.44 min |
| 10 | Et | 6-Me-3-Py | 252 | 3.70 min |
| 11 | Et | 5-Br-2-Py | 316 | 4.25 min |
| 12 | Et | 6-Br-3-Py | 316 | 3.95 min |
| 13 | Et | 6-Cl-3-Py | 272 | 3.87 min |
| 14 | Et | 5-OMe-2-Pyrazin | 269 | 4.00 min |
| 15 | Et | Benzoxazol-2-yl | 278 | 4.14 min |
| 16 | Et | 2-Br-5-Thiaz | 322 | 4.03 min |

### Preparation Example 2

A mixture of 3-pyridylboronic acid (0.17 g), 3-bromo-5-(ethylsulfonyl)pyridine (0.30 g), {1,1'-bis(diphenylphosphino)ferrocene}dichloropalladium(II) (0.04 g), tripotassium phosphate (0.51 g), DME (4 mL), and water (0.04 mL) was stirred under reflux for 5 hours. To the resulting mixture was added water, and the resulting mixture was subjected to extraction with ethyl acetate. The resulting organic layer was concentrated under reduced pressure, and the resulting residue was subjected to silica gel column chromatography (hexane : ethyl acetate = 1 : 9) to give the Present compound 17 represented by the following formula (0.17 g). 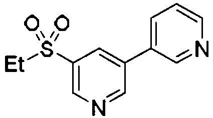 Present compound 17: LC/MS [M+H]⁺ = 249, RT = 3.07 min

### Preparation Example 2-1

The compounds prepared according to the Preparation Example 2 and physical properties thereof are shown below.

The Compound (IA-1) wherein the combination of Z^{b} and R^{1b} represents any one combination indicated in Table A-2.

**Table A-2**

| Present compound | Z^{b} | R^{1b} |
|---|---|---|
| 18 | Et | 2-OMe-4-Py |
| 19 | Et | 3-OMe-4-Py |
| 20 | 2-Thie | Benzothiophen-2-yl |
| 21 | 2-Thie | 5-F-Benzothiophen-2-yl |
| 22 | 2-Thie | Imidazo[1,2-a]pyridin-6-yl |
| 23 | 2-Thie | 6-F-5-Me-3-Py |

Present compound 18: LC/MS [M+H]⁺ = 268, RT = 3.96 min Present compound 19: LC/MS [M+H]⁺ = 268, RT = 3.73 min Present compound 20: ¹H-NMR (CDCl₃) δ: 8.14 (1H, d), 7.91 (1H, dd), 7.84-7.82 (1H, m), 7.79-7.77 (1H, m), 7.74 (1H, dd), 7.67 (1H, d), 7.38-7.33 (2H, m), 7.13 (1H, dd), 6.73 (1H, d).
Present compound 21: ¹H-NMR (CDCl₃) δ : 8.14 (1H, d), 7.92-7.91 (1H, m), 7.77-7.73 (2H, m), 7.61 (1H, s), 7.43 (1H, dd), 7.15-7.11 (2H, m), 6.72 (1H, d). Present compound 22: ¹H-NMR (CDCl₃) δ : 8.69 (1H, t), 8.18 (1H, d), 7.91 (1H, dd), 7.76 (1H, dd), 7.64 (4H, dt), 7.15 (1H, dd), 6.71 (1H, d).
Present compound 23: ¹H-NMR (CDCl₃) δ : 8.39 (1H, s), 8.16 (1H, d), 8.14-8.11 (1H, m), 7.90 (1H, dd), 7.76 (1H, dd), 7.14 (1H, dd), 6.72 (1H, d), 2.34 (3H, s).

### Preparation Example 2-2

The compounds prepared according to the Preparation Example 2 and physical properties thereof are shown below.

A compound represented by formula (IA-2) 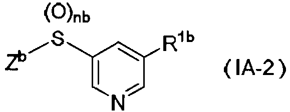 (hereinafter referred to as "Compound (IA-2)") wherein the combination of nb, Z^{b}, and R^{1b} represents any one combination indicated in Table A-3.

**Table A-3**

| Present compound | nb | Z^{b} | R^{1b} | LC/MS [M+H]⁺ | RT |
|---|---|---|---|---|---|
| 24 | 2 | Et | 5-Me-2-Thie | 268 | 4.20 min |
| 25 | 2 | Et | 4-Py | 249 | 3.06 min |
| 26 | 2 | Pr | Benzothiophen-2-yl | 318 | 4.65 min |
| 27 | 2 | Pr | 6-Me-3-Py | 277 | 3.66 min |
| 28 | 2 | Et | 2-Pyrazin | 250 | 2.99 min |
| 29 | 2 | Pr | 6-F-5-Me-3-Py | 295 | 3.88 min |
| 30 | 2 | Pr | 6-CF₃-3-Py | 331 | 3.99 min |
| 31 | 2 | Pr | 1-Et-3-Pyraz | 280 | 3.81 min |
| 32 | 2 | Pr | 1-Et-4-Pyraz | 280 | 3.59 min |
| 33 | 2 | Pr | 6-F-3-Py | 281 | 3.58 min |
| 34 | 2 | Pr | 6-F-5-Cl-3-Py | 315 | 4.07 min |
| 35 | 2 | Pr | 6-Cl-5-Me-3-Py | 311 | 4.06 min |
| 36 | 2 | Pr | 2, 6-F₂-3-Py | 299 | 3.87 min |
| 37 | 2 | Pr | 2, 6-F₂-4-Py | 299 | 3.89 min |
| 38 | 2 | Pr | 6-Cl-5-F-3-Py | 315 | 4.01 min |
| 103 | 2 | Et | 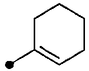 | 252 | 4.25 min |
| 104 | 2 | Et | 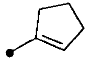 | 238 | 4.06 min |
| 105 | 2 | Pr | 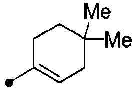 | 294 | 4.77 min |
| 106 | 2 | 2-Thie | 5-Me-1,2,4-oxadiazol-3-yl | | |
| 107 | 2 | 2-Thie | 1,2,4-oxadiazol-3-yl | | |
| 108 | 2 | Pr | 5-Me-1,2,4-oxadiazol-3-yl | | |
| 109 | 1 | Pr | 5-Me-1,2,4-oxadiazol-3-yl | | |
| 110 | 2 | Pr | 1,2,4-oxadiazol-3-yl | | |
| 111 | 1 | Pr | 1,2,4-oxadiazol-3-yl | | |

Present compound 106: ¹H-NMR (CDCl₃) δ: 9.45 (1H, d), 9.28 (1H, d), 8.89-8.87 (1H, m), 7.82-7.81 (1H, m), 7.74 (1H, dd), 7.16-7.14 (1H, m), 2.71 (3H, d).
Present compound 107: ¹H-NMR (CDCl₃) δ: 9.51 (1H, d), 9.31 (1H, d), 8.94 (1H, t), 8.88 (1H, s), 7.83 (1H, dd), 7.75 (1H, dd), 7.16 (1H, dd).
Present compound 108: ¹H-NMR (CDCl₃) δ: 9.54 (1H, d), 9.22 (1H, d), 8.84 (1H, t), 3.19-3.15 (2H, m), 2.72 (3H, s), 1.87-1.77 (2H, m), 1.05 (3H, t).
Present compound 109: ¹H-NMR (CDCl₃) δ: 9.41 (1H, d), 8.92 (1H, d), 8.64 (1H, t), 2.97-2.85 (2H, m), 2.71 (3H, s), 1.95-1.86 (1H, m), 1.78-1.66 (1H, m), 1.10 (3H, t).
Present compound 110: ¹H-NMR (CDCl₃) δ: 9.60 (1H, d), 9.25 (1H, d), 8.90-8.89 (2H, m), 3.20-3.16 (2H, m), 1.88-1.78 (2H, m), 1.06 (3H, t).
Present compound 111: ¹H-NMR (CDCl₃) δ: 9.46 (1H, d), 8.93 (1H, d), 8.87 (1H, s), 8.70 (1H, dd), 2.98-2.85 (2H, m), 1.97-1.84 (1H, m), 1.80-1.69 (1H, m), 1.10 (3H, t).

### Preparation Example 2-3

The compounds prepared according to the Preparation Example 2 and physical properties thereof are shown below.

A compound represented by formula (IA-6) 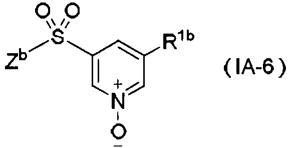 (hereinafter referred to as "Compound (IA-6)") wherein the combination of Z^{b} and R^{1b} represents any one combination indicated in Table A-15.

**Table A-15**

| Present compound | Z^{b} | R^{1b} |
|---|---|---|
| 112 | 2-Thie | 5-Me-1,2,4-oxadiazol-3-yl |
| 113 | 2-Thie | 1,2,4-oxadiazol-3-yl |

Present compound 112: ¹H-NMR (CDCl₃) δ: 8.92 (1H, t), 8.76 (1H, t), 8.35 (1H, t), 7.84-7.80 (2H, m), 7.19 (1H, dd), 2.70 (3H, s).
Present compound 113: ¹H-NMR (CDCl₃) δ: 8.98 (1H, t), 8.89 (1H, s), 8.79 (1H, t), 8.40 (1H, t), 7.83 (2H, ddd), 7.20 (1H, dd).

A compound represented by formula (IA-7) 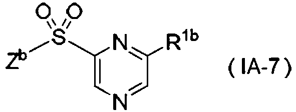 (hereinafter referred to as "Compound (IA-7)") wherein the combination of Z^{b} and R^{1b} represents any one combination indicated in Table A-16.

**Table A-16**

| Present compound | Z^{b} | R^{1b} |
|---|---|---|
| 115 | Pr | 6-F-5-Me-3-Py |
| 116 | Pr | 1-Me-3-Pyraz |

Present compound 115: ¹H-NMR (CDCl₃) δ: 9.27 (2H, d), 8.72 (1H, d), 8.35-8.32 (1H, m), 3.45-3.41 (2H, m), 2.43 (3H, s), 1.93-1.83 (2H, m), 1.09 (3H, t).
Present compound 116: ¹H-NMR (CDCl₃) δ: 9.48 (1H, s), 9.12 (1H, s), 7.47 (1H, d), 7.00 (1H, d), 4.03 (3H, s), 3.44-3.40 (2H, m), 1.92-1.81 (2H, m), 1.07 (3H, t). 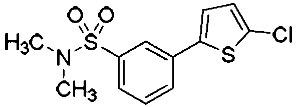
Present compound 119: ¹H-NMR (CDCl₃) δ: 7.89 (1H, s), 7.71-7.68 (2H, m), 7.56 (1H, t), 7.17 (1H, d), 6.94 (1H, d), 2.75 (6H, s). 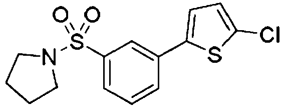 Present compound 120: ¹H-NMR (CDCl₃) δ: 7.95 (1H, t), 7.75-7.72 (1H, m), 7.70-7.68 (1H, m), 7.54 (1H, t), 7.18 (1H, d), 6.94 (1H, d), 3.30-3.26 (4H, m), 1.80-1.78 (4H, m).

### Preparation Example 3

To a mixture of the Intermediate 2 (0.32 g) and chloroform (15 mL) was added mCPBA (purity: 75%, wetted with 25% water) (0.46 g) at 0°C, and the resulting mixture was stirred at room temperature for 2 hours. To the mixture were added a saturated aqueous solution of sodium thiosulfate (10 mL) and a saturated aqueous solution of sodium hydrogen carbonate (10 mL), and the resulting mixture was stirred for 1 hour and then subjected to extraction with chloroform. The resulting organic layer was washed with saturated brine, dried over sodium sulfate, and concentrated under reduced pressure. The resulting residue was subjected to silica gel column chromatography (hexane : ethyl acetate = 1 : 1) to give the Present compound 39 represented by the following formula (0.19 g) and the Present compound 40 (0.13 g). 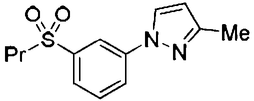 Present compound 39: LC/MS [M+H]⁺ = 265, RT = 4.03 min 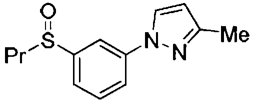 Present compound 40: LC/MS [M+H]⁺ = 249, RT = 4.01 min

### Preparation Example 3-1

The compounds prepared according to the Preparation Example 3 and physical properties thereof are shown below.

A compound represented by formula (IA-3) 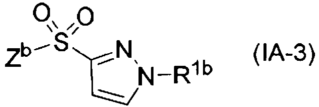 (hereinafter referred to as "Compound (IA-3)") wherein the combination of Z^{b} and R^{1b} represents any one combination indicated in Table A-4.

**Table A-4**

| Present compound | Z^{b} | R^{1b} | LC/MS [M+H]⁺ | RT |
|---|---|---|---|---|
| 41 | Et | 6-Cl-3-Py | 272 | 3.46 min |
| 42 | Et | 2-Py | 238 | 3.58 min |
| 43 | Et | 3-Py | 238 | 2.95 min |
| 44 | Et | 6-OMe-3-Py | 268 | 3.55 min |
| 45 | Et | 4-Py | 238 | 2.98 min |
| 46 | 2-Py | 2-Py | 287 | 3.69 min |

### Preparation Example 3-2

The compounds prepared according to the Preparation Example 3 and physical properties thereof are shown below.

A compound represented by formula (IA-5) 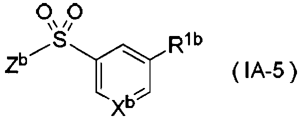 (hereinafter referred to as "Compound (IA-5)") wherein the combination of X^{b}, Z^{b}, and R^{1b} represents any one combination indicated in Table A-5.

**Table A-5**

| Present compound | X^{b} | Z^{b} | R^{1b} | LC/MS [M+H]⁺ | RT |
|---|---|---|---|---|---|
| 47 | CH | i-Pr | 3-(i-Pr)-1-Pyraz | 293 | 4.48 min |
| 48 | CH | Pr | 4-Br-3,5-Me₂-1-Pyraz | 357 | 4.52 min |
| 49 | CH | Pr | 5-Me-1-Pyraz | 265 | 3.83 min |
| 50 | CH | Pr | 5-F-6-Me-2-Py | 294 | 4.41 min |
| 51 | CH | Pr | 5-F-4-Me-2-Py | 294 | 4.36 min |
| 52 | CH | Pr | 6-F-5-Me-3-Py | 294 | 4.19 min |

A compound represented by formula (IA-8) 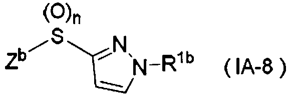 (hereinafter referred to as "Compound (IA-8)") wherein the combination of n, Z^{b}, and R^{1b} represents any one combination indicated in Table A-17.

**Table A-17**

| Present compound | n | Z^{b} | R^{1b} |
|---|---|---|---|
| 117 | 2 | 2-Thie | 6-F-5-Me-3-Py |
| 118 | 1 | 2-Thie | 6-F-5-Me-3-Py |

Present compound 117: ¹H-NMR (CDCl₃) δ: 8.30 (1H, s), 8.05 (1H, dd), 7.92 (1H, d), 7.86 (1H, dd), 7.72 (1H, dd), 7.14 (1H, dd), 7.03 (1H, d), 2.38 (3H, d).
Present compound 118: ¹H-NMR (CDCl₃) δ: 8.31 (1H, t), 8.02 (1H, ddd), 7.97 (1H, d), 7.68 (1H, dd), 7.65 (1H, dd), 7.13 (1H, dd), 6.96 (1H, d), 2.37 (3H, s).

### Preparation Example 4

To a mixture of the Present compound 87 (0.20 g) and acetonitrile (5 mL) was added N-chlorosuccinimide (0.16 g) at room temperature, and the resulting mixture was stirred at 50°C for 4 hours. The mixture was allowed to cool to room temperature, a saturated aqueous solution of sodium thiosulfate (5 mL) and a saturated aqueous solution of sodium hydrogen carbonate (5 mL) were added thereto, and the resulting mixture was stirred at room temperature for 1 hour and then subjected to extraction with ethyl acetate. The resulting organic layer was washed with saturated brine, and concentrated under reduced pressure. The resulting residue was subjected to silica gel column chromatography (hexane : ethyl acetate = 1 : 1) to give the Present compound 58 represented by the following formula (0.18 g). 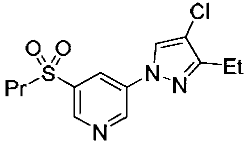 Present compound 58: LC/MS [M+H]⁺ = 314, RT = 4.57 min

### Preparation Example 4-1

The compounds prepared according to the Preparation Example 4 and physical properties thereof are shown below.

The Compound (IA-5) wherein the combination of X^{b}, Z^{b}, and R^{1b} represents any one combination indicated in Table A-6.

**Table A-6**

| Present compound | X^{b} | Z^{b} | R^{1b} | LC/MS [M+H]⁺ | RT |
|---|---|---|---|---|---|
| 53 | CH | Pr | 4-Cl-3-Me-1-Pyraz | 299 | 4.47 min |
| 54 | N | Pr | 4-Cl-3-Me-1-Pyraz | 300 | 4.33 min |
| 55 | N | Pr | 4-Cl-3-(i-Pr)-1-Pyraz | 328 | 4.75 min |
| 56 | N | Pr | 4-Cl-3-Pr-1-Pyraz | 328 | 4.74 min |
| 57 | N | Pr | 4-Cl-3-CHF₂-1-Pyraz | 336 | 4.23 min |
| 59 | N | Pr | 3,4-Cl₂-1-Pyraz | 320 | 4.46 min |
| 60 | N | Pr | 3-Br-4-Cl-1-Pyraz | 364 | 4.44 min |
| 61 | N | 2-Thie | 4-Cl-3-Me-1-Pyraz | 340 | 4.55 min |

### Preparation Example 5

To a mixture of the Present compound 39 (0.25 g) and chloroform (5 mL) was added N-bromosuccinimide (0.22 g), and the resulting mixture was stirred at room temperature for 1 hour. To the mixture were added a saturated aqueous solution of sodium thiosulfate (5 mL) and a saturated aqueous solution of sodium hydrogen carbonate (5 mL), and the resulting mixture was stirred for 1 hour and then subjected to extraction with ethyl acetate. The resulting organic layer was washed with saturated brine, dried over sodium sulfate, and concentrated under reduced pressure. The resulting residue was subjected to silica gel column chromatography (hexane : ethyl acetate = 1 : 1) to give the Present compound 62 represented by the following formula (0.28 g). 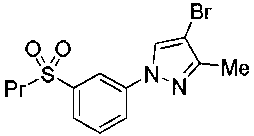 Present compound 62: LC/MS [M+H]⁺ = 343, RT = 4.53 min

### Preparation Example 5-1

The compounds prepared according to the Preparation Example 5 and physical properties thereof are shown below.

The Compound (IA-2) wherein the combination of nb, Z^{b}, and R^{1b} represents any one combination indicated in Table A-7.

**Table A-7**

| Present compound | nb | Z^{b} | R^{1b} | LC/MS [M+H]⁺ | RT |
|---|---|---|---|---|---|
| 63 | 2 | Pr | 4-Br-3-Me-1-Pyraz | 344 | 4.37 min |
| 64 | 2 | Pr | 4-Br-3-Et-1-Pyraz | 358 | 4.61 min |
| 65 | 2 | Pr | 3, 4-Br₂-1-Pyraz | 408 | 4.47 min |
| 66 | 2 | Pr | 4-Br-3-Cl-1-Pyraz | 364 | 4.47 min |
| 67 | 2 | 2-Thie | 4-Br-3-Me-1-Pyraz | 384 | 4.60 min |

### Preparation Example 5-2

The compounds prepared according to the Preparation Example 5 and physical properties thereof are shown below.

The Compound (IA-1) wherein the combination of Z^{b} and R^{1b} represents any one combination indicated in Table A-8.

**Table A-8**

| Present compound | Z^{b} | R^{1b} | LC/MS [M+H]⁺ | RT |
|---|---|---|---|---|
| 68 | Et | 5-Br-2-Fura | 305 | 4.23 min |
| 69 | Et | 5-Br-2-Thie | 321 | 4.44 min |

### Preparation Example 6

To a mixture of the Present compound 39 (0.25 g) and acetonitrile (5 mL) was added N-iodosuccinimide (0.32 g), and the resulting mixture was stirred at 60°C for 4 hours. The mixture was allowed to cool to room temperature, a saturated aqueous solution of sodium thiosulfate (5 mL) and a saturated aqueous solution of sodium hydrogen carbonate (5 mL) were added thereto, and the resulting mixture was stirred for 1 hour and then subjected to extraction with ethyl acetate. The resulting organic layer was washed with saturated brine, dried over sodium sulfate, and concentrated under reduced pressure. The resulting residue was subjected to silica gel column chromatography (hexane : ethyl acetate = 1 : 1) to give the Present compound 70 represented by the following formula (0.26 g). 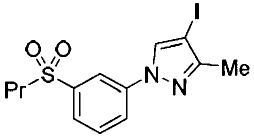 Present compound 70: LC/MS [M+H]⁺ = 391, RT = 4.55 min

### Preparation Example 6-1

The compounds prepared according to the Preparation Example 6 and physical properties thereof are shown below.

The Compound (IA-2) wherein the combination of nb, Z^{b}, and R^{1b} represents any one combination indicated in Table A-9.

**Table A-9**

| Present compound | nb | Z^{b} | R^{1b} | LC/MS [M+H]⁺ | RT |
|---|---|---|---|---|---|
| 71 | 2 | Pr | 4-I-3-Et-1-Pyraz | 406 | 4.62 min |
| 72 | 2 | Pr | 4-I-3-Me-1-Pyraz | 392 | 4.41 min |

### Preparation Example 7

Under nitrogen atmosphere, a mixture of 3-bromo-5-(propylsulfonyl)pyridine (0.40 g), bis(triphenylphosphine)palladium(II) dichloride (0.11 g), tert-butylacetylene (0.56 mL), tetrabutylammonium fluoride (a 1 mol/L solution in THF) (4.5 mL), and THF (4 mL) was stirred at 70°C for 7 hours. The resulting mixture was allowed to cool to room temperature, then a saturated aqueous solution of sodium hydrogen carbonate was added thereto, and the resulting mixture was subjected to extraction with ethyl acetate. The resulting organic layer was dried over sodium sulfate, and concentrated under reduced pressure. The resulting residue was subjected to silica gel column chromatography (ethyl acetate : hexane = 1 : 2) to give the Present compound 73 represented by the following formula (0.34 g). 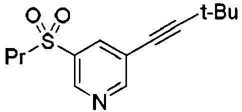 Present compound 73: LC/MS [M+H]⁺ = 266, RT = 4.63 min

### Preparation Example 7-1

The compounds prepared according to the Preparation Example 7 and physical properties thereof are shown below.

The Compound (IA-2) wherein the combination of nb, Z^{b}, and R^{1b} represents any one combination indicated in Table A-10.

**Table A-10**

| Present compound | nb | Z^{b} | R^{1b} | LC/MS [M+H]⁺ | RT |
|---|---|---|---|---|---|
| 74 | 2 | Pr | phenylethynyl | 286 | 4.61 min |
| 75 | 2 | Pr | 5-methylhex-1-yn-1-yl | 280 | 4.83 min |

### Preparation Example 8

Under nitrogen atmosphere, a mixture of 3-bromo-5-(ethylsulfonyl)pyridine (0.30 g), 4-methylpiperidine (0.17 mL), tris(dibenzylideneaceton)dipalladium(0) (0.054 g), 2-dicyclohexylphosphino-2',6'-dimethoxybiphenyl (0.030 g), sodium tert-butoxide (0.23 g), and toluene (3 mL) was stirred under reflux for 4 hours. The resulting mixture was allowed to cool to room temperature, then water was added thereto, and the resulting mixture was subjected to extraction with ethyl acetate. The resulting organic layer was dried over sodium sulfate, and concentrated under reduced pressure. The resulting residue was subjected to silica gel column chromatography (hexane : ethyl acetate = 1 : 1) to give the Present compound 76 represented by the following formula (0.13 g). 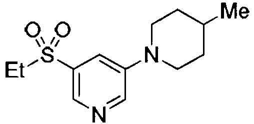 Present compound 76: LC/MS [M+H]⁺ = 269, RT = 4.14 min

### Preparation Example 8-1

The compounds prepared according to the Preparation Example 8 and physical properties thereof are shown below.

The Compound (IA-2) wherein the combination of nb, Z^{b}, and R^{1b} represents any one combination indicated in Table A-11.

**Table A-11**

| Present compound | nb | Z^{b} | R^{1b} | LC/MS [M+H]⁺ | RT |
|---|---|---|---|---|---|
| 77 | 2 | Pr | 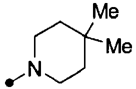 | 297 | 4.45 min |
| 78 | 2 | Pr | 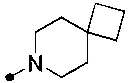 | 309 | 4.60 min |
| 79 | 2 | Pr | 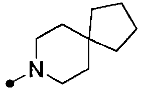 | 323 | 4.75 min |
| 80 | 2 | Pr | 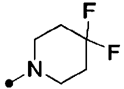 | 305 | 3.89 min |
| 81 | 2 | Pr | 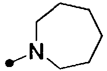 | 283 | 4.21 min |
| 82 | 2 | Pr | 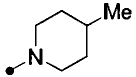 | 283 | 4.34 min |
| 114 | 2 | Pr | 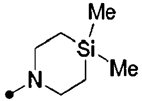 | 313 | 4.45 min |

### Preparation Example 9

Under nitrogen atmosphere, a mixture of benzoxazole (0.16 g), 3-bromo-5-(propylsulfonyl)pyridine (0.30 g), palladium(II) acetate (0.017 g), copper(II) acetate monohydrate (0.046 g), triphenylphosphine (0.15 g), potassium carbonate (0.32 g), and toluene (5 mL) was stirred under reflux for 3 hours. The resulting mixture was allowed to cool to room temperature, then ethyl acetate was added thereto, and the resulting mixture was filtered through Celite (trademark). The resulting filtrate was washed with water and saturated brine, dried over sodium sulfate, and concentrated under reduced pressure. The resulting solids were washed with methanol and hexane to give the Present compound 83 represented by the following formula (0.20 g). 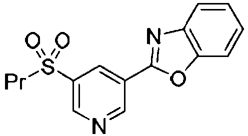 Present compound 83: LC/MS [M+H]⁺ = 303, RT = 4.36 min

### Preparation Example 9-1

The compounds prepared according to the Preparation Example 9 and physical properties thereof are shown below.

The Compound (IA-2) wherein the combination of nb, Z^{b}, and R^{1b} represents any one combination indicated in Table A-12.

**Table A-12**

| Present compound | nb | Z^{b} | R^{1b} | LC/MS [M+H]⁺ | RT |
|---|---|---|---|---|---|
| 84 | 2 | Pr | Benzothiazol-2-yl | 319 | 4.49 min |
| 85 | 2 | Pr | 4,5-Me₂-2-Thiaz | 297 | 4.27 min |

### Preparation Example 10

A mixture of 3-bromo-5-(propylsulfonyl)pyridine (0.30 g), 3,4-dimethylpyrazole (0.13 g), copper(I) iodide (0.11 g), N,N'-dimethylcyclohexane-1,2-diamine (0.1 mL), potassium carbonate (0.32 g), and DMF (5 mL) was stirred at 100°C for 6 hours. The resulting mixture was allowed to cool to room temperature, then water was added thereto, and the resulting mixture was subjected to extraction with ethyl acetate. The resulting organic layer was washed with saturated brine, and concentrated under reduced pressure. The resulting residue was subjected to silica gel column chromatography (hexane : ethyl acetate = 1 : 1) to give the Present compound 86 represented by the following formula (0.16 g). 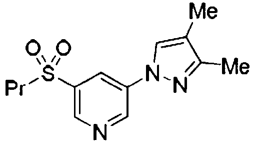 Present compound 86: LC/MS [M+H]⁺ = 280, RT = 4.09 min

### Preparation Example 10-1

The compounds prepared according to the Preparation Example 10 and physical properties thereof are shown below.

The Compound (IA-2) wherein the combination of nb, Z^{b}, and R^{1b} represents any one combination indicated in Table A-13.

**Table A-13**

| Present compound | nb | Z^{b} | R^{1b} | LC/MS [M+H]⁺ | RT |
|---|---|---|---|---|---|
| 87 | 2 | Pr | 3-Et-1-Pyraz | 280 | 4.11 min |
| 88 | 2 | Pr | 3-t-Bu-1-Pyraz | 308 | 4.57 min |
| 89 | 2 | Pr | 3-Me-1-Pyraz | 266 | 3.82 min |
| 90 | 2 | Pr | 3-(c-Pr)-1-Pyraz | 292 | 4.16 min |
| 91 | 2 | Pr | 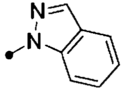 | 302 | 4.28 min |
| 92 | 2 | Pr | 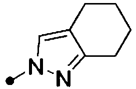 | 306 | 4.35 min |
| 93 | 2 | Pr | 3-(i-Pr)-1-Pyraz | 294 | 4.35 min |
| 94 | 2 | Pr | 3-Pr-1-Pyraz | 294 | 4.34 min |
| 95 | 2 | Pr | 3-CHF₂-1-Pyraz | 302 | 3.93 min |
| 96 | 2 | Pr | 3-Cl-1-Pyraz | 286 | 4.03 min |
| 97 | 2 | Pr | 3-Br-1-Pyraz | 330 | 4.07 min |
| 98 | 2 | 2-Thie | 3-Me-1-Pyraz | 306 | 4.12 min |

### Preparation Example 10-2

The compounds prepared according to the Preparation Example 10 and physical properties thereof are shown below.

A compound represented by formula (IA-4) 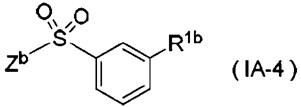 (hereinafter referred to as "Compound (IA-4)") wherein the combination of Z^{b} and R^{1b} represents any one combination indicated in Table A-14.

**Table A-14**

| Present compound | Z^{b} | R^{1b} | LC/MS [M+H]⁺ | RT |
|---|---|---|---|---|
| 99 | i-Pr | 1-Pyraz | 251 | 3.72 min |
| 100 | Bu | 1-Pyraz | 265 | 4.06 min |
| 101 | Bu | 4-Cl-1-Pyraz | 299 | 4.46 min |
| 102 | i-Pr | 4-Cl-1-Pyraz | 285 | 4.20 min |

### Reference Preparation Example 1

A mixture of 1-(1-methyl-1H-pyrazol-3-yl)ethanone (1.1 g), N,N-dimethylformamide dimethyl acetal (5.9 mL), and DMF (3 mL) was stirred under reflux for 6 hours. The resulting mixture was allowed to cool to room temperature, then water was added thereto, and the resulting mixture was subjected to extraction with chloroform. The resulting organic layer was washed with saturated brine, dried over sodium sulfate, and concentrated under reduced pressure. To the resulting solids were sequentially added ethanol (2 mL) and hydrazine monohydrate (0.12 mL), and the resulting mixture was stirred at room temperature for 8 hours. The resulting mixture was concentrated under reduced pressure. The resulting residue was subjected to silica gel column chromatography to give the Intermediate 1 represented by the following formula (0.14 g). 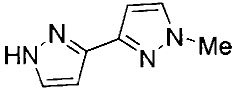 Intermediate 1: ¹H-NMR (CDCl₃) δ: 7.60 (1H, d), 7.38 (1H, d), 6.59 (1H, d), 6.53 (1H, d), 3.95 (3H, s).

### Reference Preparation Example 1-1

The compounds prepared according to the Reference Preparation Example 1 and physical properties thereof are shown below.

A compound represented by formula (IB-1) 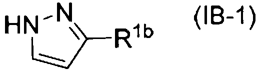 (hereinafter referred to as "Compound (IB-1)") wherein R^{1b} represents any one indicated in Table B-1.

**Table B-1**

| Intermediate | R^{1b} | Intermediate | R^{1b} |
|---|---|---|---|
| 4 | 1,5-Me₂-3-Pyraz | 5 | 2-Thie |
| 6 | 2-Fura | 7 | 5-I-2-Thie |
| 8 | 6-Me-3-Py | 9 | 5-Br-2-Py |
| 10 | 6-Br-3-Py | 11 | 6-Cl-3-Py |
| 12 | 5-OMe-2-Pyrazin | 13 | Benzoxazol-2-yl |

Intermediate 4: ¹H-NMR (CDCl₃) δ: 7.58 (1H, d), 6.53 (1H, d), 6.30 (1H, s), 3.81 (3H, s), 2.31 (3H, s).
Intermediate 5: ¹H-NMR (CDCl₃) δ: 7.60 (1H, d), 7.34 (1H, dd), 7.27 (1H, dd), 7.07 (1H, dd), 6.54 (1H, d).
Intermediate 6: ¹H-NMR (CDCl₃) δ: 7.60 (1H, d), 7.46 (1H, dd), 6.66 (1H, d), 6.55 (1H, d), 6.48 (1H, dd).
Intermediate 7: ¹H-NMR (CDCl₃) δ: 10.34 (1H, br s), 7.58 (1H, d), 7.20 (1H, d), 7.00 (1H, d), 6.49 (1H, d).
Intermediate 8: ¹H-NMR (CDCl₃) δ: 8.92 (1H, s), 8.03 (1H, br s), 7.98 (1H, dd), 7.65 (1H, d), 7.21 (1H, d), 6.65 (1H, d), 2.60 (3H, s).
Intermediate 9: ¹H-NMR (CDCl₃) δ: 8.66 (1H, d), 7.88-7.85 (1H, m), 7.69-7.65 (2H, m), 6.82 (1H, d).
Intermediate 10: ¹H-NMR (CDCl₃) δ: 10.32 (1H, br s), 8.79 (1H, d), 7.99 (1H, dd), 7.67 (1H, d), 7.53 (1H, d), 6.68 (1H, d).
Intermediate 11: ¹H-NMR (CDCl₃) δ: 10.33 (1H, br s), 8.81 (1H, d), 8.09 (1H, dd), 7.67 (1H, d), 7.38 (1H, d), 6.68 (1H, d).
Intermediate 12: ¹H-NMR (CDCl₃) δ: 8.59 (1H, s), 8.23 (1H, t), 7.66 (1H, d), 6.77 (1H, s), 4.01 (3H, s).
Intermediate 13: ¹H-NMR (CDCl₃) δ: 7.81-7.75 (2H, m), 7.63-7.60 (1H, m), 7.40-7.36 (2H, m), 7.11 (1H, d).

### Reference Preparation Example 2

A mixture of 1-bromo-3-(propylthio)benzene (2.0 g), 3-methylpyrazole (0.84 mL), copper(I) iodide (0.84 g), N,N'-dimethylcyclohexane-1,2-diamine (0.8 mL), potassium carbonate (2.4 g), and DMF (40 mL) was stirred at 120°C for 8 hours. To the resulting mixture was added water, and the resulting mixture was subjected to extraction with ethyl acetate. The resulting organic layer was washed with saturated brine, dried over sodium sulfate, and concentrated under reduced pressure. The resulting residue was subjected to silica gel column chromatography (hexane : ethyl acetate = 2 : 1) to give the Intermediate 2 represented by the following formula (1.43 g) and the Intermediate 3 (0.29 g). 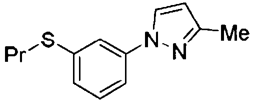 Intermediate 2: LC/MS [M+H]⁺ = 233, RT = 4.96 min 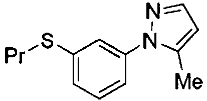 Intermediate 3: LC/MS [M+H]⁺ = 233, RT = 4.78 min

### Reference Preparation Example 2-1

The compounds prepared according to the Reference Preparation Example 2 and physical properties thereof are shown below. 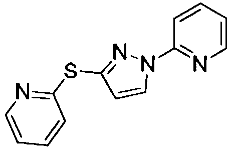 Intermediate 14: LC/MS [M+H]⁺ = 255, RT = 4.30 min 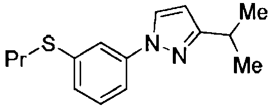 Intermediate 15: LC/MS [M+H]⁺ = 261, RT = 5.28 min

### Reference Preparation Example 3

The compounds prepared according to the Preparation Example 2 and physical properties thereof are shown below.

A compound represented by formula (IB-2) 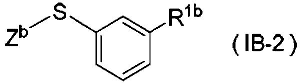 (hereinafter referred to as "Compound (IB-2)") wherein the combination of Z^{b} and R^{1b} represents any one combination indicated in Table B-2.

**Table B-2**

| Intermediate | Z^{b} | R^{1b} | LC/MS [M+H]⁺ | RT |
|---|---|---|---|---|
| 17 | Pr | 5-F-6-Me-2-Py | 262 | 5.34 min |
| 18 | Pr | 5-F-4-Me-2-Py | 262 | 5.30 min |

### Reference Preparation Example 4

Under argon atmosphere, a mixture of 1-bromo-3-(propylsulfanyl)benzene (10.0 g), [1,1'-bis(diphenylphosphino)ferrocene]palladium(II) dichloride (1.6 g), bis(pinacolato)diboron (13.2 g), potassium acetate (8.5 g), and DMSO (100 mL) was stirred at 90°C for 8 hours. The resulting mixture was cooled to room temperature, then water was added thereto, and the resulting mixture was filtered. To the resulting filtrate was added MTBE, and the resulting mixture was sequentially washed with water and saturated brine. The resulting organic layer was dried over anhydrous sodium sulfate, concentrated under reduced pressure, and the resulting residue was subjected to silica gel column chromatography (hexane : ethyl acetate = 20 : 1) to give the Intermediate 16 represented by the following formula (10.2 g). 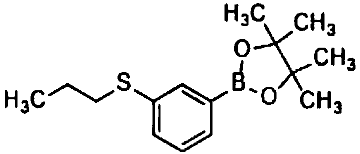 Intermediate 16: ¹H-NMR (CDCl₃) δ: 7.77 (1H, br s), 7.60 (1H, ddd), 7.42 (1H, ddd), 7.29 (1H, dd), 2.92 (2H, t), 1.66 (2H, td), 1.34 (12H, s), 1.02 (3H, t).

Examples of the Present compounds W and the Compounds W of the present invention prepared according to the above Production methods and Preparation Examples are shown below.

A compound represented by formula (A-1) 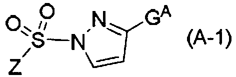 (hereinafter referred to as "Compound (A-1)") wherein Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1").

G1 to G40 represent the groups represented by the following formula G1 to formula G40, respectively.

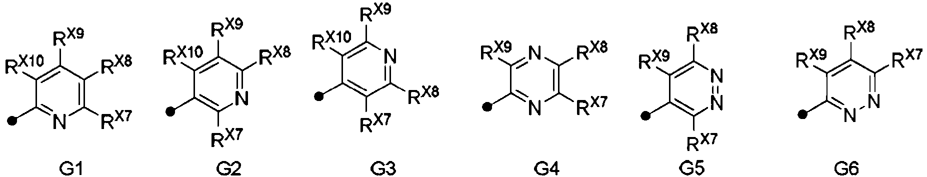

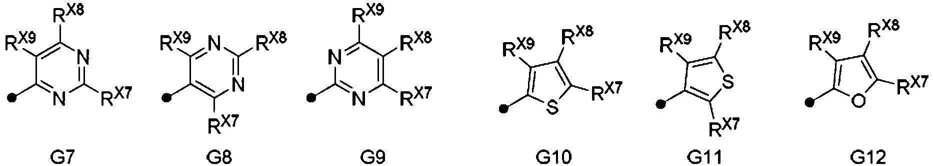

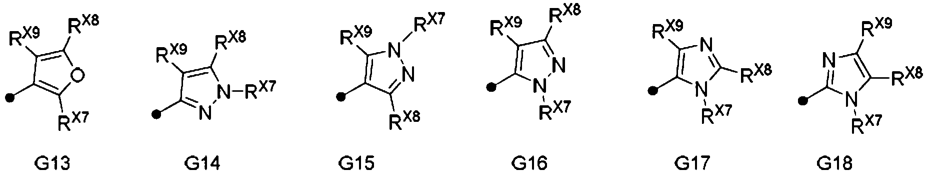

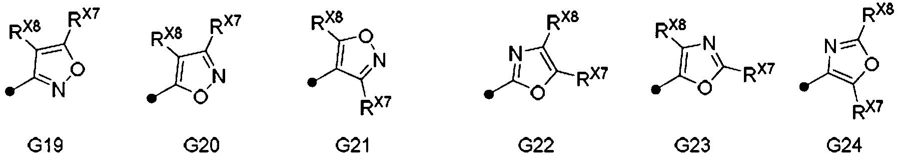

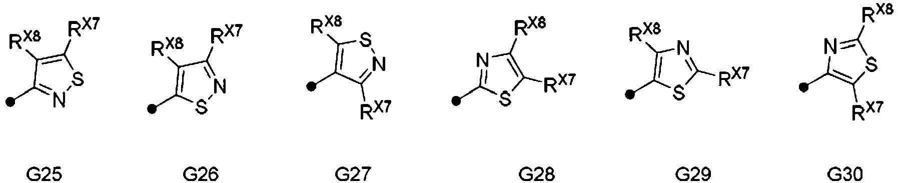

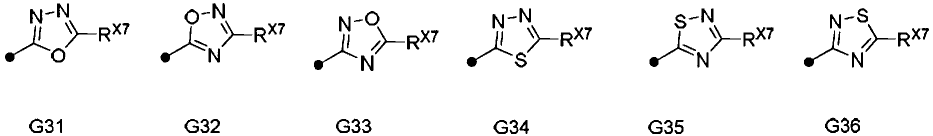

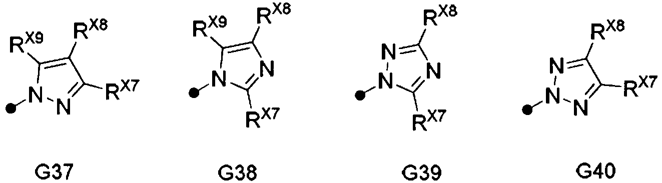

G41 to G74 represent the groups defined below, respectively. For example, G43 represents a cyclopentyl group.
G41: cyclopropyl, G42: cyclobutyl, G43: cyclopentyl, G44: cyclohexyl, G45: cycloheptyl, G46: cyclophenten-1-yl, G47: cyclohexen-1-yl, G48: cyclohepten-1-yl, G49: pyrrolidin-1-yl, G50: piperidin-1-yl, G51: 4-methylpiperidin-1-yl, G52: 4,4-dimethylpiperidin-1-yl, G53: 4,4-dimethyl-1,4-azasilinan-1-yl, G54: azepan-4-yl, G55: morpholin-4-yl, G56: 1,3-dioxolan-2-yl, G57: tetrahydrofuran-2-yl, G58: tetrahydrofuran-3-yl, G59: prop-1-yn-1-yl, G60: but-1-yn-1-yl, G61: pent-1-yn-1-yl, G62: 3-methylbut-1-yn-1-yl, G63: hex-1-yn-1-yl, G64: 4-methylpent-1-yn-1-yl, G65: 3-methylpent-1-yn-1-yl, G66: 3,3-dimethylbut-1-yn-1-yl, G67: hept-1-yn-1-yl, G68: oct-1-yn-1-yl, G69: cyclopropylethynyl, G70: cyclobutylethynyl, G71: cyclopentylethynyl, G72: cyclohexylethynyl, G73: cycloheptylethynyl, G74: phenylethynyl

Combination A consists of Substituent Numbers A1 to A1035. Substituent Numbers A1 to A1035 indicate the combinations of the group represented by G^{A} and the substituents R^{X7}, R^{X8}, R^{X9}, and R^{X10} in the group represented by G^{A} in the Compound (A-1), a compound represented by formula (A-3), a compound represented by formula (A-4), a compound represented by formula (A-5), a compound represented by formula (A-6), a compound represented by formula (A-7), a compound represented by formula (A-8), a compound represented by formula (A-9), and a compound represented by formula (A-10), and are hereinafter referred to as [Substituent Number; G^{A}, R^{X7}, R^{X8}, RX⁹, RX¹⁰].

For example, Substituent Number A5 indicates a combination wherein G^{A} represents G1, R^{X7}, R^{X8}, and R^{X9} each represent a hydrogen atom, and R^{X10} represents a fluorine atom.

Combination A: [A1;G1,H,H,H,H], [A2;G1,F,H,H,H], [A3;G1,H,F,H,H], [A4;G1,H,H,F,H], [A5;G1,H,H,H,F], [A6;G1,C1,H,H,H], [A7;G1,H,C1,H,H], [A8;G1,H,H,C1,H], [A9;G1,H,H,H,Cl], [A10;G1,Me,H,H,H], [A11;G1,H,Me,H,H], [A12;G1,H,H,Me,H], [A13;G1,H,H,H,Me], [A14;G1,F,F,H,H], [A15;G1,F,H,F,H], [A16;G1,F,H,H,F], [A17;G1,F,Cl,H,H], [A18;G1,F,H,C1,H], [A19;G1,F,H,H,Cl], [A20;G1,F,Me,H,H], [A21;G1,F,H,Me,H], [A22;G1,F,H,H,Me], [A23;G1,Cl,F,H,H], [A24;G1,C1,H,F,H], [A25;G1,Cl,H,H,F], [A26;Gl,Cl,Cl,H,H], [A27;Gl,Cl,H,Cl,H], [A28;G1,Cl,H,H,Cl], [A29;G1,Cl,Me,H,H], [A30;G1,Cl,H,Me,H], [A31;G1,Cl,H,H,Me], [A32;G1,Me,F,H,H], [A33;G1,Me,H,F,H], [A34;G1,Me,H,H,F], [A35;G1,Me,C1,H,H], [A36;G1,Me,H,Cl,H], [A37;G1,Me,H,H,Cl], [A38;G1,Me,Me,H,H], [A39;G1,Me,H,Me,H], [A40;G1,Me,H,H,Me], [A41;G1,H,F,F,H], [A42;G1,H,Cl,F,H], [A43;G1,H,Me,F,H], [A44;G1,H,F,Cl,H], [A45;G1,H,Cl,Cl,H], [A46;G1,H,Me,Cl,H], [A47;G1,H,F,Me,H], [A48;G1,H,Cl,Me,H], [A49;G1,H,Me,Me,H], [A50;G2,H,H,H,H], [A51;G2,F,H,H,H], [A52;G2,H,F,H,H], [A53;G2,H,H,F,H], [A54;G2,H,H,H,F], [A55;G2,Cl,H,H,H], [A56;G2,H,Cl,H,H], [A57;G2,H,H,Cl,H], [A58;G2,H,H,H,Cl], [A59;G2,Me,H,H,H], [A60;G2,H,Me,H,H], [A61;G2,H,H,Me,H], [A62;G2,H,H,H,Me], [A63;G2,F,F,H,H], [A64;G2,F,H,F,H], [A65;G2,F,H,H,F], [A66;G2,F,Cl,H,H], [A67;G2,F,H,Cl,H], [A68;G2,F,H,H,Cl], [A69;G2,F,Me,H,H], [A70;G2,F,H,Me,H], [A71;G2,F,H,H,Me], [A72;G2,Cl,F,H,H], [A73;G2,C1,H,F,H], [A74;G2,Cl,H,H,F], [A75;G2,Cl,Cl,H,H], [A76;G2,Cl,H,Cl,H], [A77;G2,Cl,H,H,Cl], [A78;G2,Cl,Me,H,H], [A79;G2,Cl,H,Me,H], [A80;G2,Cl,H,H,Me], [A81;G2,Me,F,H,H], [A82;G2,Me,H,F,H], [A83;G2,Me,H,H,F], [A84;G2,Me,Cl,H,H], [A85;G2,Me,H,Cl,H], [A86;G2,Me,H,H,C1], [A87;G2,Me,Me,H,H], [A88;G2,Me,H,Me,H], [A89;G2,Me,H,H,Me], [A90;G2,H,F,F,H], [A91;G2,H,Cl,F,H], [A92;G2,H,Me,F,H], [A93;G2,H,F,C1,H], [A94;G2,H,Cl,Cl,H], [A95;G2,H,Me,Cl,H], [A96;G2,H,F,Me,H], [A97;G2,H,Cl,Me,H], [A98;G2,H,Me,Me,H], [A99;G3,H,H,H,H], [A100;G3,F,H,H,H], [A101;G3,H,F,H,H], [A102;G3,H,H,F,H], [A103;G3,H,H,H,F], [A104;G3,Cl,H,H,H], [A105;G3,H,Cl,H,H], [A106;G3,H,H,Cl,H], [A107;G3,H,H,H,Cl], [A108;G3,Me,H,H,H], [A109;G3,H,Me,H,H], [A110;G3,H,H,Me,H], [A111;G3,H,H,H,Me], [Al12;G3,F,F,H,H], [A113;G3,F,H,F,H], [A114;G3,F,H,H,F], [A115;G3,F,Cl,H,H], [A116;G3,F,H,Cl,H], [A117;G3,F,H,H,Cl], [Al18;G3,F,Me,H,H], [A119;G3,F,H,Me,H], [A120;G3,F,H,H,Me], [A121;G3,Cl,F,H,H], [A122;G3,Cl,H,F,H], [A123;G3,Cl,H,H,F], [A124;G3,Cl,Cl,H,H], [A125;G3,Cl,H,Cl,H], [A126;G3,Cl,H,H,Cl], [A127;G3,Cl,Me,H,H], [A128;G3,Cl,H,Me,H], [A129;G3,Cl,H,H,Me], [A130;G3,Me,F,H,H], [A131;G3,Me,H,F,H], [A132;G3,Me,H,H,F], [A133;G3,Me,Cl,H,H], [A134;G3,Me,H,Cl,H], [A135;G3,Me,H,H,Cl], [A136;G3,Me,Me,H,H], [A137;G3,Me,H,Me,H], [A138;G3,Me,H,H,Me], [A139;G3,H,F,F,H], [A140;G3,H,Cl,F,H], [A141;G3,H,Me,F,H], [A142;G3,H,F,Cl,H], [A143;G3,H,C1,C1,H], [A144;G3,H,Me,Cl,H], [A145;G3,H,F,Me,H], [A146;G3,H,Cl,Me,H], [A147;G3,H,Me,Me,H], [A148;G4,H,H,H,-], [A149;G4,F,H,H,-], [A150;G4,H,F,H,-], [A151;G4,H,H,F,-], [A152;G4,Cl,H,H,-], [A153;G4,H,Cl,H,-], [A154;G4,H,H,Cl,-], [A155;G4,Me,H,H,-], [A156;G4,H,Me,H,-], [A157;G4,H,H,Me,-], [A158;G4,F,F,H,-], [A159;G4,F,H,F,-], [A160;G4,F,Cl,H,-], [A161;G4,F,H,Cl,-], [A162;G4,F,Me,H,-], [A163;G4,F,H,Me,-], [A164;G4,C1,F,H,-], [A165;G4,C1,H,F,-], [A166;G4,Cl,Cl,H,-], [A167;G4,Cl,H,Cl,-], [A168;G4,Cl,Me,H,-], [A169;G4,Cl,H,Me,-], [A170;G4,Me,F,H,-], [A171;G4,Me,H,F,-], [A172;G4,Me,Cl,H,-], [A173;G4,Me,H,Cl,-], [A174;G4,Me,Me,H,-], [A175;G4,Me,H,Me,-], [A176;G4,H,F,F,-], [A177;G4,H,Cl,F,-], [A178;G4,H,Me,F,-], [A179;G4,H,F,Cl,-], [A180;G4,H,Cl,Cl,-], [A181;G4,H,Me,Cl,-], [A182;G4,H,F,Me,-], [A183;G4,H,Cl,Me,-], [A184;G4,H,Me,Me,-], [A185;G5,H,H,H,-], [A186;G5,F,H,H,-], [A187;G5,H,F,H,-], [A188;G5,H,H,F,-], [A189;G5,Cl,H,H,-], [A190;G5,H,C1,H,-], [A191;G5,H,H,Cl,-], [A192;G5,Me,H,H,-], [A193;G5,H,Me,H,-], [A194;G5,H,H,Me,-], [A195;G5,F,F,H,-], [A196;G5,F,H,F,-], [A197;G5,F,Cl,H,-], [A198;G5,F,H,Cl,-], [A199;G5,F,Me,H,-], [A200;G5,F,H,Me,-], [A201;G5,Cl,F,H,-], [A202;G5,Cl,H,F,-], [A203;G5,Cl,Cl,H,-], [A204;G5,Cl,H,Cl,-], [A205;G5,Cl,Me,H,-], [A206;G5,Cl,H,Me,-], [A207;G5,Me,F,H,-], [A208;G5,Me,H,F,-], [A209;G5,Me,Cl,H,-], [A210;G5,Me,H,Cl,-], [A211;G5,Me,Me,H,-], [A212;G5,Me,H,Me,-], [A213;G5,H,F,F,-], [A214;G5,H,Cl,F,-], [A215;G5,H,Me,F,-], [A216;G5,H,F,Cl,-], [A217;G5,H,Cl,Cl,-], [A218;G5,H,Me,Cl,-], [A219;G5,H,F,Me,-], [A220;G5,H,Cl,Me,-], [A221;G5,H,Me,Me,-], [A222;G6,H,H,H,-], [A223;G6,F,H,H,-], [A224;G6,H,F,H,-], [A225;G6,H,H,F,-], [A226;G6,Cl,H,H,-], [A227;G6,H,Cl,H,-], [A228;G6,H,H,Cl,-], [A229;G6,Me,H,H,-], [A230;G6,H,Me,H,-], [A231;G6,H,H,Me,-], [A232;G6,F,F,H,-], [A233;G6,F,H,F,-], [A234;G6,F,Cl,H,-], [A235;G6,F,H,Cl,-], [A236;G6,F,Me,H,-], [A237;G6,F,H,Me,-], [A238;G6,Cl,F,H,-], [A239;G6,Cl,H,F,-], [A240;G6,Cl,Cl,H,-], [A241;G6,Cl,H,Cl,-], [A242;G6,Cl,Me,H,-], [A243;G6,Cl,H,Me,-], [A244;G6,Me,F,H,-], [A245;G6,Me,H,F,-], [A246;G6,Me,Cl,H,-], [A247;G6,Me,H,Cl,-], [A248;G6,Me,Me,H,-], [A249;G6,Me,H,Me,-], [A250;G6,H,F,F,-], [A251;G6,H,C1,F,-], [A252;G6,H,Me,F,-], [A253;G6,H,F,Cl,-], [A254;G6,H,Cl,Cl,-], [A255;G6,H,Me,Cl,-], [A256;G6,H,F,Me,-], [A257;G6,H,Cl,Me,-], [A258;G6,H,Me,Me,-], [A259;G7,H,H,H,-], [A260;G7,F,H,H,-], [A261;G7,H,F,H,-], [A262;G7,H,H,F,-], [A263;G7,Cl,H,H,-], [A264;G7,H,Cl,H,-], [A265;G7,H,H,Cl,-], [A266;G7,Me,H,H,-], [A267;G7,H,Me,H,-], [A268;G7,H,H,Me,-], [A269;G7,F,F,H,-], [A270;G7,F,H,F,-], [A271;G7,F,Cl,H,-], [A272;G7,F,H,Cl,-], [A273;G7,F,Me,H,-], [A274;G7,F,H,Me,-], [A275;G7,Cl,F,H,-], [A276;G7,Cl,H,F,-], [A277;G7,Cl,Cl,H,-], [A278;G7,Cl,H,Cl,-], [A279;G7,Cl,Me,H,-], [A280;G7,Cl,H,Me,-], [A281;G7,Me,F,H,-], [A282;G7,Me,H,F,-], [A283;G7,Me,Cl,H,-], [A284;G7,Me,H,Cl,-], [A285;G7,Me,Me,H,-], [A286;G7,Me,H,Me,-], [A287;G7,H,F,F,-], [A288;G7,H,Cl,F,-], [A289;G7,H,Me,F,-], [A290;G7,H,F,Cl,-], [A291;G7,H,Cl,Cl,-], [A292;G7,H,Me,Cl,-], [A293;G7,H,F,Me,-], [A294;G7,H,Cl,Me,-], [A295;G7,H,Me,Me,-], [A296;G8,H,H,H,-], [A297;G8,F,H,H,-], [A298;G8,H,F,H,-], [A299;G8,H,H,F,-], [A300;G8,Cl,H,H,-], [A301;G8,H,Cl,H,-], [A302;G8,H,H,Cl,-], [A303;G8,Me,H,H,-], [A304;G8,H,Me,H,-], [A305;G8,H,H,Me,-], [A306;G8,F,F,H,-], [A307;G8,F,H,F,-], [A308;G8,F,Cl,H,-], [A309;G8,F,H,Cl,-], [A310;G8,F,Me,H,-], [A311;G8,F,H,Me,-], [A312;G8,Cl,F,H,-], [A313;G8,Cl,H,F,-], [A314;G8,Cl,Cl,H,-], [A315;G8,Cl,H,Cl,-], [A316;G8,Cl,Me,H,-], [A317;G8,Cl,H,Me,-], [A318;G8,Me,F,H,-], [A319;G8,Me,H,F,-], [A320;G8,Me,Cl,H,-], [A321;G8,Me,H,Cl,-], [A322;G8,Me,Me,H,-], [A323;G8,Me,H,Me,-], [A324;G8,H,F,F,-], [A325;G8,H,Cl,F,-], [A326;G8,H,Me,F,-], [A327;G8,H,F,Cl,-], [A328;G8,H,Cl,Cl,-], [A329;G8,H,Me,Cl,-], [A330;G8,H,F,Me,-], [A331;G8,H,Cl,Me,-], [A332;G8,H,Me,Me,-], [A333;G9,H,H,H,-], [A334;G9,F,H,H,-], [A335;G9,H,F,H,-], [A336;G9,H,H,F,-], [A337;G9,Cl,H,H,-], [A338;G9,H,Cl,H,-], [A339;G9,H,H,Cl,-], [A340;G9,Me,H,H,-], [A341;G9,H,Me,H,-], [A342;G9,H,H,Me,-], [A343;G9,F,F,H,-], [A344;G9,F,H,F,-], [A345;G9,F,Cl,H,-], [A346;G9,F,H,Cl,-], [A347;G9,F,Me,H,-], [A348;G9,F,H,Me,-], [A349;G9,Cl,F,H,-], [A350;G9,Cl,H,F,-], [A351;G9,Cl,Cl,H,-], [A352;G9,Cl,H,Cl,-], [A353;G9,Cl,Me,H,-], [A354;G9,Cl,H,Me,-], [A355;G9,Me,F,H,-], [A356;G9,Me,H,F,-], [A357;G9,Me,Cl,H,-], [A358;G9,Me,H,Cl,-], [A359;G9,Me,Me,H,-], [A360;G9,Me,H,Me,-], [A361;G9,H,F,F,-], [A362;G9,H,Cl,F,-], [A363;G9,H,Me,F,-], [A364;G9,H,F,Cl,-], [A365;G9,H,Cl,Cl,-], [A366;G9,H,Me,Cl,-], [A367;G9,H,F,Me,-], [A368;G9,H,Cl,Me,-], [A369;G9,H,Me,Me,-], [A370;G10,H,H,H,-], [A371;G10,F,H,H,-], [A372;G10,H,F,H,-], [A373;G10,H,H,F,-], [A374;G10,Cl,H,H,-], [A375;G10,H,C1,H,-], [A376;G10,H,H,Cl,-], [A377;G10,Me,H,H,-], [A378;G10,H,Me,H,-], [A379;G10,H,H,Me,-], [A380;G10,F,F,H,-], [A381;G10,F,H,F,-], [A382;G10,F,Cl,H,-], [A383;G10,F,H,Cl,-], [A384;G10,F,Me,H,-], [A385;G10,F,H,Me,-], [A386;G10,Cl,F,H,-], [A387;G10,C1,H,F,-], [A388;G10,C1,C1,H,-], [A389;G10,Cl,H,Cl,-], [A390;G10,Cl,Me,H,-], [A391;G10,Cl,H,Me,-], [A392;G10,Me,F,H,-], [A393;G10,Me,H,F,-], [A394;G10,Me,Cl,H,-], [A395;G10,Me,H,Cl,-], [A396;G10,Me,Me,H,-], [A397;G10,Me,H,Me,-], [A398;G10,H,F,F,-], [A399;G10,H,C1,F,-], [A400;G10,H,Me,F,-], [A401;G10,H,F,Cl,-], [A402;G10,H,Cl,Cl,-], [A403;G10,H,Me,Cl,-], [A404;G10,H,F,Me,-], [A405;G10,H,Cl,Me,-], [A406;G10,H,Me,Me,-], [A407;G11,H,H,H,-], [A408;G11,F,H,H,-], [A409;G11,H,F,H,-], [A410;G11,H,H,F,-], [A411;G11,Cl,H,H,-], [A412;G11,H,Cl,H,-], [A413;G11,H,H,Cl,-], [A414;G11,Me,H,H,-], [A415;G11,H,Me,H,-], [A416;G11,H,H,Me,-], [A417;G11,F,F,H,-], [A418;G11,F,H,F,-], [A419;G11,F,Cl,H,-], [A420;G11,F,H,Cl,-], [A421;G11,F,Me,H,-], [A422;G11,F,H,Me,-], [A423;G11,Cl,F,H,-], [A424;G11,Cl,H,F,-], [A425;G11,Cl,Cl,H,-], [A426;G11,Cl,H,Cl,-], [A427;G11,Cl,Me,H,-], [A428;G1,Cl,H,Me,-], [A429;G11,Me,F,H,-], [A430;G11,Me,H,F,-], [A431;G11,Me,Cl,H,-], [A432;G11,Me,H,Cl,-], [A433;G11,Me,Me,H,-], [A434;G11,Me,H,Me,-], [A435;G11,H,F,F,-], [A436;G11,H,Cl,F,-], [A437;G1,H,Me,F,-], [A438;G11,H,F,Cl,-], [A439;G11,H,C1,C1,-], [A440;G11,H,Me,Cl,-], [A441;G11,H,F,Me,-], [A442;G11,H,Cl,Me,-], [A443;G11,H,Me,Me,-], [A444;G12,H,H,H,-], [A445;G12,F,H,H,-], [A446;G12,H,F,H,-], [A447;G12,H,H,F,-], [A448;G12,Cl,H,H,-], [A449;G12,H,Cl,H,-], [A450;G12,H,H,Cl,-], [A451;G12,Me,H,H,-], [A452;G12,H,Me,H,-], [A453;G12,H,H,Me,-], [A454;G12,F,F,H,-], [A455;G12,F,H,F,-], [A456;G12,F,Cl,H,-], [A457;G12,F,H,Cl,-], [A458;G12,F,Me,H,], [A459;G12,F,H,Me,-], [A460;G12,Cl,F,H,-], [A461;G12,Cl,H,F,-], [A462;G12,Cl,Cl,H,-], [A463;G12,Cl,H,Cl,-], [A464;G12,Cl,Me,H,-], [A465;G12,Cl,H,Me,-], [A466;G12,Me,F,H,-], [A467;G12,Me,H,F,-], [A468;G12,Me,Cl,H,-], [A469;G12,Me,H,Cl,-], [A470;G12,Me,Me,H,-], [A471;G12,Me,H,Me,-], [A472;G12,H,F,F,-], [A473;G12,H,Cl,F,-], [A474;G12,H,Me,F,-], [A475;G12,H,F,Cl,-], [A476;G12,H,Cl,Cl,-], [A477;G12,H,Me,Cl,-], [A478;G12,H,F,Me,-], [A479;G12,H,Cl,Me,-], [A480;G12,H,Me,Me,-], [A481;G13,H,H,H,-], [A482;G13,F,H,H,-], [A483;G13,H,F,H,-], [A484;G13,H,H,F,-], [A485;G13,Cl,H,H,-], [A486;G13,H,Cl,H,-], [A487;G13,H,H,Cl,-], [A488;G13,Me,H,H,-], [A489;G13,H,Me,H,-], [A490;G13,H,H,Me,-], [A491;G13,F,F,H,-], [A492;G13,F,H,F,-], [A493;G13,F,Cl,H,-], [A494;G13,F,H,Cl,-], [A49S;G13,F,Me,H,-], [A496;G13,F,H,Me,-], [A497;G13,Cl,F,H,-], [A498;G13,Cl,H,F,-], [A499;G13,Cl,Cl,H,-], [A500;G13,Cl,H,Cl,-], [A501;G13,Cl,Me,H,-], [A502;G13,Cl,H,Me,-], [A503;G13,Me,F,H,-], [A504;G13,Me,H,F,-], [A505;G13,Me,Cl,H,-], [A506;G13,Me,H,Cl,-], [A507;G13,Me,Me,H,-], [A508;G13,Me,H,Me,-], [A509;G13,H,F,F,-], [A510;G13,H,Cl,F,-], [A511;G13,H,Me,F,-], [A512;G13,H,F,Cl,-], [A513;G13,H,Cl,Cl,-], [A514;G13,H,Me,Cl,-], [A515;G13,H,F,Me,-], [A516;G13,H,Cl,Me,-], [A517;G13,H,Me,Me,-], [A518;G14,Me,H,H,-], [A519;G14,Me,F,H,-], [A520;G14,Me,H,F,-], [A521;G14,Me,Cl,H,-], [A522;G14,Me,H,Cl,-], [A523;G14,Me,Me,H,-], [A524;G14,Me,H,Me,-], [A525;G14,Me,F,F,-], [A526;G14,Me,F,Cl,-], [A527;G14,Me,F,Me,-], [A528;G14,Me,Cl,F,-], [A529;G14,Me,Cl,Cl,-], [A530;G14,Me,Cl,Me,-], [A531;G14,Me,Me,F,-], [A532;G14,Me,Me,Cl,-], [A533;G14,Me,Me,Me,-], [A534;G15,Me,H,H,-], [A535;G15,Me,F,H,-], [A536;G15,Me,H,F,-], [A537;G15,Me,Cl,H,-], [A538;G15,Me,H,Cl,-], [A539;G15,Me,Me,H,-], [A540;G15,Me,H,Me,-], [A541;G15,Me,F,F,-], [A542;G15,Me,F,Cl,-], [A543;G15,Me,F,Me,-], [A544;G15,Me,Cl,F,-], [A545;G15,Me,Cl,Cl,-], [A546;G15,Me,Cl,Me,-], [A547;G15,Me,Me,F,-], [A548;G15,Me,Me,Cl,-], [A549;G15,Me,Me,Me,-], [A550;G16,Me,H,H,-], [A551;G16,Me,F,H,-], [A552;G16,Me,H,F,-], [A553;G16,Me,Cl,H,-], [A554;G16,Me,H,Cl,-], [A555;G16,Me,Me,H,-], [A556;G16,Me,H,Me,-1], [A557;G16,Me,F,F,-], [A558;G16,Me,F,Cl,-], [A559;G16,Me,F,Me,-], [A560;G16,Me,Cl,F,-], [A561;G16,Me,Cl,Cl,-], [A562;G16,Me,Cl,Me,-], [A563;G16,Me,Me,F,-], [A564;G16,Me,Me,Cl,-], [A565;G16,Me,Me,Me,-], [A566;G17,Me,H,H,-], [A567;G17,Me,F,H,-], [A568;G17,Me,H,F,-], [A569;G17,Me,Cl,H,-], [A570;G17,Me,H,Cl,-], [A571;G17,Me,Me,H,-], [A572;G17,Me,H,Me,-], [A573;G17,Me,F,F,-], [A574;G17,Me,F,Cl,-], [A575;G17,Me,F,Me,-], [A576;G17,Me,Cl,F,-], [A577;G17,Me,Cl,Cl,-], [A578;G17,Me,Cl,Me,-], [A579;G17,Me,Me,F,-], [A580;G17,Me,Me,Cl,-], [A581;G17,Me,Me,Me,-], [A582;G18,Me,H,H,-], [A583;G18,Me,F,H,-], [A584;G18,Me,H,F,-], [A585;G18,Me,Cl,H,-], [A586;G18,Me,H,Cl,-], [A587;G18,Me,Me,H,-], [AS88;G18,Me,H,Me,-], [A589;G18,Me,F,F,-], [A590;G18,Me,F,Cl,-], [A591;G18,Me,F,Me,-], [A592;G18,Me,Cl,F,-], [A593;G18,Me,Cl,Cl,-], [A594;G18,Me,Cl,Me,-], [A595;G18,Me,Me,F,-], [A596;G18,Me,Me,Cl,-], [A597;G18,Me,Me,Me,-], [A598;G19,H,H,-,-], [A599;G19,F,H,-,-], [A600;G19,H,F,-,-], [A601;G19,Cl,H,-,-], [A602;G19,H,Cl,-,-], [A603;G19,Me,H,-,-], [A604;G19,H,Me,-,-], [A605;G19,F,F,-,-], [A606;G19,F,Cl,-,-], [A607;G19,F,Me,-,-], [A608;G19,Cl,F,-,-], [A609;G19,Cl,Cl,-,-], [A610;G19,Cl,Me,-,-], [A611;G19,Me,F,-,-], [A612;G19,Me,Cl,-,-], [A613;G19,Me,Me,-,-], [A614;G20,H,H,-,-], [A615;G20,F,H,-,-], [A616;G20,H,F,-,-], [A617;G20,Cl,H,-,-], [A618;G20,H,Cl,-,-], [A619;G20,Me,H,-,-], [A620;G20,H,Me,-,-], [A621;G20,F,F,-,-], [A622;G20,F,Cl,-,-], [A623;G20,F,Me,-,-], [A624;G20,Cl,F,-,-], [A625;G20,Cl,Cl,-,-], [A626;G20,Cl,Me,-,-], [A627;G20,Me,F,-,-], [A628;G20,Me,Cl,-,-], [A629;G20,Me,Me,-,-], [A630;G21,H,H,-,-], [A631;G21,F,H,-,-], [A632;G21,H,F,-,-], [A633;G21,Cl,H,-,-], [A634;G21,H,Cl,-,-], [A635;G21,Me,H,-,-], [A636;G21,H,Me,-,-], [A637;G21,F,F,-,-], [A638;G21,F,Cl,-,-], [A639;G21,F,Me,-,-], [A640;G21,Cl,F,-,-], [A641;G21,Cl,Cl,-,-], [A642;G21,Cl,Me,-,-], [A643;G21,Me,F,-,-], [A644;G21,Me,Cl,-,-], [A645;G21,Me,Me,-,-], [A646;G22,H,H,-,-], [A647;G22,F,H,-,-], [A648;G22,H,F,-,-], [A649;G22,Cl,H,-,-], [A650;G22,H,Cl,-,-], [A651;G22,Me,H,-,-], [A652;G22,H,Me,-,-], [A653;G22,F,F,-,-], [A654;G22,F,Cl,-,-], [A655;G22,F,Me,-,-], [A656;G22,Cl,F,-,-], [A657;G22,Cl,Cl,-,-], [A658;G22,Cl,Me,-,-], [A659;G22,Me,F,-,-], [A660;G22,Me,Cl,-,-], [A661;G22,Me,Me,-,-], [A662;G23,H,H,-,-], [A663;G23,F,H,-,-], [A664;G23,H,F,-,-], [A665;G23,Cl,H,-,-], [A666;G23,H,Cl,-,-], [A667;G23,Me,H,-,-], [A668;G23,H,Me,-,-], [A669;G23,F,F,-,-], [A670;G23,F,Cl,-,-], [A671;G23,F,Me,-,-], [A672;G23,Cl,F,-,-], [A673;G23,Cl,Cl,-,-], [A674;G23,Cl,Me,-,-], [A675;G23,Me,F,-,-], [A676;G23,Me,Cl,-,-], [A677;G23,Me,Me,-,-], [A678;G24,H,H,-,-], [A679;G24,F,H,-,-], [A680;G24,H,F,-,-], [A681;G24,Cl,H,-,-], [A682;G24,H,Cl,-,-], [A683;G24,Me,H,-,-], [A684;G24,H,Me,-,-], [A685;G24,F,F,-,-], [A686;G24,F,Cl,-,-], [A687;G24,F,Me,-,-], [A688;G24,Cl,F,-,-], [A689;G24,Cl,Cl,-,-], [A690;G24,Cl,Me,-,-], [A691;G24,Me,F,-,-], [A692;G24,Me,Cl,-,-], [A693;G24,Me,Me,-,-], [A694;G25,H,H,-,-], [A695;G25,F,H,-,-], [A696;G25,H,F,-,-], [A697;G25,Cl,H,-,-], [A698;G25,H,Cl,-,-], [A699;G25,Me,H,-,-], [A700;G25,H,Me,-,-], [A701;G25,F,F,-,-], [A702;G25,F,Cl,-,-], [A703;G25,F,Me,-,-], [A704;G25,Cl,F,-,-], [A705;G25,Cl,Cl,-,-], [A706;G25,Cl,Me,-,-], [A707;G25,Me,F,-,-], [A708;G25,Me,Cl,-,-], [A709;G25,Me,Me,-,-], [A710;G26,H,H,-,-], [A711;G26,F,H,-,-], [A712;G26,H,F,-,-], [A713;G26,Cl,H,-,-], [A714;G26,H,Cl,-,-], [A715;G26,Me,H,-,-], [A716;G26,H,Me,-,-], [A717;G26,F,F,-,-], [A718;G26,F,Cl,-,-], [A719;G26,F,Me,-,-], [A720;G26,Cl,F,-,-], [A721;G26,Cl,Cl,-,-], [A722;G26,Cl,Me,-,-], [A723;G26,Me,F,-,-], [A724;G26,Me,Cl,-,-], [A725;G26,Me,Me,-,-], [A726;G27,H,H,-,-], [A727;G27,F,H,-,-], [A728;G27,H,F,-,-], [A729;G27,Cl,H,-,-], [A730;G27,H,Cl,-,-], [A731;G27,Me,H,-,-], [A732;G27,H,Me,-,-], [A733;G27,F,F,-,-], [A734;G27,F,Cl,-,-], [A735;G27,F,Me,-,-], [A736;G27,Cl,F,-,-], [A737;G27,Cl,Cl,-,-], [A738;G27,Cl,Me,-,-], [A739;G27,Me,F,-,-], [A740;G27,Me,Cl,-,-], [A741;G27,Me,Me,-,-], [A742;G28,H,H,-,-], [A743;G28,F,H,-,-], [A744;G28,H,F,-,-], [A745;G28,Cl,H,-,-], [A746;G28,H,Cl,-,-], [A747;G28,Me,H,-,-], [A748;G28,H,Me,-,-], [A749;G28,F,F,-,-], [A750;G28,F,Cl,-,-], [A751;G28,F,Me,-,-], [A752;G28,Cl,F,-,-], [A753;G28,Cl,Cl,-,-], [A754;G28,Cl,Me,-,-], [A755;G28,Me,F,-,-], [A756;G28,Me,Cl,-,-], [A757;G28,Me,Me,-,-], [A758;G29,H,H,-,-], [A759;G29,F,H,-,-], [A760;G29,H,F,-,-], [A761;G29,Cl,H,-,-], [A762;G29,H,Cl,-,-], [A763;G29,Me,H,-,-], [A764;G29,H,Me,-,-], [A765;G29,F,F,-,-], [A766;G29,F,Cl,-,-], [A767;G29,F,Me,-,-], [A768;G29,Cl,F,-,-], [A769;G29,Cl,Cl,-,-], [A770;G29,Cl,Me,-,-], [A771;G29,Me,F,-,-], [A772;G29,Me,Cl,-,-], [A773;G29,Me,Me,-,-], [A774;G30,H,H,-,-], [A775;G30,F,H,-,-], [A776;G30,H,F,-,-], [A777;G30,Cl,H,-,-], [A778;G30,H,Cl,-,-], [A779;G30,Me,H,-,-], [A780;G30,H,Me,-,-], [A781;G30,F,F,-,-], [A782;G30,F,Cl,-,-], [A783;G30,F,Me,-,-], [A784;G30,Cl,F,-,-], [A785;G30,Cl,Cl,-,-], [A786;G30,Cl,Me,-,-], [A787;G30,Me,F,-,-], [A788;G30,Me,Cl,-,-], [A789;G30,Me,Me,-,-], [A790;G31,H,-,-,-], [A791;G31,F,-,-,-], [A792;G31,Cl,-,-,-], [A793;G31,Me,-,-,-], [A794;G31,OMe,-,-,-], [A795;G32,H,-,-,-], [A796;G32,F,-,-,-], [A797;G32,Cl,-,-,-], [A798;G32,Me,-,-,-], [A799;G32,OMe,-,-,-], [A800;G33,H,-,-,-], [A801;G33,F,-,-,-], [A802;G33,Cl,-,-,-], [A803;G33,Me,-,-,-], [A804;G33,OMe,-,-,-], [A805;G34,H,-,-,-], [A806;G34,F,-,-,-], [A807;G34,Cl,-,-,-], [A808;G34,Me,-,-,-], [A809;G34,OMe,-,-,-], [A810;G3S,H,-,-,-], [A811;G35,F,-,-,-], [A812;G35,Cl,-,-,-], [A813;G35,Me,-,-,-], [A814;G35,OMe,-,-,-], [A815;G36,H,-,-,-], [A816;G36,F,-,-,-], [A817;G36,Cl,-,-,-], [A818;G36,Me,-,-,-], [A819;G36,OMe,-,-,-], [A820;G37,H,H,H,-], [A821;G37,F,H,H,-], [A822;G37,H,F,H,-], [A823;G37,H,H,F,-], [A824;G37,Cl,H,H,-], [A825;G37,H,C1,H,-], [A826;G37,H,H,Cl,-], [A827;G37,Me,H,H,-], [A828;G37,H,Me,H,-], [A829;G37,H,H,Me,-], [A830;G37,F,F,H,-], [A831;G37,F,H,F,-], [A832;G37,F,Cl,H,-], [A833;G37,F,H,Cl,-], [A834;G37,F,Me,H,-], [A835;G37,F,H,Me,-], [A836;G37,Cl,F,H,-], [A837;G37,Cl,H,F,-], [A838;G37,Cl,Cl,H,-], [A839;G37,Cl,H,Cl,-], [A840;G37,Cl,Me,H,-], [A841;G37,Cl,H,Me,-], [A842;G37,Me,F,H,-], [A843;G37,Me,H,F,-], [A844;G37,Me,Cl,H,-], [A845;G37,Me,H,Cl,-], [A846;G37,Me,Me,H,-], [A847;G37,Me,H,Me,-], [A848;G37,H,F,F,-], [A849;G37,H,Cl,F,-], [A850;G37,H,Me,F,-], [A851;G37,H,F,Cl,-], [A852;G37,H,Cl,Cl,-], [A853;G37,H,Me,Cl,-], [A854;G37,H,F,Me,-], [A855;G37,H,Cl,Me,-], [A856;G37,H,Me,Me,-], [A857;G37,Br,F,H,-], [A858;G37,Br,Cl,H,-], [A859;G37,Br,Br,H,-], [A860;G37,Br,I,H,-], [A861;G37,Br,Me,H,-], [A862;G37,Br,Et,H,-], [A863;G37,I,F,H,-], [A864;G37,I,Cl,H,-], [A865;G37,I,Br,H,-], [A866;G37,I,I,H,-], [A867;G37,I,Me,H,-], [A868;G37,I,Et,H,-], [A869;G37,Et,F,H,-], [A870;G37,Et,Cl,H,-], [A871;G37,Et,Br,H,-], [A872;G37,Et,I,H,-], [A873;G37,Et,Me,H,-], [A874;G37,Et,Et,H,-], [A875;G37,Pr,F,H,-], [A876;G37,Pr,Cl,H,-], [A877;G37,Pr,Br,H,-], [A878;G37,Pr,I,H,-], [A879;G37,Pr,Me,H,-], [A880;G37,Pr,Et,H,-], [A881;G37,i-Pr,F,H,-], [A882;G37,i-Pr,Cl,H,-], [A883;G37,i-Pr,Br,H,-], [A884;G37,i-Pr,I,H,-], [A885;G37,i-Pr,Me,H,-], [A886;G37,i-Pr,Et,H,-], [A887;G37,c-Pr,F,H,-], [A888;G37,c-Pr,Cl,H,-], [A889;G37,c-Pr,Br,H,-], [A890;G37,c-Pr,I,H,-], [A891;G37,c-Pr,Me,H,-], [A892;G37,c-Pr,Et,H,-], [A893;G37,Me,Br,H,-], [A894;G37,Me,I,H,-], [A895;G38,H,H,H,-], [A896;G38,F,H,H,-], [A897;G38,H,F,H,-], [A898;G38,H,H,F,-], [A899;G38,Cl,H,H,-], [A900;G38,H,Cl,H,-], [A901;G38,H,H,Cl,-], [A902;G38,Me,H,H,-], [A903;G38,H,Me,H,-], [A904;G38,H,H,Me,-], [A905;G38,F,F,H,-], [A906;G38,F,H,F,-], [A907;G38,F,Cl,H,-], [A908;G38,F,H,Cl,-], [A909;G38,F,Me,H,-], [A910;G38,F,H,Me,-], [A911;G38,Cl,F,H,-], [A912;G38,Cl,H,F,-], [A913;G38,Cl,Cl,H,-], [A914;G38,Cl,H,Cl,-], [A915;G38,Cl,Me,H,-], [A916;G38,Cl,H,Me,-], [A917;G38,Me,F,H,-], [A918;G38,Me,H,F,-], [A919;G38,Me,Cl,H,-], [A920;G38,Me,H,Cl,-], [A921;G38,Me,Me,H,-], [A922;G38,Me,H,Me,-], [A923;G38,H,F,F,-], [A924;G38,H,Cl,F,-], [A925;G38,H,Me,F,-], [A926;G38,H,F,Cl,-], [A927;G38,H,Cl,Cl,-], [A928;G38,H,Me,Cl,-], [A929;G38,H,F,Me,-], [A930;G38,H,Cl,Me,-], [A931;G38,H,Me,Me,-], [A932;G39,H,H,-,-], [A933;G39,F,H,-,-], [A934;G39,H,F,-,-], [A935;G39,Cl,H,-,-], [A936;G39,H,Cl,-,-], [A937;G39,Me,H,-,-], [A938;G39,H,Me,-,-], [A939;G39,F,F,-,-], [A940;G39,F,Cl,-,-], [A941;G39,F,Me,-,-], [A942;G39,Cl,F,-,-], [A943;G39,Cl,Cl,-,-], [A944;G39,Cl,Me,-,-], [A945;G39,Me,F,-,-], [A946;G39,Me,Cl,-,-], [A947;G39,Me,Me,-,-], [A948;G39,H,H,-,-], [A949;G39,F,H,-,-], [A950;G40,H,F,-,-], [A951;G40,Cl,H,-,-], [A952;G40,H,Cl,-,-], [A953;G40,Me,H,-,-], [A954;G40,H,Me,-,-], [A955;G40,F,F,-,-], [A956;G40,F,Cl,-,-], [A957;G40,F,Me,-,-], [A958;G40,Cl,F,-,-], [A959;G40,Cl,Cl,-,-], [A960;G40,Cl,Me,-,-], [A961;G40,Me,F,-,-], [A962;G40,Me,Cl,-,-], [A963;G40,Me,Me,-,-], [A964;G40,Br,F,-,-], [A965;G40,Br,Cl,-,-], [A966;G40,Br,Br,-,-], [A967;G40,Br,I,-,-], [A968;G40,Br,Me,-,-], [A969;G40,Br,Et,-,-], [A970;G40,I,F,-,-], [A971;G40,I,Cl,-,-], [A972;G40,I,Br,-,-], [A973;G40,I,I,-,-], [A974;G40,I,Me,-,-], [A975;G40,I,Et,-,-], [A976;G40,Et,F,-,-], [A977;G40,Et,Cl,-,-], [A978;G40,Et,Br,-,-], [A979;G40,Et,I,-,-], [A980;G40,Et,Me,-,-], [A981;G40,Et,Et,-,-], [A982;G40,Pr,F,-,-], [A983;G40,Pr,Cl,-,-], [A984;G40,Pr,Br,-,-], [A985;G40,Pr,I,-,-], [A986;G40,Pr,Me,-,-], [A987;G40,Pr,Et,-,-], [A988;G40,i-Pr,F,-,-], [A989;G40,i-Pr,Cl,-,-], [A990;G40,i-Pr,Br,-,-], [A991;G40,i-Pr,I,-,-], [A992;G40,i-Pr,Me,-,-], [A993;G40,i-Pr,Et,-,-], [A994;G40,c-Pr,F,-,-], [A995;G40,c-Pr,Cl,-,-], [A996;G40,c-Pr,Br,-,-], [A997;G40,c-Pr,I,-,-], [A998;G40,c-Pr,Me,-,-], [A999;G40,c-Pr,Et,-,-], [A1000;G40,Me,Br,-,-], [A1001;G40,Me,I,-,-], [A1002;G41,-,-,-,-], [A1003;G42,-,-,-,-], [A1004;G43,-,-,-,-], [A1005;G44,-,-,-,-], [A1006;G45,-,-,-,-], [A1007;G46,-,-,-,-], [A1008;G47,-,-,-,-], [A1009;G48,-,-,-,-], [A1010;G49,-,-,-,-], [A1011;G50,-,-,-,-], [A1012;G51,-,-,-,-], [A1013;G52,-,-,-,-], [A1014;G53,-,-,-,-], [A1015;G54,-,-,-,-], [A1016;G55,-,-,-,-], [A1017;G56,-,-,-,-], [A1018;G57,-,-,-,-], [A1019;G58,-,-,-,-], [A1020;G59,-,-,-,-], [A1021;G60,-,-,-,-], [A1022;G61,-,-,-,-], [A1023;G62,-,-,-,-], [A1024;G63,-,-,-,-], [A1025;G64,-,-,-,-], [A1026;G65,-,-,-,-], [A1027;G66,-,-,-,-], [A1028;G67,-,-,-,-], [A1029;G68,-,-,-,-], [A1030;G69,-,-,-,-], [A1031;G70,-,-,-,-], [A1032;G71,-,-,-,-], [A1033;G72,-,-,-,-], [A1034;G73,-,-,-,-], [A1035;G74,-,-,-,-]

The Compound (A-1) wherein Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2").

The Compound (A-1) wherein Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX3").

The Compound (A-1) wherein Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX4").

The Compound (A-1) wherein Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX5").

The Compound (A-1) wherein Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX6").

The Compound (A-1) wherein Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX7").

The Compound (A-1) wherein Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX8").

The Compound (A-1) wherein Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX9").

The Compound (A-1) wherein Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX10").

The Compound (A-1) wherein Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX11").

The Compound (A-1) wherein Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX12").

The Compound (A-1) wherein Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX13").

The Compound (A-1) wherein Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX14").

The Compound (A-1) wherein Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX15").

The Compound (A-1) wherein Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX16").

The Compound (A-1) wherein Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX17").

A compound represented by formula (A-2) 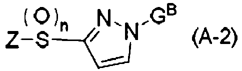 (hereinafter referred to as "Compound (A-2)") wherein n represents 1, Z represents an ethyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX18").

Combination B consists of Substituent Numbers B1 to B853. Substituent Numbers B1 to B853 indicate the combinations of the group represented by G^{B} and the substituents R^{X7}, R^{X8}, R^{X9}, and R^{X10} in the group represented by G^{B} in the compound represented by formula (A-2), and are hereinafter referred to as [Substituent Number; G^{B}, R^{X7},R^{X8},R^{X9},RX¹⁰].

For example, Substituent Number B5 indicates a combination wherein G^{B} represents G1, R^{X7}, R^{X8}, and R^{X9} each represent a hydrogen atom, and R^{X10} represents a fluorine atom.

Combination B: [B1;G1,H,H,H,H], [B2;G1,F,H,H,H], [B3;G1,H,F,H,H], [B4;G1,H,H,F,H], [B5;G1,H,H,H,F], [B6;G1,C1,H,H,H], [B7;G1,H,C1,H,H], [B8;G1,H,H,C1,H], [B9;G1,H,H,H,C1], [B10;G1,Me,H,H,H], [B11;G1,H,Me,H,H], [B12;G1,H,H,Me,H], [B13;G1,H,H,H,Me], [B14;G1,F,F,H,H], [B15;G1,F,H,F,H], [B16;G1,F,H,H,F], [B17;G1,F,Cl,H,H], [B18;G1,F,H,Cl,H], [B19;G1,F,H,H,Cl], [B20;G1,F,Me,H,H], [B21;G1,F,H,Me,H], [B22;G1,F,H,H,Me], [B23;G1,Cl,F,H,H], [B24;G1,Cl,H,F,H], [B25;G1,Cl,H,H,F], [B26;G1,Cl,Cl,H,H], [B27;G1,C1,H,C1,H], [B28;G1,C1,H,H,C1], [B29;G1,Cl,Me,H,H], [B30;G1,Cl,H,Me,H], [B31;G1,C1,H,H,Me], [B32;G1,Me,F,H,H], [B33;G1,Me,H,F,H], [B34;G1,Me,H,H,F], [B35;G1,Me,Cl,H,H], [B36;G1,Me,H,Cl,H], [B37;G1,Me,H,H,Cl], [B38;G1,Me,Me,H,H], [B39;G1,Me,H,Me,H], [B40;G1,Me,H,H,Me], [B41;G1,H,F,F,H], [B42;G1,H,Cl,F,H], [B43;G1,H,Me,F,H], [B44;G1,H,F,C1,H], [B45;G1,H,Cl,Cl,H], [B46;G1,H,Me,Cl,H], [B47;G1,H,F,Me,H], [B48;G1,H,Cl,Me,H], [B49;G1,H,Me,Me,H], [B50;G2,H,H,H,H], [B51;G2,F,H,H,H], [B52;G2,H,F,H,H], [B53;G2,H,H,F,H], [B54;G2,H,H,H,F], [B55;G2,Cl,H,H,H], [B56;G2,H,Cl,H,H], [B57;G2,H,H,Cl,H], [B58;G2,H,H,H,Cl], [B59;G2,Me,H,H,H], [B60;G2,H,Me,H,H], [B61;G2,H,H,Me,H], [B62;G2,H,H,H,Me], [B63;G2,F,F,H,H], [B64;G2,F,H,F,H], [B65;G2,F,H,H,F], [B66;G2,F,Cl,H,H], [B67;G2,F,H,Cl,H], [B68;G2,F,H,H,Cl], [B69;G2,F,Me,H,H], [B70;G2,F,H,Me,H], [B71;G2,F,H,H,Me], [B72;G2,Cl,F,H,H], [B73;G2,Cl,H,F,H], [B74;G2,Cl,H,H,F], [B75;G2,Cl,Cl,H,H], [B76;G2,Cl,H,Cl,H], [B77;G2,Cl,H,H,Cl], [B78;G2,Cl,Me,H,H], [B79;G2,Cl,H,Me,H], [B80;G2,Cl,H,H,Me], [B81;G2,Me,F,H,H], [B82;G2,Me,H,F,H], [B83;G2,Me,H,H,F], [B84;G2,Me,Cl,H,H], [B85;G2,Me,H,Cl,H], [B86;G2,Me,H,H,Cl], [B87;G2,Me,Me,H,H], [B88;G2,Me,H,Me,H], [B89;G2,Me,H,H,Me], [B90;G2,H,F,F,H], [B91;G2,H,Cl,F,H], [B92;G2,H,Me,F,H], [B93;G2,H,F,C1,H], [B94;G2,H,Cl,Cl,H], [B95;G2,H,Me,Cl,H], [B96;G2,H,F,Me,H], [B97;G2,H,Cl,Me,H], [B98;G2,H,Me,Me,H], [B99;G3,H,H,H,H], [B100;G3,F,H,H,H], [B101;G3,H,F,H,H], [B102;G3,H,H,F,H], [B103;G3,H,H,H,F], [B104;G3,Cl,H,H,H], [B105;G3,H,Cl,H,H], [B106;G3,H,H,Cl,H], [B107;G3,H,H,H,Cl], [B108;G3,Me,H,H,H], [B109;G3,H,Me,H,H], [B110;G3,H,H,Me,H], [B111;G3,H,H,H,Me], [B112;G3,F,F,H,H], [B113;G3,F,H,F,H], [B114;G3,F,H,H,F], [B115;G3,F,Cl,H,H], [B116;G3,F,H,Cl,H], [B117;G3,F,H,H,Cl], [B118;G3,F,Me,H,H], [B119;G3,F,H,Me,H], [B120;G3,F,H,H,Me], [B121;G3,Cl,F,H,H], [B122;G3,Cl,H,F,H], [B123;G3,Cl,H,H,F], [B124;G3,Cl,Cl,H,H], [B125;G3,Cl,H,Cl,H], [B126;G3,Cl,H,H,Cl], [B127;G3,Cl,Me,H,H], [B128;G3,Cl,H,Me,H], [B129;G3,Cl,H,H,Me], [B130;G3,Me,F,H,H], [B131;G3,Me,H,F,H], [B132;G3,Me,H,H,F], [B133;G3,Me,Cl,H,H], [B134;G3,Me,H,Cl,H], [B135;G3,Me,H,H,Cl], [B136;G3,Me,Me,H,H], [B137;G3,Me,H,Me,H], [B138;G3,Me,H,H,Me], [B139;G3,H,F,F,H], [B140;G3,H,Cl,F,H], [B141;G3,H,Me,F,H], [B142;G3,H,F,Cl,H], [B143;G3,H,Cl,Cl,H], [B144;G3,H,Me,Cl,H], [B145;G3,H,F,Me,H], [B146;G3,H,Cl,Me,H], [B147;G3,H,Me,Me,H], [B148;G4,H,H,H,-], [B149;G4,F,H,H,-], [B150;G4,H,F,H,-], [B151;G4,H,H,F,-], [B152;G4,Cl,H,H,-], [B153;G4,H,Cl,H,-], [B154;G4,H,H,C1,-], [B155;G4,Me,H,H,-], [B156;G4,H,Me,H,-], [B157;G4,H,H,Me,-], [B158;G4,F,F,H,-], [B159;G4,F,H,F,-], [B160;G4,F,Cl,H,-], [B161;G4,F,H,Cl,-], [B162;G4,F,Me,H,-], [B163;G4,F,H,Me,-], [B164;G4,Cl,F,H,-], [B165;G4,Cl,H,F,-], [B166;G4,Cl,Cl,H,-], [B167;G4,Cl,H,Cl,-], [B168;G4,Cl,Me,H,-], [B169;G4,Cl,H,Me,-], [B170;G4,Me,F,H,-], [B171;G4,Me,H,F,-], [B172;G4,Me,Cl,H,-], [B173;G4,Me,H,Cl,-], [B174;G4,Me,Me,H,-], [B175;G4,Me,H,Me,-], [B176;G4,H,F,F,-], [B177;G4,H,Cl,F,-], [B178;G4,H,Me,F,-], [B179;G4,H,F,Cl,-], [B180;G4,H,Cl,Cl,-], [B181;G4,H,Me,Cl,-], [B182;G4,H,F,Me,-], [B183;G4,H,Cl,Me,-], [B184;G4,H,Me,Me,-], [B185;G5,H,H,H,-], [B186;GS,F,H,H,-], [B187;G5,H,F,H,-], [B188;G5,H,H,F,-], [B189;G5,Cl,H,H,-], [B190;G5,H,C1,H,-], [B191;G5,H,H,Cl,-], [B192;G5,Me,H,H,-], [B193;G5,H,Me,H,-], [B194;G5,H,H,Me,-], [B19S;G5,F,F,H,-], [B196;G5,F,H,F,-], [B197;G5,F,Cl,H,-], [B198;G5,F,H,Cl,-], [B199;G5,F,Me,H,-], [B200;G5,F,H,Me,-], [B201;G5,Cl,F,H,-], [B202;G5,Cl,H,F,-], [B203;G5,Cl,Cl,H,-], [B204;G5,Cl,H,Cl,-], [B205;G5,Cl,Me,H,-], [B206;G5,Cl,H,Me,-], [B207;G5,Me,F,H,-], [B208;G5,Me,H,F,-], [B209;G5,Me,Cl,H,-], [B210;G5,Me,H,Cl,-], [B211;G5,Me,Me,H,-], [B212;G5,Me,H,Me,-], [B213;G5,H,F,F,-], [B214;G5,H,C1,F,-], [B215;G5,H,Me,F,-], [B216;G5,H,F,Cl,-], [B217;G5,H,Cl,Cl,-], [B218;G5,H,Me,Cl,-], [B219;G5,H,F,Me,-], [B220;G5,H,Cl,Me,-], [B221;G5,H,Me,Me,-], [B222;G6,H,H,H,-], [B223;G6,F,H,H,-], [B224;G6,H,F,H,-], [B225;G6,H,H,F,-], [B226;G6,Cl,H,H,-], [B227;G6,H,Cl,H,-], [B228;G6,H,H,Cl,-], [B229;G6,Me,H,H,-], [B230;G6,H,Me,H,-], [B231;G6,H,H,Me,-], [B232;G6,F,F,H,-], [B233;G6,F,H,F,-], [B234;G6,F,Cl,H,-], [B235;G6,F,H,Cl,-], [B236;G6,F,Me,H,-], [B237;G6,F,H,Me,-], [B238;G6,Cl,F,H,-], [B239;G6,Cl,H,F,-], [B240;G6,Cl,Cl,H,-], [B241;G6,Cl,H,Cl,-], [B242;G6,Cl,Me,H,-], [B243;G6,Cl,H,Me,-], [B244;G6,Me,F,H,-], [B245;G6,Me,H,F,-], [B246;G6,Me,Cl,H,-], [B247;G6,Me,H,Cl,-], [B248;G6,Me,Me,H,-], [B249;G6,Me,H,Me,-], [B250;G6,H,F,F,-], [B251;G6,H,Cl,F,-], [B252;G6,H,Me,F,-], [B253;G6,H,F,Cl,-], [B254;G6,H,Cl,Cl,-], [B255;G6,H,Me,Cl,-], [B256;G6,H,F,Me,-], [B257;G6,H,C1,Me,-], [B258;G6,H,Me,Me,-], [B259;G7,H,H,H,-], [B260;G7,F,H,H,-], [B261;G7,H,F,H,-], [B262;G7,H,H,F,-], [B263;G7,Cl,H,H,-], [B264;G7,H,Cl,H,-], [B265;G7,H,H,Cl,-], [B266;G7,Me,H,H,-], [B267;G7,H,Me,H,-], [B268;G7,H,H,Me,-], [B269;G7,F,F,H,-], [B270;G7,F,H,F,-], [B271;G7,F,Cl,H,-], [B272;G7,F,H,Cl,-], [B273;G7,F,Me,H,-], [B274;G7,F,H,Me,-], [B275;G7,Cl,F,H,-], [B276;G7,Cl,H,F,-], [B277;G7,Cl,Cl,H,-], [B278;G7,Cl,H,Cl,-], [B279;G7,Cl,Me,H,-], [B280;G7,Cl,H,Me,-], [B281;G7,Me,F,H,-], [B282;G7,Me,H,F,-], [B283;G7,Me,Cl,H,-], [B284;G7,Me,H,Cl,-], [B285;G7,Me,Me,H,-], [B286;G7,Me,H,Me,-], [B287;G7,H,F,F,-], [B288;G7,H,Cl,F,-], [B289;G7,H,Me,F,-], [B290;G7,H,F,Cl,-], [B291;G7,H,Cl,Cl,-], [B292;G7,H,Me,Cl,-], [B293;G7,H,F,Me,-], [B294;G7,H,Cl,Me,-], [B295;G7,H,Me,Me,-], [B296;G8,H,H,H,-], [B297;G8,F,H,H,-], [B298;G8,H,F,H,-], [B299;G8,H,H,F,-], [B300;G8,Cl,H,H,-], [B301;G8,H,Cl,H,-], [B302;G8,H,H,Cl,-], [B303;G8,Me,H,H,-], [B304;G8,H,Me,H,-], [B305;G8,H,H,Me,-], [B306;G8,F,F,H,-], [B307;G8,F,H,F,-], [B308;G8,F,Cl,H,-], [B309;G8,F,H,Cl,-], [B310;G8,F,Me,H,-], [B311;G8,F,H,Me,-], [B312;G8,Cl,F,H,-], [B313;G8,Cl,H,F,-], [B314;G8,Cl,Cl,H,-], [B315;G8,Cl,H,Cl,-], [B316;G8,Cl,Me,H,-], [B317;G8,Cl,H,Me,-], [B318;G8,Me,F,H,-], [B319;G8,Me,H,F,-], [B320;G8,Me,Cl,H,-], [B321;G8,Me,H,Cl,-], [B322;G8,Me,Me,H,-], [B323;G8,Me,H,Me,-], [B324;G8,H,F,F,-], [B325;G8,H,C1,F,-], [B326;G8,H,Me,F,-], [B327;G8,H,F,Cl,-], [B328;G8,H,Cl,Cl,-], [B329;G8,H,Me,Cl,-], [B330;G8,H,F,Me,-], [B331;G8,H,C1,Me,-], [B332;G8,H,Me,Me,-], [B333;G9,H,H,H,-], [B334;G9,F,H,H,-], [B335;G9,H,F,H,-], [B336;G9,H,H,F,-], [B337;G9,Cl,H,H,-], [B338;G9,H,Cl,H,-], [B339;G9,H,H,C1,-], [B340;G9,Me,H,H,-], [B341;G9,H,Me,H,-], [B342;G9,H,H,Me,-], [B343;G9,F,F,H,-], [B344;G9,F,H,F,-], [B345;G9,F,Cl,H,-], [B346;G9,F,H,Cl,-], [B347;G9,F,Me,H,-], [B348;G9,F,H,Me,-], [B349;G9,Cl,F,H,-], [B350;G9,Cl,H,F,-], [B351;G9,Cl,Cl,H,-], [B352;G9,Cl,H,Cl,-], [B353;G9,Cl,Me,H,-], [B354;G9,Cl,H,Me,-], [B355;G9,Me,F,H,-], [B356;G9,Me,H,F,-], [B357;G9,Me,Cl,H,-], [B358;G9,Me,H,Cl,-], [B359;G9,Me,Me,H,-], [B360;G9,Me,H,Me,-], [B361;G9,H,F,F,-], [B362;G9,H,Cl,F,-], [B363;G9,H,Me,F,-], [B364;G9,H,F,Cl,-], [B365;G9,H,Cl,Cl,-], [B366;G9,H,Me,Cl,-], [B367;G9,H,F,Me,-], [B368;G9,H,Cl,Me,-], [B369;G9,H,Me,Me,-], [B370;G10,H,H,H,-], [B371;G10,F,H,H,-], [B372;G10,H,F,H,-], [B373;G10,H,H,F,-], [B374;G10,Cl,H,H,-], [B375;G10,H,Cl,H,-], [B376;G10,H,H,Cl,-], [B377;G10,Me,H,H,-], [B378;G10,H,Me,H,-], [B379;G10,H,H,Me,-], [B380;G10,F,F,H,-], [B381;G10,F,H,F,-], [B382;G10,F,Cl,H,-], [B383;G10,F,H,Cl,-], [B384;G10,F,Me,H,-], [B385;G10,F,H,Me,-], [B386;G10,Cl,F,H,-], [B387;G10,Cl,H,F,-], [B388;G10,Cl,Cl,H,-], [B389;G10,Cl,H,Cl,-], [B390;G10,Cl,Me,H,-], [B391;G10,Cl,H,Me,-], [B392;G10,Me,F,H,-], [B393;G10,Me,H,F,-], [B394;G10,Me,Cl,H,-], [B395;G10,Me,H,Cl,-], [B396;G10,Me,Me,H,-], [B397;G10,Me,H,Me,-], [B398;G10,H,F,F,-], [B399;G10,H,Cl,F,-], [B400;G10,H,Me,F,-], [B401;G10,H,F,Cl,-], [B402;G10,H,Cl,Cl,-], [B403;G10,H,Me,Cl,-], [B404;G10,H,F,Me,-], [B405;G10,H,Cl,Me,-], [B406;G10,H,Me,Me,-], [B407;G11,H,H,H,-], [B408;G11,F,H,H,-], [B409;G11,H,F,H,-], [B410;G11,H,H,F,-], [B411;G11,Cl,H,H,-], [B412;G11,H,Cl,H,-], [B413;G11,H,H,Cl,-], [B414;G11,Me,H,H,-], [B415;G11,H,Me,H,-], [B416;G11,H,H,Me,-], [B417;G11,F,F,H,-], [B418;G11,F,H,F,-], [B419;G11,F,Cl,H,-], [B420;G11,F,H,Cl,-], [B421;G11,F,Me,H,-], [B422;G11,F,H,Me,-], [B423;G11,Cl,F,H,-], [B424;G11,Cl,H,F,-], [B425;G11,Cl,Cl,H,-], [B426;G11,Cl,H,Cl,-], [B427;G11,Cl,Me,H,-], [B428;G11,Cl,H,Me,-], [B429;G11,Me,F,H,-], [B430;G11,Me,H,F,-], [B431;G11,Me,Cl,H,-], [B432;G11,Me,H,Cl,-], [B433;G11,Me,Me,H,-], [B434;G11,Me,H,Me,-], [B435;G11,H,F,F,-], [B436;G11,H,Cl,F,-], [B437;G11,H,Me,F,-], [B438;G11,H,F,Cl,-], [B439;G11,H,Cl,Cl,-], [B440;G11,H,Me,Cl,-], [B441;G11,H,F,Me,-], [B442;G11,H,Cl,Me,-], [B443;G11,H,Me,Me,-], [B444;G12,H,H,H,-], [B445;G12,F,H,H,-], [B446;G12,H,F,H,-], [B447;G12,H,H,F,-], [B448;G12,Cl,H,H,-], [B449;G12,H,Cl,H,-], [B450;G12,H,H,Cl,-], [B451;G12,Me,H,H,-], [B452;G12,H,Me,H,-], [B453;G12,H,H,Me,-], [B454;G12,F,F,H,-], [B455;G12,F,H,F,-], [B456;G12,F,Cl,H,-], [B457;G12,F,H,C1,-], [B458;G12,F,Me,H,-], [B459;G12,F,H,Me,-], [B460;G12,Cl,F,H,-], [B461;G12,Cl,H,F,-], [B462;G12,Cl,Cl,H,-], [B463;G12,Cl,H,Cl,-], [B464;G12,Cl,Me,H,-], [B465;G12,Cl,H,Me,-], [B466;G12,Me,F,H,-], [B467;G12,Me,H,F,-], [B468;G12,Me,Cl,H,-], [B469;G12,Me,H,Cl,-], [B470;G12,Me,Me,H,-], [B471;G12,Me,H,Me,-], [B472;G12,H,F,F,-], [B473;G12,H,Cl,F,-], [B474;G12,H,Me,F,-], [B475;G12,H,F,Cl,-], [B476;G12,H,Cl,Cl,-], [B477;G12,H,Me,Cl,-], [B478;G12,H,F,Me,-], [B479;G12,H,Cl,Me,-], [B480;G12,H,Me,Me,-], [B481;G13,H,H,H,-], [B482;G13,F,H,H,-], [B483;G13,H,F,H,-], [B484;G13,H,H,F,-], [B485;G13,Cl,H,H,-], [B486;G13,H,Cl,H,-], [B487;G13,H,H,Cl,-], [B488;G13,Me,H,H,-], [B489;G13,H,Me,H,-], [B490;G13,H,H,Me,-], [B491;G13,F,F,H,-], [B492;G13,F,H,F,-], [B493;G13,F,Cl,H,-], [B494;G13,F,H,Cl,-], [B495;G13,F,Me,H,-], [B496;G13,F,H,Me,-], [B497;G13,Cl,F,H,-], [B498;G13,Cl,H,F,-], [B499;G13,Cl,Cl,H,-], [B500;G13,Cl,H,Cl,-], [B501;G13,Cl,Me,H,-], [B502;G13,Cl,H,Me,-], [B503;G13,Me,F,H,-], [B504;G13,Me,H,F,-], [B505;G13,Me,Cl,H,-], [B506;G13,Me,H,Cl,-], [B507;G13,Me,Me,H,-], [B508;G13,Me,H,Me,-], [B509;G13,H,F,F,-], [B510;G13,H,Cl,F,-], [B511;G13,H,Me,F,-], [B512;G13,H,F,C1,-], [B513;G13,H,Cl,Cl,-], [B514;G13,H,Me,Cl,-], [B515;G13,H,F,Me,-], [B516;G13,H,Cl,Me,-], [B517;G13,H,Me,Me,-], [B518;G14,Me,H,H,-], [B519;G14,Me,F,H,-], [B520;G14,Me,H,F,-], [B521;G14,Me,Cl,H,-], [B522;G14,Me,H,Cl,-], [B523;G14,Me,Me,H,-], [B524;G14,Me,H,Me,-], [B525;G14,Me,F,F,-], [B526;G14,Me,F,Cl,-], [B527;G14,Me,F,Me,-], [B528;G14,Me,Cl,F,-], [B529;G14,Me,Cl,Cl,-], [B530;G14,Me,Cl,Me,-], [B531;G14,Me,Me,F,-], [B532;G14,Me,Me,Cl,-], [B533;G14,Me,Me,Me,-], [B534;G15,Me,H,H,-], [B535;G15,Me,F,H,-], [B536;G15,Me,H,F,-], [B537;G15,Me,Cl,H,-], [B538;G15,Me,H,Cl,-], [B539;G15,Me,Me,H,-], [B540;G15,Me,H,Me,-], [B541;G15,Me,F,F,-], [B542;G15,Me,F,Cl,-], [B543;G15,Me,F,Me,-], [B544;G15,Me,Cl,F,-], [B545;G15,Me,Cl,Cl,-], [B546;G15,Me,Cl,Me,-], [B547;G15,Me,Me,F,-], [B548;G15,Me,Me,Cl,-], [B549;G15,Me,Me,Me,-], [B550;G16,Me,H,H,-], [B551;G16,Me,F,H,-], [B552;G16,Me,H,F,-], [B553;G16,Me,Cl,H,-], [B554;G16,Me,H,Cl,-], [B555;G16,Me,Me,H,-], [B556;G16,Me,H,Me,-], [B557;G16,Me,F,F,-], [B558;G16,Me,F,Cl,-], [B559;G16,Me,F,Me,-], [B560;G16,Me,Cl,F,-], [B561;G16,Me,Cl,Cl,-], [B562;G16,Me,Cl,Me,-], [B563;G16,Me,Me,F,-], [B564;G16,Me,Me,Cl,-], [B565;G16,Me,Me,Me,-], [B566;G17,Me,H,H,-], [B567;G17,Me,F,H,-], [B568;G17,Me,H,F,-], [B569;G17,Me,Cl,H,-], [B570;G17,Me,H,Cl,-], [B571;G17,Me,Me,H,-], [B572;G17,Me,H,Me,-], [B573;G17,Me,F,F,-], [B574;G17,Me,F,Cl,-], [B575;G17,Me,F,Me,-], [B576;G17,Me,Cl,F,-], [B577;G17,Me,Cl,Cl,-], [B578;G17,Me,Cl,Me,-], [B579;G17,Me,Me,F,-], [B580;G17,Me,Me,Cl,-], [B581;G17,Me,Me,Me,-], [B582;G18,Me,H,H,-], [B583;G18,Me,F,H,-], [B584;G18,Me,H,F,-], [B585;G18,Me,Cl,H,-], [B586;G18,Me,H,Cl,-], [B587;G18,Me,Me,H,-], [B588;G18,Me,H,Me,-], [B589;G18,Me,F,F,-], [B590;G18,Me,F,Cl,-], [B591;G18,Me,F,Me,-], [B592;G18,Me,Cl,F,-], [B593;G18,Me,Cl,Cl,-], [B594;G18,Me,Cl,Me,-], [B595;G18,Me,Me,F,-], [B596;G18,Me,Me,Cl,-], [B597;G18,Me,Me,Me,-], [B598;G19,H,H,-,-], [B599;G19,F,H,-,-], [B600;G19,H,F,-,-], [B601;G19,Cl,H,-,-], [B602;G19,H,Cl,-,-], [B603;G19,Me,H,-,-], [B604;G19,H,Me,-,-], [B605;G19,F,F,-,-], [B606;G19,F,Cl,-,-], [B607;G19,F,Me,-,-], [B608;G19,Cl,F,-,-], [B609;G19,Cl,Cl,-,-], [B610;G19,Cl,Me,-,-], [B611;G19,Me,F,-,-], [B612;G19,Me,Cl,-,-], [B613;G19,Me,Me,-,-], [B614;G20,H,H,-,-], [B615;G20,F,H,-,-], [B616;G20,H,F,-,-], [B617;G20,Cl,H,-,-], [B618;G20,H,Cl,-,-], [B619;G20,Me,H,-,-], [B620;G20,H,Me,-,-], [B621;G20,F,F,-,-], [B622;G20,F,Cl,-,-], [B623;G20,F,Me,-,-], [B624;G20,Cl,F,-,-], [B625;G20,Cl,Cl,-,-], [B626;G20,Cl,Me,-,-], [B627;G20,Me,F,-,-], [B628;G20,Me,Cl,-,-], [B629;G20,Me,Me,-,-], [B630;G21,H,H,-,-], [B631;G21,F,H,-,-], [B632;G21,H,F,-,-], [B633;G21,Cl,H,-,-], [B634;G21,H,Cl,-,-], [B635;G21,Me,H,-,-], [B636;G21,H,Me,-,-], [B637;G21,F,F,-,-], [B638;G21,F,Cl,-,-], [B639;G21,F,Me,-,-], [B640;G21,Cl,F,-,-], [B641;G21,Cl,Cl,-,-], [B642;G21,Cl,Me,-,-], [B643;G21,Me,F,-,-], [B644;G21,Me,Cl,-,-], [B645;G21,Me,Me,-,-], [B646;G22,H,H,-,-], [B647;G22,F,H,-,-], [B648;G22,H,F,-,-], [B649;G22,Cl,H,-,-], [B650;G22,H,Cl,-,-], [B651;G22,Me,H,-,-], [B652;G22,H,Me,-,-], [B653;G22,F,F,-,-], [B654;G22,F,Cl,-,-], [B655;G22,F,Me,-,-], [B656;G22,Cl,F,-,-], [B657;G22,Cl,Cl,-,-], [B658;G22,Cl,Me,-,-], [B659;G22,Me,F,-,-], [B660;G22,Me,Cl,-,-], [B661;G22,Me,Me,-,-], [B662;G23,H,H,-,-], [B663;G23,F,H,-,-], [B664;G23,H,F,-,-], [B665;G23,Cl,H,-,-], [B666;G23,H,Cl,-,-], [B667;G23,Me,H,-,-], [B668;G23,H,Me,-,-], [B669;G23,F,F,-,-], [B670;G23,F,Cl,-,-], [B671;G23,F,Me,-,-], [B672;G23,Cl,F,-,-], [B673;G23,Cl,Cl,-,-], [B674;G23,Cl,Me,-,-], [B675;G23,Me,F,-,-], [B676;G23,Me,Cl,-,-], [B677;G23,Me,Me,-,-], [B678;G24,H,H,-,-], [B679;G24,F,H,-,-], [B680;G24,H,F,-,-], [B681;G24,Cl,H,-,-], [B682;G24,H,Cl,-,-], [B683;G24,Me,H,-,-], [B684;G24,H,Me,-,-], [B685;G24,F,F,-,-], [B686;G24,F,Cl,-,-], [B687;G24,F,Me,-,-], [B688;G24,Cl,F,-,-], [B689;G24,Cl,Cl,-,-], [B690;G24,Cl,Me,-,-], [B691;G24,Me,F,-,-], [B692;G24,Me,Cl,-,-], [B693;G24,Me,Me,-,-], [B694;G25,H,H,-,-], [B695;G25,F,H,-,-], [B696;G25,H,F,-,-], [B697;G25,Cl,H,-,-], [B698;G25,H,Cl,-,-], [B699;G25,Me,H,-,-], [B700;G25,H,Me,-,-], [B701;G25,F,F,-,-], [B702;G25,F,Cl,-,-], [B703;G25,F,Me,-,-], [B704;G25,Cl,F,-,-], [B705;G25,Cl,Cl,-,-], [B706;G25,Cl,Me,-,-], [B707;G25,Me,F,-,-], [B708;G25,Me,Cl,-,-], [B709;G25,Me,Me,-,-], [B710;G26,H,H,-,-], [B711;G26,F,H,-,-], [B712;G26,H,F,-,-], [B713;G26,Cl,H,-,-], [B714;G26,H,Cl,-,-], [B715;G26,Me,H,-,-], [B716;G26,H,Me,-,-], [B717;G26,F,F,-,-], [B718;G26,F,Cl,-,-], [B719;G26,F,Me,-,-], [B720;G26,Cl,F,-,-], [B721;G26,Cl,Cl,-,-], [B722;G26,Cl,Me,-,-], [B723;G26,Me,F,-,-], [B724;G26,Me,Cl,-,-], [B725;G26,Me,Me,-,-], [B726;G27,H,H,-,-], [B727;G27,F,H,-,-], [B728;G27,H,F,-,-], [B729;G27,Cl,H,-,-], [B730;G27,H,Cl,-,-], [B731;G27,Me,H,-,-], [B732;G27,H,Me,-,-], [B733;G27,F,F,-,-], [B734;G27,F,Cl,-,-], [B735;G27,F,Me,-,-], [B736;G27,Cl,F,-,-], [B737;G27,Cl,Cl,-,-], [B738;G27,Cl,Me,-,-], [B739;G27,Me,F,-,-], [B740;G27,Me,Cl,-,-], [B741;G27,Me,Me,-,-], [B742;G28,H,H,-,-], [B743;G28,F,H,-,-], [B744;G28,H,F,-,-], [B745;G28,Cl,H,-,-], [B746;G28,H,Cl,-,-], [B747;G28,Me,H,-,-], [B748;G28,H,Me,-,-], [B749;G28,F,F,-,-], [B750;G28,F,Cl,-,-], [B751;G28,F,Me,-,-], [B752;G28,Cl,F,-,-], [B753;G28,Cl,Cl,-,-], [B754;G28,Cl,Me,-,-], [B755;G28,Me,F,-,-], [B756;G28,Me,Cl,-,-], [B757;G28,Me,Me,-,-], [B758;G29,H,H,-,-], [B759;G29,F,H,-,-], [B760;G29,H,F,-,-], [B761;G29,Cl,H,-,-], [B762;G29,H,Cl,-,-], [B763;G29,Me,H,-,-], [B764;G29,H,Me,-,-], [B765;G29,F,F,-,-], [B766;G29,F,Cl,-,-], [B767;G29,F,Me,-,-], [B768;G29,Cl,F,-,-], [B769;G29,Cl,Cl,-,-], [B770;G29,Cl,Me,-,-], [B771;G29,Me,F,-,-], [B772;G29,Me,Cl,-,-], [B773;G29,Me,Me,-,-], [B774;G30,H,H,-,-], [B775;G30,F,H,-,-], [B776;G30,H,F,-,-], [B777;G30,Cl,H,-,-], [B778;G30,H,Cl,-,-], [B779;G30,Me,H,-,-], [B780;G30,H,Me,-,-], [B781;G30,F,F,-,-], [B782;G30,F,Cl,-,-], [B783;G30,F,Me,-,-], [B784;G30,Cl,F,-,-], [B785;G30,Cl,Cl,-,-], [B786;G30,Cl,Me,-,-], [B787;G30,Me,F,-,-], [B788;G30,Me,Cl,-,-], [B789;G30,Me,Me,-,-], [B790;G31,H,-,-,-], [B791;G31,F,-,-,-], [B792;G31,Cl,-,-,-], [B793;G31,Me,-,-,-], [B794;G31,OMe,-,-,-], [B795;G32,H,-,-,-], [B796;G32,F,-,-,-], [B797;G32,Cl,-,-,-], [B798;G32,Me,-,-,-], [B799;G32,OMe,-,-,-], [B800;G33,H,-,-,-], [B801;G33,F,-,-,-], [B802;G33,Cl,-,-,-], [B803;G33,Me,-,-,-], [B804;G33,OMe,-,-,-], [B805;G34,H,-,-,-], [B806;G34,F,-,-,-], [B807;G34,Cl,-,-,-], [B808;G34,Me,-,-,-], [B809;G34,OMe,-,-,-], [B810;G35,H,-,-,-], [B811;G35,F,-,-,-], [B812;G35,Cl,-,-,-], [B813;G35,Me,-,-,-], [B814;G35,OMe,-,-,-], [B815;G36,H,-,-,-], [B816;G36,F,-,-,-], [B817;G36,Cl,-,-,-], [B818;G36,Me,-,-,-], [B819;G36,OMe,-,-,-], [B820;G41,-,-,-,-], [B821;G42,-,-,-,-], [B822;G43,-,-,-,-], [B823;G44,-,-,-,-], [B824;G45,-,-,-,-], [B825;G46,-,-,-,-], [B826;G47,-,-,-,-], [B827;G48,-,-,-,-], [B828;G49,-,-,-,-], [B829;G50,-,-,-,-], [B830;G51,-,-,-,-], [B831;G52,-,-,-,-], [B832;G53,-,-,-,-], [B833;G54,-,-,-,-], [B834;G55,-,-,-,-], [B835;G56,-,-,-,-], [B836;G57,-,-,-,-], [B837;G58,-,-,-,-], [B838;G59,-,-,-,-], [B839;G60,-,-,-,-], [B840;G61,-,-,-,-], [B841;G62,-,-,-,-], [B842;G63,-,-,-,-], [B843;G64,-,-,-,-], [B844;G65,-,-,-,-], [B845;G66,-,-,-,-], [B846;G67,-,-,-,-], [B847;G68,-,-,-,-], [B848;G69,-,-,-,-], [B849;G70,-,-,-,-], [B850;G71,-,-,-,-], [B851;G72,-,-,-,-], [B852;G73,-,-,-,-], [B853;G74,-,-,-,-]

The Compound (A-2) wherein n represents 1, Z represents a propyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX19").

The Compound (A-2) wherein n represents 1, Z represents an isopropyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX20").

The Compound (A-2) wherein n represents 1, Z represents -CH=CHCH₃, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX21").

The Compound (A-2) wherein n represents 1, Z represents -CH₂CH=CH₂, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX22").

The Compound (A-2) wherein n represents 1, Z represents a butyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX23").

The Compound (A-2) wherein n represents 1, Z represents a cyclopropyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX24").

The Compound (A-2) wherein n represents 1, Z represents a 2-thienyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX25").

The Compound (A-2) wherein n represents 1, Z represents a 3-thienyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX26").

The Compound (A-2) wherein n represents 1, Z represents a 2-furanyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX27").

The Compound (A-2) wherein n represents 1, Z represents a 3-furanyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX28").

The Compound (A-2) wherein n represents 1, Z represents a 2-pyridyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX29").

The Compound (A-2) wherein n represents 1, Z represents a 3-pyridyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX30").

The Compound (A-2) wherein n represents 1, Z represents a 4-pyridyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX31").

The Compound (A-2) wherein n represents 1, Z represents a 2-pyrimidyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX32").

The Compound (A-2) wherein n represents 1, Z represents a 4-pyrimidyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX33").

The Compound (A-2) wherein n represents 1, Z represents a 2-pyrazinyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX34").

The Compound (A-2) wherein n represents 2, Z represents an ethyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX35").

The Compound (A-2) wherein n represents 2, Z represents a propyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX36").

The Compound (A-2) wherein n represents 2, Z represents an isopropyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX37").

The Compound (A-2) wherein n represents 2, Z represents -CH=CHCH₃, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX38").

The Compound (A-2) wherein n represents 2, Z represents -CH₂CH=CH₂, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX39").

The Compound (A-2) wherein n represents 2, Z represents a butyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX40").

The Compound (A-2) wherein n represents 2, Z represents a cyclopropyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX41").

The Compound (A-2) wherein n represents 2, Z represents a 2-thienyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX42").

The Compound (A-2) wherein n represents 2, Z represents a 3-thienyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX43").

The Compound (A-2) wherein n represents 2, Z represents a 2-furanyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX44").

The Compound (A-2) wherein n represents 2, Z represents a 3-furanyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX45").

The Compound (A-2) wherein n represents 2, Z represents a 2-pyridyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX46").

The Compound (A-2) wherein n represents 2, Z represents a 3-pyridyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX47").

The Compound (A-2) wherein n represents 2, Z represents a 4-pyridyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX48").

The Compound (A-2) wherein n represents 2, Z represents a 2-pyrimidyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX49").

The Compound (A-2) wherein n represents 2, Z represents a 4-pyrimidyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX50").

The Compound (A-2) wherein n represents 2, Z represents a 2-pyrazinyl group, and the combination of the group represented by G^{B} and the substituents R^{X7}, R^{X8}, R^{X9}, and R^{X10} in the group represented by G^{B} represents any one combination indicated in the Combination B (hereinafter referred to as "Compound group SX51").

A compound represented by formula (A-3) 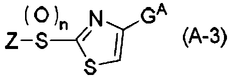 (hereinafter referred to as "Compound (A-3)") wherein n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX52").

The Compound (A-3) wherein n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX53").

The Compound (A-3) wherein n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX54").

The Compound (A-3) wherein n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX55").

The Compound (A-3) wherein n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX56").

The Compound (A-3) wherein n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX57").

The Compound (A-3) wherein n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX58").

The Compound (A-3) wherein n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX59").

The Compound (A-3) wherein n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX60").

The Compound (A-3) wherein n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX61").

The Compound (A-3) wherein n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX62").

The Compound (A-3) wherein n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX63").

The Compound (A-3) wherein n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX64").

The Compound (A-3) wherein n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX65").

The Compound (A-3) wherein n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX66").

The Compound (A-3) wherein n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX67").

The Compound (A-3) wherein n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX68").

The Compound (A-3) wherein n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX69").

The Compound (A-3) wherein n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX70").

The Compound (A-3) wherein n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX71").

The Compound (A-3) wherein n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX72").

The Compound (A-3) wherein n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX73").

The Compound (A-3) wherein n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX74").

The Compound (A-3) wherein n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX75").

The Compound (A-3) wherein n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX76").

The Compound (A-3) wherein n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX77").

The Compound (A-3) wherein n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX78").

The Compound (A-3) wherein n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX79").

The Compound (A-3) wherein n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX80").

The Compound (A-3) wherein n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX81").

The Compound (A-3) wherein n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX82").

The Compound (A-3) wherein n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX83").

The Compound (A-3) wherein n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX84").

The Compound (A-3) wherein n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX85").

A compound represented by formula (A-4) 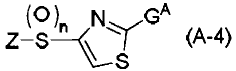 (hereinafter referred to as "Compound (A-4)") wherein n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX86").

The Compound (A-4) wherein n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX87").

The Compound (A-4) wherein n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX88").

The Compound (A-4) wherein n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX89").

The Compound (A-4) wherein n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX90").

The Compound (A-4) wherein n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX91").

The Compound (A-4) wherein n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX92").

The Compound (A-4) wherein n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX93").

The Compound (A-4) wherein n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX94").

The Compound (A-4) wherein n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX95").

The Compound (A-4) wherein n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX96").

The Compound (A-4) wherein n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX97").

The Compound (A-4) wherein n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX98").

The Compound (A-4) wherein n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX99").

The Compound (A-4) wherein n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX100").

The Compound (A-4) wherein n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX101").

The Compound (A-4) wherein n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX102").

The Compound (A-4) wherein n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX103").

The Compound (A-4) wherein n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX104").

The Compound (A-4) wherein n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX105").

The Compound (A-4) wherein n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX106").

The Compound (A-4) wherein n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX107").

The Compound (A-4) wherein n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX108").

The Compound (A-4) wherein n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX109").

The Compound (A-4) wherein n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX110").

The Compound (A-4) wherein n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX111").

The Compound (A-4) wherein n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX112").

The Compound (A-4) wherein n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX113").

The Compound (A-4) wherein n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX114").

The Compound (A-4) wherein n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX115").

The Compound (A-4) wherein n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX116").

The Compound (A-4) wherein n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX117").

The Compound (A-4) wherein n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX118").

The Compound (A-4) wherein n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX119").

A compound represented by formula (A-5) 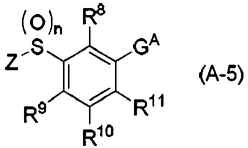 (hereinafter referred to as "Compound (A-5)") wherein n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX120").

The Compound (A-5) wherein R⁸, R⁹, R¹⁰, and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX121").

The Compound (A-5) wherein R⁸, R⁹, R¹⁰, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX122").

The Compound (A-5) wherein R⁸, R⁹, R¹⁰, and R¹¹ each represent a hydrogen atom, n represents 1, Z represents - CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX123").

The Compound (A-5) wherein R⁸, R⁹, R¹⁰, and R¹¹ each represent a hydrogen atom, n represents 1, Z represents - CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX124").

The Compound (A-5) wherein R⁸, R⁹, R¹⁰, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX125").

The Compound (A-5) wherein R⁸, R⁹, R¹⁰, and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX126").

The Compound (A-5) wherein R⁸, R⁹, R¹⁰, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX127").

The Compound (A-5) wherein R⁸, R⁹, R¹⁰, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX128").

The Compound (A-5) wherein R⁸, R⁹, R¹⁰, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX129").

The Compound (A-5) wherein R⁸, R⁹, R¹⁰, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX130").

The Compound (A-5) wherein R⁸, R⁹, R¹⁰, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX131").

The Compound (A-5) wherein R⁸, R⁹, R¹⁰, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX132").

The Compound (A-5) wherein R⁸, R⁹, R¹⁰, and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX133").

The Compound (A-5) wherein R⁸, R⁹, R¹⁰, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX134").

The Compound (A-5) wherein R⁸, R⁹, R¹⁰, and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX135").

The Compound (A-5) wherein R⁸, R⁹, R¹⁰, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX136").

The Compound (A-5) wherein R⁸, R⁹, R¹⁰, and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX137").

The Compound (A-5) wherein R⁸, R⁹, R¹⁰, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX138").

The Compound (A-5) wherein R⁸, R⁹, R¹⁰, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX139").

The Compound (A-5) wherein R⁸, R⁹, R¹⁰, and R¹¹ each represent a hydrogen atom, n represents 2, Z represents - CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX140").

The Compound (A-5) wherein R⁸, R⁹, R¹⁰, and R¹¹ each represent a hydrogen atom, n represents 2, Z represents - CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX141").

The Compound (A-5) wherein R⁸, R⁹, R¹⁰, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX142").

The Compound (A-5) wherein R⁸, R⁹, R¹⁰, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX143").

The Compound (A-5) wherein R⁸, R⁹, R¹⁰, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX144").

The Compound (A-5) wherein R⁸, R⁹, R¹⁰, and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX145").

The Compound (A-5) wherein R⁸, R⁹, R¹⁰, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX146").

The Compound (A-5) wherein R⁸, R⁹, R¹⁰, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX147").

The Compound (A-5) wherein R⁸, R⁹, R¹⁰, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX148").

The Compound (A-5) wherein R⁸, R⁹, R¹⁰, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX149").

The Compound (A-5) wherein R⁸, R⁹, R¹⁰, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX150").

The Compound (A-5) wherein R⁸, R⁹, R¹⁰, and R¹¹ each represent a hydrogen atom, n represents 2, Z represents a 2-pyrimidyl group, and the combination of the group represented by G^{A} and the substituents R^{X7}, R^{X8}, R^{X9}, and ^{R}X¹⁰ in the group represented by G^{A} represents any one combination indicated in the Combination A (hereinafter referred to as "Compound group SX151").

The Compound (A-5) wherein R⁸, R⁹, R¹⁰, and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX152").

The Compound (A-5) wherein R⁸, R⁹, R¹⁰, and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX153")

The Compound (A-5) wherein R⁸ represents a fluorine atom, R⁹, R¹⁰, and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX154").

The Compound (A-5) wherein R⁸ represents a fluorine atom, R⁹, R¹⁰, and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX155").

The Compound (A-5) wherein R⁸ represents a fluorine atom, R⁹, R¹⁰, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX156").

The Compound (A-5) wherein R⁸ represents a fluorine atom, R⁹, R¹⁰, and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX157").

The Compound (A-5) wherein R⁸ represents a fluorine atom, R⁹, R¹⁰, and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX158").

The Compound (A-5) wherein R⁸ represents a fluorine atom, R⁹, R¹⁰, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX159").

The Compound (A-5) wherein R⁸ represents a fluorine atom, R⁹, R¹⁰, and R¹¹ each represent a hydrogen atom, n represents 1, Z represents a cyclopropyl group, and the combination of the group represented by G^{A} and the substituents R^{X7}, R^{XB}, R^{X9}, and R^{X10} in the group represented by G^{A} represents any one combination indicated in the Combination A (hereinafter referred to as "Compound group SX160").

The Compound (A-5) wherein R⁸ represents a fluorine atom, R⁹, R¹⁰, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX161").

The Compound (A-5) wherein R⁸ represents a fluorine atom, R⁹, R¹⁰, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX162").

The Compound (A-5) wherein R⁸ represents a fluorine atom, R⁹, R¹⁰, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX163").

The Compound (A-5) wherein R⁸ represents a fluorine atom, R⁹, R¹⁰, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX164").

The Compound (A-5) wherein R⁸ represents a fluorine atom, R⁹, R¹⁰, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX165").

The Compound (A-5) wherein R⁸ represents a fluorine atom, R⁹, R¹⁰, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX166").

The Compound (A-5) wherein R⁸ represents a fluorine atom, R⁹, R¹⁰, and R¹¹ each represent a hydrogen atom, n represents 1, Z represents a 4-pyridyl group, and the combination of the group represented by G^{A} and the substituents R^{X7} , R^{X8}, R^{X9}, and R^{X10} in the group represented by G^{A} represents any one combination indicated in the Combination A (hereinafter referred to as "Compound group SX167").

The Compound (A-5) wherein R⁸ represents a fluorine atom, R⁹, R¹⁰, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX168").

The Compound (A-5) wherein R⁸ represents a fluorine atom, R⁹, R¹⁰, and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX169").

The Compound (A-5) wherein R⁸ represents a fluorine atom, R⁹, R¹⁰, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX170").

The Compound (A-5) wherein R⁸ represents a fluorine atom, R⁹, R¹⁰, and R¹¹ each represent a hydrogen atom, n represents 2, Z represents an ethyl group, and the combination of the group represented by G^{A} and the substituents R^{X7}, R^{XB}, R^{X9}, and R^{X10} in the group represented by G^{A} represents any one combination indicated in the Combination A (hereinafter referred to as "Compound group SX171").

The Compound (A-5) wherein R⁸ represents a fluorine atom, R⁹, R¹⁰, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX172").

The Compound (A-5) wherein R⁸ represents a fluorine atom, R⁹, R¹⁰, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX173").

The Compound (A-5) wherein R⁸ represents a fluorine atom, R⁹, R¹⁰, and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX174").

The Compound (A-5) wherein R⁸ represents a fluorine atom, R⁹, R¹⁰, and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX175").

The Compound (A-5) wherein R⁸ represents a fluorine atom, R⁹, R¹⁰, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX176").

The Compound (A-5) wherein R⁸ represents a fluorine atom, R⁹, R¹⁰, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX177").

The Compound (A-5) wherein R⁸ represents a fluorine atom, R⁹, R¹⁰, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX178").

The Compound (A-5) wherein R⁸ represents a fluorine atom, R⁹, R¹⁰, and R¹¹ each represent a hydrogen atom, n represents 2, Z represents a 3-thienyl group, and the combination of the group represented by G^{A} and the substituents R^{X7}, R^{XB}, R^{X9}, and R^{X10} in the group represented by G^{A} represents any one combination indicated in the Combination A (hereinafter referred to as "Compound group SX179").

The Compound (A-5) wherein R⁸ represents a fluorine atom, R⁹, R¹⁰, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX180").

The Compound (A-5) wherein R⁸ represents a fluorine atom, R⁹, R¹⁰, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX181").

The Compound (A-5) wherein R⁸ represents a fluorine atom, R⁹, R¹⁰, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX182").

The Compound (A-5) wherein R⁸ represents a fluorine atom, R⁹, R¹⁰, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX183").

The Compound (A-5) wherein R⁸ represents a fluorine atom, R⁹, R¹⁰, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX184").

The Compound (A-5) wherein R⁸ represents a fluorine atom, R⁹, R¹⁰, and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX185").

The Compound (A-5) wherein R⁸ represents a fluorine atom, R⁹, R¹⁰, and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX186").

The Compound (A-5) wherein R⁸ represents a fluorine atom, R⁹, R¹⁰, and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX187").

The Compound (A-5) wherein R⁸ represents a chlorine atom, R⁹, R¹⁰, and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX188").

The Compound (A-5) wherein R⁸ represents a chlorine atom, R⁹, R¹⁰, and R¹¹ each represent a hydrogen atom, n represents 1, Z represents a propyl group, and the combination of the group represented by G^{A} and the substituents R^{X7} , R^{X8}, R^{X9}, and R^{X10} in the group represented by G^{A} represents any one combination indicated in the Combination A (hereinafter referred to as "Compound group SX189").

The Compound (A-5) wherein R⁸ represents a chlorine atom, R⁹, R¹⁰, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX190").

The Compound (A-5) wherein R⁸ represents a chlorine atom, R⁹, R¹⁰, and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX191").

The Compound (A-5) wherein R⁸ represents a chlorine atom, R⁹, R¹⁰, and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX192").

The Compound (A-5) wherein R⁸ represents a chlorine atom, R⁹, R¹⁰, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX193").

The Compound (A-5) wherein R⁸ represents a chlorine atom, R⁹, R¹⁰, and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX194").

The Compound (A-5) wherein R⁸ represents a chlorine atom, R⁹, R¹⁰, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX195").

The Compound (A-5) wherein R⁸ represents a chlorine atom, R⁹, R¹⁰, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX196").

The Compound (A-5) wherein R⁸ represents a chlorine atom, R⁹, R¹⁰, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX197").

The Compound (A-5) wherein R⁸ represents a chlorine atom, R⁹, R¹⁰, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX198").

The Compound (A-5) wherein R⁸ represents a chlorine atom, R⁹, R¹⁰, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX199").

The Compound (A-5) wherein R⁸ represents a chlorine atom, R⁹, R¹⁰, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX200").

The Compound (A-5) wherein R⁸ represents a chlorine atom, R⁹, R¹⁰, and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX201").

The Compound (A-5) wherein R⁸ represents a chlorine atom, R⁹, R¹⁰, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX202").

The Compound (A-5) wherein R⁸ represents a chlorine atom, R⁹, R¹⁰, and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX203").

The Compound (A-5) wherein R⁸ represents a chlorine atom, R⁹, R¹⁰, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX204").

The Compound (A-5) wherein R⁸ represents a chlorine atom, R⁹, R¹⁰, and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX205").

The Compound (A-5) wherein R⁸ represents a chlorine atom, R⁹, R¹⁰, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX206").

The Compound (A-5) wherein R⁸ represents a chlorine atom, R⁹, R¹⁰, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX207").

The Compound (A-5) wherein R⁸ represents a chlorine atom, R⁹, R¹⁰, and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX208").

The Compound (A-5) wherein R⁸ represents a chlorine atom, R⁹, R¹⁰, and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX209").

The Compound (A-5) wherein R⁸ represents a chlorine atom, R⁹, R¹⁰, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX210").

The Compound (A-5) wherein R⁸ represents a chlorine atom, R⁹, R¹⁰, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX211").

The Compound (A-5) wherein R⁸ represents a chlorine atom, R⁹, R¹⁰, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX212").

The Compound (A-5) wherein R⁸ represents a chlorine atom, R⁹, R¹⁰, and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX213").

The Compound (A-5) wherein R⁸ represents a chlorine atom, R⁹, R¹⁰, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX214").

The Compound (A-5) wherein R⁸ represents a chlorine atom, R⁹, R¹⁰, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX215").

The Compound (A-5) wherein R⁸ represents a chlorine atom, R⁹, R¹⁰, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX216").

The Compound (A-5) wherein R⁸ represents a chlorine atom, R⁹, R¹⁰, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX217").

The Compound (A-5) wherein R⁸ represents a chlorine atom, R⁹, R¹⁰, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX218").

The Compound (A-5) wherein R⁸ represents a chlorine atom, R⁹, R¹⁰, and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX219").

The Compound (A-5) wherein R⁸ represents a chlorine atom, R⁹, R¹⁰, and R¹¹ each represent a hydrogen atom, n represents 2, Z represents a 3-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX220").

The Compound (A-5) wherein R⁸ represents a chlorine atom, R⁹, R¹⁰, and R¹¹ each represent a hydrogen atom, n represents 2, Z represents a 4-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX221").

The Compound (A-5) wherein R⁸ represents a methyl group, R⁹, R¹⁰, and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX222").

The Compound (A-5) wherein R⁸ represents a methyl group, R⁹, R¹⁰, and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX223").

The Compound (A-5) wherein R⁸ represents a methyl group, R⁹, R¹⁰, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX224").

The Compound (A-5) wherein R⁸ represents a methyl group, R⁹, R¹⁰, and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX225").

The Compound (A-5) wherein R⁸ represents a methyl group, R⁹, R¹⁰, and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX226").

The Compound (A-5) wherein R⁸ represents a methyl group, R⁹, R¹⁰, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX227").

The Compound (A-5) wherein R⁸ represents a methyl group, R⁹, R¹⁰, and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX228").

The Compound (A-5) wherein R⁸ represents a methyl group, R⁹, R¹⁰, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX229").

The Compound (A-5) wherein R⁸ represents a methyl group, R⁹, R¹⁰, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX230").

The Compound (A-5) wherein R⁸ represents a methyl group, R⁹, R¹⁰, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX231").

The Compound (A-5) wherein R⁸ represents a methyl group, R⁹, R¹⁰, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX232").

The Compound (A-5) wherein R⁸ represents a methyl group, R⁹, R¹⁰, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX233").

The Compound (A-5) wherein R⁸ represents a methyl group, R⁹, R¹⁰, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX234").

The Compound (A-5) wherein R⁸ represents a methyl group, R⁹, R¹⁰, and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX235").

The Compound (A-5) wherein R⁸ represents a methyl group, R⁹, R¹⁰, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX236").

The Compound (A-5) wherein R⁸ represents a methyl group, R⁹, R¹⁰, and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX237").

The Compound (A-5) wherein R⁸ represents a methyl group, R⁹, R¹⁰, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX238").

The Compound (A-5) wherein R⁸ represents a methyl group, R⁹, R¹⁰, and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX239").

The Compound (A-5) wherein R⁸ represents a methyl group, R⁹, R¹⁰, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX240").

The Compound (A-5) wherein R⁸ represents a methyl group, R⁹, R¹⁰, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX241").

The Compound (A-5) wherein R⁸ represents a methyl group, R⁹, R¹⁰, and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX242").

The Compound (A-5) wherein R⁸ represents a methyl group, R⁹, R¹⁰, and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX243").

The Compound (A-5) wherein R⁸ represents a methyl group, R⁹, R¹⁰, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX244").

The Compound (A-5) wherein R⁸ represents a methyl group, R⁹, R¹⁰, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX245").

The Compound (A-5) wherein R⁸ represents a methyl group, R⁹, R¹⁰, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX246").

The Compound (A-5) wherein R⁸ represents a methyl group, R⁹, R¹⁰, and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX247").

The Compound (A-5) wherein R⁸ represents a methyl group, R⁹, R¹⁰, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX248").

The Compound (A-5) wherein R⁸ represents a methyl group, R⁹, R¹⁰, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX249").

The Compound (A-5) wherein R⁸ represents a methyl group, R⁹, R¹⁰, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX250").

The Compound (A-5) wherein R⁸ represents a methyl group, R⁹, R¹⁰, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX251").

The Compound (A-5) wherein R⁸ represents a methyl group, R⁹, R¹⁰, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX252").

The Compound (A-5) wherein R⁸ represents a methyl group, R⁹, R¹⁰, and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX253").

The Compound (A-5) wherein R⁸ represents a methyl group, R⁹, R¹⁰, and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX254").

The Compound (A-5) wherein R⁸ represents a methyl group, R⁹, R¹⁰, and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX255").

The Compound (A-5) wherein R⁹ represents a fluorine atom, R⁸, R¹⁰, and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX256").

The Compound (A-5) wherein R⁹ represents a fluorine atom, R⁸, R¹⁰, and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX257").

The Compound (A-5) wherein R⁹ represents a fluorine atom, R⁸, R¹⁰, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX258").

The Compound (A-5) wherein R⁹ represents a fluorine atom, R⁸, R¹⁰, and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX259").

The Compound (A-5) wherein R⁹ represents a fluorine atom, R⁸, R¹⁰, and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX260").

The Compound (A-5) wherein R⁹ represents a fluorine atom, R⁸, R¹⁰, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX261").

The Compound (A-5) wherein R⁹ represents a fluorine atom, R⁸, R¹⁰, and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX262").

The Compound (A-5) wherein R⁹ represents a fluorine atom, R⁸, R¹⁰, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX263").

The Compound (A-5) wherein R⁹ represents a fluorine atom, R⁸, R¹⁰, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX264").

The Compound (A-5) wherein R⁹ represents a fluorine atom, R⁸, R¹⁰, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX265").

The Compound (A-5) wherein R⁹ represents a fluorine atom, R⁸, R¹⁰, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX266").

The Compound (A-5) wherein R⁹ represents a fluorine atom, R⁸, R¹⁰, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX267").

The Compound (A-5) wherein R⁹ represents a fluorine atom, R⁸, R¹⁰, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX268").

The Compound (A-5) wherein R⁹ represents a fluorine atom, R⁸, R¹⁰, and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX269").

The Compound (A-5) wherein R⁹ represents a fluorine atom, R⁸, R¹⁰, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX270").

The Compound (A-5) wherein R⁹ represents a fluorine atom, R⁸, R¹⁰, and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX271").

The Compound (A-5) wherein R⁹ represents a fluorine atom, R⁸, R¹⁰, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX272").

The Compound (A-5) wherein R⁹ represents a fluorine atom, R⁸, R¹⁰, and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX273").

The Compound (A-5) wherein R⁹ represents a fluorine atom, R⁸, R¹⁰, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX274").

The Compound (A-5) wherein R⁹ represents a fluorine atom, R⁸, R¹⁰, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX275").

The Compound (A-5) wherein R⁹ represents a fluorine atom, R⁸, R¹⁰, and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX276").

The Compound (A-5) wherein R⁹ represents a fluorine atom, R⁸, R¹⁰, and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX277").

The Compound (A-5) wherein R⁹ represents a fluorine atom, R⁸, R¹⁰, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX278").

The Compound (A-5) wherein R⁹ represents a fluorine atom, R⁸, R¹⁰, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX279").

The Compound (A-5) wherein R⁹ represents a fluorine atom, R⁸, R¹⁰, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX280").

The Compound (A-5) wherein R⁹ represents a fluorine atom, R⁸, R¹⁰, and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX281").

The Compound (A-5) wherein R⁹ represents a fluorine atom, R⁸, R¹⁰, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX282").

The Compound (A-5) wherein R⁹ represents a fluorine atom, R⁸, R¹⁰, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX283").

The Compound (A-5) wherein R⁹ represents a fluorine atom, R⁸, R¹⁰, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX284").

The Compound (A-5) wherein R⁹ represents a fluorine atom, R⁸, R¹⁰, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX285").

The Compound (A-5) wherein R⁹ represents a fluorine atom, R⁸, R¹⁰, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX286").

The Compound (A-5) wherein R⁹ represents a fluorine atom, R⁸, R¹⁰, and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX287").

The Compound (A-5) wherein R⁹ represents a fluorine atom, R⁸, R¹⁰, and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX288").

The Compound (A-5) wherein R⁹ represents a fluorine atom, R⁸, R¹⁰, and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX289").

The Compound (A-5) wherein R⁹ represents a chlorine atom, R⁸, R¹⁰, and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX290").

The Compound (A-5) wherein R⁹ represents a chlorine atom, R⁸, R¹⁰, and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX291").

The Compound (A-5) wherein R⁹ represents a chlorine atom, R⁸, R¹⁰, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX292").

The Compound (A-5) wherein R⁹ represents a chlorine atom, R⁸, R¹⁰, and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX293").

The Compound (A-5) wherein R⁹ represents a chlorine atom, R⁸, R¹⁰, and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX294").

The Compound (A-5) wherein R⁹ represents a chlorine atom, R⁸, R¹⁰, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX295").

The Compound (A-5) wherein R⁹ represents a chlorine atom, R⁸, R¹⁰, and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX296").

The Compound (A-5) wherein R⁹ represents a chlorine atom, R⁸, R¹⁰, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX297").

The Compound (A-5) wherein R⁹ represents a chlorine atom, R⁸, R¹⁰, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX298").

The Compound (A-5) wherein R⁹ represents a chlorine atom, R⁸, R¹⁰, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX299").

The Compound (A-5) wherein R⁹ represents a chlorine atom, R⁸, R¹⁰, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX300").

The Compound (A-5) wherein R⁹ represents a chlorine atom, R⁸, R¹⁰, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX301").

The Compound (A-5) wherein R⁹ represents a chlorine atom, R⁸, R¹⁰, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX302").

The Compound (A-5) wherein R⁹ represents a chlorine atom, R⁸, R¹⁰, and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX303").

The Compound (A-5) wherein R⁹ represents a chlorine atom, R⁸, R¹⁰, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX304").

The Compound (A-5) wherein R⁹ represents a chlorine atom, R⁸, R¹⁰, and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX305").

The Compound (A-5) wherein R⁹ represents a chlorine atom, R⁸, R¹⁰, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX306").

The Compound (A-5) wherein R⁹ represents a chlorine atom, R⁸, R¹⁰, and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX307").

The Compound (A-5) wherein R⁹ represents a chlorine atom, R⁸, R¹⁰, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX308").

The Compound (A-5) wherein R⁹ represents a chlorine atom, R⁸, R¹⁰, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX309").

The Compound (A-5) wherein R⁹ represents a chlorine atom, R⁸, R¹⁰, and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX310").

The Compound (A-5) wherein R⁹ represents a chlorine atom, R⁸, R¹⁰, and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX311").

The Compound (A-5) wherein R⁹ represents a chlorine atom, R⁸, R¹⁰, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX312").

The Compound (A-5) wherein R⁹ represents a chlorine atom, R⁸, R¹⁰, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX313").

The Compound (A-5) wherein R⁹ represents a chlorine atom, R⁸, R¹⁰, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX314").

The Compound (A-5) wherein R⁹ represents a chlorine atom, R⁸, R¹⁰, and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX315").

The Compound (A-5) wherein R⁹ represents a chlorine atom, R⁸, R¹⁰, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX316").

The Compound (A-5) wherein R⁹ represents a chlorine atom, R⁸, R¹⁰, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX317").

The Compound (A-5) wherein R⁹ represents a chlorine atom, R⁸, R¹⁰, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX318").

The Compound (A-5) wherein R⁹ represents a chlorine atom, R⁸, R¹⁰, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX319").

The Compound (A-5) wherein R⁹ represents a chlorine atom, R⁸, R¹⁰, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX320").

The Compound (A-5) wherein R⁹ represents a chlorine atom, R⁸, R¹⁰, and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX321").

The Compound (A-5) wherein R⁹ represents a chlorine atom, R⁸, R¹⁰, and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX322").

The Compound (A-5) wherein R⁹ represents a chlorine atom, R⁸, R¹⁰, and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX323").

The Compound (A-5) wherein R⁹ represents a methyl group, R⁸, R¹⁰, and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX324").

The Compound (A-5) wherein R⁹ represents a methyl group, R⁸, R¹⁰, and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX325").

The Compound (A-5) wherein R⁹ represents a methyl group, R⁸, R¹⁰, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX326").

The Compound (A-5) wherein R⁹ represents a methyl group, R⁸, R¹⁰, and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX327").

The Compound (A-5) wherein R⁹ represents a methyl group, R⁸, R¹⁰, and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX328").

The Compound (A-5) wherein R⁹ represents a methyl group, R⁸, R¹⁰, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX329").

The Compound (A-5) wherein R⁹ represents a methyl group, R⁸, R¹⁰, and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX330").

The Compound (A-5) wherein R⁹ represents a methyl group, R⁸, R¹⁰, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX331").

The Compound (A-5) wherein R⁹ represents a methyl group, R⁸, R¹⁰, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX332").

The Compound (A-5) wherein R⁹ represents a methyl group, R⁸, R¹⁰, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX333").

The Compound (A-5) wherein R⁹ represents a methyl group, R⁸, R¹⁰, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX334").

The Compound (A-5) wherein R⁹ represents a methyl group, R⁸, R¹⁰, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX335").

The Compound (A-5) wherein R⁹ represents a methyl group, R⁸, R¹⁰, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX336").

The Compound (A-5) wherein R⁹ represents a methyl group, R⁸, R¹⁰, and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX337").

The Compound (A-5) wherein R⁹ represents a methyl group, R⁸, R¹⁰, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX338").

The Compound (A-5) wherein R⁹ represents a methyl group, R⁸, R¹⁰, and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX339").

The Compound (A-5) wherein R⁹ represents a methyl group, R⁸, R¹⁰, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX340").

The Compound (A-5) wherein R⁹ represents a methyl group, R⁸, R¹⁰, and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX341").

The Compound (A-5) wherein R⁹ represents a methyl group, R⁸, R¹⁰, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX342").

The Compound (A-5) wherein R⁹ represents a methyl group, R⁸, R¹⁰, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX343").

The Compound (A-5) wherein R⁹ represents a methyl group, R⁸, R¹⁰, and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX344").

The Compound (A-5) wherein R⁹ represents a methyl group, R⁸, R¹⁰, and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX345").

The Compound (A-5) wherein R⁹ represents a methyl group, R⁸, R¹⁰, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX346").

The Compound (A-5) wherein R⁹ represents a methyl group, R⁸, R¹⁰, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX347").

The Compound (A-5) wherein R⁹ represents a methyl group, R⁸, R¹⁰, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX348").

The Compound (A-5) wherein R⁹ represents a methyl group, R⁸, R¹⁰, and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX349").

The Compound (A-5) wherein R⁹ represents a methyl group, R⁸, R¹⁰, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX350").

The Compound (A-5) wherein R⁹ represents a methyl group, R⁸, R¹⁰, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX351").

The Compound (A-5) wherein R⁹ represents a methyl group, R⁸, R¹⁰, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX352").

The Compound (A-5) wherein R⁹ represents a methyl group, R⁸, R¹⁰, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX353").

The Compound (A-5) wherein R⁹ represents a methyl group, R⁸, R¹⁰, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX354").

The Compound (A-5) wherein R⁹ represents a methyl group, R⁸, R¹⁰, and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX355").

The Compound (A-5) wherein R⁹ represents a methyl group, R⁸, R¹⁰, and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX356").

The Compound (A-5) wherein R⁹ represents a methyl group, R⁸, R¹⁰, and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX357").

The Compound (A-5) wherein R¹⁰ represents a fluorine atom, R⁸, R⁹, and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX358").

The Compound (A-5) wherein R¹⁰ represents a fluorine atom, R⁸, R⁹, and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX359").

The Compound (A-5) wherein R¹⁰ represents a fluorine atom, R⁸, R⁹, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX360").

The Compound (A-5) wherein R¹⁰ represents a fluorine atom, R⁸, R⁹, and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX361").

The Compound (A-5) wherein R¹⁰ represents a fluorine atom, R⁸, R⁹, and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX362").

The Compound (A-5) wherein R¹⁰ represents a fluorine atom, R⁸, R⁹, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX363").

The Compound (A-5) wherein R¹⁰ represents a fluorine atom, R⁸, R⁹, and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX364").

The Compound (A-5) wherein R¹⁰ represents a fluorine atom, R⁸, R⁹, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX365").

The Compound (A-5) wherein R¹⁰ represents a fluorine atom, R⁸, R⁹, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX366").

The Compound (A-5) wherein R¹⁰ represents a fluorine atom, R⁸, R⁹, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX367").

The Compound (A-5) wherein R¹⁰ represents a fluorine atom, R⁸, R⁹, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX368").

The Compound (A-5) wherein R¹⁰ represents a fluorine atom, R⁸, R⁹, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX369").

The Compound (A-5) wherein R¹⁰ represents a fluorine atom, R⁸, R⁹, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX370").

The Compound (A-5) wherein R¹⁰ represents a fluorine atom, R⁸, R⁹, and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX371").

The Compound (A-5) wherein R¹⁰ represents a fluorine atom, R⁸, R⁹, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX372").

The Compound (A-5) wherein R¹⁰ represents a fluorine atom, R⁸, R⁹, and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX373").

The Compound (A-5) wherein R¹⁰ represents a fluorine atom, R⁸, R⁹, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX374").

The Compound (A-5) wherein R¹⁰ represents a fluorine atom, R⁸, R⁹, and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX375").

The Compound (A-5) wherein R¹⁰ represents a fluorine atom, R⁸, R⁹, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX376").

The Compound (A-5) wherein R¹⁰ represents a fluorine atom, R⁸, R⁹, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX377").

The Compound (A-5) wherein R¹⁰ represents a fluorine atom, R⁸, R⁹, and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX378").

The Compound (A-5) wherein R¹⁰ represents a fluorine atom, R⁸, R⁹, and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX379").

The Compound (A-5) wherein R¹⁰ represents a fluorine atom, R⁸, R⁹, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX380").

The Compound (A-5) wherein R¹⁰ represents a fluorine atom, R⁸, R⁹, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX381").

The Compound (A-5) wherein R¹⁰ represents a fluorine atom, R⁸, R⁹, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX382").

The Compound (A-5) wherein R¹⁰ represents a fluorine atom, R⁸, R⁹, and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX383").

The Compound (A-5) wherein R¹⁰ represents a fluorine atom, R⁸, R⁹, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX384").

The Compound (A-5) wherein R¹⁰ represents a fluorine atom, R⁸, R⁹, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX385").

The Compound (A-5) wherein R¹⁰ represents a fluorine atom, R⁸, R⁹, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX386").

The Compound (A-5) wherein R¹⁰ represents a fluorine atom, R⁸, R⁹, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX387").

The Compound (A-5) wherein R¹⁰ represents a fluorine atom, R⁸, R⁹, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX388").

The Compound (A-5) wherein R¹⁰ represents a fluorine atom, R⁸, R⁹, and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX389").

The Compound (A-5) wherein R¹⁰ represents a fluorine atom, R⁸, R⁹, and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX390").

The Compound (A-5) wherein R¹⁰ represents a fluorine atom, R⁸, R⁹, and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX391").

The Compound (A-5) wherein R¹⁰ represents a chlorine atom, R⁸, R⁹, and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX392").

The Compound (A-5) wherein R¹⁰ represents a chlorine atom, R⁸, R⁹, and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX393").

The Compound (A-5) wherein R¹⁰ represents a chlorine atom, R⁸, R⁹, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX394").

The Compound (A-5) wherein R¹⁰ represents a chlorine atom, R⁸, R⁹, and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX395").

The Compound (A-5) wherein R¹⁰ represents a chlorine atom, R⁸, R⁹, and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX396").

The Compound (A-5) wherein R¹⁰ represents a chlorine atom, R⁸, R⁹, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX397").

The Compound (A-5) wherein R¹⁰ represents a chlorine atom, R⁸, R⁹, and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX398").

The Compound (A-5) wherein R¹⁰ represents a chlorine atom, R⁸, R⁹, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX399").

The Compound (A-5) wherein R¹⁰ represents a chlorine atom, R⁸, R⁹, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX400").

The Compound (A-5) wherein R¹⁰ represents a chlorine atom, R⁸, R⁹, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX401").

The Compound (A-5) wherein R¹⁰ represents a chlorine atom, R⁸, R⁹, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX402").

The Compound (A-5) wherein R¹⁰ represents a chlorine atom, R⁸, R⁹, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX403").

The Compound (A-5) wherein R¹⁰ represents a chlorine atom, R⁸, R⁹, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX404").

The Compound (A-5) wherein R¹⁰ represents a chlorine atom, R⁸, R⁹, and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX405").

The Compound (A-5) wherein R¹⁰ represents a chlorine atom, R⁸, R⁹, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX406").

The Compound (A-5) wherein R¹⁰ represents a chlorine atom, R⁸, R⁹, and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX407").

The Compound (A-5) wherein R¹⁰ represents a chlorine atom, R⁸, R⁹, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX408").

The Compound (A-5) wherein R¹⁰ represents a chlorine atom, R⁸, R⁹, and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX409").

The Compound (A-5) wherein R¹⁰ represents a chlorine atom, R⁸, R⁹, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX410").

The Compound (A-5) wherein R¹⁰ represents a chlorine atom, R⁸, R⁹, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX411").

The Compound (A-5) wherein R¹⁰ represents a chlorine atom, R⁸, R⁹, and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX412").

The Compound (A-5) wherein R¹⁰ represents a chlorine atom, R⁸, R⁹, and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX413").

The Compound (A-5) wherein R¹⁰ represents a chlorine atom, R⁸, R⁹, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX414").

The Compound (A-5) wherein R¹⁰ represents a chlorine atom, R⁸, R⁹, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX415").

The Compound (A-5) wherein R¹⁰ represents a chlorine atom, R⁸, R⁹, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX416").

The Compound (A-5) wherein R¹⁰ represents a chlorine atom, R⁸, R⁹, and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX417").

The Compound (A-5) wherein R¹⁰ represents a chlorine atom, R⁸, R⁹, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX418").

The Compound (A-5) wherein R¹⁰ represents a chlorine atom, R⁸, R⁹, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX419").

The Compound (A-5) wherein R¹⁰ represents a chlorine atom, R⁸, R⁹, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX420").

The Compound (A-5) wherein R¹⁰ represents a chlorine atom, R⁸, R⁹, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX421").

The Compound (A-5) wherein R¹⁰ represents a chlorine atom, R⁸, R⁹, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX422").

The Compound (A-5) wherein R¹⁰ represents a chlorine atom, R⁸, R⁹, and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX423").

The Compound (A-5) wherein R¹⁰ represents a chlorine atom, R⁸, R⁹, and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX424").

The Compound (A-5) wherein R¹⁰ represents a chlorine atom, R⁸, R⁹, and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX425").

The Compound (A-5) wherein R¹⁰ represents a methyl group, R⁸, R⁹, and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX426").

The Compound (A-5) wherein R¹⁰ represents a methyl group, R⁸, R⁹, and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX427").

The Compound (A-5) wherein R¹⁰ represents a methyl group, R⁸, R⁹, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX428").

The Compound (A-5) wherein R¹⁰ represents a methyl group, R⁸, R⁹, and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX429").

The Compound (A-5) wherein R¹⁰ represents a methyl group, R⁸, R⁹, and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX430").

The Compound (A-5) wherein R¹⁰ represents a methyl group, R⁸, R⁹, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX431").

The Compound (A-5) wherein R¹⁰ represents a methyl group, R⁸, R⁹, and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX432").

The Compound (A-5) wherein R¹⁰ represents a methyl group, R⁸, R⁹, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX433").

The Compound (A-5) wherein R¹⁰ represents a methyl group, R⁸, R⁹, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX434").

The Compound (A-5) wherein R¹⁰ represents a methyl group, R⁸, R⁹, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX435").

The Compound (A-5) wherein R¹⁰ represents a methyl group, R⁸, R⁹, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX436").

The Compound (A-5) wherein R¹⁰ represents a methyl group, R⁸, R⁹, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX437").

The Compound (A-5) wherein R¹⁰ represents a methyl group, R⁸, R⁹, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX438").

The Compound (A-5) wherein R¹⁰ represents a methyl group, R⁸, R⁹, and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX439").

The Compound (A-5) wherein R¹⁰ represents a methyl group, R⁸, R⁹, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX440").

The Compound (A-5) wherein R¹⁰ represents a methyl group, R⁸, R⁹, and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX441").

The Compound (A-5) wherein R¹⁰ represents a methyl group, R⁸, R⁹, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX442").

The Compound (A-5) wherein R¹⁰ represents a methyl group, R⁸, R⁹, and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX443").

The Compound (A-5) wherein R¹⁰ represents a methyl group, R⁸, R⁹, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX444").

The Compound (A-5) wherein R¹⁰ represents a methyl group, R⁸, R⁹, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX445").

The Compound (A-5) wherein R¹⁰ represents a methyl group, R⁸, R⁹, and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX446").

The Compound (A-5) wherein R¹⁰ represents a methyl group, R⁸, R⁹, and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX447").

The Compound (A-5) wherein R¹⁰ represents a methyl group, R⁸, R⁹, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX448").

The Compound (A-5) wherein R¹⁰ represents a methyl group, R⁸, R⁹, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX449").

The Compound (A-5) wherein R¹⁰ represents a methyl group, R⁸, R⁹, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX450").

The Compound (A-5) wherein R¹⁰ represents a methyl group, R⁸, R⁹, and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX451").

The Compound (A-5) wherein R¹⁰ represents a methyl group, R⁸, R⁹, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX452").

The Compound (A-5) wherein R¹⁰ represents a methyl group, R⁸, R⁹, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX453").

The Compound (A-5) wherein R¹⁰ represents a methyl group, R⁸, R⁹, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX454").

The Compound (A-5) wherein R¹⁰ represents a methyl group, R⁸, R⁹, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX455").

The Compound (A-5) wherein R¹⁰ represents a methyl group, R⁸, R⁹, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX456").

The Compound (A-5) wherein R¹⁰ represents a methyl group, R⁸, R⁹, and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX457").

The Compound (A-5) wherein R¹⁰ represents a methyl group, R⁸, R⁹, and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX458").

The Compound (A-5) wherein R¹⁰ represents a methyl group, R⁸, R⁹, and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX459").

The Compound (A-5) wherein R¹¹ represents a fluorine atom, R⁸, R⁹, and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX460").

The Compound (A-5) wherein R¹¹ represents a fluorine atom, R⁸, R⁹, and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX461").

The Compound (A-5) wherein R¹¹ represents a fluorine atom, R⁸, R⁹, and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX462").

The Compound (A-5) wherein R¹¹ represents a fluorine atom, R⁸, R⁹, and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX463").

The Compound (A-5) wherein R¹¹ represents a fluorine atom, R⁸, R⁹, and R¹⁰ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX464").

The Compound (A-5) wherein R¹¹ represents a fluorine atom, R⁸, R⁹, and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX465").

The Compound (A-5) wherein R¹¹ represents a fluorine atom, R⁸, R⁹, and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX466").

The Compound (A-5) wherein R¹¹ represents a fluorine atom, R⁸, R⁹, and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX467").

The Compound (A-5) wherein R¹¹ represents a fluorine atom, R⁸, R⁹, and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX468").

The Compound (A-5) wherein R¹¹ represents a fluorine atom, R⁸, R⁹, and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX469").

The Compound (A-5) wherein R¹¹ represents a fluorine atom, R⁸, R⁹, and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX470").

The Compound (A-5) wherein R¹¹ represents a fluorine atom, R⁸, R⁹, and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX471").

The Compound (A-5) wherein R¹¹ represents a fluorine atom, R⁸, R⁹, and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX472").

The Compound (A-5) wherein R¹¹ represents a fluorine atom, R⁸, R⁹, and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX473").

The Compound (A-5) wherein R¹¹ represents a fluorine atom, R⁸, R⁹, and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX474").

The Compound (A-5) wherein R¹¹ represents a fluorine atom, R⁸, R⁹, and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX475").

The Compound (A-5) wherein R¹¹ represents a fluorine atom, R⁸, R⁹, and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX476").

The Compound (A-5) wherein R¹¹ represents a fluorine atom, R⁸, R⁹, and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX477").

The Compound (A-5) wherein R¹¹ represents a fluorine atom, R⁸, R⁹, and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX478").

The Compound (A-5) wherein R¹¹ represents a fluorine atom, R⁸, R⁹, and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX479").

The Compound (A-5) wherein R¹¹ represents a fluorine atom, R⁸, R⁹, and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX480").

The Compound (A-5) wherein R¹¹ represents a fluorine atom, R⁸, R⁹, and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX481").

The Compound (A-5) wherein R¹¹ represents a fluorine atom, R⁸, R⁹, and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX482").

The Compound (A-5) wherein R¹¹ represents a fluorine atom, R⁸, R⁹, and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX483").

The Compound (A-5) wherein R¹¹ represents a fluorine atom, R⁸, R⁹, and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX484").

The Compound (A-5) wherein R¹¹ represents a fluorine atom, R⁸, R⁹, and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX485").

The Compound (A-5) wherein R¹¹ represents a fluorine atom, R⁸, R⁹, and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX486").

The Compound (A-5) wherein R¹¹ represents a fluorine atom, R⁸, R⁹, and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX487").

The Compound (A-5) wherein R¹¹ represents a fluorine atom, R⁸, R⁹, and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX488").

The Compound (A-5) wherein R¹¹ represents a fluorine atom, R⁸, R⁹, and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX489").

The Compound (A-5) wherein R¹¹ represents a fluorine atom, R⁸, R⁹, and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX490").

The Compound (A-5) wherein R¹¹ represents a fluorine atom, R⁸, R⁹, and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX491").

The Compound (A-5) wherein R¹¹ represents a fluorine atom, R⁸, R⁹, and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX492").

The Compound (A-5) wherein R¹¹ represents a fluorine atom, R⁸, R⁹, and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX493").

The Compound (A-5) wherein R¹¹ represents a chlorine atom, R⁸, R⁹, and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX494").

The Compound (A-5) wherein R¹¹ represents a chlorine atom, R⁸, R⁹, and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX495").

The Compound (A-5) wherein R¹¹ represents a chlorine atom, R⁸, R⁹, and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX496").

The Compound (A-5) wherein R¹¹ represents a chlorine atom, R⁸, R⁹, and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX497").

The Compound (A-5) wherein R¹¹ represents a chlorine atom, R⁸, R⁹, and R¹⁰ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX498").

The Compound (A-5) wherein R¹¹ represents a chlorine atom, R⁸, R⁹, and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX499").

The Compound (A-5) wherein R¹¹ represents a chlorine atom, R⁸, R⁹, and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX500").

The Compound (A-5) wherein R¹¹ represents a chlorine atom, R⁸, R⁹, and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX501").

The Compound (A-5) wherein R¹¹ represents a chlorine atom, R⁸, R⁹, and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX502").

The Compound (A-5) wherein R¹¹ represents a chlorine atom, R⁸, R⁹, and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX503").

The Compound (A-5) wherein R¹¹ represents a chlorine atom, R⁸, R⁹, and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX504").

The Compound (A-5) wherein R¹¹ represents a chlorine atom, R⁸, R⁹, and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX505").

The Compound (A-5) wherein R¹¹ represents a chlorine atom, R⁸, R⁹, and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX506").

The Compound (A-5) wherein R¹¹ represents a chlorine atom, R⁸, R⁹, and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX507").

The Compound (A-5) wherein R¹¹ represents a chlorine atom, R⁸, R⁹, and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX508").

The Compound (A-5) wherein R¹¹ represents a chlorine atom, R⁸, R⁹, and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX509").

The Compound (A-5) wherein R¹¹ represents a chlorine atom, R⁸, R⁹, and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX510").

The Compound (A-5) wherein R¹¹ represents a chlorine atom, R⁸, R⁹, and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX511").

The Compound (A-5) wherein R¹¹ represents a chlorine atom, R⁸, R⁹, and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX512").

The Compound (A-5) wherein R¹¹ represents a chlorine atom, R⁸, R⁹, and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX513").

The Compound (A-5) wherein R¹¹ represents a chlorine atom, R⁸, R⁹, and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX514").

The Compound (A-5) wherein R¹¹ represents a chlorine atom, R⁸, R⁹, and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX515").

The Compound (A-5) wherein R¹¹ represents a chlorine atom, R⁸, R⁹, and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX516").

The Compound (A-5) wherein R¹¹ represents a chlorine atom, R⁸, R⁹, and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX517").

The Compound (A-5) wherein R¹¹ represents a chlorine atom, R⁸, R⁹, and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX518").

The Compound (A-5) wherein R¹¹ represents a chlorine atom, R⁸, R⁹, and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX519").

The Compound (A-5) wherein R¹¹ represents a chlorine atom, R⁸, R⁹, and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX520").

The Compound (A-5) wherein R¹¹ represents a chlorine atom, R⁸, R⁹, and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX521").

The Compound (A-5) wherein R¹¹ represents a chlorine atom, R⁸, R⁹, and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX522").

The Compound (A-5) wherein R¹¹ represents a chlorine atom, R⁸, R⁹, and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX523").

The Compound (A-5) wherein R¹¹ represents a chlorine atom, R⁸, R⁹, and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX524").

The Compound (A-5) wherein R¹¹ represents a chlorine atom, R⁸, R⁹, and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX525").

The Compound (A-5) wherein R¹¹ represents a chlorine atom, R⁸, R⁹, and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX526").

The Compound (A-5) wherein R¹¹ represents a chlorine atom, R⁸, R⁹, and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX527").

The Compound (A-5) wherein R¹¹ represents a methyl group, R⁸, R⁹, and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX528").

The Compound (A-5) wherein R¹¹ represents a methyl group, R⁸, R⁹, and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX529").

The Compound (A-5) wherein R¹¹ represents a methyl group, R⁸, R⁹, and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX530").

The Compound (A-5) wherein R¹¹ represents a methyl group, R⁸, R⁹, and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX531").

The Compound (A-5) wherein R¹¹ represents a methyl group, R⁸, R⁹, and R¹⁰ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX532").

The Compound (A-5) wherein R¹¹ represents a methyl group, R⁸, R⁹, and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX533").

The Compound (A-5) wherein R¹¹ represents a methyl group, R⁸, R⁹, and R¹⁰ each represent a hydrogen atom, n represents 1, Z represents a cyclopropyl group, and the combination of the group represented by G^{A} and the substituents R^{X7}, R^{X8}, R^{X9}, and R¹⁰ in the group represented by G^{A} represents any one combination indicated in the Combination A (hereinafter referred to as "Compound group SX534").

The Compound (A-5) wherein R¹¹ represents a methyl group, R⁸, R⁹, and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX535").

The Compound (A-5) wherein R¹¹ represents a methyl group, R⁸, R⁹, and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX536").

The Compound (A-5) wherein R¹¹ represents a methyl group, R⁸, R⁹, and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX537").

The Compound (A-5) wherein R¹¹ represents a methyl group, R⁸, R⁹, and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX538").

The Compound (A-5) wherein R¹¹ represents a methyl group, R⁸, R⁹, and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX539").

The Compound (A-5) wherein R¹¹ represents a methyl group, R⁸, R⁹, and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX540").

The Compound (A-5) wherein R¹¹ represents a methyl group, R⁸, R⁹, and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX541").

The Compound (A-5) wherein R¹¹ represents a methyl group, R⁸, R⁹, and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX542").

The Compound (A-5) wherein R¹¹ represents a methyl group, R⁸, R⁹, and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX543").

The Compound (A-5) wherein R¹¹ represents a methyl group, R⁸, R⁹, and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX544").

The Compound (A-5) wherein R¹¹ represents a methyl group, R⁸, R⁹, and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX545").

The Compound (A-5) wherein R¹¹ represents a methyl group, R⁸, R⁹, and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX546").

The Compound (A-5) wherein R¹¹ represents a methyl group, R⁸, R⁹, and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX547").

The Compound (A-5) wherein R¹¹ represents a methyl group, R⁸, R⁹, and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX548").

The Compound (A-5) wherein R¹¹ represents a methyl group, R⁸, R⁹, and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX549").

The Compound (A-5) wherein R¹¹ represents a methyl group, R⁸, R⁹, and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX550").

The Compound (A-5) wherein R¹¹ represents a methyl group, R⁸, R⁹, and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX551").

The Compound (A-5) wherein R¹¹ represents a methyl group, R⁸, R⁹, and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX552").

The Compound (A-5) wherein R¹¹ represents a methyl group, R⁸, R⁹, and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX553").

The Compound (A-5) wherein R¹¹ represents a methyl group, R⁸, R⁹, and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX554").

The Compound (A-5) wherein R¹¹ represents a methyl group, R⁸, R⁹, and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX555").

The Compound (A-5) wherein R¹¹ represents a methyl group, R⁸, R⁹, and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX556").

The Compound (A-5) wherein R¹¹ represents a methyl group, R⁸, R⁹, and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX557").

The Compound (A-5) wherein R¹¹ represents a methyl group, R⁸, R⁹, and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX558").

The Compound (A-5) wherein R¹¹ represents a methyl group, R⁸, R⁹, and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX559").

The Compound (A-5) wherein R¹¹ represents a methyl group, R⁸, R⁹, and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX560").

The Compound (A-5) wherein R¹¹ represents a methyl group, R⁸, R⁹, and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX561").

A compound represented by formula (A-6) 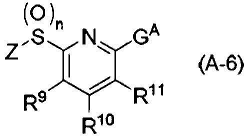 (hereinafter referred to as "Compound (A-6)") wherein R⁹, R¹⁰, and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX562").

The Compound (A-6) wherein R⁹, R¹⁰, and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX563").

The Compound (A-6) wherein R⁹, R¹⁰, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX564").

The Compound (A-6) wherein R⁹, R¹⁰, and R¹¹ each represent a hydrogen atom, n represents 1, Z represents - CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX565").

The Compound (A-6) wherein R⁹, R¹⁰, and R¹¹ each represent a hydrogen atom, n represents 1, Z represents - CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX566").

The Compound (A-6) wherein R⁹, R¹⁰, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX567").

The Compound (A-6) wherein R⁹, R¹⁰, and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX568").

The Compound (A-6) wherein R⁹, R¹⁰, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX569").

The Compound (A-6) wherein R⁹, R¹⁰, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX570").

The Compound (A-6) wherein R⁹, R¹⁰, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX571").

The Compound (A-6) wherein R⁹, R¹⁰, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX572").

The Compound (A-6) wherein R⁹, R¹⁰, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX573").

The Compound (A-6) wherein R⁹, R¹⁰, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX574").

The Compound (A-6) wherein R⁹, R¹⁰, and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX575").

The Compound (A-6) wherein R⁹, R¹⁰, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX576").

The Compound (A-6) wherein R⁹, R¹⁰, and R¹¹ each represent a hydrogen atom, n represents 1, Z represents a 3-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX577").

The Compound (A-6) wherein R⁹, R¹⁰, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX578").

The Compound (A-6) wherein R⁹, R¹⁰, and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX579").

The Compound (A-6) wherein R⁹, R¹⁰, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX580").

The Compound (A-6) wherein R⁹, R¹⁰, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX581").

The Compound (A-6) wherein R⁹, R¹⁰, and R¹¹ each represent a hydrogen atom, n represents 2, Z represents - CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX582").

The Compound (A-6) wherein R⁹, R¹⁰, and R¹¹ each represent a hydrogen atom, n represents 2, Z represents - CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX583").

The Compound (A-6) wherein R⁹, R¹⁰, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX584").

The Compound (A-6) wherein R⁹, R¹⁰, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX585").

The Compound (A-6) wherein R⁹, R¹⁰, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX586").

The Compound (A-6) wherein R⁹, R¹⁰, and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX587").

The Compound (A-6) wherein R⁹, R¹⁰, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX588").

The Compound (A-6) wherein R⁹, R¹⁰, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX589").

The Compound (A-6) wherein R⁹, R¹⁰, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX590").

The Compound (A-6) wherein R⁹, R¹⁰, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX591").

The Compound (A-6) wherein R⁹, R¹⁰, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX592").

The Compound (A-6) wherein R⁹, R¹⁰, and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX593").

The Compound (A-6) wherein R⁹, R¹⁰, and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX594").

The Compound (A-6) wherein R⁹, R¹⁰, and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX595").

The Compound (A-6) wherein R⁹ represents a fluorine atom, R¹⁰ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX596").

The Compound (A-6) wherein R⁹ represents a fluorine atom, R¹⁰ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX597").

The Compound (A-6) wherein R⁹ represents a fluorine atom, R¹⁰ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX598").

The Compound (A-6) wherein R⁹ represents a fluorine atom, R¹⁰ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX599").

The Compound (A-6) wherein R⁹ represents a fluorine atom, R¹⁰ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX600").

The Compound (A-6) wherein R⁹ represents a fluorine atom, R¹⁰ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX601").

The Compound (A-6) wherein R⁹ represents a fluorine atom, R¹⁰ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX602").

The Compound (A-6) wherein R⁹ represents a fluorine atom, R¹⁰ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX603").

The Compound (A-6) wherein R⁹ represents a fluorine atom, R¹⁰ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX604").

The Compound (A-6) wherein R⁹ represents a fluorine atom, R¹⁰ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX605").

The Compound (A-6) wherein R⁹ represents a fluorine atom, R¹⁰ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX606").

The Compound (A-6) wherein R⁹ represents a fluorine atom, R¹⁰ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX607").

The Compound (A-6) wherein R⁹ represents a fluorine atom, R¹⁰ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX608").

The Compound (A-6) wherein R⁹ represents a fluorine atom, R¹⁰ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX609").

The Compound (A-6) wherein R⁹ represents a fluorine atom, R¹⁰ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX610").

The Compound (A-6) wherein R⁹ represents a fluorine atom, R¹⁰ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX611").

The Compound (A-6) wherein R⁹ represents a fluorine atom, R¹⁰ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX612").

The Compound (A-6) wherein R⁹ represents a fluorine atom, R¹⁰ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX613").

The Compound (A-6) wherein R⁹ represents a fluorine atom, R¹⁰ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX614").

The Compound (A-6) wherein R⁹ represents a fluorine atom, R¹⁰ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX615").

The Compound (A-6) wherein R⁹ represents a fluorine atom, R¹⁰ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX616").

The Compound (A-6) wherein R⁹ represents a fluorine atom, R¹⁰ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX617").

The Compound (A-6) wherein R⁹ represents a fluorine atom, R¹⁰ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX618").

The Compound (A-6) wherein R⁹ represents a fluorine atom, R¹⁰ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX619").

The Compound (A-6) wherein R⁹ represents a fluorine atom, R¹⁰ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX620").

The Compound (A-6) wherein R⁹ represents a fluorine atom, R¹⁰ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX621").

The Compound (A-6) wherein R⁹ represents a fluorine atom, R¹⁰ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX622").

The Compound (A-6) wherein R⁹ represents a fluorine atom, R¹⁰ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX623").

The Compound (A-6) wherein R⁹ represents a fluorine atom, R¹⁰ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX624").

The Compound (A-6) wherein R⁹ represents a fluorine atom, R¹⁰ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX625").

The Compound (A-6) wherein R⁹ represents a fluorine atom, R¹⁰ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX626").

The Compound (A-6) wherein R⁹ represents a fluorine atom, R¹⁰ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX627").

The Compound (A-6) wherein R⁹ represents a fluorine atom, R¹⁰ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX628").

The Compound (A-6) wherein R⁹ represents a fluorine atom, R¹⁰ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX629").

The Compound (A-6) wherein R⁹ represents a chlorine atom, R¹⁰ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX630").

The Compound (A-6) wherein R⁹ represents a chlorine atom, R¹⁰ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX631").

The Compound (A-6) wherein R⁹ represents a chlorine atom, R¹⁰ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX632").

The Compound (A-6) wherein R⁹ represents a chlorine atom, R¹⁰ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX633").

The Compound (A-6) wherein R⁹ represents a chlorine atom, R¹⁰ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX634").

The Compound (A-6) wherein R⁹ represents a chlorine atom, R¹⁰ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX635").

The Compound (A-6) wherein R⁹ represents a chlorine atom, R¹⁰ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX636").

The Compound (A-6) wherein R⁹ represents a chlorine atom, R¹⁰ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX637").

The Compound (A-6) wherein R⁹ represents a chlorine atom, R¹⁰ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX638").

The Compound (A-6) wherein R⁹ represents a chlorine atom, R¹⁰ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX639").

The Compound (A-6) wherein R⁹ represents a chlorine atom, R¹⁰ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX640").

The Compound (A-6) wherein R⁹ represents a chlorine atom, R¹⁰ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX641").

The Compound (A-6) wherein R⁹ represents a chlorine atom, R¹⁰ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX642").

The Compound (A-6) wherein R⁹ represents a chlorine atom, R¹⁰ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX643").

The Compound (A-6) wherein R⁹ represents a chlorine atom, R¹⁰ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX644").

The Compound (A-6) wherein R⁹ represents a chlorine atom, R¹⁰ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX645").

The Compound (A-6) wherein R⁹ represents a chlorine atom, R¹⁰ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX646").

The Compound (A-6) wherein R⁹ represents a chlorine atom, R¹⁰ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX647").

The Compound (A-6) wherein R⁹ represents a chlorine atom, R¹⁰ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX648").

The Compound (A-6) wherein R⁹ represents a chlorine atom, R¹⁰ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX649").

The Compound (A-6) wherein R⁹ represents a chlorine atom, R¹⁰ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX650").

The Compound (A-6) wherein R⁹ represents a chlorine atom, R¹⁰ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX651").

The Compound (A-6) wherein R⁹ represents a chlorine atom, R¹⁰ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX652").

The Compound (A-6) wherein R⁹ represents a chlorine atom, R¹⁰ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX653").

The Compound (A-6) wherein R⁹ represents a chlorine atom, R¹⁰ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX654").

The Compound (A-6) wherein R⁹ represents a chlorine atom, R¹⁰ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX655").

The Compound (A-6) wherein R⁹ represents a chlorine atom, R¹⁰ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX656").

The Compound (A-6) wherein R⁹ represents a chlorine atom, R¹⁰ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX657").

The Compound (A-6) wherein R⁹ represents a chlorine atom, R¹⁰ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX658").

The Compound (A-6) wherein R⁹ represents a chlorine atom, R¹⁰ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX659").

The Compound (A-6) wherein R⁹ represents a chlorine atom, R¹⁰ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX660").

The Compound (A-6) wherein R⁹ represents a chlorine atom, R¹⁰ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX661").

The Compound (A-6) wherein R⁹ represents a chlorine atom, R¹⁰ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX662").

The Compound (A-6) wherein R⁹ represents a chlorine atom, R¹⁰ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX663").

The Compound (A-6) wherein R⁹ represents a methyl group, R¹⁰ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX664").

The Compound (A-6) wherein R⁹ represents a methyl group, R¹⁰ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX665").

The Compound (A-6) wherein R⁹ represents a methyl group, R¹⁰ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX666").

The Compound (A-6) wherein R⁹ represents a methyl group, R¹⁰ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX667").

The Compound (A-6) wherein R⁹ represents a methyl group, R¹⁰ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX668").

The Compound (A-6) wherein R⁹ represents a methyl group, R¹⁰ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX669").

The Compound (A-6) wherein R⁹ represents a methyl group, R¹⁰ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX670").

The Compound (A-6) wherein R⁹ represents a methyl group, R¹⁰ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX671").

The Compound (A-6) wherein R⁹ represents a methyl group, R¹⁰ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX672").

The Compound (A-6) wherein R⁹ represents a methyl group, R¹⁰ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX673").

The Compound (A-6) wherein R⁹ represents a methyl group, R¹⁰ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX674").

The Compound (A-6) wherein R⁹ represents a methyl group, R¹⁰ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX675").

The Compound (A-6) wherein R⁹ represents a methyl group, R¹⁰ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX676").

The Compound (A-6) wherein R⁹ represents a methyl group, R¹⁰ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX677").

The Compound (A-6) wherein R⁹ represents a methyl group, R¹⁰ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX678").

The Compound (A-6) wherein R⁹ represents a methyl group, R¹⁰ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX679").

The Compound (A-6) wherein R⁹ represents a methyl group, R¹⁰ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX680").

The Compound (A-6) wherein R⁹ represents a methyl group, R¹⁰ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX681").

The Compound (A-6) wherein R⁹ represents a methyl group, R¹⁰ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX682").

The Compound (A-6) wherein R⁹ represents a methyl group, R¹⁰ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX683").

The Compound (A-6) wherein R⁹ represents a methyl group, R¹⁰ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX684").

The Compound (A-6) wherein R⁹ represents a methyl group, R¹⁰ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX685").

The Compound (A-6) wherein R⁹ represents a methyl group, R¹⁰ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX686").

The Compound (A-6) wherein R⁹ represents a methyl group, R¹⁰ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX687").

The Compound (A-6) wherein R⁹ represents a methyl group, R¹⁰ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX688").

The Compound (A-6) wherein R⁹ represents a methyl group, R¹⁰ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX689").

The Compound (A-6) wherein R⁹ represents a methyl group, R¹⁰ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX690").

The Compound (A-6) wherein R⁹ represents a methyl group, R¹⁰ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX691") .

The Compound (A-6) wherein R⁹ represents a methyl group, R¹⁰ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX692").

The Compound (A-6) wherein R⁹ represents a methyl group, R¹⁰ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX693").

The Compound (A-6) wherein R⁹ represents a methyl group, R¹⁰ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX694").

The Compound (A-6) wherein R⁹ represents a methyl group, R¹⁰ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX695").

The Compound (A-6) wherein R⁹ represents a methyl group, R¹⁰ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX696").

The Compound (A-6) wherein R⁹ represents a methyl group, R¹⁰ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX697").

The Compound (A-6) wherein R¹⁰ represents a fluorine atom, R⁹ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX698").

The Compound (A-6) wherein R¹⁰ represents a fluorine atom, R⁹ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX699").

The Compound (A-6) wherein R¹⁰ represents a fluorine atom, R⁹ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX700").

The Compound (A-6) wherein R¹⁰ represents a fluorine atom, R⁹ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX701").

The Compound (A-6) wherein R¹⁰ represents a fluorine atom, R⁹ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX702").

The Compound (A-6) wherein R¹⁰ represents a fluorine atom, R⁹ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX703").

The Compound (A-6) wherein R¹⁰ represents a fluorine atom, R⁹ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX704").

The Compound (A-6) wherein R¹⁰ represents a fluorine atom, R⁹ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX705").

The Compound (A-6) wherein R¹⁰ represents a fluorine atom, R⁹ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX706").

The Compound (A-6) wherein R¹⁰ represents a fluorine atom, R⁹ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX707").

The Compound (A-6) wherein R¹⁰ represents a fluorine atom, R⁹ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX708").

The Compound (A-6) wherein R¹⁰ represents a fluorine atom, R⁹ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX709").

The Compound (A-6) wherein R¹⁰ represents a fluorine atom, R⁹ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX710").

The Compound (A-6) wherein R¹⁰ represents a fluorine atom, R⁹ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX711").

The Compound (A-6) wherein R¹⁰ represents a fluorine atom, R⁹ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX712").

The Compound (A-6) wherein R¹⁰ represents a fluorine atom, R⁹ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX713").

The Compound (A-6) wherein R¹⁰ represents a fluorine atom, R⁹ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX714").

The Compound (A-6) wherein R¹⁰ represents a fluorine atom, R⁹ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX715").

The Compound (A-6) wherein R¹⁰ represents a fluorine atom, R⁹ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX716").

The Compound (A-6) wherein R¹⁰ represents a fluorine atom, R⁹ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX717").

The Compound (A-6) wherein R¹⁰ represents a fluorine atom, R⁹ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX718").

The Compound (A-6) wherein R¹⁰ represents a fluorine atom, R⁹ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX719").

The Compound (A-6) wherein R¹⁰ represents a fluorine atom, R⁹ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX720").

The Compound (A-6) wherein R¹⁰ represents a fluorine atom, R⁹ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX721").

The Compound (A-6) wherein R¹⁰ represents a fluorine atom, R⁹ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX722").

The Compound (A-6) wherein R¹⁰ represents a fluorine atom, R⁹ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX723").

The Compound (A-6) wherein R¹⁰ represents a fluorine atom, R⁹ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX724").

The Compound (A-6) wherein R¹⁰ represents a fluorine atom, R⁹ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX725").

The Compound (A-6) wherein R¹⁰ represents a fluorine atom, R⁹ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX726").

The Compound (A-6) wherein R¹⁰ represents a fluorine atom, R⁹ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX727").

The Compound (A-6) wherein R¹⁰ represents a fluorine atom, R⁹ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX728").

The Compound (A-6) wherein R¹⁰ represents a fluorine atom, R⁹ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX729").

The Compound (A-6) wherein R¹⁰ represents a fluorine atom, R⁹ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX730").

The Compound (A-6) wherein R¹⁰ represents a fluorine atom, R⁹ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX731").

The Compound (A-6) wherein R¹⁰ represents a chlorine atom, R⁹ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX732").

The Compound (A-6) wherein R¹⁰ represents a chlorine atom, R⁹ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX733").

The Compound (A-6) wherein R¹⁰ represents a chlorine atom, R⁹ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX734").

The Compound (A-6) wherein R¹⁰ represents a chlorine atom, R⁹ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX735").

The Compound (A-6) wherein R¹⁰ represents a chlorine atom, R⁹ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX736").

The Compound (A-6) wherein R¹⁰ represents a chlorine atom, R⁹ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX737").

The Compound (A-6) wherein R¹⁰ represents a chlorine atom, R⁹ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX738").

The Compound (A-6) wherein R¹⁰ represents a chlorine atom, R⁹ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX739").

The Compound (A-6) wherein R¹⁰ represents a chlorine atom, R⁹ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX740").

The Compound (A-6) wherein R¹⁰ represents a chlorine atom, R⁹ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX741").

The Compound (A-6) wherein R¹⁰ represents a chlorine atom, R⁹ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX742").

The Compound (A-6) wherein R¹⁰ represents a chlorine atom, R⁹ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX743").

The Compound (A-6) wherein R¹⁰ represents a chlorine atom, R⁹ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX744").

The Compound (A-6) wherein R¹⁰ represents a chlorine atom, R⁹ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX745").

The Compound (A-6) wherein R¹⁰ represents a chlorine atom, R⁹ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX746").

The Compound (A-6) wherein R¹⁰ represents a chlorine atom, R⁹ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX747").

The Compound (A-6) wherein R¹⁰ represents a chlorine atom, R⁹ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX748").

The Compound (A-6) wherein R¹⁰ represents a chlorine atom, R⁹ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX749").

The Compound (A-6) wherein R¹⁰ represents a chlorine atom, R⁹ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX750").

The Compound (A-6) wherein R¹⁰ represents a chlorine atom, R⁹ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX751").

The Compound (A-6) wherein R¹⁰ represents a chlorine atom, R⁹ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX752").

The Compound (A-6) wherein R¹⁰ represents a chlorine atom, R⁹ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX753").

The Compound (A-6) wherein R¹⁰ represents a chlorine atom, R⁹ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX754").

The Compound (A-6) wherein R¹⁰ represents a chlorine atom, R⁹ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX755").

The Compound (A-6) wherein R¹⁰ represents a chlorine atom, R⁹ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX756").

The Compound (A-6) wherein R¹⁰ represents a chlorine atom, R⁹ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX757").

The Compound (A-6) wherein R¹⁰ represents a chlorine atom, R⁹ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX758").

The Compound (A-6) wherein R¹⁰ represents a chlorine atom, R⁹ and R¹¹ each represent a hydrogen atom, n represents 2, Z represents a 3-furanyl group, and the combination of the group represented by G^{A} and the substituents R^{X7}, R^{x8}, R^{X9}, and R^{X10} in the group represented by G^{A} represents any one combination indicated in the Combination A (hereinafter referred to as "Compound group SX759").

The Compound (A-6) wherein R¹⁰ represents a chlorine atom, R⁹ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX760").

The Compound (A-6) wherein R¹⁰ represents a chlorine atom, R⁹ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX761").

The Compound (A-6) wherein R¹⁰ represents a chlorine atom, R⁹ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX762").

The Compound (A-6) wherein R¹⁰ represents a chlorine atom, R⁹ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX763").

The Compound (A-6) wherein R¹⁰ represents a chlorine atom, R⁹ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX764").

The Compound (A-6) wherein R¹⁰ represents a chlorine atom, R⁹ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX765").

The Compound (A-6) wherein R¹⁰ represents a methyl group, R⁹ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX766").

The Compound (A-6) wherein R¹⁰ represents a methyl group, R⁹ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX767").

The Compound (A-6) wherein R¹⁰ represents a methyl group, R⁹ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX768").

The Compound (A-6) wherein R¹⁰ represents a methyl group, R⁹ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX769").

The Compound (A-6) wherein R¹⁰ represents a methyl group, R⁹ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX770").

The Compound (A-6) wherein R¹⁰ represents a methyl group, R⁹ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX771").

The Compound (A-6) wherein R¹⁰ represents a methyl group, R⁹ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX772").

The Compound (A-6) wherein R¹⁰ represents a methyl group, R⁹ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX773").

The Compound (A-6) wherein R¹⁰ represents a methyl group, R⁹ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX774").

The Compound (A-6) wherein R¹⁰ represents a methyl group, R⁹ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX775").

The Compound (A-6) wherein R¹⁰ represents a methyl group, R⁹ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX776").

The Compound (A-6) wherein R¹⁰ represents a methyl group, R⁹ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX777").

The Compound (A-6) wherein R¹⁰ represents a methyl group, R⁹ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX778").

The Compound (A-6) wherein R¹⁰ represents a methyl group, R⁹ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX779").

The Compound (A-6) wherein R¹⁰ represents a methyl group, R⁹ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX780").

The Compound (A-6) wherein R¹⁰ represents a methyl group, R⁹ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX781").

The Compound (A-6) wherein R¹⁰ represents a methyl group, R⁹ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX782").

The Compound (A-6) wherein R¹⁰ represents a methyl group, R⁹ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX783").

The Compound (A-6) wherein R¹⁰ represents a methyl group, R⁹ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX784").

The Compound (A-6) wherein R¹⁰ represents a methyl group, R⁹ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX785").

The Compound (A-6) wherein R¹⁰ represents a methyl group, R⁹ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX786").

The Compound (A-6) wherein R¹⁰ represents a methyl group, R⁹ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX787").

The Compound (A-6) wherein R¹⁰ represents a methyl group, R⁹ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX788").

The Compound (A-6) wherein R¹⁰ represents a methyl group, R⁹ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX789").

The Compound (A-6) wherein R¹⁰ represents a methyl group, R⁹ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX790").

The Compound (A-6) wherein R¹⁰ represents a methyl group, R⁹ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX791").

The Compound (A-6) wherein R¹⁰ represents a methyl group, R⁹ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX792").

The Compound (A-6) wherein R¹⁰ represents a methyl group, R⁹ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX793").

The Compound (A-6) wherein R¹⁰ represents a methyl group, R⁹ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX794").

The Compound (A-6) wherein R¹⁰ represents a methyl group, R⁹ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX795").

The Compound (A-6) wherein R¹⁰ represents a methyl group, R⁹ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX796").

The Compound (A-6) wherein R¹⁰ represents a methyl group, R⁹ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX797").

The Compound (A-6) wherein R¹⁰ represents a methyl group, R⁹ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX798").

The Compound (A-6) wherein R¹⁰ represents a methyl group, R⁹ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX799").

The Compound (A-6) wherein R¹¹ represents a fluorine atom, R⁹ and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX800").

The Compound (A-6) wherein R¹¹ represents a fluorine atom, R⁹ and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX801").

The Compound (A-6) wherein R¹¹ represents a fluorine atom, R⁹ and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX802").

The Compound (A-6) wherein R¹¹ represents a fluorine atom, R⁹ and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX803").

The Compound (A-6) wherein R¹¹ represents a fluorine atom, R⁹ and R¹⁰ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX804").

The Compound (A-6) wherein R¹¹ represents a fluorine atom, R⁹ and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX805").

The Compound (A-6) wherein R¹¹ represents a fluorine atom, R⁹ and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX806").

The Compound (A-6) wherein R¹¹ represents a fluorine atom, R⁹ and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX807").

The Compound (A-6) wherein R¹¹ represents a fluorine atom, R⁹ and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX808").

The Compound (A-6) wherein R¹¹ represents a fluorine atom, R⁹ and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX809").

The Compound (A-6) wherein R¹¹ represents a fluorine atom, R⁹ and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX810").

The Compound (A-6) wherein R¹¹ represents a fluorine atom, R⁹ and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX811").

The Compound (A-6) wherein R¹¹ represents a fluorine atom, R⁹ and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX812").

The Compound (A-6) wherein R¹¹ represents a fluorine atom, R⁹ and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX813").

The Compound (A-6) wherein R¹¹ represents a fluorine atom, R⁹ and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX814").

The Compound (A-6) wherein R¹¹ represents a fluorine atom, R⁹ and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX815").

The Compound (A-6) wherein R¹¹ represents a fluorine atom, R⁹ and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX816").

The Compound (A-6) wherein R¹¹ represents a fluorine atom, R⁹ and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX817").

The Compound (A-6) wherein R¹¹ represents a fluorine atom, R⁹ and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX818").

The Compound (A-6) wherein R¹¹ represents a fluorine atom, R⁹ and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX819").

The Compound (A-6) wherein R¹¹ represents a fluorine atom, R⁹ and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX820").

The Compound (A-6) wherein R¹¹ represents a fluorine atom, R⁹ and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX821").

The Compound (A-6) wherein R¹¹ represents a fluorine atom, R⁹ and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX822").

The Compound (A-6) wherein R¹¹ represents a fluorine atom, R⁹ and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX823").

The Compound (A-6) wherein R¹¹ represents a fluorine atom, R⁹ and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX824").

The Compound (A-6) wherein R¹¹ represents a fluorine atom, R⁹ and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX825").

The Compound (A-6) wherein R¹¹ represents a fluorine atom, R⁹ and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX826").

The Compound (A-6) wherein R¹¹ represents a fluorine atom, R⁹ and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX827").

The Compound (A-6) wherein R¹¹ represents a fluorine atom, R⁹ and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX828").

The Compound (A-6) wherein R¹¹ represents a fluorine atom, R⁹ and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX829").

The Compound (A-6) wherein R¹¹ represents a fluorine atom, R⁹ and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX830").

The Compound (A-6) wherein R¹¹ represents a fluorine atom, R⁹ and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX831").

The Compound (A-6) wherein R¹¹ represents a fluorine atom, R⁹ and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX832").

The Compound (A-6) wherein R¹¹ represents a fluorine atom, R⁹ and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX833").

The Compound (A-6) wherein R¹¹ represents a chlorine atom, R⁹ and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX834").

The Compound (A-6) wherein R¹¹ represents a chlorine atom, R⁹ and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX835").

The Compound (A-6) wherein R¹¹ represents a chlorine atom, R⁹ and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX836").

The Compound (A-6) wherein R¹¹ represents a chlorine atom, R⁹ and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX837").

The Compound (A-6) wherein R¹¹ represents a chlorine atom, R⁹ and R¹⁰ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX838").

The Compound (A-6) wherein R¹¹ represents a chlorine atom, R⁹ and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX839").

The Compound (A-6) wherein R¹¹ represents a chlorine atom, R⁹ and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX840").

The Compound (A-6) wherein R¹¹ represents a chlorine atom, R⁹ and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX841").

The Compound (A-6) wherein R¹¹ represents a chlorine atom, R⁹ and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX842").

The Compound (A-6) wherein R¹¹ represents a chlorine atom, R⁹ and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX843").

The Compound (A-6) wherein R¹¹ represents a chlorine atom, R⁹ and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX844").

The Compound (A-6) wherein R¹¹ represents a chlorine atom, R⁹ and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX845").

The Compound (A-6) wherein R¹¹ represents a chlorine atom, R⁹ and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX846").

The Compound (A-6) wherein R¹¹ represents a chlorine atom, R⁹ and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX847").

The Compound (A-6) wherein R¹¹ represents a chlorine atom, R⁹ and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX848").

The Compound (A-6) wherein R¹¹ represents a chlorine atom, R⁹ and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX849").

The Compound (A-6) wherein R¹¹ represents a chlorine atom, R⁹ and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX850").

The Compound (A-6) wherein R¹¹ represents a chlorine atom, R⁹ and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX851").

The Compound (A-6) wherein R¹¹ represents a chlorine atom, R⁹ and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX852").

The Compound (A-6) wherein R¹¹ represents a chlorine atom, R⁹ and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX853").

The Compound (A-6) wherein R¹¹ represents a chlorine atom, R⁹ and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX854").

The Compound (A-6) wherein R¹¹ represents a chlorine atom, R⁹ and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX855").

The Compound (A-6) wherein R¹¹ represents a chlorine atom, R⁹ and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX856").

The Compound (A-6) wherein R¹¹ represents a chlorine atom, R⁹ and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX857").

The Compound (A-6) wherein R¹¹ represents a chlorine atom, R⁹ and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX858").

The Compound (A-6) wherein R¹¹ represents a chlorine atom, R⁹ and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX859").

The Compound (A-6) wherein R¹¹ represents a chlorine atom, R⁹ and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX860").

The Compound (A-6) wherein R¹¹ represents a chlorine atom, R⁹ and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX861").

The Compound (A-6) wherein R¹¹ represents a chlorine atom, R⁹ and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX862").

The Compound (A-6) wherein R¹¹ represents a chlorine atom, R⁹ and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX863").

The Compound (A-6) wherein R¹¹ represents a chlorine atom, R⁹ and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX864").

The Compound (A-6) wherein R¹¹ represents a chlorine atom, R⁹ and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX865").

The Compound (A-6) wherein R¹¹ represents a chlorine atom, R⁹ and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX866").

The Compound (A-6) wherein R¹¹ represents a chlorine atom, R⁹ and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX867").

The Compound (A-6) wherein R¹¹ represents a methyl group, R⁹ and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX868").

The Compound (A-6) wherein R¹¹ represents a methyl group, R⁹ and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX869").

The Compound (A-6) wherein R¹¹ represents a methyl group, R⁹ and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX870").

The Compound (A-6) wherein R¹¹ represents a methyl group, R⁹ and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX871").

The Compound (A-6) wherein R¹¹ represents a methyl group, R⁹ and R¹⁰ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX872").

The Compound (A-6) wherein R¹¹ represents a methyl group, R⁹ and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX873").

The Compound (A-6) wherein R¹¹ represents a methyl group, R⁹ and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX874").

The Compound (A-6) wherein R¹¹ represents a methyl group, R⁹ and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX875").

The Compound (A-6) wherein R¹¹ represents a methyl group, R⁹ and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX876").

The Compound (A-6) wherein R¹¹ represents a methyl group, R⁹ and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX877").

The Compound (A-6) wherein R¹¹ represents a methyl group, R⁹ and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX878").

The Compound (A-6) wherein R¹¹ represents a methyl group, R⁹ and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX879").

The Compound (A-6) wherein R¹¹ represents a methyl group, R⁹ and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX880").

The Compound (A-6) wherein R¹¹ represents a methyl group, R⁹ and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX881").

The Compound (A-6) wherein R¹¹ represents a methyl group, R⁹ and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX882").

The Compound (A-6) wherein R¹¹ represents a methyl group, R⁹ and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX883").

The Compound (A-6) wherein R¹¹ represents a methyl group, R⁹ and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX884").

The Compound (A-6) wherein R¹¹ represents a methyl group, R⁹ and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX885").

The Compound (A-6) wherein R¹¹ represents a methyl group, R⁹ and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX886").

The Compound (A-6) wherein R¹¹ represents a methyl group, R⁹ and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX887").

The Compound (A-6) wherein R¹¹ represents a methyl group, R⁹ and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX888").

The Compound (A-6) wherein R¹¹ represents a methyl group, R⁹ and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX889").

The Compound (A-6) wherein R¹¹ represents a methyl group, R⁹ and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX890").

The Compound (A-6) wherein R¹¹ represents a methyl group, R⁹ and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX891").

The Compound (A-6) wherein R¹¹ represents a methyl group, R⁹ and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX892").

The Compound (A-6) wherein R¹¹ represents a methyl group, R⁹ and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX893").

The Compound (A-6) wherein R¹¹ represents a methyl group, R⁹ and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX894").

The Compound (A-6) wherein R¹¹ represents a methyl group, R⁹ and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX895").

The Compound (A-6) wherein R¹¹ represents a methyl group, R⁹ and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX896").

The Compound (A-6) wherein R¹¹ represents a methyl group, R⁹ and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX897").

The Compound (A-6) wherein R¹¹ represents a methyl group, R⁹ and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX898").

The Compound (A-6) wherein R¹¹ represents a methyl group, R⁹ and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX899").

The Compound (A-6) wherein R¹¹ represents a methyl group, R⁹ and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX900").

The Compound (A-6) wherein R¹¹ represents a methyl group, R⁹ and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX901").

A compound represented by formula (A-7) 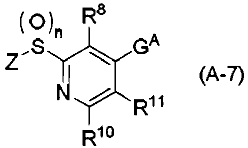 (hereinafter referred to as "Compound (A-7)") wherein R⁸, R¹⁰, and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX902").

The Compound (A-7) wherein R⁸, R¹⁰, and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX903").

The Compound (A-7) wherein R⁸, R¹⁰, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX904").

The Compound (A-7) wherein R⁸, R¹⁰, and R¹¹ each represent a hydrogen atom, n represents 1, Z represents - CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX905").

The Compound (A-7) wherein R⁸, R¹⁰, and R¹¹ each represent a hydrogen atom, n represents 1, Z represents - CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX906").

The Compound (A-7) wherein R⁸, R¹⁰, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX907").

The Compound (A-7) wherein R⁸, R¹⁰, and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX908").

The Compound (A-7) wherein R⁸, R¹⁰, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX909").

The Compound (A-7) wherein R⁸, R¹⁰, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX910").

The Compound (A-7) wherein R⁹, R¹⁰, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX911").

The Compound (A-7) wherein R⁸, R¹⁰, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX912").

The Compound (A-7) wherein R⁸, R¹⁰, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX913").

The Compound (A-7) wherein R⁸, R¹⁰, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX914").

The Compound (A-7) wherein R⁸, R¹⁰, and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX915").

The Compound (A-7) wherein R⁸, R¹⁰, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX916").

The Compound (A-7) wherein R⁸, R¹⁰, and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX917").

The Compound (A-7) wherein R⁸, R¹⁰, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX918").

The Compound (A-7) wherein R⁸, R¹⁰, and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX919").

The Compound (A-7) wherein R⁸, R¹⁰, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX920").

The Compound (A-7) wherein R⁸, R¹⁰, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX921").

The Compound (A-7) wherein R⁸, R¹⁰, and R¹¹ each represent a hydrogen atom, n represents 2, Z represents - CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX922").

The Compound (A-7) wherein R⁸, R¹⁰, and R¹¹ each represent a hydrogen atom, n represents 2, Z represents - CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX923").

The Compound (A-7) wherein R⁸, R¹⁰, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX924").

The Compound (A-7) wherein R⁸, R¹⁰, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX925").

The Compound (A-7) wherein R⁸, R¹⁰, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX926").

The Compound (A-7) wherein R⁸, R¹⁰, and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX927").

The Compound (A-7) wherein R⁸, R¹⁰, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX928").

The Compound (A-7) wherein R⁸, R¹⁰, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX929").

The Compound (A-7) wherein R⁸, R¹⁰, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX930").

The Compound (A-7) wherein R⁸, R¹⁰, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX931").

The Compound (A-7) wherein R⁸, R¹⁰, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX932").

The Compound (A-7) wherein R⁸, R¹⁰, and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX933").

The Compound (A-7) wherein R⁸, R¹⁰, and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX934").

The Compound (A-7) wherein R⁸, R¹⁰, and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX935").

The Compound (A-7) wherein R⁸ represents a fluorine atom, R¹⁰ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX936").

The Compound (A-7) wherein R⁸ represents a fluorine atom, R¹⁰ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX937").

The Compound (A-7) wherein R⁸ represents a fluorine atom, R¹⁰ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX938").

The Compound (A-7) wherein R⁸ represents a fluorine atom, R¹⁰ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX939").

The Compound (A-7) wherein R⁸ represents a fluorine atom, R¹⁰ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX940").

The Compound (A-7) wherein R⁸ represents a fluorine atom, R¹⁰ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX941").

The Compound (A-7) wherein R⁸ represents a fluorine atom, R¹⁰ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX942").

The Compound (A-7) wherein R⁸ represents a fluorine atom, R¹⁰ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX943").

The Compound (A-7) wherein R⁸ represents a fluorine atom, R¹⁰ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX944").

The Compound (A-7) wherein R⁸ represents a fluorine atom, R¹⁰ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX945").

The Compound (A-7) wherein R⁸ represents a fluorine atom, R¹⁰ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX946").

The Compound (A-7) wherein R⁸ represents a fluorine atom, R¹⁰ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX947").

The Compound (A-7) wherein R⁸ represents a fluorine atom, R¹⁰ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX948").

The Compound (A-7) wherein R⁸ represents a fluorine atom, R¹⁰ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX949").

The Compound (A-7) wherein R⁸ represents a fluorine atom, R¹⁰ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX950").

The Compound (A-7) wherein R⁸ represents a fluorine atom, R¹⁰ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX951").

The Compound (A-7) wherein R⁸ represents a fluorine atom, R¹⁰ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX952").

The Compound (A-7) wherein R⁸ represents a fluorine atom, R¹⁰ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX953").

The Compound (A-7) wherein R⁸ represents a fluorine atom, R¹⁰ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX954").

The Compound (A-7) wherein R⁸ represents a fluorine atom, R¹⁰ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX955").

The Compound (A-7) wherein R⁸ represents a fluorine atom, R¹⁰ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX956").

The Compound (A-7) wherein R⁸ represents a fluorine atom, R¹⁰ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX957").

The Compound (A-7) wherein R⁸ represents a fluorine atom, R¹⁰ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX958").

The Compound (A-7) wherein R⁸ represents a fluorine atom, R¹⁰ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX959").

The Compound (A-7) wherein R⁸ represents a fluorine atom, R¹⁰ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX960").

The Compound (A-7) wherein R⁸ represents a fluorine atom, R¹⁰ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX961").

The Compound (A-7) wherein R⁸ represents a fluorine atom, R¹⁰ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX962").

The Compound (A-7) wherein R⁸ represents a fluorine atom, R¹⁰ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX963").

The Compound (A-7) wherein R⁸ represents a fluorine atom, R¹⁰ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX964").

The Compound (A-7) wherein R⁸ represents a fluorine atom, R¹⁰ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX965").

The Compound (A-7) wherein R⁸ represents a fluorine atom, R¹⁰ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX966").

The Compound (A-7) wherein R⁸ represents a fluorine atom, R¹⁰ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX967").

The Compound (A-7) wherein R⁸ represents a fluorine atom, R¹⁰ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX968").

The Compound (A-7) wherein R⁸ represents a fluorine atom, R¹⁰ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX969").

The Compound (A-7) wherein R⁸ represents a chlorine atom, R¹⁰ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX970").

The Compound (A-7) wherein R⁸ represents a chlorine atom, R¹⁰ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX971").

The Compound (A-7) wherein R⁸ represents a chlorine atom, R¹⁰ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX972").

The Compound (A-7) wherein R⁸ represents a chlorine atom, R¹⁰ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX973").

The Compound (A-7) wherein R⁸ represents a chlorine atom, R¹⁰ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX974").

The Compound (A-7) wherein R⁸ represents a chlorine atom, R¹⁰ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX975").

The Compound (A-7) wherein R⁸ represents a chlorine atom, R¹⁰ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX976").

The Compound (A-7) wherein R⁸ represents a chlorine atom, R¹⁰ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX977").

The Compound (A-7) wherein R⁸ represents a chlorine atom, R¹⁰ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX978").

The Compound (A-7) wherein R⁸ represents a chlorine atom, R¹⁰ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX979").

The Compound (A-7) wherein R⁸ represents a chlorine atom, R¹⁰ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX980").

The Compound (A-7) wherein R⁸ represents a chlorine atom, R¹⁰ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX981").

The Compound (A-7) wherein R⁸ represents a chlorine atom, R¹⁰ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX982").

The Compound (A-7) wherein R⁸ represents a chlorine atom, R¹⁰ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX983").

The Compound (A-7) wherein R⁸ represents a chlorine atom, R¹⁰ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX984").

The Compound (A-7) wherein R⁸ represents a chlorine atom, R¹⁰ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX985").

The Compound (A-7) wherein R⁸ represents a chlorine atom, R¹⁰ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX986").

The Compound (A-7) wherein R⁸ represents a chlorine atom, R¹⁰ and R¹¹ each represent a hydrogen atom, n represents 2, Z represents an ethyl group, and the combination of the group represented by G^{A} and the substituents R^{X7}, R^{X8} , R^{X9}, and R^{X10} in the group represented by G^{A} represents any one combination indicated in the Combination A (hereinafter referred to as "Compound group SX987").

The Compound (A-7) wherein R⁸ represents a chlorine atom, R¹⁰ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX988").

The Compound (A-7) wherein R⁸ represents a chlorine atom, R¹⁰ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX989").

The Compound (A-7) wherein R⁸ represents a chlorine atom, R¹⁰ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX990").

The Compound (A-7) wherein R⁸ represents a chlorine atom, R¹⁰ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX991").

The Compound (A-7) wherein R⁸ represents a chlorine atom, R¹⁰ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX992").

The Compound (A-7) wherein R⁸ represents a chlorine atom, R¹⁰ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX993").

The Compound (A-7) wherein R⁸ represents a chlorine atom, R¹⁰ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX994").

The Compound (A-7) wherein R⁸ represents a chlorine atom, R¹⁰ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX995").

The Compound (A-7) wherein R⁸ represents a chlorine atom, R¹⁰ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX996").

The Compound (A-7) wherein R⁸ represents a chlorine atom, R¹⁰ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX997").

The Compound (A-7) wherein R⁸ represents a chlorine atom, R¹⁰ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX998").

The Compound (A-7) wherein R⁸ represents a chlorine atom, R¹⁰ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX999").

The Compound (A-7) wherein R⁸ represents a chlorine atom, R¹⁰ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1000").

The Compound (A-7) wherein R⁸ represents a chlorine atom, R¹⁰ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1001").

The Compound (A-7) wherein R⁸ represents a chlorine atom, R¹⁰ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1002").

The Compound (A-7) wherein R⁸ represents a chlorine atom, R¹⁰ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1003").

The Compound (A-7) wherein R⁸ represents a methyl group, R¹⁰ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1004").

The Compound (A-7) wherein R⁸ represents a methyl group, R¹⁰ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1005").

The Compound (A-7) wherein R⁸ represents a methyl group, R¹⁰ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1006").

The Compound (A-7) wherein R⁸ represents a methyl group, R¹⁰ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1007").

The Compound (A-7) wherein R⁸ represents a methyl group, R¹⁰ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1008").

The Compound (A-7) wherein R⁸ represents a methyl group, R¹⁰ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1009").

The Compound (A-7) wherein R⁸ represents a methyl group, R¹⁰ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1010").

The Compound (A-7) wherein R⁸ represents a methyl group, R¹⁰ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1011").

The Compound (A-7) wherein R⁸ represents a methyl group, R¹⁰ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1012").

The Compound (A-7) wherein R⁸ represents a methyl group, R¹⁰ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1013").

The Compound (A-7) wherein R⁸ represents a methyl group, R¹⁰ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1014").

The Compound (A-7) wherein R⁸ represents a methyl group, R¹⁰ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1015").

The Compound (A-7) wherein R⁸ represents a methyl group, R¹⁰ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1016").

The Compound (A-7) wherein R⁸ represents a methyl group, R¹⁰ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1017").

The Compound (A-7) wherein R⁸ represents a methyl group, R¹⁰ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1018").

The Compound (A-7) wherein R⁸ represents a methyl group, R¹⁰ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1019").

The Compound (A-7) wherein R⁸ represents a methyl group, R¹⁰ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1020").

The Compound (A-7) wherein R⁸ represents a methyl group, R¹⁰ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1021").

The Compound (A-7) wherein R⁸ represents a methyl group, R¹⁰ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1022").

The Compound (A-7) wherein R⁸ represents a methyl group, R¹⁰ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1023").

The Compound (A-7) wherein R⁸ represents a methyl group, R¹⁰ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1024").

The Compound (A-7) wherein R⁸ represents a methyl group, R¹⁰ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1025").

The Compound (A-7) wherein R⁸ represents a methyl group, R¹⁰ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1026").

The Compound (A-7) wherein R⁸ represents a methyl group, R¹⁰ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1027").

The Compound (A-7) wherein R⁸ represents a methyl group, R¹⁰ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1028").

The Compound (A-7) wherein R⁸ represents a methyl group, R¹⁰ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1029").

The Compound (A-7) wherein R⁸ represents a methyl group, R¹⁰ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1030").

The Compound (A-7) wherein R⁸ represents a methyl group, R¹⁰ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1031").

The Compound (A-7) wherein R⁸ represents a methyl group, R¹⁰ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1032").

The Compound (A-7) wherein R⁸ represents a methyl group, R¹⁰ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1033").

The Compound (A-7) wherein R⁸ represents a methyl group, R¹⁰ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1034").

The Compound (A-7) wherein R⁸ represents a methyl group, R¹⁰ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1035").

The Compound (A-7) wherein R⁸ represents a methyl group, R¹⁰ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1036").

The Compound (A-7) wherein R⁸ represents a methyl group, R¹⁰ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1037").

The Compound (A-7) wherein R¹⁰ represents a fluorine atom, R⁸ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1038").

The Compound (A-7) wherein R¹⁰ represents a fluorine atom, R⁸ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1039").

The Compound (A-7) wherein R¹⁰ represents a fluorine atom, R⁸ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1040").

The Compound (A-7) wherein R¹⁰ represents a fluorine atom, R⁸ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1041").

The Compound (A-7) wherein R¹⁰ represents a fluorine atom, R⁸ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1042").

The Compound (A-7) wherein R¹⁰ represents a fluorine atom, R⁸ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1043").

The Compound (A-7) wherein R¹⁰ represents a fluorine atom, R⁸ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1044").

The Compound (A-7) wherein R¹⁰ represents a fluorine atom, R⁸ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1045").

The Compound (A-7) wherein R¹⁰ represents a fluorine atom, R⁸ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1046").

The Compound (A-7) wherein R¹⁰ represents a fluorine atom, R⁸ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1047").

The Compound (A-7) wherein R¹⁰ represents a fluorine atom, R⁸ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1048").

The Compound (A-7) wherein R¹⁰ represents a fluorine atom, R⁸ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1049").

The Compound (A-7) wherein R¹⁰ represents a fluorine atom, R⁸ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1050").

The Compound (A-7) wherein R¹⁰ represents a fluorine atom, R⁸ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1051").

The Compound (A-7) wherein R¹⁰ represents a fluorine atom, R⁸ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1052").

The Compound (A-7) wherein R¹⁰ represents a fluorine atom, R⁸ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1053").

The Compound (A-7) wherein R¹⁰ represents a fluorine atom, R⁸ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1054").

The Compound (A-7) wherein R¹⁰ represents a fluorine atom, R⁸ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1055").

The Compound (A-7) wherein R¹⁰ represents a fluorine atom, R⁸ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1056").

The Compound (A-7) wherein R¹⁰ represents a fluorine atom, R⁸ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1057").

The Compound (A-7) wherein R¹⁰ represents a fluorine atom, R⁸ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1058").

The Compound (A-7) wherein R¹⁰ represents a fluorine atom, R⁸ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1059").

The Compound (A-7) wherein R¹⁰ represents a fluorine atom, R⁸ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1060").

The Compound (A-7) wherein R¹⁰ represents a fluorine atom, R⁸ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1061").

The Compound (A-7) wherein R¹⁰ represents a fluorine atom, R⁸ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1062").

The Compound (A-7) wherein R¹⁰ represents a fluorine atom, R⁸ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1063").

The Compound (A-7) wherein R¹⁰ represents a fluorine atom, R⁸ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1064").

The Compound (A-7) wherein R¹⁰ represents a fluorine atom, R⁸ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1065").

The Compound (A-7) wherein R¹⁰ represents a fluorine atom, R⁸ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1066").

The Compound (A-7) wherein R¹⁰ represents a fluorine atom, R⁸ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1067").

The Compound (A-7) wherein R¹⁰ represents a fluorine atom, R⁸ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1068").

The Compound (A-7) wherein R¹⁰ represents a fluorine atom, R⁸ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1069").

The Compound (A-7) wherein R¹⁰ represents a fluorine atom, R⁸ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1070").

The Compound (A-7) wherein R¹⁰ represents a fluorine atom, R⁸ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1071").

The Compound (A-7) wherein R¹⁰ represents a chlorine atom, R⁸ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1072").

The Compound (A-7) wherein R¹⁰ represents a chlorine atom, R⁸ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1073").

The Compound (A-7) wherein R¹⁰ represents a chlorine atom, R⁸ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1074").

The Compound (A-7) wherein R¹⁰ represents a chlorine atom, R⁸ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1075").

The Compound (A-7) wherein R¹⁰ represents a chlorine atom, R⁸ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1076").

The Compound (A-7) wherein R¹⁰ represents a chlorine atom, R⁸ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1077").

The Compound (A-7) wherein R¹⁰ represents a chlorine atom, R⁸ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1078").

The Compound (A-7) wherein R¹⁰ represents a chlorine atom, R⁸ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1079").

The Compound (A-7) wherein R¹⁰ represents a chlorine atom, R⁸ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1080").

The Compound (A-7) wherein R¹⁰ represents a chlorine atom, R⁸ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1081").

The Compound (A-7) wherein R¹⁰ represents a chlorine atom, R⁸ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1082").

The Compound (A-7) wherein R¹⁰ represents a chlorine atom, R⁸ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1083").

The Compound (A-7) wherein R¹⁰ represents a chlorine atom, R⁸ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1084").

The Compound (A-7) wherein R¹⁰ represents a chlorine atom, R⁸ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1085").

The Compound (A-7) wherein R¹⁰ represents a chlorine atom, R⁸ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1086").

The Compound (A-7) wherein R¹⁰ represents a chlorine atom, R⁸ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1087").

The Compound (A-7) wherein R¹⁰ represents a chlorine atom, R⁸ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1088").

The Compound (A-7) wherein R¹⁰ represents a chlorine atom, R⁸ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1089").

The Compound (A-7) wherein R¹⁰ represents a chlorine atom, R⁸ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1090").

The Compound (A-7) wherein R¹⁰ represents a chlorine atom, R⁸ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1091").

The Compound (A-7) wherein R¹⁰ represents a chlorine atom, R⁸ and R¹¹ each represent a hydrogen atom, n represents 2, Z represents -CH=CHCH₃, and the combination of the group represented by G^{A} and the substituents R^{X7}, R^{XB}, R^{X9}, and R^{X10} in the group represented by G^{A} represents any one combination indicated in the Combination A (hereinafter referred to as "Compound group SX1092").

The Compound (A-7) wherein R¹⁰ represents a chlorine atom, R⁸ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1093").

The Compound (A-7) wherein R¹⁰ represents a chlorine atom, R⁸ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1094").

The Compound (A-7) wherein R¹⁰ represents a chlorine atom, R⁸ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1095").

The Compound (A-7) wherein R¹⁰ represents a chlorine atom, R⁸ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1096").

The Compound (A-7) wherein R¹⁰ represents a chlorine atom, R⁸ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1097").

The Compound (A-7) wherein R¹⁰ represents a chlorine atom, R⁸ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1098").

The Compound (A-7) wherein R¹⁰ represents a chlorine atom, R⁸ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1099").

The Compound (A-7) wherein R¹⁰ represents a chlorine atom, R⁸ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1100").

The Compound (A-7) wherein R¹⁰ represents a chlorine atom, R⁸ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1101").

The Compound (A-7) wherein R¹⁰ represents a chlorine atom, R⁸ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1102").

The Compound (A-7) wherein R¹⁰ represents a chlorine atom, R⁸ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1103").

The Compound (A-7) wherein R¹⁰ represents a chlorine atom, R⁸ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1104").

The Compound (A-7) wherein R¹⁰ represents a chlorine atom, R⁸ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1105").

The Compound (A-7) wherein R¹⁰ represents a methyl group, R⁸ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1106").

The Compound (A-7) wherein R¹⁰ represents a methyl group, R⁸ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1107").

The Compound (A-7) wherein R¹⁰ represents a methyl group, R⁸ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1108").

The Compound (A-7) wherein R¹⁰ represents a methyl group, R⁸ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1109").

The Compound (A-7) wherein R¹⁰ represents a methyl group, R⁸ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1110").

The Compound (A-7) wherein R¹⁰ represents a methyl group, R⁸ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1111").

The Compound (A-7) wherein R¹⁰ represents a methyl group, R⁸ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1112").

The Compound (A-7) wherein R¹⁰ represents a methyl group, R⁸ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1113").

The Compound (A-7) wherein R¹⁰ represents a methyl group, R⁸ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1114").

The Compound (A-7) wherein R¹⁰ represents a methyl group, R⁸ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1115").

The Compound (A-7) wherein R¹⁰ represents a methyl group, R⁸ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1116").

The Compound (A-7) wherein R¹⁰ represents a methyl group, R⁸ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1117").

The Compound (A-7) wherein R¹⁰ represents a methyl group, R⁸ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1118").

The Compound (A-7) wherein R¹⁰ represents a methyl group, R⁸ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1119").

The Compound (A-7) wherein R¹⁰ represents a methyl group, R⁸ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1120").

The Compound (A-7) wherein R¹⁰ represents a methyl group, R⁸ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1121").

The Compound (A-7) wherein R¹⁰ represents a methyl group, R⁸ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1122").

The Compound (A-7) wherein R¹⁰ represents a methyl group, R⁸ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1123").

The Compound (A-7) wherein R¹⁰ represents a methyl group, R⁸ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1124").

The Compound (A-7) wherein R¹⁰ represents a methyl group, R⁸ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1125").

The Compound (A-7) wherein R¹⁰ represents a methyl group, R⁸ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1126").

The Compound (A-7) wherein R¹⁰ represents a methyl group, R⁸ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1127").

The Compound (A-7) wherein R¹⁰ represents a methyl group, R⁸ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1128").

The Compound (A-7) wherein R¹⁰ represents a methyl group, R⁸ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1129").

The Compound (A-7) wherein R¹⁰ represents a methyl group, R⁸ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1130").

The Compound (A-7) wherein R¹⁰ represents a methyl group, R⁸ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1131").

The Compound (A-7) wherein R¹⁰ represents a methyl group, R⁸ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1132").

The Compound (A-7) wherein R¹⁰ represents a methyl group, R⁸ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1133").

The Compound (A-7) wherein R¹⁰ represents a methyl group, R⁸ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1134").

The Compound (A-7) wherein R¹⁰ represents a methyl group, R⁸ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1135").

The Compound (A-7) wherein R¹⁰ represents a methyl group, R⁸ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1136").

The Compound (A-7) wherein R¹⁰ represents a methyl group, R⁸ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1137").

The Compound (A-7) wherein R¹⁰ represents a methyl group, R⁸ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1138").

The Compound (A-7) wherein R¹⁰ represents a methyl group, R⁸ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1139").

The Compound (A-7) wherein R¹¹ represents a fluorine atom, R⁸ and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1140").

The Compound (A-7) wherein R¹¹ represents a fluorine atom, R⁸ and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1141").

The Compound (A-7) wherein R¹¹ represents a fluorine atom, R⁸ and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1142").

The Compound (A-7) wherein R¹¹ represents a fluorine atom, R⁸ and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1143").

The Compound (A-7) wherein R¹¹ represents a fluorine atom, R⁸ and R¹⁰ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1144").

The Compound (A-7) wherein R¹¹ represents a fluorine atom, R⁸ and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1145").

The Compound (A-7) wherein R¹¹ represents a fluorine atom, R⁸ and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1146").

The Compound (A-7) wherein R¹¹ represents a fluorine atom, R⁸ and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1147").

The Compound (A-7) wherein R¹¹ represents a fluorine atom, R⁸ and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1148").

The Compound (A-7) wherein R¹¹ represents a fluorine atom, R⁸ and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1149").

The Compound (A-7) wherein R¹¹ represents a fluorine atom, R⁸ and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1150").

The Compound (A-7) wherein R¹¹ represents a fluorine atom, R⁸ and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1151").

The Compound (A-7) wherein R¹¹ represents a fluorine atom, R⁸ and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1152").

The Compound (A-7) wherein R¹¹ represents a fluorine atom, R⁸ and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1153").

The Compound (A-7) wherein R¹¹ represents a fluorine atom, R⁸ and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1154").

The Compound (A-7) wherein R¹¹ represents a fluorine atom, R⁸ and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1155").

The Compound (A-7) wherein R¹¹ represents a fluorine atom, R⁸ and R¹⁰ each represent a hydrogen atom, n represents 1, Z represents a 2-pyrazinyl group, and the combination of the group represented by G^{A} and the substituents R^{X7}, R^{XB}, R^{X9}, and R^{X10} in the group represented by G^{A} represents any one combination indicated in the Combination A (hereinafter referred to as "Compound group SX1156").

The Compound (A-7) wherein R¹¹ represents a fluorine atom, R⁸ and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1157").

The Compound (A-7) wherein R¹¹ represents a fluorine atom, R⁸ and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1158").

The Compound (A-7) wherein R¹¹ represents a fluorine atom, R⁸ and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1159").

The Compound (A-7) wherein R¹¹ represents a fluorine atom, R⁸ and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1160").

The Compound (A-7) wherein R¹¹ represents a fluorine atom, R⁸ and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1161").

The Compound (A-7) wherein R¹¹ represents a fluorine atom, R⁸ and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1162").

The Compound (A-7) wherein R¹¹ represents a fluorine atom, R⁸ and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1163").

The Compound (A-7) wherein R¹¹ represents a fluorine atom, R⁸ and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1164").

The Compound (A-7) wherein R¹¹ represents a fluorine atom, R⁸ and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1165").

The Compound (A-7) wherein R¹¹ represents a fluorine atom, R⁸ and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1166").

The Compound (A-7) wherein R¹¹ represents a fluorine atom, R⁸ and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1167").

The Compound (A-7) wherein R¹¹ represents a fluorine atom, R⁸ and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1168").

The Compound (A-7) wherein R¹¹ represents a fluorine atom, R⁸ and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1169").

The Compound (A-7) wherein R¹¹ represents a fluorine atom, R⁸ and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1170").

The Compound (A-7) wherein R¹¹ represents a fluorine atom, R⁸ and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1171").

The Compound (A-7) wherein R¹¹ represents a fluorine atom, R⁸ and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1172").

The Compound (A-7) wherein R¹¹ represents a fluorine atom, R⁸ and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1173").

The Compound (A-7) wherein R¹¹ represents a chlorine atom, R⁸ and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1174").

The Compound (A-7) wherein R¹¹ represents a chlorine atom, R⁸ and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1175").

The Compound (A-7) wherein R¹¹ represents a chlorine atom, R⁸ and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1176").

The Compound (A-7) wherein R¹¹ represents a chlorine atom, R⁸ and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1177").

The Compound (A-7) wherein R¹¹ represents a chlorine atom, R⁸ and R¹⁰ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1178").

The Compound (A-7) wherein R¹¹ represents a chlorine atom, R⁸ and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1179").

The Compound (A-7) wherein R¹¹ represents a chlorine atom, R⁸ and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1180").

The Compound (A-7) wherein R¹¹ represents a chlorine atom, R⁸ and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1181").

The Compound (A-7) wherein R¹¹ represents a chlorine atom, R⁸ and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1182").

The Compound (A-7) wherein R¹¹ represents a chlorine atom, R⁸ and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1183").

The Compound (A-7) wherein R¹¹ represents a chlorine atom, R⁸ and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1184").

The Compound (A-7) wherein R¹¹ represents a chlorine atom, R⁸ and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1185").

The Compound (A-7) wherein R¹¹ represents a chlorine atom, R⁸ and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1186").

The Compound (A-7) wherein R¹¹ represents a chlorine atom, R⁸ and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1187").

The Compound (A-7) wherein R¹¹ represents a chlorine atom, R⁸ and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1188").

The Compound (A-7) wherein R¹¹ represents a chlorine atom, R⁸ and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1189").

The Compound (A-7) wherein R¹¹ represents a chlorine atom, R⁸ and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1190").

The Compound (A-7) wherein R¹¹ represents a chlorine atom, R⁸ and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1191").

The Compound (A-7) wherein R¹¹ represents a chlorine atom, R⁸ and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1192").

The Compound (A-7) wherein R¹¹ represents a chlorine atom, R⁸ and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1193").

The Compound (A-7) wherein R¹¹ represents a chlorine atom, R⁸ and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1194").

The Compound (A-7) wherein R¹¹ represents a chlorine atom, R⁸ and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1195").

The Compound (A-7) wherein R¹¹ represents a chlorine atom, R⁸ and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1196").

The Compound (A-7) wherein R¹¹ represents a chlorine atom, R⁸ and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1197").

The Compound (A-7) wherein R¹¹ represents a chlorine atom, R⁸ and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1198").

The Compound (A-7) wherein R¹¹ represents a chlorine atom, R⁸ and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1199").

The Compound (A-7) wherein R¹¹ represents a chlorine atom, R⁸ and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1200").

The Compound (A-7) wherein R¹¹ represents a chlorine atom, R⁸ and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1201").

The Compound (A-7) wherein R¹¹ represents a chlorine atom, R⁸ and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1202").

The Compound (A-7) wherein R¹¹ represents a chlorine atom, R⁸ and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1203").

The Compound (A-7) wherein R¹¹ represents a chlorine atom, R⁸ and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1204").

The Compound (A-7) wherein R¹¹ represents a chlorine atom, R⁸ and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1205").

The Compound (A-7) wherein R¹¹ represents a chlorine atom, R⁸ and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1206").

The Compound (A-7) wherein R¹¹ represents a chlorine atom, R⁸ and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1207").

The Compound (A-7) wherein R¹¹ represents a methyl group, R⁸ and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1208").

The Compound (A-7) wherein R¹¹ represents a methyl group, R⁸ and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1209").

The Compound (A-7) wherein R¹¹ represents a methyl group, R⁸ and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1210").

The Compound (A-7) wherein R¹¹ represents a methyl group, R⁸ and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1211").

The Compound (A-7) wherein R¹¹ represents a methyl group, R⁸ and R¹⁰ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1212").

The Compound (A-7) wherein R¹¹ represents a methyl group, R⁸ and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1213").

The Compound (A-7) wherein R¹¹ represents a methyl group, R⁸ and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1214").

The Compound (A-7) wherein R¹¹ represents a methyl group, R⁸ and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1215").

The Compound (A-7) wherein R¹¹ represents a methyl group, R⁸ and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1216").

The Compound (A-7) wherein R¹¹ represents a methyl group, R⁸ and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1217").

The Compound (A-7) wherein R¹¹ represents a methyl group, R⁸ and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1218").

The Compound (A-7) wherein R¹¹ represents a methyl group, R⁸ and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1219").

The Compound (A-7) wherein R¹¹ represents a methyl group, R⁸ and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1220").

The Compound (A-7) wherein R¹¹ represents a methyl group, R⁸ and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1221").

The Compound (A-7) wherein R¹¹ represents a methyl group, R⁸ and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1222").

The Compound (A-7) wherein R¹¹ represents a methyl group, R⁸ and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1223").

The Compound (A-7) wherein R¹¹ represents a methyl group, R⁸ and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1224").

The Compound (A-7) wherein R¹¹ represents a methyl group, R⁸ and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1225").

The Compound (A-7) wherein R¹¹ represents a methyl group, R⁸ and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1226").

The Compound (A-7) wherein R¹¹ represents a methyl group, R⁸ and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1227").

The Compound (A-7) wherein R¹¹ represents a methyl group, R⁸ and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1228").

The Compound (A-7) wherein R¹¹ represents a methyl group, R⁸ and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1229").

The Compound (A-7) wherein R¹¹ represents a methyl group, R⁸ and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1230").

The Compound (A-7) wherein R¹¹ represents a methyl group, R⁸ and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1231").

The Compound (A-7) wherein R¹¹ represents a methyl group, R⁸ and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1232").

The Compound (A-7) wherein R¹¹ represents a methyl group, R⁸ and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1233").

The Compound (A-7) wherein R¹¹ represents a methyl group, R⁸ and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1234").

The Compound (A-7) wherein R¹¹ represents a methyl group, R⁸ and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1235").

The Compound (A-7) wherein R¹¹ represents a methyl group, R⁸ and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1236").

The Compound (A-7) wherein R¹¹ represents a methyl group, R⁸ and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1237").

The Compound (A-7) wherein R¹¹ represents a methyl group, R⁸ and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1238").

The Compound (A-7) wherein R¹¹ represents a methyl group, R⁸ and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1239").

The Compound (A-7) wherein R¹¹ represents a methyl group, R⁸ and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1240").

The Compound (A-7) wherein R¹¹ represents a methyl group, R⁸ and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1241").

A compound represented by formula (A-8) 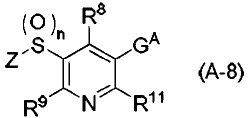 (hereinafter referred to as "Compound (A-8)") wherein R⁸, R⁹, and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1242").

The Compound (A-8) wherein R⁸, R⁹, and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1243").

The Compound (A-8) wherein R⁸, R⁹, and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1244").

The Compound (A-8) wherein R⁸, R⁹, and R¹¹ each represent a hydrogen atom, n represents 1, Z represents - CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1245").

The Compound (A-8) wherein R⁸, R⁹, and R¹¹ each represent a hydrogen atom, n represents 1, Z represents - CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1246").

The Compound (A-8) wherein R⁸, R⁹, and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1247").

The Compound (A-8) wherein R⁸, R⁹, and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1248").

The Compound (A-8) wherein R⁸, R⁹, and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1249").

The Compound (A-8) wherein R⁸, R⁹, and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1250").

The Compound (A-8) wherein R⁸, R⁹, and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1251").

The Compound (A-8) wherein R⁸, R⁹, and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1252").

The Compound (A-8) wherein R⁸, R⁹, and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1253").

The Compound (A-8) wherein R⁸, R⁹, and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1254").

The Compound (A-8) wherein R⁸, R⁹, and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1255").

The Compound (A-8) wherein R⁸, R⁹, and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1256").

The Compound (A-8) wherein R⁸, R⁹, and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1257").

The Compound (A-8) wherein R⁸, R⁹, and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1258").

The Compound (A-8) wherein R⁸, R⁹, and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1259").

The Compound (A-8) wherein R⁸, R⁹, and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1260").

The Compound (A-8) wherein R⁸, R⁹, and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1261").

The Compound (A-8) wherein R⁸, R⁹, and R¹¹ each represent a hydrogen atom, n represents 2, Z represents - CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1262").

The Compound (A-8) wherein R⁸, R⁹, and R¹¹ each represent a hydrogen atom, n represents 2, Z represents - CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1263").

The Compound (A-8) wherein R⁸, R⁹, and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1264").

The Compound (A-8) wherein R⁸, R⁹, and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1265").

The Compound (A-8) wherein R⁸, R⁹, and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1266").

The Compound (A-8) wherein R⁸, R⁹, and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1267").

The Compound (A-8) wherein R⁸, R⁹, and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1268").

The Compound (A-8) wherein R⁸, R⁹, and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1269").

The Compound (A-8) wherein R⁸, R⁹, and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1270").

The Compound (A-8) wherein R⁸, R⁹, and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1271").

The Compound (A-8) wherein R⁸, R⁹, and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1272").

The Compound (A-8) wherein R⁸, R⁹, and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1273").

The Compound (A-8) wherein R⁸, R⁹, and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1274").

The Compound (A-8) wherein R⁸, R⁹, and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1275").

The Compound (A-8) wherein R⁸ represents a fluorine atom, R⁹ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1276").

The Compound (A-8) wherein R⁸ represents a fluorine atom, R⁹ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1277").

The Compound (A-8) wherein R⁸ represents a fluorine atom, R⁹ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1278").

The Compound (A-8) wherein R⁸ represents a fluorine atom, R⁹ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1279").

The Compound (A-8) wherein R⁸ represents a fluorine atom, R⁹ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1280").

The Compound (A-8) wherein R⁸ represents a fluorine atom, R⁹ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1281").

The Compound (A-8) wherein R⁸ represents a fluorine atom, R⁹ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1282").

The Compound (A-8) wherein R⁸ represents a fluorine atom, R⁹ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1283").

The Compound (A-8) wherein R⁸ represents a fluorine atom, R⁹ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1284").

The Compound (A-8) wherein R⁸ represents a fluorine atom, R⁹ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1285").

The Compound (A-8) wherein R⁸ represents a fluorine atom, R⁹ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1286").

The Compound (A-8) wherein R⁸ represents a fluorine atom, R⁹ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1287").

The Compound (A-8) wherein R⁸ represents a fluorine atom, R⁹ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1288").

The Compound (A-8) wherein R⁸ represents a fluorine atom, R⁹ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1289").

The Compound (A-8) wherein R⁸ represents a fluorine atom, R⁹ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1290").

The Compound (A-8) wherein R⁸ represents a fluorine atom, R⁹ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1291").

The Compound (A-8) wherein R⁸ represents a fluorine atom, R⁹ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1292").

The Compound (A-8) wherein R⁸ represents a fluorine atom, R⁹ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1293").

The Compound (A-8) wherein R⁸ represents a fluorine atom, R⁹ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1294").

The Compound (A-8) wherein R⁸ represents a fluorine atom, R⁹ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1295").

The Compound (A-8) wherein R⁸ represents a fluorine atom, R⁹ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1296").

The Compound (A-8) wherein R⁸ represents a fluorine atom, R⁹ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1297").

The Compound (A-8) wherein R⁸ represents a fluorine atom, R⁹ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1298").

The Compound (A-8) wherein R⁸ represents a fluorine atom, R⁹ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1299").

The Compound (A-8) wherein R⁸ represents a fluorine atom, R⁹ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1300").

The Compound (A-8) wherein R⁸ represents a fluorine atom, R⁹ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1301").

The Compound (A-8) wherein R⁸ represents a fluorine atom, R⁹ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1302").

The Compound (A-8) wherein R⁸ represents a fluorine atom, R⁹ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1303").

The Compound (A-8) wherein R⁸ represents a fluorine atom, R⁹ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1304").

The Compound (A-8) wherein R⁸ represents a fluorine atom, R⁹ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1305").

The Compound (A-8) wherein R⁸ represents a fluorine atom, R⁹ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1306").

The Compound (A-8) wherein R⁸ represents a fluorine atom, R⁹ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1307").

The Compound (A-8) wherein R⁸ represents a fluorine atom, R⁹ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1308").

The Compound (A-8) wherein R⁸ represents a fluorine atom, R⁹ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1309").

The Compound (A-8) wherein R⁸ represents a chlorine atom, R⁹ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1310").

The Compound (A-8) wherein R⁸ represents a chlorine atom, R⁹ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1311").

The Compound (A-8) wherein R⁸ represents a chlorine atom, R⁹ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1312").

The Compound (A-8) wherein R⁸ represents a chlorine atom, R⁹ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1313").

The Compound (A-8) wherein R⁸ represents a chlorine atom, R⁹ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1314").

The Compound (A-8) wherein R⁸ represents a chlorine atom, R⁹ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1315").

The Compound (A-8) wherein R⁸ represents a chlorine atom, R⁹ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1316").

The Compound (A-8) wherein R⁸ represents a chlorine atom, R⁹ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1317").

The Compound (A-8) wherein R⁸ represents a chlorine atom, R⁹ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1318").

The Compound (A-8) wherein R⁸ represents a chlorine atom, R⁹ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1319").

The Compound (A-8) wherein R⁸ represents a chlorine atom, R⁹ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1320").

The Compound (A-8) wherein R⁸ represents a chlorine atom, R⁹ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1321").

The Compound (A-8) wherein R⁸ represents a chlorine atom, R⁹ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1322").

The Compound (A-8) wherein R⁸ represents a chlorine atom, R⁹ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1323").

The Compound (A-8) wherein R⁸ represents a chlorine atom, R⁹ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1324").

The Compound (A-8) wherein R⁸ represents a chlorine atom, R⁹ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1325").

The Compound (A-8) wherein R⁸ represents a chlorine atom, R⁹ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1326").

The Compound (A-8) wherein R⁸ represents a chlorine atom, R⁹ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1327").

The Compound (A-8) wherein R⁸ represents a chlorine atom, R⁹ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1328").

The Compound (A-8) wherein R⁸ represents a chlorine atom, R⁹ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1329").

The Compound (A-8) wherein R⁸ represents a chlorine atom, R⁹ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1330").

The Compound (A-8) wherein R⁸ represents a chlorine atom, R⁹ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1331").

The Compound (A-8) wherein R⁸ represents a chlorine atom, R⁹ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1332").

The Compound (A-8) wherein R⁸ represents a chlorine atom, R⁹ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1333").

The Compound (A-8) wherein R⁸ represents a chlorine atom, R⁹ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1334").

The Compound (A-8) wherein R⁸ represents a chlorine atom, R⁹ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1335").

The Compound (A-8) wherein R⁸ represents a chlorine atom, R⁹ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1336").

The Compound (A-8) wherein R⁸ represents a chlorine atom, R⁹ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1337").

The Compound (A-8) wherein R⁸ represents a chlorine atom, R⁹ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1338").

The Compound (A-8) wherein R⁸ represents a chlorine atom, R⁹ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1339").

The Compound (A-8) wherein R⁸ represents a chlorine atom, R⁹ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1340").

The Compound (A-8) wherein R⁸ represents a chlorine atom, R⁹ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1341").

The Compound (A-8) wherein R⁸ represents a chlorine atom, R⁹ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1342").

The Compound (A-8) wherein R⁸ represents a chlorine atom, R⁹ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1343").

The Compound (A-8) wherein R⁸ represents a methyl group, R⁹ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1344").

The Compound (A-8) wherein R⁸ represents a methyl group, R⁹ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1345").

The Compound (A-8) wherein R⁸ represents a methyl group, R⁹ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1346").

The Compound (A-8) wherein R⁸ represents a methyl group, R⁹ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1347").

The Compound (A-8) wherein R⁸ represents a methyl group, R⁹ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1348").

The Compound (A-8) wherein R⁸ represents a methyl group, R⁹ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1349").

The Compound (A-8) wherein R⁸ represents a methyl group, R⁹ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1350").

The Compound (A-8) wherein R⁸ represents a methyl group, R⁹ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1351").

The Compound (A-8) wherein R⁸ represents a methyl group, R⁹ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1352").

The Compound (A-8) wherein R⁸ represents a methyl group, R⁹ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1353").

The Compound (A-8) wherein R⁸ represents a methyl group, R⁹ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1354").

The Compound (A-8) wherein R⁸ represents a methyl group, R⁹ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1355").

The Compound (A-8) wherein R⁸ represents a methyl group, R⁹ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1356").

The Compound (A-8) wherein R⁸ represents a methyl group, R⁹ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1357").

The Compound (A-8) wherein R⁸ represents a methyl group, R⁹ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1358").

The Compound (A-8) wherein R⁸ represents a methyl group, R⁹ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1359").

The Compound (A-8) wherein R⁸ represents a methyl group, R⁹ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1360").

The Compound (A-8) wherein R⁸ represents a methyl group, R⁹ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1361").

The Compound (A-8) wherein R⁸ represents a methyl group, R⁹ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1362").

The Compound (A-8) wherein R⁸ represents a methyl group, R⁹ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1363").

The Compound (A-8) wherein R⁸ represents a methyl group, R⁹ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1364").

The Compound (A-8) wherein R⁸ represents a methyl group, R⁹ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1365").

The Compound (A-8) wherein R⁸ represents a methyl group, R⁹ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1366").

The Compound (A-8) wherein R⁸ represents a methyl group, R⁹ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1367").

The Compound (A-8) wherein R⁸ represents a methyl group, R⁹ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1368").

The Compound (A-8) wherein R⁸ represents a methyl group, R⁹ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1369").

The Compound (A-8) wherein R⁸ represents a methyl group, R⁹ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1370").

The Compound (A-8) wherein R⁸ represents a methyl group, R⁹ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1371").

The Compound (A-8) wherein R⁸ represents a methyl group, R⁹ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1372").

The Compound (A-8) wherein R⁸ represents a methyl group, R⁹ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1373").

The Compound (A-8) wherein R⁸ represents a methyl group, R⁹ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1374").

The Compound (A-8) wherein R⁸ represents a methyl group, R⁹ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1375").

The Compound (A-8) wherein R⁸ represents a methyl group, R⁹ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1376").

The Compound (A-8) wherein R⁸ represents a methyl group, R⁹ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1377").

The Compound (A-8) wherein R⁹ represents a fluorine atom, R⁸ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1378").

The Compound (A-8) wherein R⁹ represents a fluorine atom, R⁸ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1379").

The Compound (A-8) wherein R⁹ represents a fluorine atom, R⁸ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1380").

The Compound (A-8) wherein R⁹ represents a fluorine atom, R⁸ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1381").

The Compound (A-8) wherein R⁹ represents a fluorine atom, R⁸ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1382").

The Compound (A-8) wherein R⁹ represents a fluorine atom, R⁸ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1383").

The Compound (A-8) wherein R⁹ represents a fluorine atom, R⁸ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1384").

The Compound (A-8) wherein R⁹ represents a fluorine atom, R⁹ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1385").

The Compound (A-8) wherein R⁹ represents a fluorine atom, R⁸ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1386").

The Compound (A-8) wherein R⁹ represents a fluorine atom, R⁸ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1387").

The Compound (A-8) wherein R⁹ represents a fluorine atom, R⁸ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1388").

The Compound (A-8) wherein R⁹ represents a fluorine atom, R⁸ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1389").

The Compound (A-8) wherein R⁹ represents a fluorine atom, R⁸ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1390").

The Compound (A-8) wherein R⁹ represents a fluorine atom, R⁸ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1391").

The Compound (A-8) wherein R⁹ represents a fluorine atom, R⁸ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1392").

The Compound (A-8) wherein R⁹ represents a fluorine atom, R⁸ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1393").

The Compound (A-8) wherein R⁹ represents a fluorine atom, R⁸ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1394").

The Compound (A-8) wherein R⁹ represents a fluorine atom, R⁸ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1395").

The Compound (A-8) wherein R⁹ represents a fluorine atom, R⁸ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1396").

The Compound (A-8) wherein R⁹ represents a fluorine atom, R⁸ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1397").

The Compound (A-8) wherein R⁹ represents a fluorine atom, R⁸ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1398").

The Compound (A-8) wherein R⁹ represents a fluorine atom, R⁸ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1399").

The Compound (A-8) wherein R⁹ represents a fluorine atom, R⁸ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1400").

The Compound (A-8) wherein R⁹ represents a fluorine atom, R⁸ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1401").

The Compound (A-8) wherein R⁹ represents a fluorine atom, R⁸ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1402").

The Compound (A-8) wherein R⁹ represents a fluorine atom, R⁸ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1403").

The Compound (A-8) wherein R⁹ represents a fluorine atom, R⁹ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1404").

The Compound (A-8) wherein R⁹ represents a fluorine atom, R⁸ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1405").

The Compound (A-8) wherein R⁹ represents a fluorine atom, R⁸ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1406").

The Compound (A-8) wherein R⁹ represents a fluorine atom, R⁸ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1407").

The Compound (A-8) wherein R⁹ represents a fluorine atom, R⁸ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1408").

The Compound (A-8) wherein R⁹ represents a fluorine atom, R⁸ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1409").

The Compound (A-8) wherein R⁹ represents a fluorine atom, R⁸ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1410").

The Compound (A-8) wherein R⁹ represents a fluorine atom, R⁸ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1411").

The Compound (A-8) wherein R⁹ represents a chlorine atom, R⁸ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1412").

The Compound (A-8) wherein R⁹ represents a chlorine atom, R⁸ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1413").

The Compound (A-8) wherein R⁹ represents a chlorine atom, R⁸ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1414").

The Compound (A-8) wherein R⁹ represents a chlorine atom, R⁸ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1415").

The Compound (A-8) wherein R⁹ represents a chlorine atom, R⁸ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1416").

The Compound (A-8) wherein R⁹ represents a chlorine atom, R⁸ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1417").

The Compound (A-8) wherein R⁹ represents a chlorine atom, R⁸ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1418").

The Compound (A-8) wherein R⁹ represents a chlorine atom, R⁸ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1419").

The Compound (A-8) wherein R⁹ represents a chlorine atom, R⁸ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1420").

The Compound (A-8) wherein R⁹ represents a chlorine atom, R⁸ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1421").

The Compound (A-8) wherein R⁹ represents a chlorine atom, R⁸ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1422").

The Compound (A-8) wherein R⁹ represents a chlorine atom, R⁸ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1423").

The Compound (A-8) wherein R⁹ represents a chlorine atom, R⁸ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1424").

The Compound (A-8) wherein R⁹ represents a chlorine atom, R⁸ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1425").

The Compound (A-8) wherein R⁹ represents a chlorine atom, R⁹ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1426").

The Compound (A-8) wherein R⁹ represents a chlorine atom, R⁸ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1427").

The Compound (A-8) wherein R⁹ represents a chlorine atom, R⁸ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1428").

The Compound (A-8) wherein R⁹ represents a chlorine atom, R⁸ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1429").

The Compound (A-8) wherein R⁹ represents a chlorine atom, R⁸ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1430").

The Compound (A-8) wherein R⁹ represents a chlorine atom, R⁸ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1431").

The Compound (A-8) wherein R⁹ represents a chlorine atom, R⁸ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1432").

The Compound (A-8) wherein R⁹ represents a chlorine atom, R⁸ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1433").

The Compound (A-8) wherein R⁹ represents a chlorine atom, R⁸ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1434").

The Compound (A-8) wherein R⁹ represents a chlorine atom, R⁸ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1435").

The Compound (A-8) wherein R⁹ represents a chlorine atom, R⁸ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1436").

The Compound (A-8) wherein R⁹ represents a chlorine atom, R⁸ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1437").

The Compound (A-8) wherein R⁹ represents a chlorine atom, R⁸ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1438").

The Compound (A-8) wherein R⁹ represents a chlorine atom, R⁸ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1439").

The Compound (A-8) wherein R⁹ represents a chlorine atom, R⁸ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1440").

The Compound (A-8) wherein R⁹ represents a chlorine atom, R⁸ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1441").

The Compound (A-8) wherein R⁹ represents a chlorine atom, R⁸ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1442").

The Compound (A-8) wherein R⁹ represents a chlorine atom, R⁸ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1443").

The Compound (A-8) wherein R⁹ represents a chlorine atom, R⁸ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1444").

The Compound (A-8) wherein R⁹ represents a chlorine atom, R⁹ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1445").

The Compound (A-8) wherein R⁹ represents a methyl group, R⁸ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1446").

The Compound (A-8) wherein R⁹ represents a methyl group, R⁸ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1447").

The Compound (A-8) wherein R⁹ represents a methyl group, R⁸ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1448").

The Compound (A-8) wherein R⁹ represents a methyl group, R⁸ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1449").

The Compound (A-8) wherein R⁹ represents a methyl group, R⁸ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1450").

The Compound (A-8) wherein R⁹ represents a methyl group, R⁸ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1451").

The Compound (A-8) wherein R⁹ represents a methyl group, R⁸ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1452").

The Compound (A-8) wherein R⁹ represents a methyl group, R⁸ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1453").

The Compound (A-8) wherein R⁹ represents a methyl group, R⁸ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1454").

The Compound (A-8) wherein R⁹ represents a methyl group, R⁸ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1455").

The Compound (A-8) wherein R⁹ represents a methyl group, R⁸ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1456").

The Compound (A-8) wherein R⁹ represents a methyl group, R⁸ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1457").

The Compound (A-8) wherein R⁹ represents a methyl group, R⁸ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1458").

The Compound (A-8) wherein R⁹ represents a methyl group, R⁸ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1459").

The Compound (A-8) wherein R⁹ represents a methyl group, R⁸ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1460").

The Compound (A-8) wherein R⁹ represents a methyl group, R⁸ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1461").

The Compound (A-8) wherein R⁹ represents a methyl group, R⁸ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1462").

The Compound (A-8) wherein R⁹ represents a methyl group, R⁸ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1463").

The Compound (A-8) wherein R⁹ represents a methyl group, R⁸ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1464").

The Compound (A-8) wherein R⁹ represents a methyl group, R⁸ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1465").

The Compound (A-8) wherein R⁹ represents a methyl group, R⁸ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1466").

The Compound (A-8) wherein R⁹ represents a methyl group, R⁸ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1467").

The Compound (A-8) wherein R⁹ represents a methyl group, R⁸ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1468").

The Compound (A-8) wherein R⁹ represents a methyl group, R⁸ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1469").

The Compound (A-8) wherein R⁹ represents a methyl group, R⁸ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1470").

The Compound (A-8) wherein R⁹ represents a methyl group, R⁸ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1471").

The Compound (A-8) wherein R⁹ represents a methyl group, R⁸ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1472").

The Compound (A-8) wherein R⁹ represents a methyl group, R⁸ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1473").

The Compound (A-8) wherein R⁹ represents a methyl group, R⁸ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1474").

The Compound (A-8) wherein R⁹ represents a methyl group, R⁸ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1475").

The Compound (A-8) wherein R⁹ represents a methyl group, R⁸ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1476").

The Compound (A-8) wherein R⁹ represents a methyl group, R⁸ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1477").

The Compound (A-8) wherein R⁹ represents a methyl group, R⁸ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1478").

The Compound (A-8) wherein R⁹ represents a methyl group, R⁸ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1479").

The Compound (A-8) wherein R¹¹ represents a fluorine atom, R⁸ and R⁹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1480").

The Compound (A-8) wherein R¹¹ represents a fluorine atom, R⁸ and R⁹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1481").

The Compound (A-8) wherein R¹¹ represents a fluorine atom, R⁸ and R⁹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1482").

The Compound (A-8) wherein R¹¹ represents a fluorine atom, R⁸ and R⁹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1483").

The Compound (A-8) wherein R¹¹ represents a fluorine atom, R⁸ and R⁹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1484").

The Compound (A-8) wherein R¹¹ represents a fluorine atom, R⁸ and R⁹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1485").

The Compound (A-8) wherein R¹¹ represents a fluorine atom, R⁸ and R⁹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1486").

The Compound (A-8) wherein R¹¹ represents a fluorine atom, R⁸ and R⁹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1487").

The Compound (A-8) wherein R¹¹ represents a fluorine atom, R⁸ and R⁹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1488").

The Compound (A-8) wherein R¹¹ represents a fluorine atom, R⁸ and R⁹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1489").

The Compound (A-8) wherein R¹¹ represents a fluorine atom, R⁸ and R⁹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1490").

The Compound (A-8) wherein R¹¹ represents a fluorine atom, R⁸ and R⁹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1491").

The Compound (A-8) wherein R¹¹ represents a fluorine atom, R⁸ and R⁹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1492").

The Compound (A-8) wherein R¹¹ represents a fluorine atom, R⁸ and R⁹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1493").

The Compound (A-8) wherein R¹¹ represents a fluorine atom, R⁸ and R⁹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1494").

The Compound (A-8) wherein R¹¹ represents a fluorine atom, R⁸ and R⁹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1495").

The Compound (A-8) wherein R¹¹ represents a fluorine atom, R⁸ and R⁹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1496").

The Compound (A-8) wherein R¹¹ represents a fluorine atom, R⁸ and R⁹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1497").

The Compound (A-8) wherein R¹¹ represents a fluorine atom, R⁸ and R⁹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1498").

The Compound (A-8) wherein R¹¹ represents a fluorine atom, R⁸ and R⁹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1499").

The Compound (A-8) wherein R¹¹ represents a fluorine atom, R⁸ and R⁹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1500").

The Compound (A-8) wherein R¹¹ represents a fluorine atom, R⁸ and R⁹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1501").

The Compound (A-8) wherein R¹¹ represents a fluorine atom, R⁸ and R⁹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1502").

The Compound (A-8) wherein R¹¹ represents a fluorine atom, R⁸ and R⁹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1503").

The Compound (A-8) wherein R¹¹ represents a fluorine atom, R⁸ and R⁹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1504").

The Compound (A-8) wherein R¹¹ represents a fluorine atom, R⁸ and R⁹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1505").

The Compound (A-8) wherein R¹¹ represents a fluorine atom, R⁸ and R⁹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1506").

The Compound (A-8) wherein R¹¹ represents a fluorine atom, R⁸ and R⁹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1507").

The Compound (A-8) wherein R¹¹ represents a fluorine atom, R⁸ and R⁹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1508").

The Compound (A-8) wherein R¹¹ represents a fluorine atom, R⁸ and R⁹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1509").

The Compound (A-8) wherein R¹¹ represents a fluorine atom, R⁸ and R⁹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1510").

The Compound (A-8) wherein R¹¹ represents a fluorine atom, R⁸ and R⁹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1511").

The Compound (A-8) wherein R¹¹ represents a fluorine atom, R⁸ and R⁹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1512").

The Compound (A-8) wherein R¹¹ represents a fluorine atom, R⁸ and R⁹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1513").

The Compound (A-8) wherein R¹¹ represents a chlorine atom, R⁸ and R⁹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1514").

The Compound (A-8) wherein R¹¹ represents a chlorine atom, R⁸ and R⁹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1515").

The Compound (A-8) wherein R¹¹ represents a chlorine atom, R⁸ and R⁹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1516").

The Compound (A-8) wherein R¹¹ represents a chlorine atom, R⁸ and R⁹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1517").

The Compound (A-8) wherein R¹¹ represents a chlorine atom, R⁸ and R⁹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1518").

The Compound (A-8) wherein R¹¹ represents a chlorine atom, R⁸ and R⁹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1519").

The Compound (A-8) wherein R¹¹ represents a chlorine atom, R⁸ and R⁹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1520").

The Compound (A-8) wherein R¹¹ represents a chlorine atom, R⁸ and R⁹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1521").

The Compound (A-8) wherein R¹¹ represents a chlorine atom, R⁸ and R⁹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1522").

The Compound (A-8) wherein R¹¹ represents a chlorine atom, R⁸ and R⁹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1523").

The Compound (A-8) wherein R¹¹ represents a chlorine atom, R⁸ and R⁹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1524").

The Compound (A-8) wherein R¹¹ represents a chlorine atom, R⁸ and R⁹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1525").

The Compound (A-8) wherein R¹¹ represents a chlorine atom, R⁸ and R⁹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1526").

The Compound (A-8) wherein R¹¹ represents a chlorine atom, R⁸ and R⁹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1527").

The Compound (A-8) wherein R¹¹ represents a chlorine atom, R⁸ and R⁹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1528").

The Compound (A-8) wherein R¹¹ represents a chlorine atom, R⁸ and R⁹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1529").

The Compound (A-8) wherein R¹¹ represents a chlorine atom, R⁸ and R⁹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1530").

The Compound (A-8) wherein R¹¹ represents a chlorine atom, R⁸ and R⁹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1531").

The Compound (A-8) wherein R¹¹ represents a chlorine atom, R⁸ and R⁹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1532").

The Compound (A-8) wherein R¹¹ represents a chlorine atom, R⁸ and R⁹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1533").

The Compound (A-8) wherein R¹¹ represents a chlorine atom, R⁸ and R⁹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1534").

The Compound (A-8) wherein R¹¹ represents a chlorine atom, R⁸ and R⁹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1535").

The Compound (A-8) wherein R¹¹ represents a chlorine atom, R⁸ and R⁹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1536").

The Compound (A-8) wherein R¹¹ represents a chlorine atom, R⁸ and R⁹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1537").

The Compound (A-8) wherein R¹¹ represents a chlorine atom, R⁸ and R⁹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1538").

The Compound (A-8) wherein R¹¹ represents a chlorine atom, R⁸ and R⁹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1539").

The Compound (A-8) wherein R¹¹ represents a chlorine atom, R⁸ and R⁹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1540").

The Compound (A-8) wherein R¹¹ represents a chlorine atom, R⁸ and R⁹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1541").

The Compound (A-8) wherein R¹¹ represents a chlorine atom, R⁸ and R⁹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1542").

The Compound (A-8) wherein R¹¹ represents a chlorine atom, R⁸ and R⁹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1543").

The Compound (A-8) wherein R¹¹ represents a chlorine atom, R⁸ and R⁹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1544").

The Compound (A-8) wherein R¹¹ represents a chlorine atom, R⁸ and R⁹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1545").

The Compound (A-8) wherein R¹¹ represents a chlorine atom, R⁸ and R⁹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1546").

The Compound (A-8) wherein R¹¹ represents a chlorine atom, R⁸ and R⁹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1547").

The Compound (A-8) wherein R¹¹ represents a methyl group, R⁸ and R⁹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1548").

The Compound (A-8) wherein R¹¹ represents a methyl group, R⁸ and R⁹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1549").

The Compound (A-8) wherein R¹¹ represents a methyl group, R⁸ and R⁹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1550").

The Compound (A-8) wherein R¹¹ represents a methyl group, R⁸ and R⁹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1551").

The Compound (A-8) wherein R¹¹ represents a methyl group, R⁸ and R⁹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1552").

The Compound (A-8) wherein R¹¹ represents a methyl group, R⁸ and R⁹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1553").

The Compound (A-8) wherein R¹¹ represents a methyl group, R⁸ and R⁹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1554").

The Compound (A-8) wherein R¹¹ represents a methyl group, R⁸ and R⁹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1555").

The Compound (A-8) wherein R¹¹ represents a methyl group, R⁸ and R⁹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1556").

The Compound (A-8) wherein R¹¹ represents a methyl group, R⁸ and R⁹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1557").

The Compound (A-8) wherein R¹¹ represents a methyl group, R⁸ and R⁹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1558").

The Compound (A-8) wherein R¹¹ represents a methyl group, R⁸ and R⁹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1559").

The Compound (A-8) wherein R¹¹ represents a methyl group, R⁸ and R⁹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1560").

The Compound (A-8) wherein R¹¹ represents a methyl group, R⁸ and R⁹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1561").

The Compound (A-8) wherein R¹¹ represents a methyl group, R⁸ and R⁹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1562").

The Compound (A-8) wherein R¹¹ represents a methyl group, R⁸ and R⁹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1563").

The Compound (A-8) wherein R¹¹ represents a methyl group, R⁸ and R⁹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1564").

The Compound (A-8) wherein R¹¹ represents a methyl group, R⁸ and R⁹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1565").

The Compound (A-8) wherein R¹¹ represents a methyl group, R⁸ and R⁹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1566").

The Compound (A-8) wherein R¹¹ represents a methyl group, R⁸ and R⁹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1567").

The Compound (A-8) wherein R¹¹ represents a methyl group, R⁸ and R⁹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1568").

The Compound (A-8) wherein R¹¹ represents a methyl group, R⁸ and R⁹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1569").

The Compound (A-8) wherein R¹¹ represents a methyl group, R⁸ and R⁹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1570").

The Compound (A-8) wherein R¹¹ represents a methyl group, R⁸ and R⁹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1571").

The Compound (A-8) wherein R¹¹ represents a methyl group, R⁸ and R⁹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1572").

The Compound (A-8) wherein R¹¹ represents a methyl group, R⁸ and R⁹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1573").

The Compound (A-8) wherein R¹¹ represents a methyl group, R⁸ and R⁹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1574").

The Compound (A-8) wherein R¹¹ represents a methyl group, R⁸ and R⁹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1575").

The Compound (A-8) wherein R¹¹ represents a methyl group, R⁸ and R⁹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1576").

The Compound (A-8) wherein R¹¹ represents a methyl group, R⁸ and R⁹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1577").

The Compound (A-8) wherein R¹¹ represents a methyl group, R⁸ and R⁹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1578").

The Compound (A-8) wherein R¹¹ represents a methyl group, R⁸ and R⁹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1579").

The Compound (A-8) wherein R¹¹ represents a methyl group, R⁸ and R⁹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1580").

The Compound (A-8) wherein R¹¹ represents a methyl group, R⁸ and R⁹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1581").

A compound represented by formula (A-9) 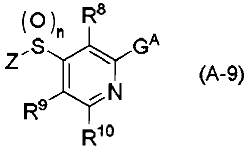 (hereinafter referred to as "Compound (A-9)") wherein R⁸, R⁹, and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1582").

The Compound (A-9) wherein R⁸, R⁹, and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1583").

The Compound (A-9) wherein R⁸, R⁹, and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1584").

The Compound (A-9) wherein R^{B}, R⁹, and R¹⁰ each represent a hydrogen atom, n represents 1, Z represents - CH=CHCH₃, and the combination of the group represented by G^{A} and the substituents R^{X7}, R^{x8}, R^{X9}, and R^{X10} in the group represented by G^{A} represents any one combination indicated in the Combination A (hereinafter referred to as "Compound group SX1585").

The Compound (A-9) wherein R⁸, R⁹, and R¹⁰ each represent a hydrogen atom, n represents 1, Z represents - CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1586").

The Compound (A-9) wherein R⁸, R⁹, and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1587").

The Compound (A-9) wherein R⁸, R⁹, and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1588").

The Compound (A-9) wherein R⁸, R⁹, and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1589").

The Compound (A-9) wherein R⁸, R⁹, and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1590").

The Compound (A-9) wherein R⁸, R⁹, and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1591").

The Compound (A-9) wherein R⁸, R⁹, and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1592").

The Compound (A-9) wherein R⁸, R⁹, and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1593").

The Compound (A-9) wherein R⁸, R⁹, and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1594").

The Compound (A-9) wherein R⁸, R⁹, and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1595").

The Compound (A-9) wherein R⁸, R⁹, and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1596").

The Compound (A-9) wherein R⁸, R⁹, and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1597").

The Compound (A-9) wherein R⁸, R⁹, and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1598").

The Compound (A-9) wherein R⁸, R⁹, and R¹⁰ each represent a hydrogen atom, n represents 2, Z represents an ethyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1599").

The Compound (A-9) wherein R⁸, R⁹, and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1600").

The Compound (A-9) wherein R⁸, R⁹, and R¹⁰ each represent a hydrogen atom, n represents 2, Z represents an isopropyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1601").

The Compound (A-9) wherein R⁸, R⁹, and R¹⁰ each represent a hydrogen atom, n represents 2, Z represents - CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1602").

The Compound (A-9) wherein R⁸, R⁹, and R¹⁰ each represent a hydrogen atom, n represents 2, Z represents - CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1603").

The Compound (A-9) wherein R⁸, R⁹, and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1604").

The Compound (A-9) wherein R⁸, R⁹, and R¹⁰ each represent a hydrogen atom, n represents 2, Z represents a cyclopropyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1605").

The Compound (A-9) wherein R⁸, R⁹, and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1606").

The Compound (A-9) wherein R⁸, R⁹, and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1607").

The Compound (A-9) wherein R⁸, R⁹, and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1608").

The Compound (A-9) wherein R⁸, R⁹, and R¹⁰ each represent a hydrogen atom, n represents 2, Z represents a 3-furanyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1609").

The Compound (A-9) wherein R⁸, R⁹, and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1610").

The Compound (A-9) wherein R⁸, R⁹, and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1611").

The Compound (A-9) wherein R⁸, R⁹, and R¹⁰ each represent a hydrogen atom, n represents 2, Z represents a 4-pyridyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1612").

The Compound (A-9) wherein R⁸, R⁹, and R¹⁰ each represent a hydrogen atom, n represents 2, Z represents a 2-pyrimidyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1613").

The Compound (A-9) wherein R⁸, R⁹, and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1614").

The Compound (A-9) wherein R⁸, R⁹, and R¹⁰ each represent a hydrogen atom, n represents 2, Z represents a 2-pyrazinyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1615").

The Compound (A-9) wherein R⁸ represents a fluorine atom, R⁹ and R¹⁰ each represent a hydrogen atom, n represents 1, Z represents an ethyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1616").

The Compound (A-9) wherein R⁸ represents a fluorine atom, R⁹ and R¹⁰ each represent a hydrogen atom, n represents 1, Z represents a propyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1617").

The Compound (A-9) wherein R⁸ represents a fluorine atom, R⁹ and R¹⁰ each represent a hydrogen atom, n represents 1, Z represents an isopropyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1618").

The Compound (A-9) wherein R⁸ represents a fluorine atom, R⁹ and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1619").

The Compound (A-9) wherein R⁸ represents a fluorine atom, R⁹ and R¹⁰ each represent a hydrogen atom, n represents 1, Z represents -CH₂CH=CH₂, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1620").

The Compound (A-9) wherein R⁸ represents a fluorine atom, R⁹ and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1621").

The Compound (A-9) wherein R⁸ represents a fluorine atom, R⁹ and R¹⁰ each represent a hydrogen atom, n represents 1, Z represents a cyclopropyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1622").

The Compound (A-9) wherein R⁸ represents a fluorine atom, R⁹ and R¹⁰ each represent a hydrogen atom, n represents 1, Z represents a 2-thienyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1623").

The Compound (A-9) wherein R⁸ represents a fluorine atom, R⁹ and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1624").

The Compound (A-9) wherein R⁸ represents a fluorine atom, R⁹ and R¹⁰ each represent a hydrogen atom, n represents 1, Z represents a 2-furanyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1625").

The Compound (A-9) wherein R⁸ represents a fluorine atom, R⁹ and R¹⁰ each represent a hydrogen atom, n represents 1, Z represents a 3-furanyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1626").

The Compound (A-9) wherein R⁸ represents a fluorine atom, R⁹ and R¹⁰ each represent a hydrogen atom, n represents 1, Z represents a 2-pyridyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1627").

The Compound (A-9) wherein R⁸ represents a fluorine atom, R⁹ and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1628").

The Compound (A-9) wherein R⁸ represents a fluorine atom, R⁹ and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1629").

The Compound (A-9) wherein R⁸ represents a fluorine atom, R⁹ and R¹⁰ each represent a hydrogen atom, n represents 1, Z represents a 2-pyrimidyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1630").

The Compound (A-9) wherein R⁸ represents a fluorine atom, R⁹ and R¹⁰ each represent a hydrogen atom, n represents 1, Z represents a 4-pyrimidyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1631").

The Compound (A-9) wherein R⁸ represents a fluorine atom, R⁹ and R¹⁰ each represent a hydrogen atom, n represents 1, Z represents a 2-pyrazinyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1632").

The Compound (A-9) wherein R⁸ represents a fluorine atom, R⁹ and R¹⁰ each represent a hydrogen atom, n represents 2, Z represents an ethyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1633").

The Compound (A-9) wherein R⁸ represents a fluorine atom, R⁹ and R¹⁰ each represent a hydrogen atom, n represents 2, Z represents a propyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1634").

The Compound (A-9) wherein R⁸ represents a fluorine atom, R⁹ and R¹⁰ each represent a hydrogen atom, n represents 2, Z represents an isopropyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1635").

The Compound (A-9) wherein R⁸ represents a fluorine atom, R⁹ and R¹⁰ each represent a hydrogen atom, n represents 2, Z represents -CH=CHCH₃, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1636").

The Compound (A-9) wherein R⁸ represents a fluorine atom, R⁹ and R¹⁰ each represent a hydrogen atom, n represents 2, Z represents -CH₂CH=CH₂, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1637").

The Compound (A-9) wherein R⁸ represents a fluorine atom, R⁹ and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1638").

The Compound (A-9) wherein R⁸ represents a fluorine atom, R⁹ and R¹⁰ each represent a hydrogen atom, n represents 2, Z represents a cyclopropyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1639").

The Compound (A-9) wherein R⁸ represents a fluorine atom, R⁹ and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1640").

The Compound (A-9) wherein R⁸ represents a fluorine atom, R⁹ and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1641").

The Compound (A-9) wherein R⁸ represents a fluorine atom, R⁹ and R¹⁰ each represent a hydrogen atom, n represents 2, Z represents a 2-furanyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1642").

The Compound (A-9) wherein R⁸ represents a fluorine atom, R⁹ and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1643").

The Compound (A-9) wherein R⁸ represents a fluorine atom, R⁹ and R¹⁰ each represent a hydrogen atom, n represents 2, Z represents a 2-pyridyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1644").

The Compound (A-9) wherein R⁸ represents a fluorine atom, R⁹ and R¹⁰ each represent a hydrogen atom, n represents 2, Z represents a 3-pyridyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1645").

The Compound (A-9) wherein R⁸ represents a fluorine atom, R⁹ and R¹⁰ each represent a hydrogen atom, n represents 2, Z represents a 4-pyridyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1646").

The Compound (A-9) wherein R⁸ represents a fluorine atom, R⁹ and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1647").

The Compound (A-9) wherein R⁸ represents a fluorine atom, R⁹ and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1648").

The Compound (A-9) wherein R⁸ represents a fluorine atom, R⁹ and R¹⁰ each represent a hydrogen atom, n represents 2, Z represents a 2-pyrazinyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1649").

The Compound (A-9) wherein R⁸ represents a chlorine atom, R⁹ and R¹⁰ each represent a hydrogen atom, n represents 1, Z represents an ethyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1650").

The Compound (A-9) wherein R⁸ represents a chlorine atom, R⁹ and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1651").

The Compound (A-9) wherein R⁸ represents a chlorine atom, R⁹ and R¹⁰ each represent a hydrogen atom, n represents 1, Z represents an isopropyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1652").

The Compound (A-9) wherein R⁸ represents a chlorine atom, R⁹ and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1653").

The Compound (A-9) wherein R⁸ represents a chlorine atom, R⁹ and R¹⁰ each represent a hydrogen atom, n represents 1, Z represents -CH₂CH=CH₂, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1654").

The Compound (A-9) wherein R⁸ represents a chlorine atom, R⁹ and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1655").

The Compound (A-9) wherein R⁸ represents a chlorine atom, R⁹ and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1656").

The Compound (A-9) wherein R⁸ represents a chlorine atom, R⁹ and R¹⁰ each represent a hydrogen atom, n represents 1, Z represents a 2-thienyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1657").

The Compound (A-9) wherein R⁸ represents a chlorine atom, R⁹ and R¹⁰ each represent a hydrogen atom, n represents 1, Z represents a 3-thienyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1658").

The Compound (A-9) wherein R⁸ represents a chlorine atom, R⁹ and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1659").

The Compound (A-9) wherein R⁸ represents a chlorine atom, R⁹ and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1660").

The Compound (A-9) wherein R⁸ represents a chlorine atom, R⁹ and R¹⁰ each represent a hydrogen atom, n represents 1, Z represents a 2-pyridyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1661").

The Compound (A-9) wherein R⁸ represents a chlorine atom, R⁹ and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1662").

The Compound (A-9) wherein R⁸ represents a chlorine atom, R⁹ and R¹⁰ each represent a hydrogen atom, n represents 1, Z represents a 4-pyridyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1663").

The Compound (A-9) wherein R⁸ represents a chlorine atom, R⁹ and R¹⁰ each represent a hydrogen atom, n represents 1, Z represents a 2-pyrimidyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1664").

The Compound (A-9) wherein R⁸ represents a chlorine atom, R⁹ and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1665").

The Compound (A-9) wherein R⁸ represents a chlorine atom, R⁹ and R¹⁰ each represent a hydrogen atom, n represents 1, Z represents a 2-pyrazinyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1666").

The Compound (A-9) wherein R⁸ represents a chlorine atom, R⁹ and R¹⁰ each represent a hydrogen atom, n represents 2, Z represents an ethyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1667").

The Compound (A-9) wherein R⁸ represents a chlorine atom, R⁹ and R¹⁰ each represent a hydrogen atom, n represents 2, Z represents a propyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1668").

The Compound (A-9) wherein R⁸ represents a chlorine atom, R⁹ and R¹⁰ each represent a hydrogen atom, n represents 2, Z represents an isopropyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1669").

The Compound (A-9) wherein R⁸ represents a chlorine atom, R⁹ and R¹⁰ each represent a hydrogen atom, n represents 2, Z represents -CH=CHCH₃, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1670").

The Compound (A-9) wherein R⁸ represents a chlorine atom, R⁹ and R¹⁰ each represent a hydrogen atom, n represents 2, Z represents -CH₂CH=CH₂, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1671").

The Compound (A-9) wherein R⁸ represents a chlorine atom, R⁹ and R¹⁰ each represent a hydrogen atom, n represents 2, Z represents a butyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1672").

The Compound (A-9) wherein R⁸ represents a chlorine atom, R⁹ and R¹⁰ each represent a hydrogen atom, n represents 2, Z represents a cyclopropyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1673").

The Compound (A-9) wherein R⁸ represents a chlorine atom, R⁹ and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1674").

The Compound (A-9) wherein R⁸ represents a chlorine atom, R⁹ and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1675").

The Compound (A-9) wherein R⁸ represents a chlorine atom, R⁹ and R¹⁰ each represent a hydrogen atom, n represents 2, Z represents a 2-furanyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1676").

The Compound (A-9) wherein R⁸ represents a chlorine atom, R⁹ and R¹⁰ each represent a hydrogen atom, n represents 2, Z represents a 3-furanyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1677").

The Compound (A-9) wherein R⁸ represents a chlorine atom, R⁹ and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1678").

The Compound (A-9) wherein R⁸ represents a chlorine atom, R⁹ and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1679").

The Compound (A-9) wherein R⁸ represents a chlorine atom, R⁹ and R¹⁰ each represent a hydrogen atom, n represents 2, Z represents a 4-pyridyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1680").

The Compound (A-9) wherein R⁸ represents a chlorine atom, R⁹ and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1681").

The Compound (A-9) wherein R⁸ represents a chlorine atom, R⁹ and R¹⁰ each represent a hydrogen atom, n represents 2, Z represents a 4-pyrimidyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1682").

The Compound (A-9) wherein R⁸ represents a chlorine atom, R⁹ and R¹⁰ each represent a hydrogen atom, n represents 2, Z represents a 2-pyrazinyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1683").

The Compound (A-9) wherein R⁸ represents a methyl group, R⁹ and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1684").

The Compound (A-9) wherein R⁸ represents a methyl group, R⁹ and R¹⁰ each represent a hydrogen atom, n represents 1, Z represents a propyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1685").

The Compound (A-9) wherein R⁸ represents a methyl group, R⁹ and R¹⁰ each represent a hydrogen atom, n represents 1, Z represents an isopropyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1686").

The Compound (A-9) wherein R⁸ represents a methyl group, R⁹ and R¹⁰ each represent a hydrogen atom, n represents 1, Z represents -CH=CHCH₃, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1687").

The Compound (A-9) wherein R⁸ represents a methyl group, R⁹ and R¹⁰ each represent a hydrogen atom, n represents 1, Z represents -CH₂CH=CH₂, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1688").

The Compound (A-9) wherein R⁸ represents a methyl group, R⁹ and R¹⁰ each represent a hydrogen atom, n represents 1, Z represents a butyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1689").

The Compound (A-9) wherein R⁸ represents a methyl group, R⁹ and R¹⁰ each represent a hydrogen atom, n represents 1, Z represents a cyclopropyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1690").

The Compound (A-9) wherein R⁸ represents a methyl group, R⁹ and R¹⁰ each represent a hydrogen atom, n represents 1, Z represents a 2-thienyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1691").

The Compound (A-9) wherein R⁸ represents a methyl group, R⁹ and R¹⁰ each represent a hydrogen atom, n represents 1, Z represents a 3-thienyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1692").

The Compound (A-9) wherein R⁸ represents a methyl group, R⁹ and R¹⁰ each represent a hydrogen atom, n represents 1, Z represents a 2-furanyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1693").

The Compound (A-9) wherein R⁸ represents a methyl group, R⁹ and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1694").

The Compound (A-9) wherein R⁸ represents a methyl group, R⁹ and R¹⁰ each represent a hydrogen atom, n represents 1, Z represents a 2-pyridyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1695").

The Compound (A-9) wherein R⁸ represents a methyl group, R⁹ and R¹⁰ each represent a hydrogen atom, n represents 1, Z represents a 3-pyridyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1696").

The Compound (A-9) wherein R⁸ represents a methyl group, R⁹ and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1697").

The Compound (A-9) wherein R⁸ represents a methyl group, R⁹ and R¹⁰ each represent a hydrogen atom, n represents 1, Z represents a 2-pyrimidyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1698").

The Compound (A-9) wherein R⁸ represents a methyl group, R⁹ and R¹⁰ each represent a hydrogen atom, n represents 1, Z represents a 4-pyrimidyl group, and the combination of the group represented by G^{A} and the substituents R^{X7}, R^{X8}, R^{X9}, and R^{x10} in the group represented by G^{A} represents any one combination indicated in the Combination A (hereinafter referred to as "Compound group SX1699").

The Compound (A-9) wherein R⁸ represents a methyl group, R⁹ and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1700").

The Compound (A-9) wherein R⁸ represents a methyl group, R⁹ and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1701").

The Compound (A-9) wherein R⁸ represents a methyl group, R⁹ and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1702").

The Compound (A-9) wherein R⁸ represents a methyl group, R⁹ and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1703").

The Compound (A-9) wherein R⁸ represents a methyl group, R⁹ and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1704").

The Compound (A-9) wherein R⁸ represents a methyl group, R⁹ and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1705").

The Compound (A-9) wherein R⁸ represents a methyl group, R⁹ and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1706").

The Compound (A-9) wherein R⁸ represents a methyl group, R⁹ and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1707").

The Compound (A-9) wherein R⁸ represents a methyl group, R⁹ and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1708").

The Compound (A-9) wherein R⁸ represents a methyl group, R⁹ and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1709").

The Compound (A-9) wherein R⁸ represents a methyl group, R⁹ and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1710").

The Compound (A-9) wherein R⁸ represents a methyl group, R⁹ and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1711").

The Compound (A-9) wherein R⁸ represents a methyl group, R⁹ and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1712").

The Compound (A-9) wherein R⁸ represents a methyl group, R⁹ and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1713").

The Compound (A-9) wherein R⁸ represents a methyl group, R⁹ and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1714").

The Compound (A-9) wherein R⁸ represents a methyl group, R⁹ and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1715").

The Compound (A-9) wherein R⁸ represents a methyl group, R⁹ and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1716").

The Compound (A-9) wherein R⁸ represents a methyl group, R⁹ and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1717").

The Compound (A-9) wherein R⁹ represents a fluorine atom, R⁸ and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1718").

The Compound (A-9) wherein R⁹ represents a fluorine atom, R⁸ and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1719").

The Compound (A-9) wherein R⁹ represents a fluorine atom, R⁸ and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1720").

The Compound (A-9) wherein R⁹ represents a fluorine atom, R⁸ and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1721").

The Compound (A-9) wherein R⁹ represents a fluorine atom, R⁸ and R¹⁰ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1722").

The Compound (A-9) wherein R⁹ represents a fluorine atom, R⁸ and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1723").

The Compound (A-9) wherein R⁹ represents a fluorine atom, R⁸ and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1724").

The Compound (A-9) wherein R⁹ represents a fluorine atom, R⁸ and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1725").

The Compound (A-9) wherein R⁹ represents a fluorine atom, R⁸ and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1726").

The Compound (A-9) wherein R⁹ represents a fluorine atom, R⁸ and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1727").

The Compound (A-9) wherein R⁹ represents a fluorine atom, R⁸ and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1728").

The Compound (A-9) wherein R⁹ represents a fluorine atom, R⁸ and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1729").

The Compound (A-9) wherein R⁹ represents a fluorine atom, R⁸ and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1730").

The Compound (A-9) wherein R⁹ represents a fluorine atom, R⁸ and R¹⁰ each represent a hydrogen atom, n represents 1, Z represents a 4-pyridyl group, and the combination of the group represented by G^{A} and the substituents R^{X7} , R^{X8}, R^{X9}, and R^{X10} in the group represented by G^{A} represents any one combination indicated in the Combination A (hereinafter referred to as "Compound group SX1731").

The Compound (A-9) wherein R⁹ represents a fluorine atom, R⁸ and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1732").

The Compound (A-9) wherein R⁹ represents a fluorine atom, R⁸ and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1733").

The Compound (A-9) wherein R⁹ represents a fluorine atom, R⁸ and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1734").

The Compound (A-9) wherein R⁹ represents a fluorine atom, R⁸ and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1735").

The Compound (A-9) wherein R⁹ represents a fluorine atom, R⁸ and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1736").

The Compound (A-9) wherein R⁹ represents a fluorine atom, R⁸ and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1737").

The Compound (A-9) wherein R⁹ represents a fluorine atom, R⁸ and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1738").

The Compound (A-9) wherein R⁹ represents a fluorine atom, R⁸ and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1739").

The Compound (A-9) wherein R⁹ represents a fluorine atom, R⁸ and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1740").

The Compound (A-9) wherein R⁹ represents a fluorine atom, R⁸ and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1741").

The Compound (A-9) wherein R⁹ represents a fluorine atom, R⁸ and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1742").

The Compound (A-9) wherein R⁹ represents a fluorine atom, R⁸ and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1743").

The Compound (A-9) wherein R⁹ represents a fluorine atom, R⁸ and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1744").

The Compound (A-9) wherein R⁹ represents a fluorine atom, R⁸ and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1745").

The Compound (A-9) wherein R⁹ represents a fluorine atom, R⁸ and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1746").

The Compound (A-9) wherein R⁹ represents a fluorine atom, R⁸ and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1747").

The Compound (A-9) wherein R⁹ represents a fluorine atom, R⁸ and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1748").

The Compound (A-9) wherein R⁹ represents a fluorine atom, R⁸ and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1749").

The Compound (A-9) wherein R⁹ represents a fluorine atom, R⁸ and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1750").

The Compound (A-9) wherein R⁹ represents a fluorine atom, R⁸ and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1751").

The Compound (A-9) wherein R⁹ represents a chlorine atom, R⁸ and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1752").

The Compound (A-9) wherein R⁹ represents a chlorine atom, R⁸ and R¹⁰ each represent a hydrogen atom, n represents 1, Z represents a propyl group, and the combination of the group represented by G^{A} and the substituents R^{X7} , R^{X8}, R^{X9}, and R^{X10} in the group represented by G^{A} represents any one combination indicated in the Combination A (hereinafter referred to as "Compound group SX1753").

The Compound (A-9) wherein R⁹ represents a chlorine atom, R⁸ and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1754").

The Compound (A-9) wherein R⁹ represents a chlorine atom, R⁸ and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1755").

The Compound (A-9) wherein R⁹ represents a chlorine atom, R⁸ and R¹⁰ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1756").

The Compound (A-9) wherein R⁹ represents a chlorine atom, R⁸ and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1757").

The Compound (A-9) wherein R⁹ represents a chlorine atom, R⁸ and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1758").

The Compound (A-9) wherein R⁹ represents a chlorine atom, R⁸ and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1759").

The Compound (A-9) wherein R⁹ represents a chlorine atom, R⁸ and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1760").

The Compound (A-9) wherein R⁹ represents a chlorine atom, R⁸ and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1761").

The Compound (A-9) wherein R⁹ represents a chlorine atom, R⁸ and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1762").

The Compound (A-9) wherein R⁹ represents a chlorine atom, R⁸ and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1763").

The Compound (A-9) wherein R⁹ represents a chlorine atom, R⁸ and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1764").

The Compound (A-9) wherein R⁹ represents a chlorine atom, R⁸ and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1765").

The Compound (A-9) wherein R⁹ represents a chlorine atom, R⁸ and R¹⁰ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1766").

The Compound (A-9) wherein R⁹ represents a chlorine atom, R⁸ and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1767").

The Compound (A-9) wherein R⁹ represents a chlorine atom, R⁸ and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1768").

The Compound (A-9) wherein R⁹ represents a chlorine atom, R⁸ and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1769").

The Compound (A-9) wherein R⁹ represents a chlorine atom, R⁸ and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1770").

The Compound (A-9) wherein R⁹ represents a chlorine atom, R⁸ and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1771").

The Compound (A-9) wherein R⁹ represents a chlorine atom, R⁸ and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1772").

The Compound (A-9) wherein R⁹ represents a chlorine atom, R⁸ and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1773").

The Compound (A-9) wherein R⁹ represents a chlorine atom, R⁸ and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1774").

The Compound (A-9) wherein R⁹ represents a chlorine atom, R⁸ and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1775").

The Compound (A-9) wherein R⁹ represents a chlorine atom, R⁸ and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1776").

The Compound (A-9) wherein R⁹ represents a chlorine atom, R⁸ and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1777").

The Compound (A-9) wherein R⁹ represents a chlorine atom, R⁸ and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1778").

The Compound (A-9) wherein R⁹ represents a chlorine atom, R⁸ and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1779").

The Compound (A-9) wherein R⁹ represents a chlorine atom, R⁸ and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1780").

The Compound (A-9) wherein R⁹ represents a chlorine atom, R⁸ and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1781").

The Compound (A-9) wherein R⁹ represents a chlorine atom, R⁸ and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1782").

The Compound (A-9) wherein R⁹ represents a chlorine atom, R⁸ and R¹⁰ each represent a hydrogen atom, n represents 2, Z represents a 2-pyrimidyl group, and the combination of the group represented by G^{A} and the substituents R^{X7}, R^{X9}, R^{X9}, and R^{X10} in the group represented by G^{A} represents any one combination indicated in the Combination A (hereinafter referred to as "Compound group SX1783").

The Compound (A-9) wherein R⁹ represents a chlorine atom, R⁸ and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1784").

The Compound (A-9) wherein R⁹ represents a chlorine atom, R⁸ and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1785").

The Compound (A-9) wherein R⁹ represents a methyl group, R⁸ and R¹⁰ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1786").

The Compound (A-9) wherein R⁹ represents a methyl group, R⁸ and R¹⁰ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1787").

The Compound (A-9) wherein R⁹ represents a methyl group, R⁸ and R¹⁰ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1788").

The Compound (A-9) wherein R⁹ represents a methyl group, R⁸ and R¹⁰ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1789").

The Compound (A-9) wherein R⁹ represents a methyl group, R⁸ and R¹⁰ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1790").

The Compound (A-9) wherein R⁹ represents a methyl group, R⁸ and R¹⁰ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1791").

The Compound (A-9) wherein R⁹ represents a methyl group, R⁸ and R¹⁰ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1792").

The Compound (A-9) wherein R⁹ represents a methyl group, R⁸ and R¹⁰ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1793").

The Compound (A-9) wherein R⁹ represents a methyl group, R⁸ and R¹⁰ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1794").

The Compound (A-9) wherein R⁹ represents a methyl group, R⁸ and R¹⁰ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1795").

The Compound (A-9) wherein R⁹ represents a methyl group, R⁸ and R¹⁰ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1796").

The Compound (A-9) wherein R⁹ represents a methyl group, R⁸ and R¹⁰ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1797").

The Compound (A-9) wherein R⁹ represents a methyl group, R⁸ and R¹⁰ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1798").

The Compound (A-9) wherein R⁹ represents a methyl group, R⁸ and R¹⁰ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1799").

The Compound (A-9) wherein R⁹ represents a methyl group, R⁸ and R¹⁰ each represent a hydrogen atom, n represents 1, Z represents a 2-pyrimidinyl group, and the combination of the group represented by G^{A} and the substituents R^{X7}, R^{X8} R^{X9}, and R^{X10} in the group represented by G^{A} represents any one combination indicated in the Combination A (hereinafter referred to as "Compound group SX1800").

The Compound (A-9) wherein R⁹ represents a methyl group, R⁸ and R¹⁰ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1801").

The Compound (A-9) wherein R⁹ represents a methyl group, R⁸ and R¹⁰ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1802").

The Compound (A-9) wherein R⁹ represents a methyl group, R⁸ and R¹⁰ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1803").

The Compound (A-9) wherein R⁹ represents a methyl group, R⁸ and R¹⁰ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1804").

The Compound (A-9) wherein R⁹ represents a methyl group, R⁸ and R¹⁰ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1805").

The Compound (A-9) wherein R⁹ represents a methyl group, R⁸ and R¹⁰ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1806").

The Compound (A-9) wherein R⁹ represents a methyl group, R⁸ and R¹⁰ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1807").

The Compound (A-9) wherein R⁹ represents a methyl group, R⁸ and R¹⁰ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1808").

The Compound (A-9) wherein R⁹ represents a methyl group, R⁸ and R¹⁰ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1809").

The Compound (A-9) wherein R⁹ represents a methyl group, R⁸ and R¹⁰ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1810").

The Compound (A-9) wherein R⁹ represents a methyl group, R⁸ and R¹⁰ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1811").

The Compound (A-9) wherein R⁹ represents a methyl group, R⁸ and R¹⁰ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1812").

The Compound (A-9) wherein R⁹ represents a methyl group, R⁸ and R¹⁰ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1813").

The Compound (A-9) wherein R⁹ represents a methyl group, R⁸ and R¹⁰ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1814").

The Compound (A-9) wherein R⁹ represents a methyl group, R⁸ and R¹⁰ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1815").

The Compound (A-9) wherein R⁹ represents a methyl group, R⁸ and R¹⁰ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1816").

The Compound (A-9) wherein R⁹ represents a methyl group, R⁸ and R¹⁰ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1817").

The Compound (A-9) wherein R⁹ represents a methyl group, R⁸ and R¹⁰ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1818").

The Compound (A-9) wherein R⁹ represents a methyl group, R⁸ and R¹⁰ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1819").

The Compound (A-9) wherein R¹⁰ represents a fluorine atom, R⁸ and R⁹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1820").

The Compound (A-9) wherein R¹⁰ represents a fluorine atom, R⁸ and R⁹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1821").

The Compound (A-9) wherein R¹⁰ represents a fluorine atom, R⁸ and R⁹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1822").

The Compound (A-9) wherein R¹⁰ represents a fluorine atom, R⁸ and R⁹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1823").

The Compound (A-9) wherein R¹⁰ represents a fluorine atom, R⁸ and R⁹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1824").

The Compound (A-9) wherein R¹⁰ represents a fluorine atom, R⁸ and R⁹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1825").

The Compound (A-9) wherein R¹⁰ represents a fluorine atom, R⁸ and R⁹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1826").

The Compound (A-9) wherein R¹⁰ represents a fluorine atom, R⁸ and R⁹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1827").

The Compound (A-9) wherein R¹⁰ represents a fluorine atom, R⁸ and R⁹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1828").

The Compound (A-9) wherein R¹⁰ represents a fluorine atom, R⁸ and R⁹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1829").

The Compound (A-9) wherein R¹⁰ represents a fluorine atom, R⁸ and R⁹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1830").

The Compound (A-9) wherein R¹⁰ represents a fluorine atom, R⁸ and R⁹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1831").

The Compound (A-9) wherein R¹⁰ represents a fluorine atom, R⁸ and R⁹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1832").

The Compound (A-9) wherein R¹⁰ represents a fluorine atom, R⁸ and R⁹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1833").

The Compound (A-9) wherein R¹⁰ represents a fluorine atom, R⁸ and R⁹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1834").

The Compound (A-9) wherein R¹⁰ represents a fluorine atom, R⁸ and R⁹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1835").

The Compound (A-9) wherein R¹⁰ represents a fluorine atom, R⁸ and R⁹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1836").

The Compound (A-9) wherein R¹⁰ represents a fluorine atom, R⁸ and R⁹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1837").

The Compound (A-9) wherein R¹⁰ represents a fluorine atom, R⁸ and R⁹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1838").

The Compound (A-9) wherein R¹⁰ represents a fluorine atom, R⁸ and R⁹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1839").

The Compound (A-9) wherein R¹⁰ represents a fluorine atom, R⁸ and R⁹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1840").

The Compound (A-9) wherein R¹⁰ represents a fluorine atom, R⁸ and R⁹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1841").

The Compound (A-9) wherein R¹⁰ represents a fluorine atom, R⁸ and R⁹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1842").

The Compound (A-9) wherein R¹⁰ represents a fluorine atom, R⁸ and R⁹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1843").

The Compound (A-9) wherein R¹⁰ represents a fluorine atom, R⁸ and R⁹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1844").

The Compound (A-9) wherein R¹⁰ represents a fluorine atom, R⁸ and R⁹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1845").

The Compound (A-9) wherein R¹⁰ represents a fluorine atom, R⁸ and R⁹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1846").

The Compound (A-9) wherein R¹⁰ represents a fluorine atom, R⁸ and R⁹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1847").

The Compound (A-9) wherein R¹⁰ represents a fluorine atom, R⁸ and R⁹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1848").

The Compound (A-9) wherein R¹⁰ represents a fluorine atom, R⁸ and R⁹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1849").

The Compound (A-9) wherein R¹⁰ represents a fluorine atom, R⁸ and R⁹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1850").

The Compound (A-9) wherein R¹⁰ represents a fluorine atom, R⁸ and R⁹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1851").

The Compound (A-9) wherein R¹⁰ represents a fluorine atom, R⁸ and R⁹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1852").

The Compound (A-9) wherein R¹⁰ represents a fluorine atom, R⁸ and R⁹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1853").

The Compound (A-9) wherein R¹⁰ represents a chlorine atom, R⁸ and R⁹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1854").

The Compound (A-9) wherein R¹⁰ represents a chlorine atom, R⁸ and R⁹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1855").

The Compound (A-9) wherein R¹⁰ represents a chlorine atom, R⁸ and R⁹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1856").

The Compound (A-9) wherein R¹⁰ represents a chlorine atom, R⁸ and R⁹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1857").

The Compound (A-9) wherein R¹⁰ represents a chlorine atom, R⁸ and R⁹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1858").

The Compound (A-9) wherein R¹⁰ represents a chlorine atom, R⁸ and R⁹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1859").

The Compound (A-9) wherein R¹⁰ represents a chlorine atom, R⁸ and R⁹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1860").

The Compound (A-9) wherein R¹⁰ represents a chlorine atom, R⁸ and R⁹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1861").

The Compound (A-9) wherein R¹⁰ represents a chlorine atom, R⁸ and R⁹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1862").

The Compound (A-9) wherein R¹⁰ represents a chlorine atom, R⁸ and R⁹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1863").

The Compound (A-9) wherein R¹⁰ represents a chlorine atom, R⁸ and R⁹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1864").

The Compound (A-9) wherein R¹⁰ represents a chlorine atom, R⁸ and R⁹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1865").

The Compound (A-9) wherein R¹⁰ represents a chlorine atom, R⁸ and R⁹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1866").

The Compound (A-9) wherein R¹⁰ represents a chlorine atom, R⁸ and R⁹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1867").

The Compound (A-9) wherein R¹⁰ represents a chlorine atom, R⁸ and R⁹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1868").

The Compound (A-9) wherein R¹⁰ represents a chlorine atom, R⁸ and R⁹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1869").

The Compound (A-9) wherein R¹⁰ represents a chlorine atom, R⁸ and R⁹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1870").

The Compound (A-9) wherein R¹⁰ represents a chlorine atom, R⁸ and R⁹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1871").

The Compound (A-9) wherein R¹⁰ represents a chlorine atom, R⁸ and R⁹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1872").

The Compound (A-9) wherein R¹⁰ represents a chlorine atom, R⁸ and R⁹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1873").

The Compound (A-9) wherein R¹⁰ represents a chlorine atom, R⁸ and R⁹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1874").

The Compound (A-9) wherein R¹⁰ represents a chlorine atom, R⁸ and R⁹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1875").

The Compound (A-9) wherein R¹⁰ represents a chlorine atom, R⁸ and R⁹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1876").

The Compound (A-9) wherein R¹⁰ represents a chlorine atom, R⁸ and R⁹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1877").

The Compound (A-9) wherein R¹⁰ represents a chlorine atom, R⁸ and R⁹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1878").

The Compound (A-9) wherein R¹⁰ represents a chlorine atom, R⁸ and R⁹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1879").

The Compound (A-9) wherein R¹⁰ represents a chlorine atom, R⁸ and R⁹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1880").

The Compound (A-9) wherein R¹⁰ represents a chlorine atom, R⁸ and R⁹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1881").

The Compound (A-9) wherein R¹⁰ represents a chlorine atom, R⁸ and R⁹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1882").

The Compound (A-9) wherein R¹⁰ represents a chlorine atom, R⁸ and R⁹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1883").

The Compound (A-9) wherein R¹⁰ represents a chlorine atom, R⁸ and R⁹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1884").

The Compound (A-9) wherein R¹⁰ represents a chlorine atom, R⁸ and R⁹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1885").

The Compound (A-9) wherein R¹⁰ represents a chlorine atom, R⁸ and R⁹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1886").

The Compound (A-9) wherein R¹⁰ represents a chlorine atom, R⁸ and R⁹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1887").

The Compound (A-9) wherein R¹⁰ represents a methyl group, R⁸ and R⁹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1888").

The Compound (A-9) wherein R¹⁰ represents a methyl group, R⁸ and R⁹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1889").

The Compound (A-9) wherein R¹⁰ represents a methyl group, R⁸ and R⁹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1890").

The Compound (A-9) wherein R¹⁰ represents a methyl group, R⁸ and R⁹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1891").

The Compound (A-9) wherein R¹⁰ represents a methyl group, R⁸ and R⁹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1892").

The Compound (A-9) wherein R¹⁰ represents a methyl group, R⁸ and R⁹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1893").

The Compound (A-9) wherein R¹⁰ represents a methyl group, R⁸ and R⁹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1894").

The Compound (A-9) wherein R¹⁰ represents a methyl group, R⁸ and R⁹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1895").

The Compound (A-9) wherein R¹⁰ represents a methyl group, R⁸ and R⁹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1896").

The Compound (A-9) wherein R¹⁰ represents a methyl group, R⁸ and R⁹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1897").

The Compound (A-9) wherein R¹⁰ represents a methyl group, R⁸ and R⁹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1898").

The Compound (A-9) wherein R¹⁰ represents a methyl group, R⁸ and R⁹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1899").

The Compound (A-9) wherein R¹⁰ represents a methyl group, R⁸ and R⁹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1900").

The Compound (A-9) wherein R¹⁰ represents a methyl group, R⁸ and R⁹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1901").

The Compound (A-9) wherein R¹⁰ represents a methyl group, R⁸ and R⁹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1902").

The Compound (A-9) wherein R¹⁰ represents a methyl group, R⁸ and R⁹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1903").

The Compound (A-9) wherein R¹⁰ represents a methyl group, R⁸ and R⁹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1904").

The Compound (A-9) wherein R¹⁰ represents a methyl group, R⁸ and R⁹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1905").

The Compound (A-9) wherein R¹⁰ represents a methyl group, R⁸ and R⁹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1906").

The Compound (A-9) wherein R¹⁰ represents a methyl group, R⁸ and R⁹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1907").

The Compound (A-9) wherein R¹⁰ represents a methyl group, R⁸ and R⁹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1908").

The Compound (A-9) wherein R¹⁰ represents a methyl group, R⁸ and R⁹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1909").

The Compound (A-9) wherein R¹⁰ represents a methyl group, R⁸ and R⁹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1910").

The Compound (A-9) wherein R¹⁰ represents a methyl group, R⁸ and R⁹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1911").

The Compound (A-9) wherein R¹⁰ represents a methyl group, R⁸ and R⁹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1912").

The Compound (A-9) wherein R¹⁰ represents a methyl group, R⁸ and R⁹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1913").

The Compound (A-9) wherein R¹⁰ represents a methyl group, R⁸ and R⁹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1914").

The Compound (A-9) wherein R¹⁰ represents a methyl group, R⁸ and R⁹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1915").

The Compound (A-9) wherein R¹⁰ represents a methyl group, R⁸ and R⁹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1916").

The Compound (A-9) wherein R¹⁰ represents a methyl group, R⁸ and R⁹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1917").

The Compound (A-9) wherein R¹⁰ represents a methyl group, R⁸ and R⁹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1918").

The Compound (A-9) wherein R¹⁰ represents a methyl group, R⁸ and R⁹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1919").

The Compound (A-9) wherein R¹⁰ represents a methyl group, R⁸ and R⁹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1920").

The Compound (A-9) wherein R¹⁰ represents a methyl group, R⁸ and R⁹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1921").

A compound represented by formula (A-10) 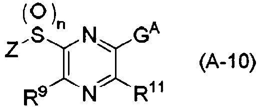 (hereinafter referred to as "Compound (A-10)") wherein R⁹ and R¹¹ each represent a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1922").

The Compound (A-10) wherein R⁹ and R¹¹ each represent a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1923").

The Compound (A-10) wherein R⁹ and R¹¹ each represent a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1924").

The Compound (A-10) wherein R⁹ and R¹¹ each represent a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1925").

The Compound (A-10) wherein R⁹ and R¹¹ each represent a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1926").

The Compound (A-10) wherein R⁹ and R¹¹ each represent a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1927").

The Compound (A-10) wherein R⁹ and R¹¹ each represent a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1928").

The Compound (A-10) wherein R⁹ and R¹¹ each represent a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1929").

The Compound (A-10) wherein R⁹ and R¹¹ each represent a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1930").

The Compound (A-10) wherein R⁹ and R¹¹ each represent a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1931").

The Compound (A-10) wherein R⁹ and R¹¹ each represent a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1932").

The Compound (A-10) wherein R⁹ and R¹¹ each represent a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1933").

The Compound (A-10) wherein R⁹ and R¹¹ each represent a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1934").

The Compound (A-10) wherein R⁹ and R¹¹ each represent a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1935").

The Compound (A-10) wherein R⁹ and R¹¹ each represent a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1936").

The Compound (A-10) wherein R⁹ and R¹¹ each represent a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1937").

The Compound (A-10) wherein R⁹ and R¹¹ each represent a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1938").

The Compound (A-10) wherein R⁹ and R¹¹ each represent a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1939").

The Compound (A-10) wherein R⁹ and R¹¹ each represent a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1940").

The Compound (A-10) wherein R⁹ and R¹¹ each represent a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1941").

The Compound (A-10) wherein R⁹ and R¹¹ each represent a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1942").

The Compound (A-10) wherein R⁹ and R¹¹ each represent a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1943").

The Compound (A-10) wherein R⁹ and R¹¹ each represent a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1944").

The Compound (A-10) wherein R⁹ and R¹¹ each represent a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1945").

The Compound (A-10) wherein R⁹ and R¹¹ each represent a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1946").

The Compound (A-10) wherein R⁹ and R¹¹ each represent a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1947").

The Compound (A-10) wherein R⁹ and R¹¹ each represent a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1948").

The Compound (A-10) wherein R⁹ and R¹¹ each represent a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1949").

The Compound (A-10) wherein R⁹ and R¹¹ each represent a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1950").

The Compound (A-10) wherein R⁹ and R¹¹ each represent a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1951").

The Compound (A-10) wherein R⁹ and R¹¹ each represent a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1952").

The Compound (A-10) wherein R⁹ and R¹¹ each represent a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1953").

The Compound (A-10) wherein R⁹ and R¹¹ each represent a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1954").

The Compound (A-10) wherein R⁹ and R¹¹ each represent a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1955").

The Compound (A-10) wherein R⁹ represents a fluorine atom, R¹¹ represents a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1956").

The Compound (A-10) wherein R⁹ represents a fluorine atom, R¹¹ represents a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1957").

The Compound (A-10) wherein R⁹ represents a fluorine atom, R¹¹ represents a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1958").

The Compound (A-10) wherein R⁹ represents a fluorine atom, R¹¹ represents a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1959").

The Compound (A-10) wherein R⁹ represents a fluorine atom, R¹¹ represents a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1960").

The Compound (A-10) wherein R⁹ represents a fluorine atom, R¹¹ represents a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1961").

The Compound (A-10) wherein R⁹ represents a fluorine atom, R¹¹ represents a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1962").

The Compound (A-10) wherein R⁹ represents a fluorine atom, R¹¹ represents a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1963").

The Compound (A-10) wherein R⁹ represents a fluorine atom, R¹¹ represents a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1964").

The Compound (A-10) wherein R⁹ represents a fluorine atom, R¹¹ represents a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1965").

The Compound (A-10) wherein R⁹ represents a fluorine atom, R¹¹ represents a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1966").

The Compound (A-10) wherein R⁹ represents a fluorine atom, R¹¹ represents a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1967").

The Compound (A-10) wherein R⁹ represents a fluorine atom, R¹¹ represents a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1968").

The Compound (A-10) wherein R⁹ represents a fluorine atom, R¹¹ represents a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1969").

The Compound (A-10) wherein R⁹ represents a fluorine atom, R¹¹ represents a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1970").

The Compound (A-10) wherein R⁹ represents a fluorine atom, R¹¹ represents a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1971").

The Compound (A-10) wherein R⁹ represents a fluorine atom, R¹¹ represents a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1972").

The Compound (A-10) wherein R⁹ represents a fluorine atom, R¹¹ represents a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1973").

The Compound (A-10) wherein R⁹ represents a fluorine atom, R¹¹ represents a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1974").

The Compound (A-10) wherein R⁹ represents a fluorine atom, R¹¹ represents a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1975").

The Compound (A-10) wherein R⁹ represents a fluorine atom, R¹¹ represents a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1976").

The Compound (A-10) wherein R⁹ represents a fluorine atom, R¹¹ represents a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1977").

The Compound (A-10) wherein R⁹ represents a fluorine atom, R¹¹ represents a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1978").

The Compound (A-10) wherein R⁹ represents a fluorine atom, R¹¹ represents a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1979").

The Compound (A-10) wherein R⁹ represents a fluorine atom, R¹¹ represents a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1980").

The Compound (A-10) wherein R⁹ represents a fluorine atom, R¹¹ represents a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1981").

The Compound (A-10) wherein R⁹ represents a fluorine atom, R¹¹ represents a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1982").

The Compound (A-10) wherein R⁹ represents a fluorine atom, R¹¹ represents a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1983").

The Compound (A-10) wherein R⁹ represents a fluorine atom, R¹¹ represents a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1984").

The Compound (A-10) wherein R⁹ represents a fluorine atom, R¹¹ represents a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1985").

The Compound (A-10) wherein R⁹ represents a fluorine atom, R¹¹ represents a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1986").

The Compound (A-10) wherein R⁹ represents a fluorine atom, R¹¹ represents a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1987").

The Compound (A-10) wherein R⁹ represents a fluorine atom, R¹¹ represents a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1988").

The Compound (A-10) wherein R⁹ represents a fluorine atom, R¹¹ represents a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1989").

The Compound (A-10) wherein R⁹ represents a chlorine atom, R¹¹ represents a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1990").

The Compound (A-10) wherein R⁹ represents a chlorine atom, R¹¹ represents a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1991").

The Compound (A-10) wherein R⁹ represents a chlorine atom, R¹¹ represents a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1992").

The Compound (A-10) wherein R⁹ represents a chlorine atom, R¹¹ represents a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1993").

The Compound (A-10) wherein R⁹ represents a chlorine atom, R¹¹ represents a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1994").

The Compound (A-10) wherein R⁹ represents a chlorine atom, R¹¹ represents a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1995").

The Compound (A-10) wherein R⁹ represents a chlorine atom, R¹¹ represents a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1996").

The Compound (A-10) wherein R⁹ represents a chlorine atom, R¹¹ represents a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1997").

The Compound (A-10) wherein R⁹ represents a chlorine atom, R¹¹ represents a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1998").

The Compound (A-10) wherein R⁹ represents a chlorine atom, R¹¹ represents a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX1999").

The Compound (A-10) wherein R⁹ represents a chlorine atom, R¹¹ represents a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2000").

The Compound (A-10) wherein R⁹ represents a chlorine atom, R¹¹ represents a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2001").

The Compound (A-10) wherein R⁹ represents a chlorine atom, R¹¹ represents a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2002").

The Compound (A-10) wherein R⁹ represents a chlorine atom, R¹¹ represents a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2003").

The Compound (A-10) wherein R⁹ represents a chlorine atom, R¹¹ represents a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2004").

The Compound (A-10) wherein R⁹ represents a chlorine atom, R¹¹ represents a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2005").

The Compound (A-10) wherein R⁹ represents a chlorine atom, R¹¹ represents a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2006").

The Compound (A-10) wherein R⁹ represents a chlorine atom, R¹¹ represents a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2007").

The Compound (A-10) wherein R⁹ represents a chlorine atom, R¹¹ represents a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2008").

The Compound (A-10) wherein R⁹ represents a chlorine atom, R¹¹ represents a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2009").

The Compound (A-10) wherein R⁹ represents a chlorine atom, R¹¹ represents a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2010").

The Compound (A-10) wherein R⁹ represents a chlorine atom, R¹¹ represents a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2011").

The Compound (A-10) wherein R⁹ represents a chlorine atom, R¹¹ represents a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2012").

The Compound (A-10) wherein R⁹ represents a chlorine atom, R¹¹ represents a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2013").

The Compound (A-10) wherein R⁹ represents a chlorine atom, R¹¹ represents a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2014").

The Compound (A-10) wherein R⁹ represents a chlorine atom, R¹¹ represents a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2015").

The Compound (A-10) wherein R⁹ represents a chlorine atom, R¹¹ represents a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2016").

The Compound (A-10) wherein R⁹ represents a chlorine atom, R¹¹ represents a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2017").

The Compound (A-10) wherein R⁹ represents a chlorine atom, R¹¹ represents a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2018").

The Compound (A-10) wherein R⁹ represents a chlorine atom, R¹¹ represents a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2019").

The Compound (A-10) wherein R⁹ represents a chlorine atom, R¹¹ represents a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2020").

The Compound (A-10) wherein R⁹ represents a chlorine atom, R¹¹ represents a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2021").

The Compound (A-10) wherein R⁹ represents a chlorine atom, R¹¹ represents a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2022").

The Compound (A-10) wherein R⁹ represents a chlorine atom, R¹¹ represents a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2023").

The Compound (A-10) wherein R⁹ represents a methyl group, R¹¹ represents a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2024").

The Compound (A-10) wherein R⁹ represents a methyl group, R¹¹ represents a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2025").

The Compound (A-10) wherein R⁹ represents a methyl group, R¹¹ represents a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2026").

The Compound (A-10) wherein R⁹ represents a methyl group, R¹¹ represents a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2027").

The Compound (A-10) wherein R⁹ represents a methyl group, R¹¹ represents a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2028").

The Compound (A-10) wherein R⁹ represents a methyl group, R¹¹ represents a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2029").

The Compound (A-10) wherein R⁹ represents a methyl group, R¹¹ represents a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2030").

The Compound (A-10) wherein R⁹ represents a methyl group, R¹¹ represents a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2031").

The Compound (A-10) wherein R⁹ represents a methyl group, R¹¹ represents a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2032").

The Compound (A-10) wherein R⁹ represents a methyl group, R¹¹ represents a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2033").

The Compound (A-10) wherein R⁹ represents a methyl group, R¹¹ represents a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2034").

The Compound (A-10) wherein R⁹ represents a methyl group, R¹¹ represents a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2035").

The Compound (A-10) wherein R⁹ represents a methyl group, R¹¹ represents a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2036").

The Compound (A-10) wherein R⁹ represents a methyl group, R¹¹ represents a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2037").

The Compound (A-10) wherein R⁹ represents a methyl group, R¹¹ represents a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2038").

The Compound (A-10) wherein R⁹ represents a methyl group, R¹¹ represents a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2039").

The Compound (A-10) wherein R⁹ represents a methyl group, R¹¹ represents a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2040").

The Compound (A-10) wherein R⁹ represents a methyl group, R¹¹ represents a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2041").

The Compound (A-10) wherein R⁹ represents a methyl group, R¹¹ represents a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2042").

The Compound (A-10) wherein R⁹ represents a methyl group, R¹¹ represents a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2043").

The Compound (A-10) wherein R⁹ represents a methyl group, R¹¹ represents a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2044").

The Compound (A-10) wherein R⁹ represents a methyl group, R¹¹ represents a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2045").

The Compound (A-10) wherein R⁹ represents a methyl group, R¹¹ represents a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2046").

The Compound (A-10) wherein R⁹ represents a methyl group, R¹¹ represents a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2047").

The Compound (A-10) wherein R⁹ represents a methyl group, R¹¹ represents a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2048").

The Compound (A-10) wherein R⁹ represents a methyl group, R¹¹ represents a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2049").

The Compound (A-10) wherein R⁹ represents a methyl group, R¹¹ represents a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2050").

The Compound (A-10) wherein R⁹ represents a methyl group, R¹¹ represents a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2051").

The Compound (A-10) wherein R⁹ represents a methyl group, R¹¹ represents a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2052").

The Compound (A-10) wherein R⁹ represents a methyl group, R¹¹ represents a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2053").

The Compound (A-10) wherein R⁹ represents a methyl group, R¹¹ represents a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2054").

The Compound (A-10) wherein R⁹ represents a methyl group, R¹¹ represents a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2055").

The Compound (A-10) wherein R⁹ represents a methyl group, R¹¹ represents a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2056").

The Compound (A-10) wherein R⁹ represents a methyl group, R¹¹ represents a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2057").

The Compound (A-10) wherein R¹¹ represents a fluorine atom, R⁹ represents a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2058").

The Compound (A-10) wherein R¹¹ represents a fluorine atom, R⁹ represents a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2059").

The Compound (A-10) wherein R¹¹ represents a fluorine atom, R⁹ represents a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2060").

The Compound (A-10) wherein R¹¹ represents a fluorine atom, R⁹ represents a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2061").

The Compound (A-10) wherein R¹¹ represents a fluorine atom, R⁹ represents a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2062").

The Compound (A-10) wherein R¹¹ represents a fluorine atom, R⁹ represents a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2063").

The Compound (A-10) wherein R¹¹ represents a fluorine atom, R⁹ represents a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2064").

The Compound (A-10) wherein R¹¹ represents a fluorine atom, R⁹ represents a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2065").

The Compound (A-10) wherein R¹¹ represents a fluorine atom, R⁹ represents a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2066").

The Compound (A-10) wherein R¹¹ represents a fluorine atom, R⁹ represents a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2067").

The Compound (A-10) wherein R¹¹ represents a fluorine atom, R⁹ represents a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2068").

The Compound (A-10) wherein R¹¹ represents a fluorine atom, R⁹ represents a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2069").

The Compound (A-10) wherein R¹¹ represents a fluorine atom, R⁹ represents a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2070").

The Compound (A-10) wherein R¹¹ represents a fluorine atom, R⁹ represents a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2071").

The Compound (A-10) wherein R¹¹ represents a fluorine atom, R⁹ represents a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2072").

The Compound (A-10) wherein R¹¹ represents a fluorine atom, R⁹ represents a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2073").

The Compound (A-10) wherein R¹¹ represents a fluorine atom, R⁹ represents a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2074").

The Compound (A-10) wherein R¹¹ represents a fluorine atom, R⁹ represents a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2075").

The Compound (A-10) wherein R¹¹ represents a fluorine atom, R⁹ represents a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2076").

The Compound (A-10) wherein R¹¹ represents a fluorine atom, R⁹ represents a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2077").

The Compound (A-10) wherein R¹¹ represents a fluorine atom, R⁹ represents a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2078").

The Compound (A-10) wherein R¹¹ represents a fluorine atom, R⁹ represents a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2079").

The Compound (A-10) wherein R¹¹ represents a fluorine atom, R⁹ represents a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2080").

The Compound (A-10) wherein R¹¹ represents a fluorine atom, R⁹ represents a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2081").

The Compound (A-10) wherein R¹¹ represents a fluorine atom, R⁹ represents a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2082").

The Compound (A-10) wherein R¹¹ represents a fluorine atom, R⁹ represents a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2083").

The Compound (A-10) wherein R¹¹ represents a fluorine atom, R⁹ represents a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2084").

The Compound (A-10) wherein R¹¹ represents a fluorine atom, R⁹ represents a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2085").

The Compound (A-10) wherein R¹¹ represents a fluorine atom, R⁹ represents a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2086").

The Compound (A-10) wherein R¹¹ represents a fluorine atom, R⁹ represents a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2087").

The Compound (A-10) wherein R¹¹ represents a fluorine atom, R⁹ represents a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2088").

The Compound (A-10) wherein R¹¹ represents a fluorine atom, R⁹ represents a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2089").

The Compound (A-10) wherein R¹¹ represents a fluorine atom, R⁹ represents a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2090").

The Compound (A-10) wherein R¹¹ represents a fluorine atom, R⁹ represents a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2091").

The Compound (A-10) wherein R¹¹ represents a chlorine atom, R⁹ represents a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2092").

The Compound (A-10) wherein R¹¹ represents a chlorine atom, R⁹ represents a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2093").

The Compound (A-10) wherein R¹¹ represents a chlorine atom, R⁹ represents a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2094").

The Compound (A-10) wherein R¹¹ represents a chlorine atom, R⁹ represents a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2095").

The Compound (A-10) wherein R¹¹ represents a chlorine atom, R⁹ represents a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2096").

The Compound (A-10) wherein R¹¹ represents a chlorine atom, R⁹ represents a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2097").

The Compound (A-10) wherein R¹¹ represents a chlorine atom, R⁹ represents a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2098").

The Compound (A-10) wherein R¹¹ represents a chlorine atom, R⁹ represents a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2099").

The Compound (A-10) wherein R¹¹ represents a chlorine atom, R⁹ represents a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2100").

The Compound (A-10) wherein R¹¹ represents a chlorine atom, R⁹ represents a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2101").

The Compound (A-10) wherein R¹¹ represents a chlorine atom, R⁹ represents a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2102").

The Compound (A-10) wherein R¹¹ represents a chlorine atom, R⁹ represents a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2103").

The Compound (A-10) wherein R¹¹ represents a chlorine atom, R⁹ represents a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2104").

The Compound (A-10) wherein R¹¹ represents a chlorine atom, R⁹ represents a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2105").

The Compound (A-10) wherein R¹¹ represents a chlorine atom, R⁹ represents a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2106").

The Compound (A-10) wherein R¹¹ represents a chlorine atom, R⁹ represents a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2107").

The Compound (A-10) wherein R¹¹ represents a chlorine atom, R⁹ represents a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2108").

The Compound (A-10) wherein R¹¹ represents a chlorine atom, R⁹ represents a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2109").

The Compound (A-10) wherein R¹¹ represents a chlorine atom, R⁹ represents a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2110").

The Compound (A-10) wherein R¹¹ represents a chlorine atom, R⁹ represents a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2111").

The Compound (A-10) wherein R¹¹ represents a chlorine atom, R⁹ represents a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2112").

The Compound (A-10) wherein R¹¹ represents a chlorine atom, R⁹ represents a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2113").

The Compound (A-10) wherein R¹¹ represents a chlorine atom, R⁹ represents a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2114").

The Compound (A-10) wherein R¹¹ represents a chlorine atom, R⁹ represents a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2115").

The Compound (A-10) wherein R¹¹ represents a chlorine atom, R⁹ represents a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2116").

The Compound (A-10) wherein R¹¹ represents a chlorine atom, R⁹ represents a hydrogen atom, n represents 2, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2117").

The Compound (A-10) wherein R¹¹ represents a chlorine atom, R⁹ represents a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2118").

The Compound (A-10) wherein R¹¹ represents a chlorine atom, R⁹ represents a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2119").

The Compound (A-10) wherein R¹¹ represents a chlorine atom, R⁹ represents a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2120").

The Compound (A-10) wherein R¹¹ represents a chlorine atom, R⁹ represents a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2121").

The Compound (A-10) wherein R¹¹ represents a chlorine atom, R⁹ represents a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2122").

The Compound (A-10) wherein R¹¹ represents a chlorine atom, R⁹ represents a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2123").

The Compound (A-10) wherein R¹¹ represents a chlorine atom, R⁹ represents a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2124").

The Compound (A-10) wherein R¹¹ represents a chlorine atom, R⁹ represents a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2125").

The Compound (A-10) wherein R¹¹ represents a methyl group, R⁹ represents a hydrogen atom, n represents 1, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2126").

The Compound (A-10) wherein R¹¹ represents a methyl group, R⁹ represents a hydrogen atom, n represents 1, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2127").

The Compound (A-10) wherein R¹¹ represents a methyl group, R⁹ represents a hydrogen atom, n represents 1, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2128").

The Compound (A-10) wherein R¹¹ represents a methyl group, R⁹ represents a hydrogen atom, n represents 1, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2129").

The Compound (A-10) wherein R¹¹ represents a methyl group, R⁹ represents a hydrogen atom, n represents 1, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2130").

The Compound (A-10) wherein R¹¹ represents a methyl group, R⁹ represents a hydrogen atom, n represents 1, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2131").

The Compound (A-10) wherein R¹¹ represents a methyl group, R⁹ represents a hydrogen atom, n represents 1, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2132").

The Compound (A-10) wherein R¹¹ represents a methyl group, R⁹ represents a hydrogen atom, n represents 1, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2133").

The Compound (A-10) wherein R¹¹ represents a methyl group, R⁹ represents a hydrogen atom, n represents 1, Z represents a 3-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2134").

The Compound (A-10) wherein R¹¹ represents a methyl group, R⁹ represents a hydrogen atom, n represents 1, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2135").

The Compound (A-10) wherein R¹¹ represents a methyl group, R⁹ represents a hydrogen atom, n represents 1, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2136").

The Compound (A-10) wherein R¹¹ represents a methyl group, R⁹ represents a hydrogen atom, n represents 1, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2137").

The Compound (A-10) wherein R¹¹ represents a methyl group, R⁹ represents a hydrogen atom, n represents 1, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2138").

The Compound (A-10) wherein R¹¹ represents a methyl group, R⁹ represents a hydrogen atom, n represents 1, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2139").

The Compound (A-10) wherein R¹¹ represents a methyl group, R⁹ represents a hydrogen atom, n represents 1, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2140").

The Compound (A-10) wherein R¹¹ represents a methyl group, R⁹ represents a hydrogen atom, n represents 1, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2141").

The Compound (A-10) wherein R¹¹ represents a methyl group, R⁹ represents a hydrogen atom, n represents 1, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2142").

The Compound (A-10) wherein R¹¹ represents a methyl group, R⁹ represents a hydrogen atom, n represents 2, Z represents an ethyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2143").

The Compound (A-10) wherein R¹¹ represents a methyl group, R⁹ represents a hydrogen atom, n represents 2, Z represents a propyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2144").

The Compound (A-10) wherein R¹¹ represents a methyl group, R⁹ represents a hydrogen atom, n represents 2, Z represents an isopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2145").

The Compound (A-10) wherein R¹¹ represents a methyl group, R⁹ represents a hydrogen atom, n represents 2, Z represents -CH=CHCH₃, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2146").

The Compound (A-10) wherein R¹¹ represents a methyl group, R⁹ represents a hydrogen atom, n represents 2, Z represents -CH₂CH=CH₂, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2147").

The Compound (A-10) wherein R¹¹ represents a methyl group, R⁹ represents a hydrogen atom, n represents 2, Z represents a butyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2148").

The Compound (A-10) wherein R¹¹ represents a methyl group, R⁹ represents a hydrogen atom, n represents 2, Z represents a cyclopropyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2149").

The Compound (A-10) wherein R¹¹ represents a methyl group, R⁹ represents a hydrogen atom, n represents 2, Z represents a 2-thienyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2150").

The Compound (A-10) wherein R¹¹ represents a methyl group, R⁹ represents a hydrogen atom, n represents 2, Z represents a 3-thienyl group, and the combination of the group represented by G^{A} and the substituents R^{X7}, R^{X8}, R^{X9,} and R^{X10} in the group represented by G^{A} represents any one combination indicated in the Combination A (hereinafter referred to as "Compound group SX2151").

The Compound (A-10) wherein R¹¹ represents a methyl group, R⁹ represents a hydrogen atom, n represents 2, Z represents a 2-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2152").

The Compound (A-10) wherein R¹¹ represents a methyl group, R⁹ represents a hydrogen atom, n represents 2, Z represents a 3-furanyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2153").

The Compound (A-10) wherein R¹¹ represents a methyl group, R⁹ represents a hydrogen atom, n represents 2, Z represents a 2-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2154").

The Compound (A-10) wherein R¹¹ represents a methyl group, R⁹ represents a hydrogen atom, n represents 2, Z represents a 3-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2155").

The Compound (A-10) wherein R¹¹ represents a methyl group, R⁹ represents a hydrogen atom, n represents 2, Z represents a 4-pyridyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2156").

The Compound (A-10) wherein R¹¹ represents a methyl group, R⁹ represents a hydrogen atom, n represents 2, Z represents a 2-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2157").

The Compound (A-10) wherein R¹¹ represents a methyl group, R⁹ represents a hydrogen atom, n represents 2, Z represents a 4-pyrimidyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2158").

The Compound (A-10) wherein R¹¹ represents a methyl group, R⁹ represents a hydrogen atom, n represents 2, Z represents a 2-pyrazinyl group, and the combination of the group represented by G^{A} and the substituents R^{X7}, R^{X8}, R^{X9}, and R^{X10} in the group represented by G^{A} represents any one combination indicated in the Combination A (hereinafter referred to as "Compound group SX2159").

Next, Formulation Examples are shown below. The "part(s)" represents "part(s) by weight". Also, the expression of "Present compound S" represents the compounds described in the Compound groups SX1 to SX2159 and the Present compounds 1 to 120.

### Formulation Example 1

A mixture of polyoxyethylene alkyl ether sulfate ammonium salt and silica (weight ratio of 1 : 1) (35 parts), any one of the Present compound S (10 parts), and water (55 parts) are mixed, and the resulting mixture is subjected to fine grinding according to a wet grinding method to obtain each formulation.

### Formulation Example 2

Any one of the Present compound S (50 parts), calcium lignin sulfonate (3 parts), sodium lauryl sulfate (2 parts), and silica (45 parts) are ground and mixed to obtain each formulation.

### Formulation Example 3

Any one of the Present compound S (5 parts), polyoxyethylene styryl phenyl ether (9 parts), polyoxyethylene decyl ether (number of added ethyleneoxide: 5) (5 parts), calcium dodecylbenzene sulfonate (6 parts), and xylene (75 parts) are mixed to obtain each formulation.

### Formulation Example 4

Any one of the Present compound S (2 parts), silica (1 part), calcium lignin sulfonate (2 parts), bentonite (30 parts), and kaolin clay (65 parts) are ground and mixed, an appropriate amount of water is added thereto, the resulting mixture is kneaded, subjected to granulation with a granulator, and then dried to obtain each formulation.

### Formulation Example 5

Any one of the Present compound S (10 parts), and a mixture of benzyl alcohol (18 parts) and DMSO (9 parts) are mixed, GERONOL (registered trademark) TE250 (6.3 parts), Ethylan (registered trademark) NS-500LQ (2.7 parts), and solvent naphtha (54 parts) are added thereto, and the resulting mixture is mixed to obtain each formulation.

Next, Test Examples are shown below.

The term of "non-treated well" in Test Example 1-2 and Test Example 15 represents a well wherein a test was carried out under the same conditions as those described in the Test Example except for dispensing DMSO instead of a diluted solution with DMSO comprising the Present compound W. The term of "non-treated" in Test Example 16 represents a test which was carried out under the same conditions as those described in the Test Example except for using DMSO instead of a diluted solution with DMSO comprising 10000 ppm of the Present compound W. Also, the term of "non-treated leaf disk" in Test Example 7 and Test Example 7-1 represents a leaf disk wherein a test was carried out under the same conditions as those described in the Test Example except for dispensing DMSO instead of a diluted solution with DMSO comprising the Present compound W. Also, the term of "non-treated" in Test Example 8, Test Example 9, Test Example 9-1, Test Example 10, Test Example 10-1, and Test Example 11 means that a diluted solution with water of a formulation comprising the Present compound W was not sprayed.

### Test Example 1 Control test against wheat leaf blotch fungus (Septoria tritici)

Each Present compound W is diluted with DMSO to obtain a diluted solution containing 150 ppm of the compound, 1 µL of the diluted solution is dispensed into a titer plate (96 well), and thereto is then dispensed 150 µL of a YBG medium to which spores of Septoria tritici are inoculated in advance. This plate is cultured at 18°C for 3 days, thereby allowing Septoria tritici to undergo proliferation, and the absorbance at 550 nm of each well of the titer plate is then measured to determine a degree of growth of Septoria tritici. As a result, each Present compound W shows a control effect against the plant disease.

### Test Example 1-2 Control test against wheat leaf blotch fungus (Septoria tritici)

The Present compound 46 was diluted with DMSO to obtain a diluted solution containing 1500 ppm of the compound, 1 µL of the diluted solution was dispensed into a titer plate (96 well), and thereto was then dispensed 150 µL of a YBG medium to which spores of Septoria tritici were inoculated in advance. This plate was cultured at 18°C for 3 days, thereby allowing Septoria tritici to undergo proliferation, and the absorbance at 550 nm of each well of the titer plate was then measured to determine a degree of growth of Septoria tritici. As a result, the degree of growth in the well treated with the above Present compound W was 50% or less relative to the degree of growth in the non-treated well.

### Test Example 2 Control test against corn smut fungus (Ustilago maydis)

Each Present compound W is diluted with DMSO to obtain a diluted solution containing 150 ppm of the compound, 1 µL of the diluted solution is dispensed into a titer plate (96 well), and thereto is then dispensed 150 µL of a potato dextrose broth medium (PDB medium) to which spores of Ustilago maydis are inoculated in advance. This plate is cultured at 18°C for 4 days, thereby allowing Ustilago maydis to undergo proliferation, and the absorbance at 550 nm of each well of the titer plate is then measured to determine a degree of growth of Ustilago maydis. As a result, each Present compound W shows a control effect against the plant disease.

### Test Example 3 Control test against barley scald fungus (Rhynchosporium secalis)

Each Present compound W is diluted with DMSO to obtain a diluted solution containing 150 ppm of the compound, 1 µL of the diluted solution is dispensed into a titer plate (96 well), and thereto is then dispensed 150 µL of a potato dextrose broth medium (PDB medium) to which spores of Rhynchosporium secalis are inoculated in advance. This plate is cultured at 18°C for 7 days, thereby allowing Rhynchosporium secalis to undergo proliferation, and the absorbance at 550 nm of each well of the titer plate is then measured to determine a degree of growth of Rhynchosporium secalis. As a result, each Present compound W shows a control effect against the plant disease.

### Test Example 4 Control test against cucumber Botrytis rot fungus (Botrytis cinerea)

Each Present compound W is diluted with DMSO to obtain a diluted solution containing 150 ppm of the compound, 1 µL of the diluted solution is dispensed into a titer plate (96 well), and thereto is then dispensed 150 µL of a complete medium to which spores of Botrytis cinerea are inoculated in advance. This plate is cultured at 18°C for 4 days, thereby allowing Botrytis cinerea to undergo proliferation, and the absorbance at 550 nm of each well of the titer plate is then measured to determine a degree of growth of Botrytis cinerea. As a result, each Present compound W shows a control effect against the plant disease.

### Test Example 5 Control test against peach scab fungus (Cladosporium carpophilum)

Each Present compound W is diluted with DMSO to obtain a diluted solution containing 150 ppm of the compound, 1 µL of the diluted solution is dispensed into a titer plate (96 well), and thereto is then dispensed 150 µL of a potato dextrose broth medium (PDB medium) to which spores of Cladosporium carpophilum are inoculated in advance. This plate is cultured at 18°C for 5 days, thereby allowing Cladosporium carpophilum to undergo proliferation, and the absorbance at 550 nm of each well of the titer plate is then measured to determine a degree of growth of Cladosporium carpophilum. As a result, each Present compound W shows a control effect against the plant disease.

### Test Example 6 Control test against rice brown spot fungus (Cochliobolus miyabeanus)

Each Present compound W is diluted with DMSO to obtain a diluted solution containing 150 ppm of the compound, 1 µL of the diluted solution is dispensed into a titer plate (96 well), and thereto is then dispensed 150 µL of a YB liquid medium to which spores of Cochliobolus miyabeanus are inoculated in advance. This plate is cultured at 23°C for 3 days, thereby allowing Cochliobolus miyabeanus to undergo proliferation, and the absorbance at 550 nm of each well of the titer plate is then measured to determine a degree of growth of Cochliobolus miyabeanus. As a result, each Present compound W shows a control effect against the plant disease.

### Test Example 7 Control test against soybean rust (Phakopsora pachyrhizi)

A true leaf of a soybean (cultivar: Kurosengoku) was cut into a diameter of 1 cm to prepare each leaf disk. To each well of a 24 well microplate was dispensed 1 mL of an agar medium (agar concentration: 1.2%), and then one of said leaf disk was placed on the agar medium in each well. To a mixture of Sorpol (registered trademark) 1200KX (0.5 µL), DMSO (4.5 µL), and xylene (5 µL) was added a DMSO solution (20 µL) comprising a test compound (10000 ppm), and the resulting mixture was mixed. The resulting mixture was diluted with ion exchange water to prepare a mixture comprising a prescribed concentration of the test compound. The resulting mixture was sprayed into each leaf disk at a ratio of 10 µL per leaf disk. After 1 day, an aqueous suspension of spores of soybean rust fungus (Phakopsora pachyrhizi) having an amino acid substitution of F129L in a mitochondrial cytochrome b protein (1.0 × 10⁵ / mL) was inoculated by spraying on each leaf disk. After the inoculation, the microplate was placed into an artificial climate chamber (Lighting: 18 hours, Lights-out: 6 hours, Temperature: 23°C, Humidity: 60%). After 1 day, the leaf disks were air-dried until water droplets on the surfaces of the leaf disks disappeared, and the microplate was placed into the artificial climate chamber again for 12 days. Then, lesion area of soybean rust was investigated. As a result, when the prescribed concentration was 200 ppm, each lesion area of the leaf disk treated with any one of the Present compound 5, 17, 20, 23, 24, 25, 26, 27, 29, 30, 31, 32, 33, 34, 35, 36, 37, 38, 48, 50, 51, 52, 53, 54, 55, 57, 58, 59, 61, 62, 63, 64, 67, 70, 72, 73, 76, 77, 80, 82, 83, 85, 86, 87, 88, 89, 90, 91, 92, 93, 96, 97, 98, 103, 104, 105, 106, 108, 109, 114, 115, 116, 117, or 118 as the test compound was 30% or less relative to the lesion area of the non-treated leaf disk.

### Test Example 7-1 Control test against soybean rust (Phakopsora pachyrhizi)

A true leaf of a soybean (cultivar: Kurosengoku) was cut into a diameter of 1 cm to prepare each leaf disk. To each well of a 24 well microplate was dispensed 1 mL of an agar medium (agar concentration: 1.2%), and then one of said leaf disk was placed on the agar medium in each well. To a mixture of Sorpol (registered trademark) 1200KX (0.5 µL), DMSO (4.5 µL), and xylene (5 µL) was added a DMSO solution (20 µL) comprising a test compound (10000 ppm), and the resulting mixture was mixed. The resulting mixture was diluted with ion exchange water to prepare a mixture comprising a prescribed concentration of the test compound. The resulting mixture was sprayed into each leaf disk at a ratio of 10 µL per leaf disk. After 1 day, an aqueous suspension of spores of soybean rust fungus (Phakopsora pachyrhizi) (1.0 × 10⁵ / mL) was inoculated by spraying on each leaf disk. After the inoculation, the microplate was placed into an artificial climate chamber (Lighting: 18 hours, Lights-out: 6 hours, Temperature: 23°C, Humidity: 60%). After 1 day, the leaf disks were air-dried until water droplets on the surfaces of the leaf disks disappeared, and the microplate was placed into the artificial climate chamber again for 12 days. Then, lesion area of soybean rust was investigated. As a result, when the prescribed concentration was 200 ppm, each lesion area of the leaf disk treated with any one of the Present compound 71 or 107 as the test compound was 30% or less relative to the lesion area of the non-treated leaf disk.

### Test Example 8 Control test against barley net blotch (Pyrenophora teres)

A plastic pot was filled with soil, barley (cultivar: Nishinohoshi) was seeded thereto, and grown in a greenhouse for 7 days. The Present compound 4 formulated according to the method described in the Formulation Example 1 was mixed with water in such a way that the concentration thereof was 200 ppm, and the resulting mixture was sprayed to foliage of the above barley so as to adequately adhere onto the surfaces of leaves of the above barley. After spraying the mixture, the barley was air-dried. After 1 day, an aqueous suspension of spores of Pyrenophora teres was inoculated by spraying to the barley. After the inoculation, the barley was placed in a greenhouse at 23°C in daytime and at 20°C in nighttime under a humid condition for 3 days, then cultured in a greenhouse for 7 days, and then a lesion area was investigated. As a result, the lesion area in the barley treated with the above Present compound W was 30% or less relative to the lesion area in the non-treated barley.

### Test Example 9 Control test against wheat leaf rust (Puccinia recondita)

A plastic pot was filled with soil, wheat (cultivar: Shirogane) was seeded thereto, and grown in a greenhouse for 9 days. The Present compound 41 formulated according to the method described in the Formulation Example 1 was mixed with water in such a way that the concentration thereof was 200 ppm, and the resulting mixture was sprayed to foliage of the above wheat so as to adequately adhere onto the surfaces of leaves of the above wheat. After spraying the mixture, the wheat was air-dried, cultured at 20°C under lighting for 5 to 7 days, and then spores of Puccinia recondita were inoculated by dusting thereto. After the inoculation, the wheat was placed at 23°C under a dark humid condition for 1 day, then cultured at 20°C under lighting for 8 days, and a lesion area was investigated. As a result, the lesion area in the wheat treated with the above Present compound W was 30% or less relative to the lesion area in the non-treated wheat.

### Test Example 9-1 Control test against wheat leaf rust (Puccinia recondita)

A plastic pot was filled with soil, wheat (cultivar: Shirogane) was seeded thereto, and grown in a greenhouse for 9 days. The Present compound 7 or 68 formulated according to the method described in the Formulation Example 1 was mixed with water in such a way that the concentration thereof was 500 ppm, and the resulting mixture was sprayed to foliage of the above wheat so as to adequately adhere onto the surfaces of leaves of the above wheat. After spraying the mixture, the wheat was air-dried, cultured at 20°C under lighting for 5 to 7 days, and then spores of Puccinia recondita were inoculated by dusting thereto. After the inoculation, the wheat was placed at 23°C under a dark humid condition for 1 day, then cultured at 20°C under lighting for 8 days, and a lesion area was investigated. As a result, the lesion area in the wheat treated with each of the above Present compound W was 30% or less relative to the lesion area in the non-treated wheat.

### Test Example 10 Control test against wheat leaf blotch (Septoria tritici)

A plastic pot was filled with soil, wheat (cultivar: Apogee) was seeded thereto, and cultured in a greenhouse for 10 days. The Present compound 3, 53, 62, or 102 formulated according to the method described in the Formulation Example 1 was mixed with water in such a way that the concentration thereof was 200 ppm, and the resulting mixture was sprayed to foliage of the above wheat so as to adequately adhere onto the surfaces of leaves of the above wheat. After spraying the mixture, the wheat was air-dried. After 4 days, an aqueous suspension comprising spores of Septoria tritici was inoculated by spraying to the wheat. After the inoculation, the wheat was placed at 18°C under a humid condition for 3 days, then cultured under lighting for 14 to 18 days, and then a lesion area was investigated. As a result, the lesion area in the wheat treated with each of the above Present compound W was 30% or less relative to the lesion area in the non-treated wheat.

### Test Example 10-1 Control test against wheat leaf blotch (Septoria tritici)

A plastic pot was filled with soil, wheat (cultivar: Apogee) was seeded thereto, and cultured in a greenhouse for 10 days. The Present compound 11, 12, 13, or 14 formulated according to the method described in the Formulation Example 1 was mixed with water in such a way that the concentration thereof was 500 ppm, and the resulting mixture was sprayed to foliage of the above wheat so as to adequately adhere onto the surfaces of leaves of the above wheat. After spraying the mixture, the wheat was air-dried. After 4 days, an aqueous suspension comprising spores of Septoria tritici was inoculated by spraying to the wheat. After the inoculation, the wheat was placed at 18°C under a humid condition for 3 days, then cultured under lighting for 14 to 18 days, and then a lesion area was investigated. As a result, the lesion area in the wheat treated with each of the above Present compound W was 30% or less relative to the lesion area in the non-treated wheat.

### Test Example 11 Control test against soybean rust (Phakopsora pachyrhizi)

A plastic pot was filled with soil, soybeans (cultivar: Kurosengoku) were seeded thereto, and cultured in a greenhouse for 10 to 14 days. The Present compound 5, 9, 12, 13, 15, 16, 17, 20, 22, 23, 24, 26, 27, 28, 29, 30, 32, 33, 34, 35, 36, 37, 38, 50, 51, 52, 53, 54, 55, 56, 57, 58, 59, 60, 61, 62, 63, 64, 65, 66, 69, 70, 72, 73, 75, 76, 77, 78, 80, 82, 83, 85, 86, 87, 88, 89, 90, 91, 92, 93, 94, 95, 96, 97, 98, 103, 104, 105, 106, 108, 109, 111, or 114 formulated according to the method described in the Formulation Example 1 was mixed with water in such a way that the concentration thereof was 200 ppm, and the resulting mixture was sprayed to foliage of the above soybeans so as to adequately adhere onto the surfaces of leaves of the above soybeans. After spraying the mixture, the soybeans were air-dried. After 2 to 5 days, an aqueous suspension of spores of Phakopsora pachyrhizi was inoculated by spraying to the soybeans. After the inoculation, the soybeans were placed in a greenhouse at 23°C in daytime and at 20°C in nighttime under a humid condition for 1 to 2 day(s), then cultured in a greenhouse for 12 days, and then a lesion area was investigated. As a result, the lesion area in the soybeans treated with each of the above Present compound W was 30% or less relative to the lesion area in the non-treated soybeans.

### Test Example 12 Control test against soybean rust (Phakopsora pachyrhizi)

A plastic pot is filled with soil, soybeans (cultivar: Kurosengoku) are seeded thereto, and cultured in a greenhouse for 10 days. An aqueous suspension comprising spores of Phakopsora pachyrhizi is inoculated by spraying to the soybeans. After the inoculation, the soybeans are placed in a greenhouse at 23°C in daytime and at 20°C in nighttime under a humid condition for 1 day, and then cultured in a greenhouse for 2 days. Then, the Present compound W formulated according to the method described in the Formulation Example 1 is mixed with water in such a way that the concentration thereof is 200 ppm, and the resulting mixture is sprayed to foliage of the above soybeans so as to adequately adhere onto the surfaces of leaves of the above soybeans. After spraying the mixture, the soybeans are air-dried, then cultured in a greenhouse for 8 days, and then a lesion area is investigated. As a result, each Present compound W shows a control effect against the plant disease.

### Test Example 13 Control test against soybean Cercospora leaf spot (Cercospora sojina)

A plastic pot is filled with soil, soybeans (cultivar: Tachinagaha) are seeded thereto, and cultured in a greenhouse for 13 days. The Present compound W formulated according to the method described in the Formulation Example 1 is mixed with water in such a way that the concentration thereof is 200 ppm, and the resulting mixture is sprayed to foliage of the above soybeans so as to adequately adhere onto the surfaces of leaves of the above soybeans. After spraying the mixture, the soybeans are air-dried. After 1 day, an aqueous suspension of spores of Cercospora sojina is inoculated by spraying to the soybeans. After the inoculation, the soybeans are placed in a greenhouse at 23°C in daytime and at 20°C in nighttime under a humid condition for 3 days, then cultured in a greenhouse for 16 days, and then a lesion area is investigated. As a result, each Present compound W shows a control effect against the plant disease.

### Test Example 14 Control test against tomato early blight (Alternaria solani)

A plastic pot is filled with soil, tomatoes (cultivar: Patio) are seeded thereto, and cultured in a greenhouse for 20 days. The Present compound W formulated according to the method described in the Formulation Example 1 is mixed with water in such a way that the concentration thereof is 200 ppm, and the resulting mixture is sprayed to foliage of the above tomatoes so as to adequately adhere onto the surfaces of leaves of the above tomatoes. After spraying the mixture, the tomatoes are air-dried. After 1 day, an aqueous suspension of spores of Alternaria solani is inoculated by spraying to the tomatoes. After the inoculation, the tomatoes are placed at 18°C under a humid condition for 6 days, and then a lesion area is investigated. As a result, each Present compound W shows a control effect against the plant disease.

### Test Example 15 Control test against soybean rust fungus (Phakopsora pachyrhizi)

Each Present compound W was diluted with DMSO to obtain a diluted solution containing 150 ppm of the compound, 1 µL of the diluted solution was dispensed into a titer plate (96 well), and thereto was then dispensed 149 µL of an aqueous suspension of spores of Phakopsora pachyrhizi to which spores of Phakopsora pachyrhizi were suspended in advance (1.0 × 10⁴ / mL). This plate was cultured at 23°C for several hours, and then germinated spores of Phakopsora pachyrhizi were counted. As a result, when the treatment concentration was 1 ppm, the number of germinated spores in each well comprising any one of the Present compound 17, 20, 21, 23, 24, 26, 27, 29, 30, 32, 33, 34, 35, 36, 37, 38, 39, 50, 51, 52, 53, 54, 55, 57, 58, 59, 60, 61, 62, 63, 65, 66, 67, 70, 72, 73, 76, 77, 78, 79, 80, 81, 82, 84, 85, 86, 87, 89, 90, 91, 92, 93, 95, 96, 97, 98, 103, 104, 105, 114, 115, 116, 117, 118, 119, or 120 as the test compound was 50% or less relative to the number of germinated spores in the non-treated well.

### Test Example 16 Control test against soybean rust fungus (Phakopsora pachyrhizi)

An agar medium (agar concentration: 1.0%) (3 mL) was placed in a plastic petri dish having a diameter of 3.5 cm, and solidified. Each Present compound W was diluted with DMSO in such a way that the resulting diluted solution containing 10000 ppm of the compound, and said solution (1 µL) and an aqueous suspension of spores of Phakopsora pachyrhizi to which spores of Phakopsora pachyrhizi were suspended (1.0 × 10⁴ / mL) (999 µL) were mixed, and then the resulting mixture was dispensed into the plastic petri dish. This plastic petri dish was cultured at 23°C for 1 day, and then germinated spores of Phakopsora pachyrhizi were counted. As a result, when the treatment concentration was 10 ppm, and any one of the Present compound 9, 10, 13, 16, 41, 47, 48, or 69 was used as the test compound to carry out the test, the number of germinated spores in each plastic petri dish using the above each Present compound W as the test compound was 50% or less relative to the number of germinated spores in the non-treated plastic petri dish.

### Test Example 17 Control test against soybean rust fungus (Phakopsora pachyrhizi)

A test is carried out by the method according to the Test Example 16 and using each Present compound W as the test compound with the treatment concentration of 10 ppm. As a result, each Present compound W shows a control effect against the plant disease.

### INDUSTRIAL APPLICABILITY

The Present compounds W have control effects against plant diseases, and can be used in controlling plant diseases.

## Claims

1. A method for controlling a plant disease which comprises applying a compound represented by formula (I) 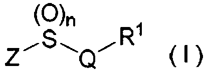 [wherein:
n represents 1 or 2;
R¹ represents a C3-C10 alicyclic hydrocarbon group, a 3-10 membered nonaromatic heterocyclic group {wherein said C3-C10 alicyclic hydrocarbon group and said 3-10 membered nonaromatic heterocyclic group are optionally substituted with one or more substituent(s) selected from Group A, and among atoms constituting said C3-C10 alicyclic hydrocarbon group and said 3-10 membered nonaromatic heterocyclic group, any atom which is not bound to Q optionally forms a C3-C7 alicyclic hydrocarbon or a 3-7 membered nonaromatic heterocycle as a spiro atom}, a 5-7 membered aromatic heterocyclic group optionally substituted with one or more substituent(s) selected from Group B1 (provided that a 1H-pyrazol-3-yl group is excluded when Q represents a group represented by Q6) {wherein among atoms constituting said 5-7 membered aromatic heterocyclic group (provided that a 1H-pyrazol-3-yl group is excluded when Q represents a group represented by Q6), any two adjacent atoms which are not bound to Q optionally form another benzene ring, C4-C7 alicyclic hydrocarbon, 4-7 membered nonaromatic heterocycle, or 5-7 membered aromatic heterocycle comprising said two adjacent atoms {wherein said benzene ring, said C4-C7 alicyclic hydrocarbon, said 4-7 membered nonaromatic heterocycle, and said 5-7 membered aromatic heterocycle are optionally substituted with one or more halogen atom(s)}}, or -C≡CR¹³;
Q represents a group represented by Q1, a group represented by Q2, a group represented by Q3, a group represented by Q4, a group represented by Q5, or a group represented by Q6;
Z represents a C2-C6 chain hydrocarbon group, a methyl group substituted with one or more fluorine atom(s), a C1-C2 alkyl group {wherein said C1-C2 alkyl group is substituted with a methoxy group or an ethoxy group}, a C3-C8 alicyclic hydrocarbon group, a 3-8 membered nonaromatic heterocyclic group {wherein said C3-C8 alicyclic hydrocarbon group and said 3-8 membered nonaromatic heterocyclic group are optionally substituted with one or more substituent(s) selected from Group C}, a 5-10 membered aromatic heterocyclic group optionally substituted with one or more substituent(s) selected from Group D, or NR¹⁴R¹⁵;
the group represented by Q1, the group represented by Q2, the group represented by Q3, the group represented by Q4, the group represented by Q5, and the group represented by Q6 represent the groups represented by the following formulae (wherein # represents the binding site to R¹, and • represents the binding site to the sulfur atom);
Q = 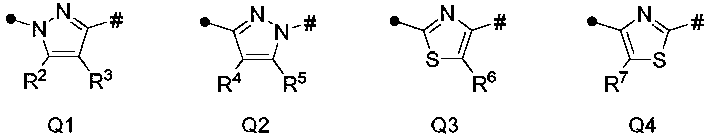 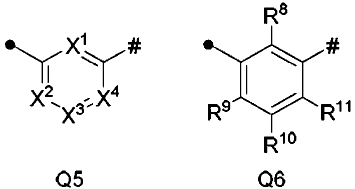
R², R³, R⁴, R⁵, R⁶, and R⁷ are identical to or different from each other, and each represent a hydrogen atom or a fluorine atom;
X¹ represents CR¹² or a nitrogen atom;
X² represents CR¹⁶ or a nitrogen atom;
X³ represents CR¹⁷ or a nitrogen atom;
X⁴ represents CR¹⁸ or a nitrogen atom (provided that a combination wherein X¹ represents CR¹², X² represents CR¹⁶, X³ represents CR¹⁷, and X⁴ represents CR¹⁸ is excluded);
R⁸, R⁹, R¹⁰, R¹¹, R¹², R¹⁶, R¹⁷, and R¹⁸ are identical to or different from each other, and each represent a methyl group optionally substituted with one or more fluorine atom(s), a halogen atom, or a hydrogen atom;
R¹³ represents a C1-C6 chain hydrocarbon group optionally substituted with one or more substituent(s) selected from Group E, a C3-C10 alicyclic hydrocarbon group, a 3-10 membered nonaromatic heterocyclic group {wherein said C3-C10 alicyclic hydrocarbon group and said 3-10 membered nonaromatic heterocyclic group are optionally substituted with one or more substituent(s) selected from Group A}, a phenyl group, or a 5-7 membered aromatic heterocyclic group {wherein said phenyl group and said 5-7 membered aromatic heterocyclic group are optionally substituted with one or more substituent(s) selected from Group D}; and
R¹⁴ and R¹⁵ are identical to or different from each other, and each represent a C1-C6 alkyl group, a methyl group substituted with one or more fluorine atom(s), or a C1-C2 alkyl group {wherein said C1-C2 alkyl group is substituted with a methoxy group or an ethoxy group};
Group A: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group E}, a hydroxy group, a halogen atom, and a cyano group;
Group B1: a group consisting of a C3-C10 alicyclic hydrocarbon group optionally substituted with one or more substituent(s) selected from Group D, a methyl group optionally substituted with one or more substituent(s) selected from Group F, a C2-C6 chain hydrocarbon group optionally substituted with one or more substituent(s) selected from Group E, a C1-C6 alkoxy group, a C1-C6 alkylsulfanyl group, a C1-C6 alkylsulfinyl group, a C1-C6 alkylsulfonyl group {wherein said C1-C6 alkoxy group, said C1-C6 alkylsulfanyl group, said C1-C6 alkylsulfinyl group, and said C1-C6 alkylsulfonyl group are optionally substituted with one or more halogen atom(s)}, a hydroxy group, a halogen atom, and a cyano group;
Group C: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group E}, an oxo group, a thioxo group, a hydroxy group, a halogen atom, and a cyano group;
Group D: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group E}, a halogen atom, and a cyano group;
Group E: a group consisting of a C1-C3 alkoxy group, a halogen atom, and a cyano group;
Group F: a group consisting of a methoxy group, a chlorine atom, and a cyano group]
or an N-oxide thereof, or a salt thereof, to a plant or soil for cultivating a plant.

2. The method for controlling a plant disease according to claim 1, wherein the compound represented by formula (I) or an N-oxide thereof, or a salt thereof in claim 1 is a compound or an N-oxide thereof, or a salt thereof wherein
R¹ represents a C3-C10 alicyclic hydrocarbon group, a 3-10 membered nonaromatic heterocyclic group {wherein said C3-C10 alicyclic hydrocarbon group and said 3-10 membered nonaromatic heterocyclic group are optionally substituted with one or more substituent(s) selected from Group A, and among atoms constituting said C3-C10 alicyclic hydrocarbon group and said 3-10 membered nonaromatic heterocyclic group, any atom which is not bound to Q optionally forms a C3-C7 alicyclic hydrocarbon or a 3-7 membered nonaromatic heterocycle as a spiro atom}, a 5-7 membered aromatic heterocyclic group optionally substituted with one or more substituent(s) selected from Group B (provided that a 1H-pyrazol-3-yl group is excluded when Q represents the group represented by Q6) {wherein among atoms constituting said 5-7 membered aromatic heterocyclic group (provided that a 1H-pyrazol-3-yl group is excluded when Q represents the group represented by Q6), any two adjacent atoms which are not bound to Q optionally form another C4-C7 alicyclic hydrocarbon, 4-7 membered nonaromatic heterocycle, or 5-7 membered aromatic heterocycle comprising said two adjacent atoms}, or -C≡CR¹³;
Group B: a group consisting of a methyl group optionally substituted with one or more substituent(s) selected from Group F, a C2-C6 chain hydrocarbon group optionally substituted with one or more substituent(s) selected from Group E, a C1-C6 alkoxy group, a C1-C6 alkylsulfanyl group, a C1-C6 alkylsulfinyl group, a C1-C6 alkylsulfonyl group {wherein said C1-C6 alkoxy group, said C1-C6 alkylsulfanyl group, said C1-C6 alkylsulfinyl group, and said C1-C6 alkylsulfonyl group are optionally substituted with one or more halogen atom(s)}, a hydroxy group, a halogen atom, and a cyano group.

3. The method for controlling a plant disease according to claim 1, wherein the compound represented by formula (I) or an N-oxide thereof, or a salt thereof in claim 1 is a compound or an N-oxide thereof, or a salt thereof wherein
Q represents the group represented by Q1, the group represented by Q2, the group represented by Q5, or the group represented by Q6;
R¹ represents a C3-C10 alicyclic hydrocarbon group, a 3-10 membered nonaromatic heterocyclic group (provided that when Q represents the group represented by Q2, then a carbon atom constituting said 3-10 membered nonaromatic heterocyclic group is bound to the group represented by Q2) {wherein said C3-C10 alicyclic hydrocarbon group and said 3-10 membered nonaromatic heterocyclic group (provided that when Q represents the group represented by Q2, then a carbon atom constituting said 3-10 membered nonaromatic heterocyclic group is bound to the group represented by Q2) are optionally substituted with one or more substituent(s) selected from Group A, and among atoms constituting said C3-C10 alicyclic hydrocarbon group and said 3-10 membered nonaromatic heterocyclic group (provided that when Q represents the group represented by Q2, then a carbon atom constituting said 3-10 membered nonaromatic heterocyclic group is bound to the group represented by Q2), any atom which is not bound to Q optionally forms a C3-C7 alicyclic hydrocarbon or a 3-7 membered nonaromatic heterocycle as a spiro atom}, a 5-7 membered aromatic heterocyclic group optionally substituted with one or more substituent(s) selected from Group B1 (provided that a 1H-pyrazol-3-yl group is excluded when Q represents a group represented by Q6, and when Q represents the group represented by Q2, then a carbon atom constituting said 5-7 membered aromatic heterocyclic group is bound to the group represented by Q2) {wherein among atoms constituting said 5-7 membered aromatic heterocyclic group (provided that a 1H-pyrazol-3-yl group is excluded when Q represents a group represented by Q6, and when Q represents the group represented by Q2, then a carbon atom constituting said 5-7 membered aromatic heterocyclic group is bound to the group represented by Q2), any two adjacent atoms which are not bound to Q optionally form another benzene ring, C4-C7 alicyclic hydrocarbon, 4-7 membered nonaromatic heterocycle, or 5-7 membered aromatic heterocycle comprising said two adjacent atoms {wherein said benzene ring, said C4-C7 alicyclic hydrocarbon, said 4-7 membered nonaromatic heterocycle, and said 5-7 membered aromatic heterocycle are optionally substituted with one or more halogen atom(s)}}, or -C≡CR¹³;
Z represents a C2-C6 chain hydrocarbon group, a 5-6 membered aromatic heterocyclic group optionally substituted with one or more substituent(s) selected from Group D, or NR¹⁴R¹⁵;
R², R³, R⁴, R⁵, R⁸, R⁹, R¹⁰, R¹¹, and R¹² each represent a hydrogen atom;
X² and X⁴ each represent CH;
X³ represents a nitrogen atom;
R¹³ represents a C1-C6 chain hydrocarbon group or a phenyl group; and
R¹⁴ and R¹⁵ are identical to or different from each other, and each represent a C1-C6 alkyl group.

4. A compound represented by formula (II) 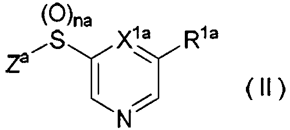 [wherein:
na represents 1 or 2;
X^{1a} represents CH or a nitrogen atom;
R^{1a} represents a C3-C10 alicyclic hydrocarbon group, a 3-7 membered nonaromatic heterocyclic group {wherein said C3-C10 alicyclic hydrocarbon group and said 3-7 membered nonaromatic heterocyclic group are optionally substituted with one or more substituent(s) selected from Group A^{a}, and among atoms constituting said C3-C10 alicyclic hydrocarbon group and said 3-7 membered nonaromatic heterocyclic group, any atom which is not bound to the pyridine or pyrazine optionally forms a C3-C7 alicyclic hydrocarbon or a 3-7 membered nonaromatic heterocycle as a spiro atom}, or a 5-7 membered aromatic heterocyclic group {wherein said 5-7 membered aromatic heterocyclic group is optionally substituted with one or more substituent(s) selected from Group B1^{a}, and among atoms constituting said 5-7 membered aromatic heterocyclic group, any two adjacent atoms which are not bound to the pyridine or pyrazine optionally form another benzene ring, C4-C7 alicyclic hydrocarbon, 4-7 membered nonaromatic heterocycle, or 5-7 membered aromatic heterocycle comprising said two adjacent atoms {wherein said benzene ring, said C4-C7 alicyclic hydrocarbon, said 4-7 membered nonaromatic heterocycle, and said 5-7 membered aromatic heterocycle are optionally substituted with one or more halogen atom(s)}}; and
Z^{a} represents a C2-C6 chain hydrocarbon group, a C3-C8 alicyclic hydrocarbon group optionally substituted with one or more substituent(s) selected from Group C^{a}, or a 5-10 membered aromatic heterocyclic group {wherein said 5-10 membered aromatic heterocyclic group is optionally substituted with one or more substituent(s) selected from Group D^{a}};
Group A^{a}: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group E^{a}}, a hydroxy group, a halogen atom, and a cyano group;
Group B1^{a}: a group consisting of a C3-C10 alicyclic hydrocarbon group optionally substituted with one or more substituent(s) selected from Group D^{a}, a methyl group optionally substituted with one or more substituent(s) selected from Group F^{a}, a C2-C6 chain hydrocarbon group optionally substituted with one or more substituent(s) selected from Group E^{a}, a C1-C6 alkoxy group, a C1-C6 alkylsulfanyl group, a C1-C6 alkylsulfinyl group, a C1-C6 alkylsulfonyl group {wherein said C1-C6 alkoxy group, said C1-C6 alkylsulfanyl group, said C1-C6 alkylsulfinyl group, and said C1-C6 alkylsulfonyl group are optionally substituted with one or more halogen atom(s)}, a hydroxy group, a halogen atom, and a cyano group;
Group C^{a}: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group E^{a}}, an oxo group, a thioxo group, a hydroxy group, a halogen atom, and a cyano group;
Group D^{a}: a group consisting of a C1-C6 chain hydrocarbon group, a C1-C6 alkoxy group {wherein said C1-C6 chain hydrocarbon group and said C1-C6 alkoxy group are optionally substituted with one or more substituent(s) selected from Group E^{a}}, a halogen atom, and a cyano group;
Group E^{a}: a group consisting of a C1-C3 alkoxy group, a halogen atom, and a cyano group;
Group F^{a}: a group consisting of a methoxy group, a chlorine atom, and a cyano group]
or an N-oxide thereof, or a salt thereof.

5. The compound or an N-oxide thereof, or a salt thereof according to claim 4, wherein
R^{1a} represents a 3-7 membered nonaromatic heterocyclic group {wherein said 3-7 membered nonaromatic heterocyclic group is optionally substituted with one or more substituent(s) selected from Group A^{a}, and among atoms constituting said 3-7 membered nonaromatic heterocyclic group, any atom which is not bound to the pyridine or pyrazine optionally forms a C3-C7 alicyclic hydrocarbon or a 3-7 membered nonaromatic heterocycle as a spiro atom}, or a 5-7 membered aromatic heterocyclic group {wherein said 5-7 membered aromatic heterocyclic group is optionally substituted with one or more substituent(s) selected from Group B^{a}, and among atoms constituting said 5-7 membered aromatic heterocyclic group, any two adjacent atoms which are not bound to the pyridine or pyrazine optionally form another C4-C7 alicyclic hydrocarbon, 4-7 membered nonaromatic heterocycle, or 5-7 membered aromatic heterocycle comprising said two adjacent atoms};
Group B^{a}: a group consisting of a methyl group optionally substituted with one or more substituent(s) selected from Group F^{a}, a C2-C6 chain hydrocarbon group optionally substituted with one or more substituent(s) selected from Group E^{a}, a C1-C6 alkoxy group, a C1-C6 alkylsulfanyl group, a C1-C6 alkylsulfinyl group, a C1-C6 alkylsulfonyl group {wherein said C1-C6 alkoxy group, said C1-C6 alkylsulfanyl group, said C1-C6 alkylsulfinyl group, and said C1-C6 alkylsulfonyl group are optionally substituted with one or more halogen atom(s)}, a hydroxy group, a halogen atom, and a cyano group.

6. The compound or an N-oxide thereof, or a salt thereof according to claim 4, wherein
R^{1a} represents a C3-C10 alicyclic hydrocarbon group optionally substituted with one or more C1-C6 chain hydrocarbon group(s), a 3-7 membered nonaromatic heterocyclic group {wherein said 3-7 membered nonaromatic heterocyclic group is optionally substituted with one or more substituent(s) selected from Group A^{a}2, and among atoms constituting said 3-7 membered nonaromatic heterocyclic group, any atom which is not bound to the pyridine or pyrazine optionally forms a C3-C7 alicyclic hydrocarbon as a spiro atom}, or a 5-7 membered aromatic heterocyclic group {wherein said 5-7 membered aromatic heterocyclic group is optionally substituted with one or more substituent(s) selected from Group B2^{a}, and among atoms constituting said 5-7 membered aromatic heterocyclic group, any two adjacent atoms which are not bound to the pyridine or pyrazine optionally form another benzene ring or C4-C7 alicyclic hydrocarbon comprising said two adjacent atoms}; and
Z^{a} represents a C2-C6 chain hydrocarbon group or a 5-10 membered aromatic heterocyclic group {wherein said 5-10 membered aromatic heterocyclic group is optionally substituted with one or more substituent(s) selected from Group D^{a}};
Group A^{a}2: a group consisting of a C1-C3 alkyl group and a halogen atom;
Group B2^{a}: a group consisting of a C3-C6 alicyclic hydrocarbon group, a C1-C6 chain hydrocarbon group, and a halogen atom.

7. A composition comprising the compound or an N-oxide thereof, or a salt thereof according to any one of claims 4 to 6, and an inert carrier.

8. A composition comprising one or more ingredient(s) selected from the group consisting of Group (a), Group (b), Group (c), and Group (d), and the compound or an N-oxide thereof, or a salt thereof according to any one of claims 4 to 6:
Group (a): a group consisting of insecticidal active ingredients, miticidal active ingredients, and nematicidal active ingredients;
Group (b): fungicidal active ingredients;
Group (c): plant growth regulatory ingredients;
Group (d): repellent ingredients.

9. A method for controlling a plant disease which comprises applying an effective amount of the compound or an N-oxide thereof, or a salt thereof according to any one of claims 4 to 6 or an effective amount of the composition according to claim 8, to a plant or soil for cultivating a plant.

10. Use of the compound or an N-oxide thereof, or a salt thereof according to any one of claims 4 to 6 or the composition according to claim 8 for controlling a plant disease.

11. A seed or a vegetative reproductive organ holding an effective amount of the compound or an N-oxide thereof, or a salt thereof according to any one of claims 4 to 6 or an effective amount of the composition according to claim 8.
